# EUROPEAN PATENT SPECIFICATION

(11) **EP 3 845 522 B1**
(45) Date of publication and mention of the grant of the patent: **21.10.2026**
(21) Application number: 20211926.9
(22) Date of filing: 04.12.2020
(51) Int. Cl.: C07D 209/88, C07C 211/54, C07D 333/76, C07D 405/12, C07D 407/12, C07D 409/12, C07C 211/58, H10K 50/15, H10K 85/60

(54) **NITROGEN-CONTAINING COMPOUND, ELECTRONIC ELEMENT AND ELECTRONIC DEVICE**
STICKSTOFFHALTIGE VERBINDUNG, ELEKTRONISCHES ELEMENT UND ELEKTRONISCHE VORRICHTUNG
COMPOSÉ CONTENANT DE L'AZOTE, ÉLÉMENT ÉLECTRONIQUE ET DISPOSITIF ÉLECTRONIQUE

(30) Priority: 31.12.2019 CN 201911415821
(43) Date of publication of application: 07.07.2021
(73) Proprietor: Shaanxi Lighte Optoelectronics Material Co., Ltd., Xi'an, Shaanxi 710065 (CN)
(72) Inventor: Nie, Qiqi, Xi'an, Shaanxi (CN); Cao, Jiamei, Xi'an, Shaanxi (CN); Ma, Tiantian, Xi'an, Shaanxi 710084 (CN)
(74) Representative: Delbarba, Andrea

(56) References cited:
- WO-A1-2020/050623
- WO-A1-2020/080849
- WO-A1-2020/080872
- WO-A1-2020/248943
- CN-A- 107 459 466
- CN-A- 110 128 279
- KR-A- 20190 035 567
- KR-A- 20190 118 515

## Description

### Technical field

The present disclosure relates to the technical field of organic materials, in particular to a nitrogen-containing compound, an electronic element using the nitrogen-containing compound, and an electronic device using the electronic element.

### Background

With the development of electronic technology and the advancement of materials science, the application range of electronic components for realizing electroluminescence or photoelectric conversion becomes more and more extensive. Such electronic component usually includes a cathode and an anode disposed opposite to each other, and a functional layer disposed between the cathode and the anode. The functional layer is composed of multiple organic or inorganic film layers, and generally includes an energy conversion layer, a hole transporting layer disposed between the energy conversion layer and the anode, and an electron transporting layer disposed between the energy conversion layer and the cathode.

For example, when the electronic component is an organic electroluminescent device, it generally includes an anode, a hole transporting layer, an electroluminescent layer as an energy conversion layer, an electron transporting layer and a cathode, which are sequentially stacked. When a voltage is applied to between anode and cathode, the two electrodes generate an electric field. Under the action of the electric field, the electrons on the cathode side move to the electroluminescent layer, while the holes on the anode side move to the electroluminescent layer, so the electrons and the holes combine in the electroluminescent layer to form excitons, and the excitons are in an excited state and release energy outwards, which in turn makes the electroluminescent layer emit light outward. In order to improve the performance of electronic components that realize electroluminescence or photoelectric conversion, an electron blocking layer may also be provided between the energy conversion layer and the hole transporting layer.

In electronic components that realize electroluminescence or photoelectric conversion, the hole transport performance of the film layer disposed between the anode and the energy conversion layer has an important influence on the performance of the electronic components. As recited in patent documents such as Chinese Patent Application CN20 171 04073 82.3, the fluorene group-containing compound may be used for the hole transporting layer. However, the performance of the existing hole transporting layer materials containing fluorene groups needs to be further improved.

Compunds for use in electroluminescence or photoelectric conversion are also disclosed in CN 107 459 466 A, KR 2019-0035567 A, CN 110 128 279 A and KR 2019-0118515 A.

The above information disclosed in the background is only for enhancing the understanding of the background of the present disclosure, so it may include information that does not constitute prior art known to those skilled in the art.

### Summary

The object of the present disclosure is to provide a nitrogen-containing compound, an electronic element and an electronic device to improve the performance of the electronic element and the electronic device.

In order to achieve the above-mentioned object of the disclosure, the present disclosure adopts the following technical solutions.

According to the first aspect of the present disclosure, there is provided a nitrogen-containing compound having the structure shown in Chemical Formula 1: 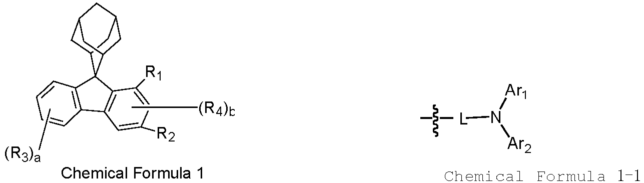
wherein 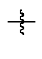 represents a chemical bond;
R₁ and R₂ are each independently selected from hydrogen or a group represented by Chemical Formula 1-1, and one and only one of R₁ and R₂ has the group represented by Chemical Formula 1-1; when R₁ or R₂ is selected from hydrogen, said R₁ and R₂ may be replaced by R₄;
R₃, R₄ are each independently selected from the group consisting of deuterium and phenyl;
a is selected from 0, 1, 2, 3, or 4; when a is greater than or equal to 2, any two R₃ are the same or different;
b is selected from 0, 1, 2, or 3; when b is greater than or equal to 2, any two R₄ are the same or different;
L is defined as laid out further below; and
Ar₁ and Ar₂ are defined as laid out further below.

According to the second aspect of the present disclosure, there is provided an electronic element including an anode and a cathode disposed opposite to each other, and a functional layer disposed between the anode and the cathode, wherein the functional layer contains the above-mentioned nitrogen-containing compound. According to an embodiment of the present disclosure, the electronic element is an organic electroluminescence device. According to another embodiment of the present disclosure, the electronic element is a solar cell.

According to the third aspect of the present disclosure, there is provided an electronic device including the above-mentioned electronic element.

The nitrogen-containing compound of the present disclosure introduces an adamantane structure at the side of the fluorene to enhance the electron density of the fluorene ring and the conjugate system of the entire compound through the hyperconjugation effect, which can enhance the hole conductivity and electron tolerance of the nitrogen-containing compound. At the same time, the luminous efficiency and lifetime of the organic electroluminescent device using the nitrogen-containing compound may be improved, and the conversion efficiency and lifetime of the photoelectric conversion device using the nitrogen-containing compound may be improved. The adamantyl group is introduced between the branches of the triarylamine which is originally a near-plane structure, rather than at the end of it. The large steric hindrance of the adamantyl group can finely adjust the bonding angle and conjugation degree of the amine and each aryl group, thereby obtain HOMO value suitable for the material of the adjacent layer. It reduces the operating voltage of the organic electroluminescent device, and increases the open circuit voltage of the photoelectric conversion device. In addition, the introduction of adamantyl can also increase the molecular weight of the nitrogen-containing compound and reduce the molecular symmetry, increase the glass transition temperature and evaporation temperature of the compound of the present disclosure, and can control the crystallinity of the nitrogen-containing compound, makes the nitrogen-containing compound have better physical and thermal stability when being mass-produced, which facilitates the mass production stability of the electronic elements such as organic electroluminescent devices and photoelectric conversion devices.

### Brief Decrption of the Drawings

The above and other features and advantages of the present disclosure will become more apparent by describing in detail exemplary embodiments thereof with reference to the drawings.
FIG. 1 is a schematic structural diagram of an organic electroluminescent device according to an embodiment of the present disclosure.
FIG. 2 is a schematic structural diagram of a photoelectric conversion device according to an embodiment of the present disclosure.
FIG. 3 is a schematic structural diagram of an electronic device according to an embodiment of the present disclosure.
FIG. 4 is a schematic structural diagram of an electronic device according to another embodiment of the present disclosure.

The reference symbols of the main elements in the figure are as follows:
100, anode;
200, cathode;
300, functional layer;
310, hole injecting layer;
321, hole transporting layer;
322, electron blocking layer;
330, organic electroluminescent layer;
340, hole blocking layer;
350, electron transporting layer;
360, electron injecting layer;
370, photoelectric conversion layer;
400, the first electronic device;
500, the second electronic device.

### Detailed Description

Exemplary embodiments will now be described more fully with reference to the drawings.

In the present disclosure, when R₁ or R₂ is selected from hydrogen, said R₁ and R₂ may be replaced by R₄. It means that when one of R₁ and R₂ is selected from hydrogen, the one selected from hydrogen may be replaced by R₄ or not. For example, when R₂ is selected from Chemical Formula 1-1 and R₁ is selected from hydrogen, R₁ may or may not be replaced by R₄. Specifically, Chemical Formula 1 may include, but not limit to, 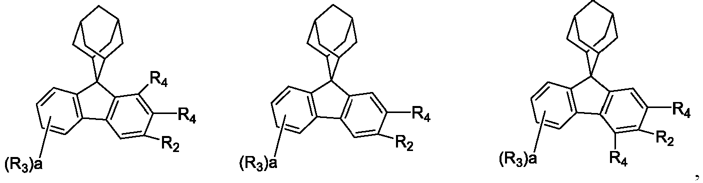 in which any two R₄ are the same or different.

In the present disclosure, since adamantane has a three-dimensional structure, in the structure diagram of the compound, because of the different drawing angles, it will show different plane shapes, among which, the ring structures formed on each 9,9-dimethylfluorene all refer to adamantine, and the connection location between the two group is the same. For example, the following four structures are the same:

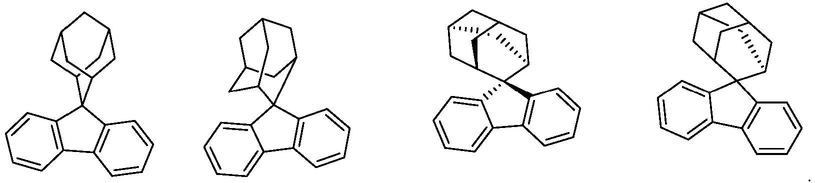

The present disclosure provides a nitrogen-containing compound having a structure shown in Chemical Formula 1: 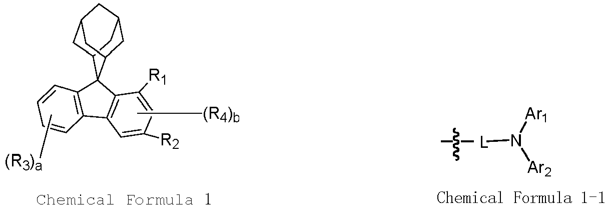
wherein 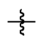 represents a chemical bond;
R₁ and R₂ are each independently selected from hydrogen or a group represented by Chemical Formula 1-1, and one and only one of R₁ and R₂ has the group represented by Chemical Formula 1-1; when R₁ or R₂ is selected from hydrogen, said R₁ and R₂ may be replaced by R₄;
R₃, R₄ are each independently selected the group consisting of deuterium and phenyl;
a is selected from 0, 1, 2, 3, or 4; when a is greater than or equal to 2, any two R₃ are the same or different;
b is selected from 0, 1, 2, or 3, when b is greater than or equal to 2, any two R₄ are the same or different;
L is defined as laid out further below; and
Ar₁ and Ar₂ are defined as laid out further below.

In the present disclosure, "alkyl" may include linear or branched alkyl. The alkyl group is an alkyl group with 1 to 10 carbon atoms. The alkyl group may also be a lower alkyl group with 1 to 6 carbon atoms.

Preferably, the alkyl group is selected from methyl, ethyl, propyl, isopropyl, n-butyl, isobutyl, sec-butyl, tert-butyl, pentyl and hexyl.

The nitrogen-containing compound of the present disclosure may be used in the preparation of organic electroluminescent devices and photoelectric conversion devices, especially suitable for the preparation of the electron blocking layer (also known as hole assist layer, or second hole transporting layer, etc.) of the organic electroluminescent devices and photoelectric conversion devices, so as to improve the efficiency and lifetime of the organic electroluminescent devices and photoelectric conversion devices, reduce the operating voltage of organic electroluminescent devices, increase the open circuit voltage of photoelectric conversion devices, and improve the mass production stability of the photoelectric conversion devices and the organic electroluminescent devices.

According to the invention, L is selected from the group consisting of single bond and the following groups:

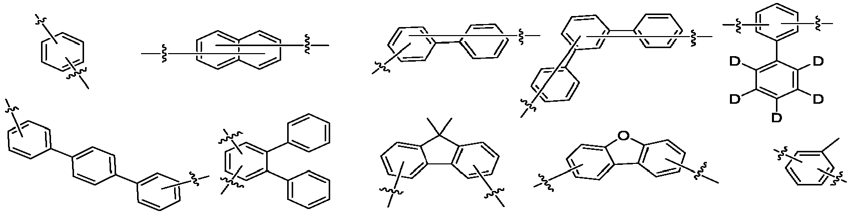

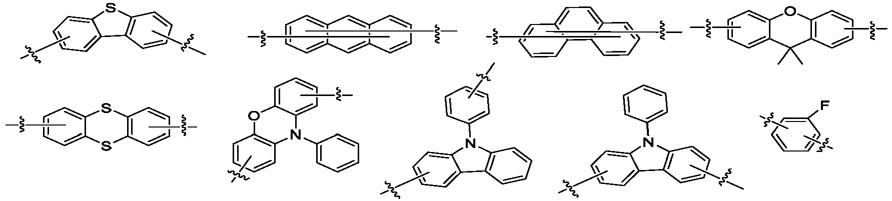

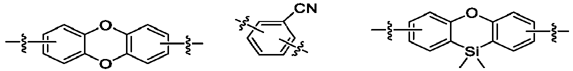

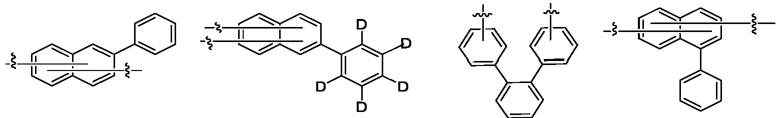

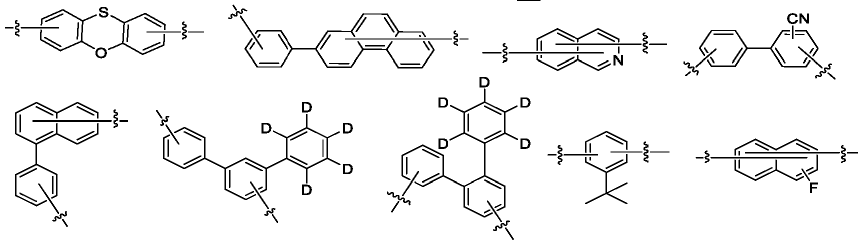

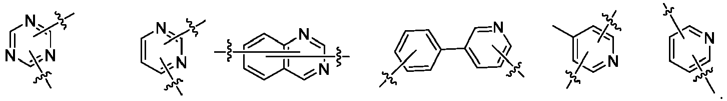

Optionally, L is selected from the group consisting of single bond and the following groups:

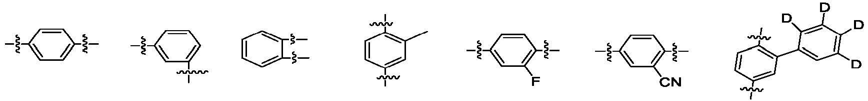

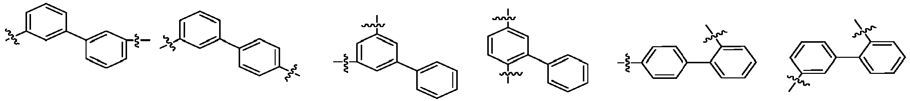

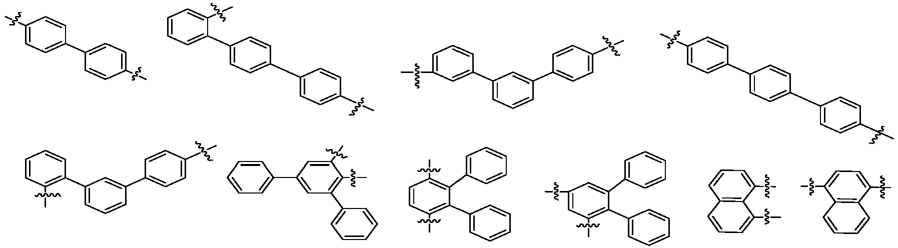

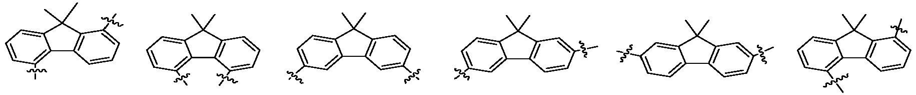

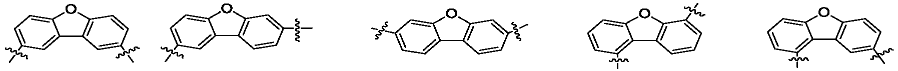

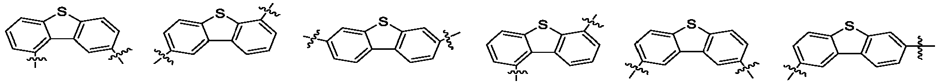

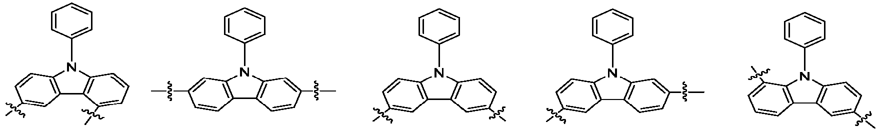

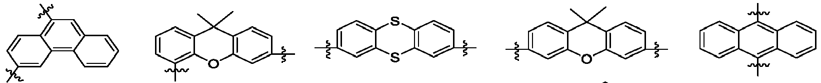

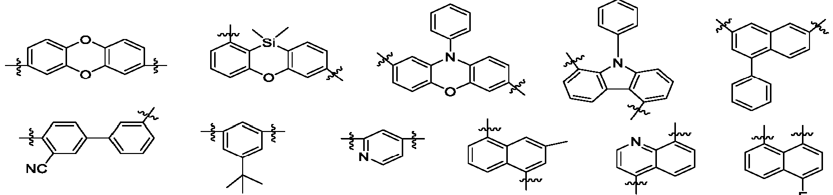

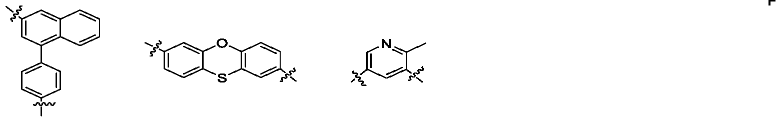

Further, according to the invention Ar₁ and Ar₂ are each independently selected from the group consisting of the following groups:

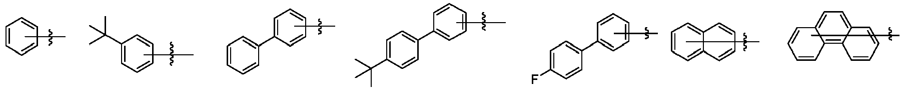

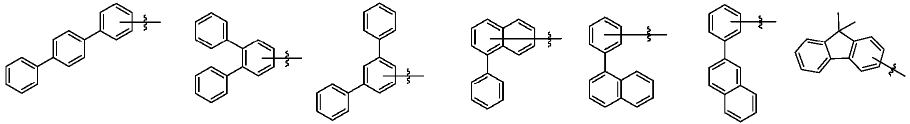

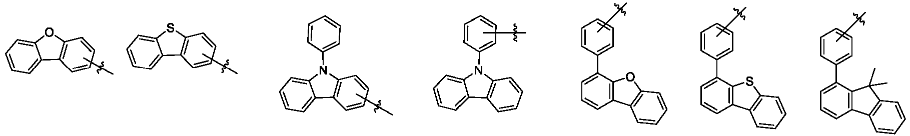

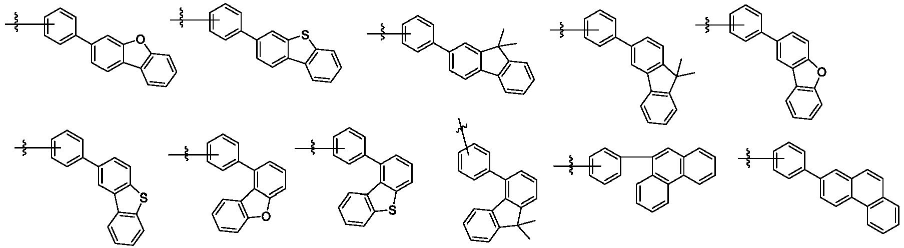

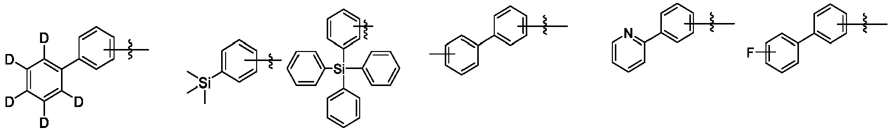

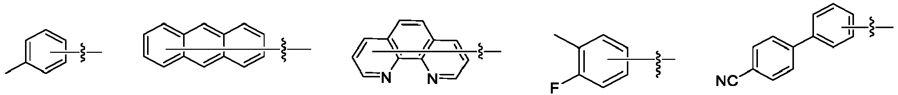

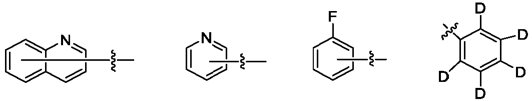

Optionally, Ar₁ and Ar₂ are each independently selected from the group consisting of the following groups:

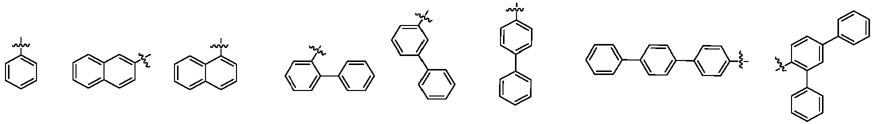

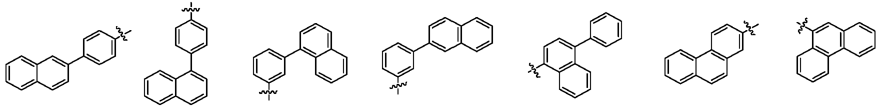

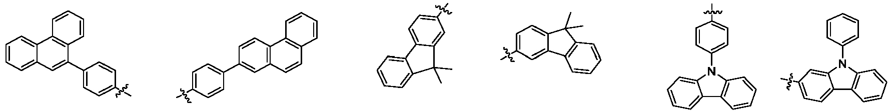

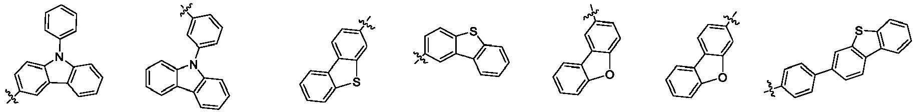

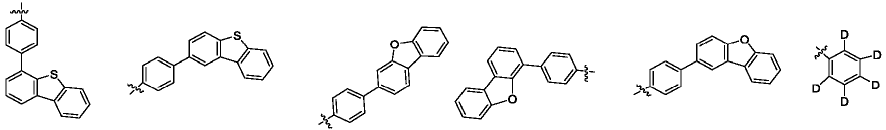

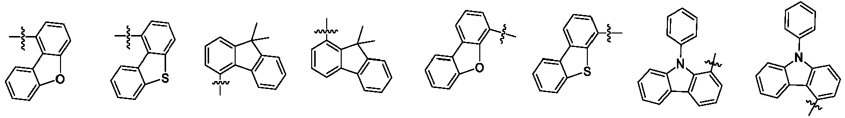

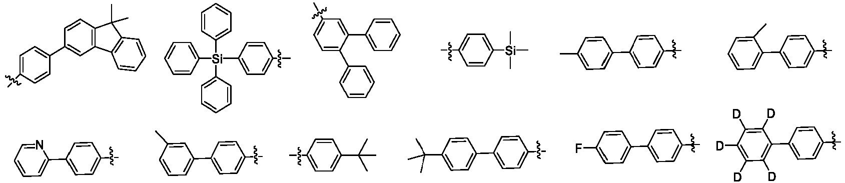

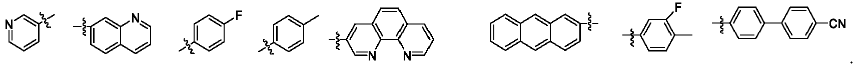

R₃ and R₄ are each independently selected from deuterium and phenyl.

Optionally, the nitrogen-containing compound is selected from the group consisiting of the following compounds:

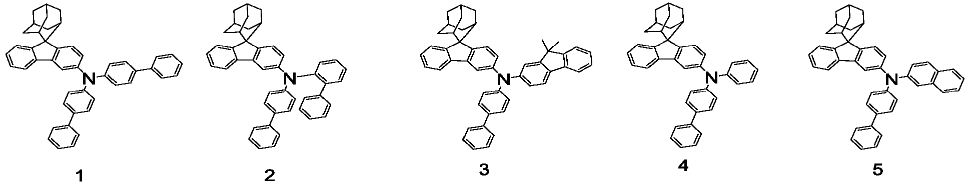

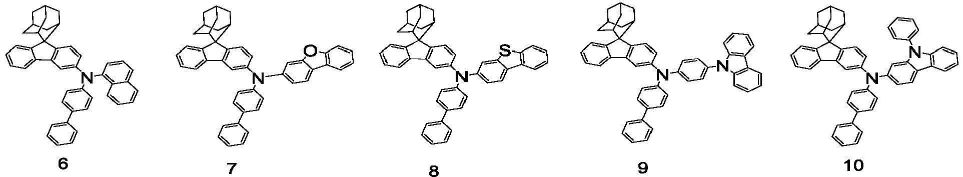

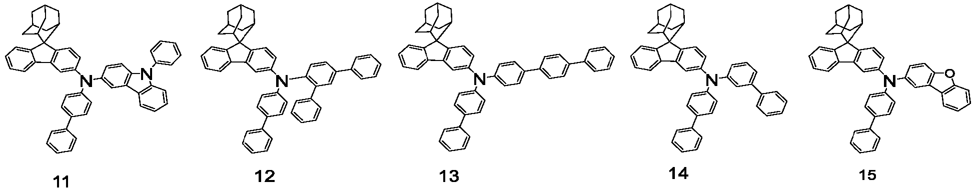

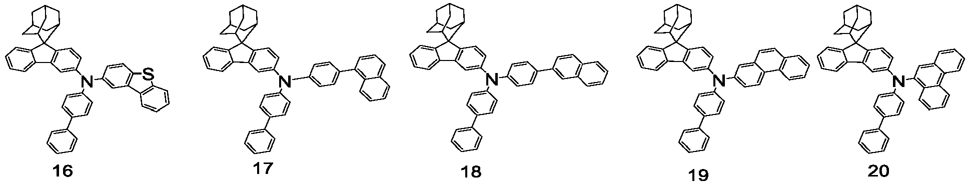

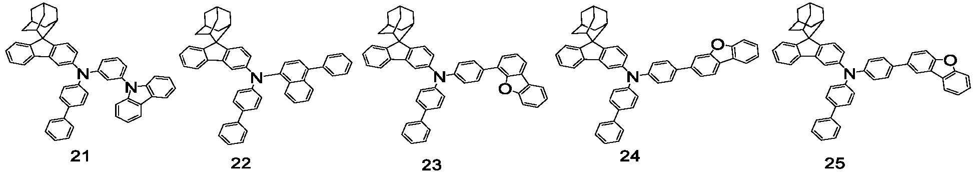

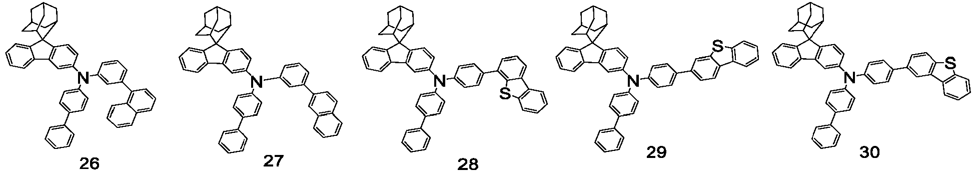

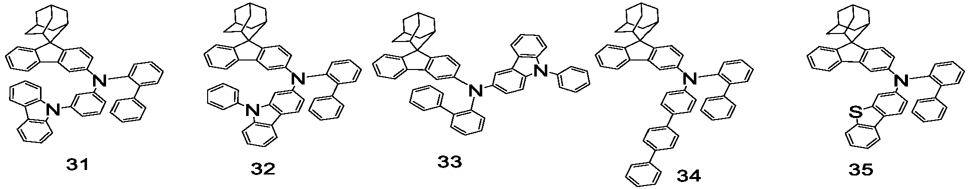

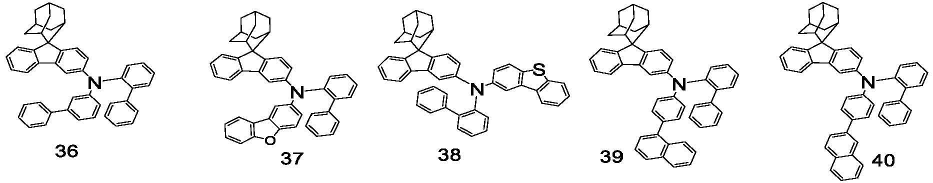

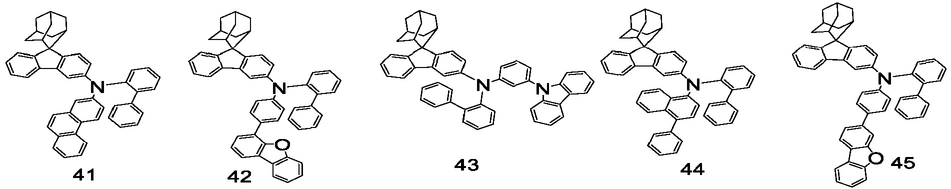

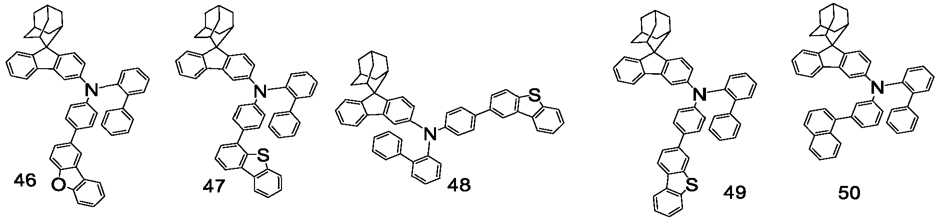

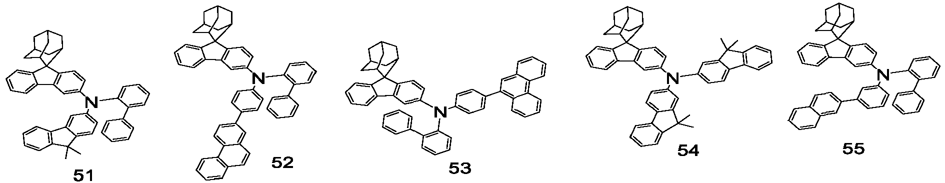

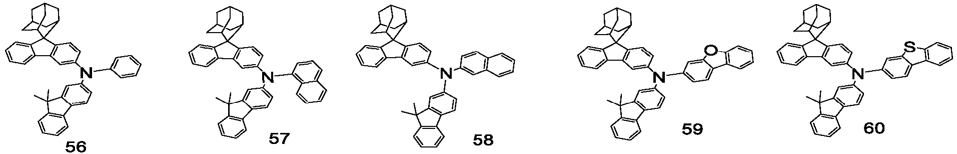

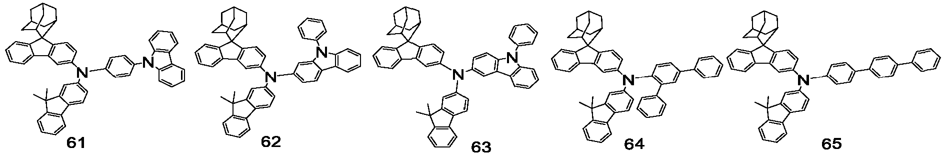

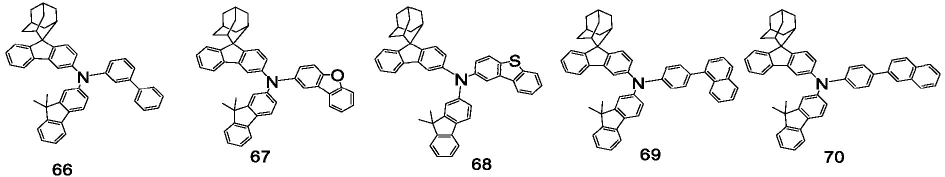

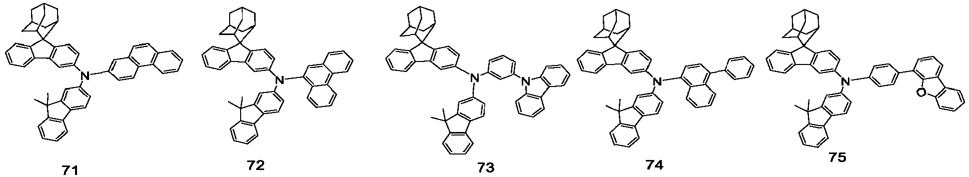

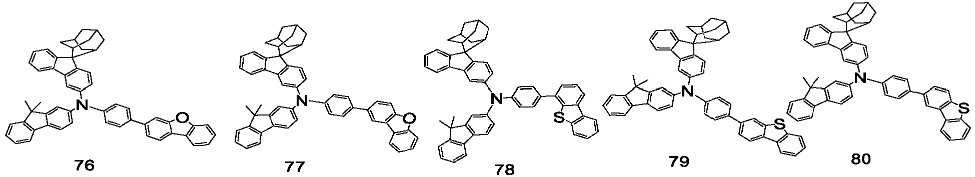

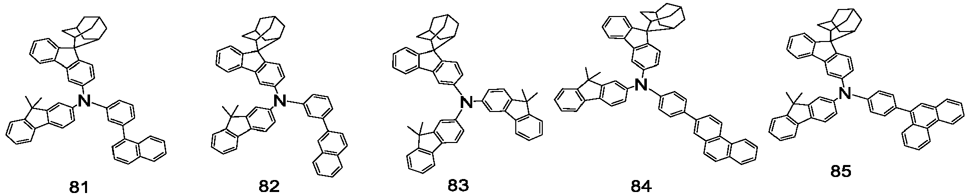

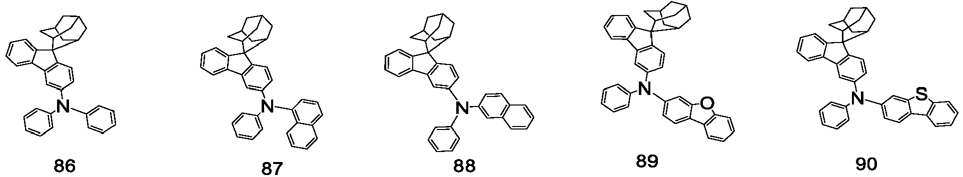

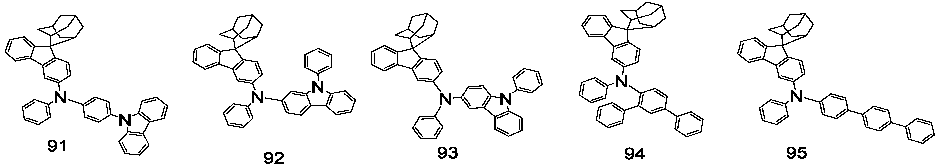

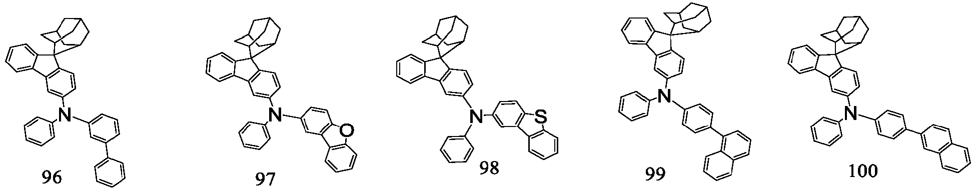

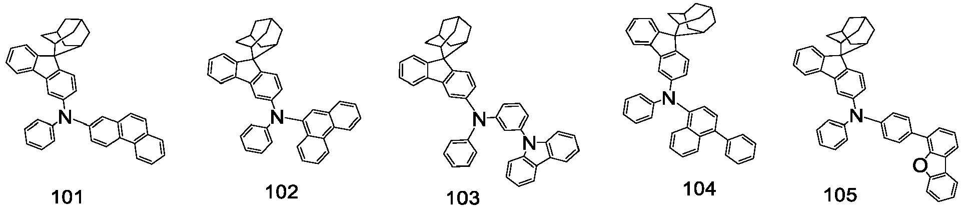

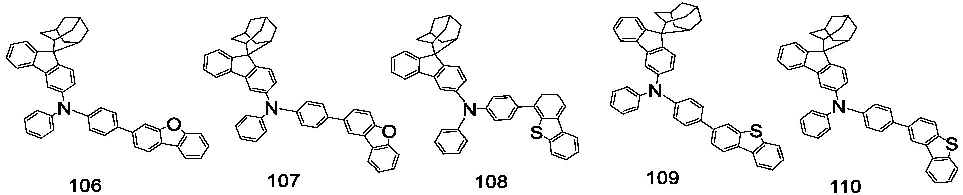

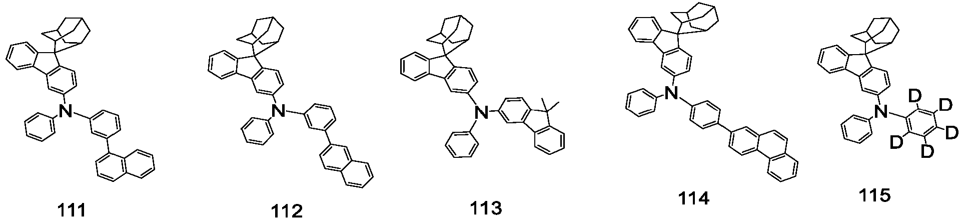

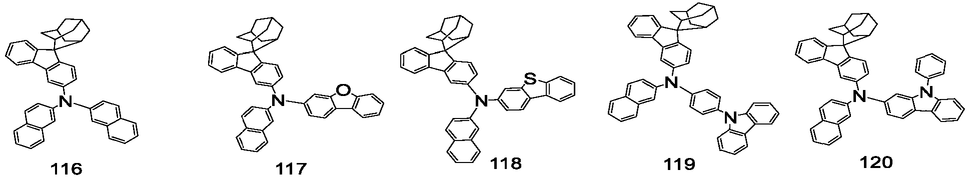

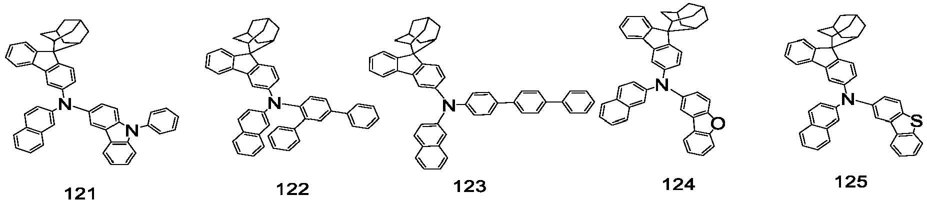

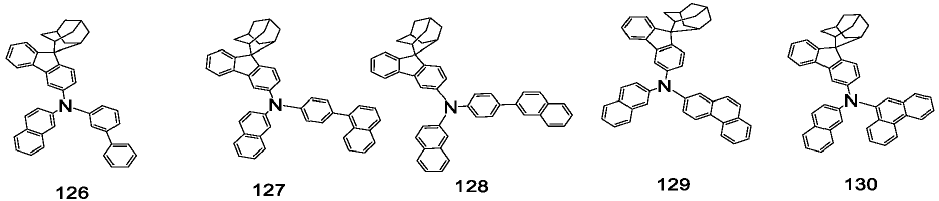

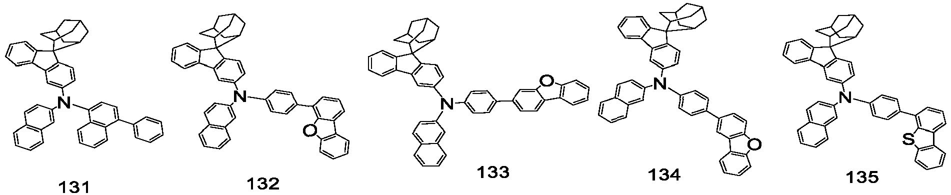

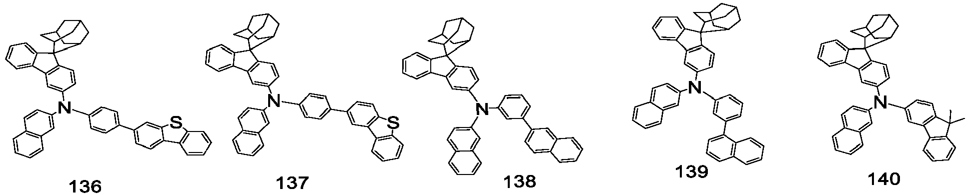

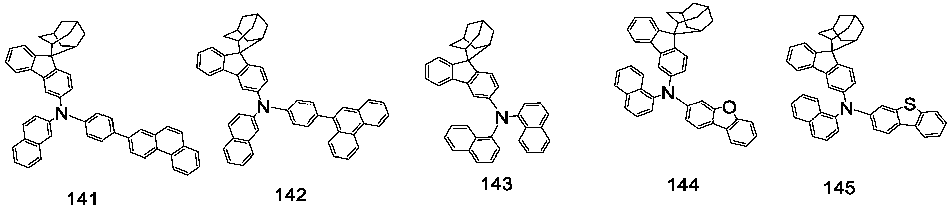

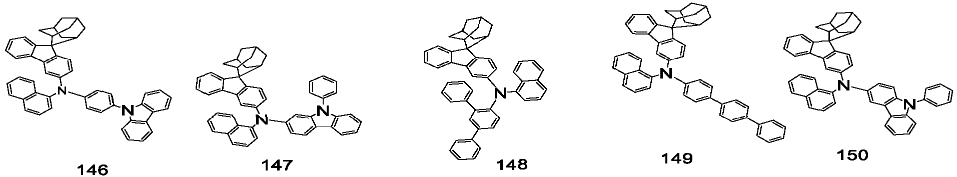

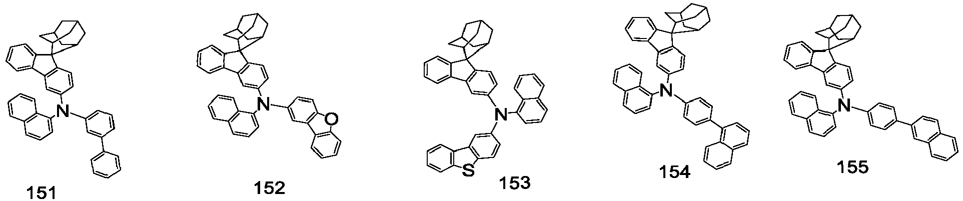

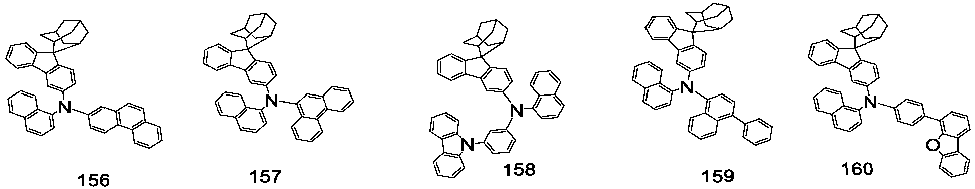

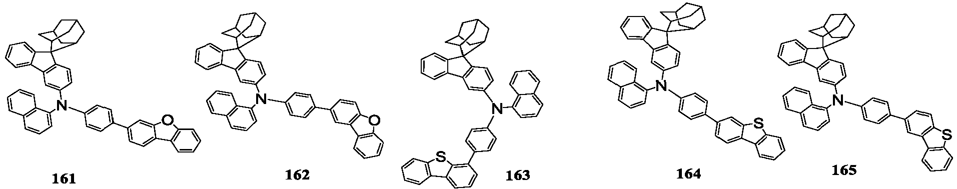

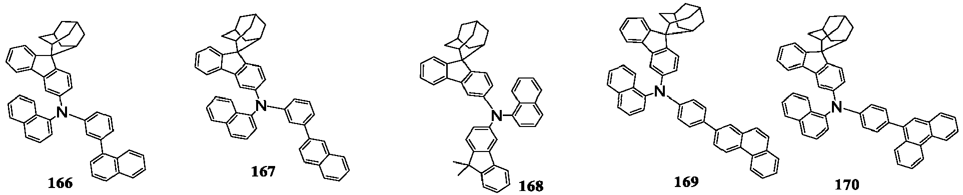

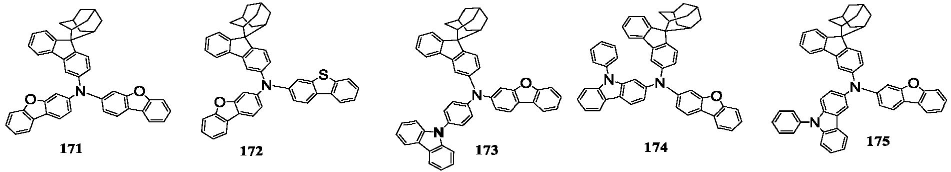

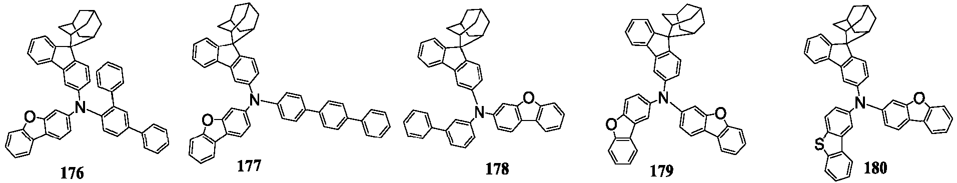

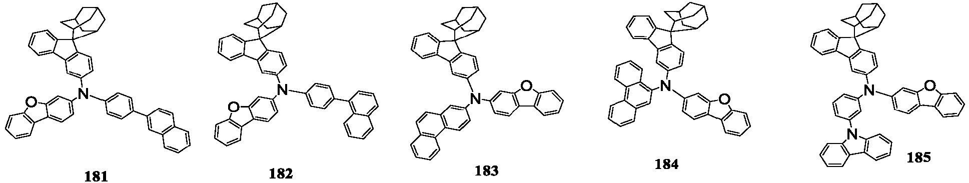

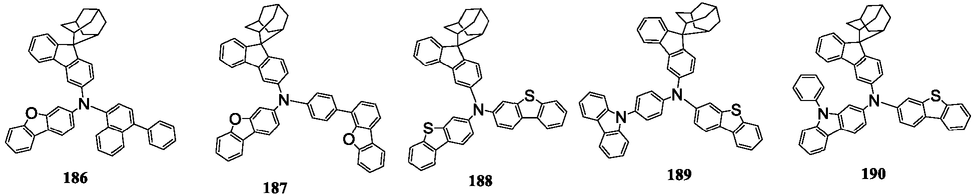

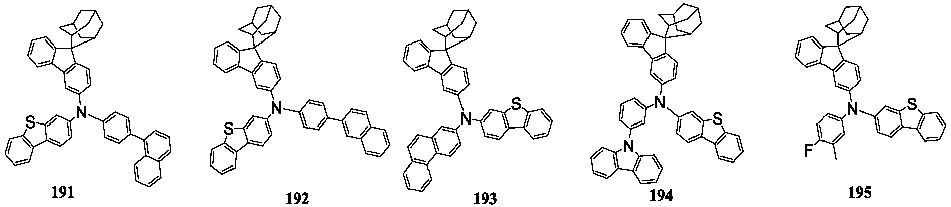

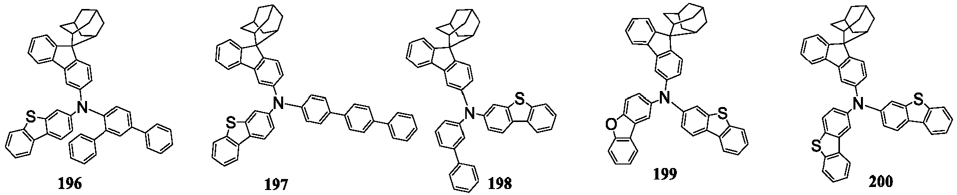

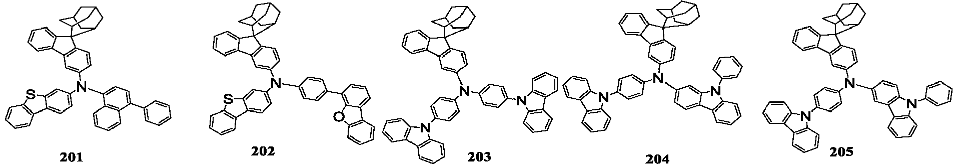

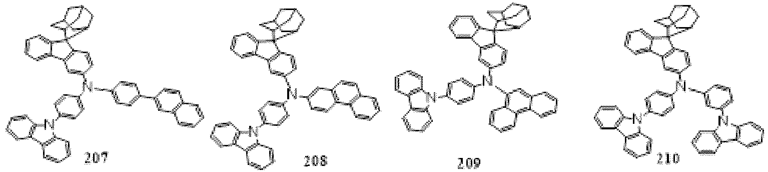

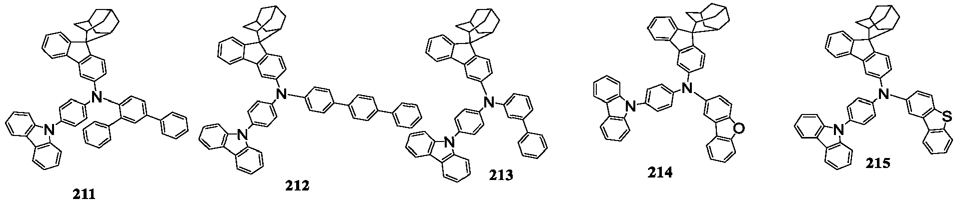

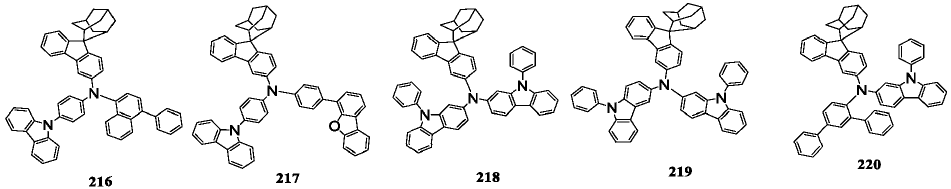

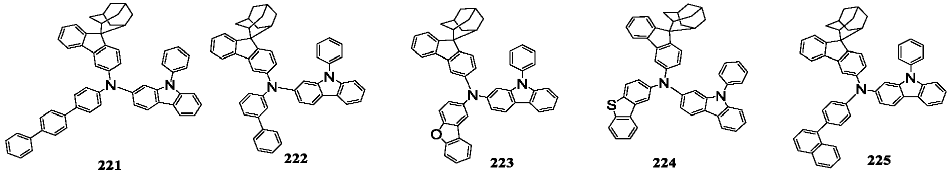

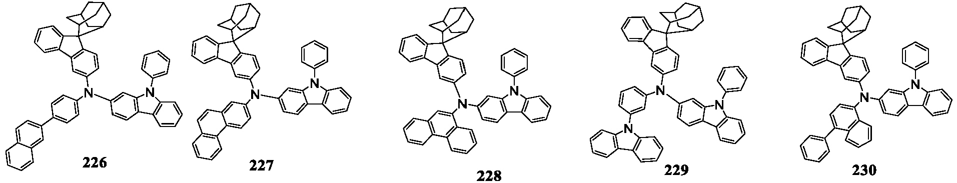

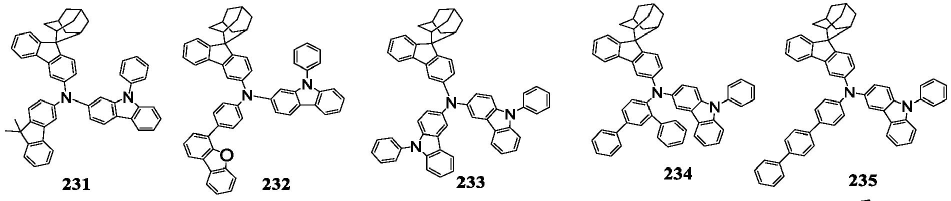

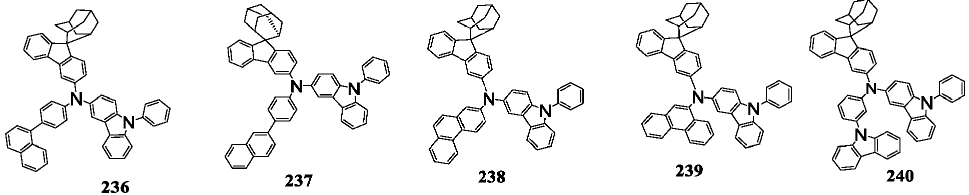

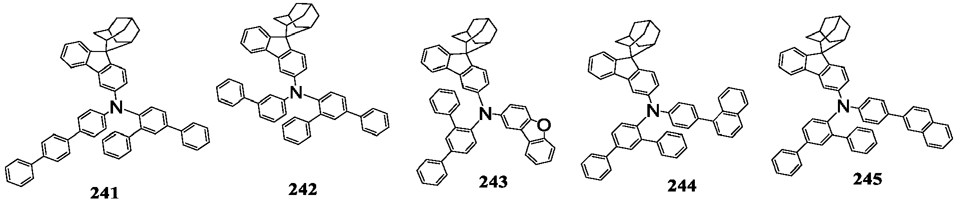

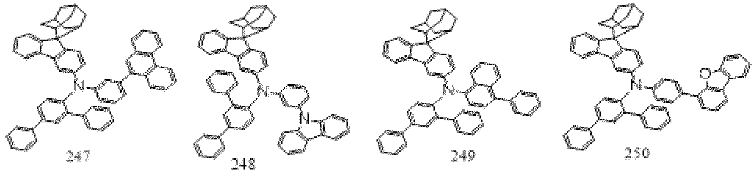

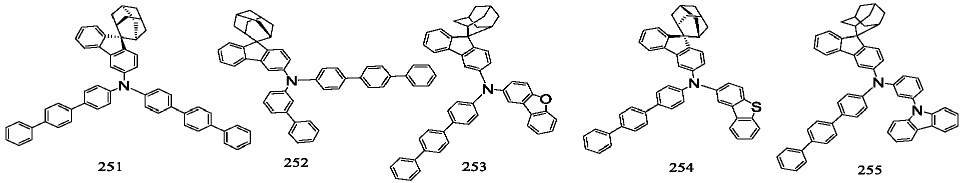

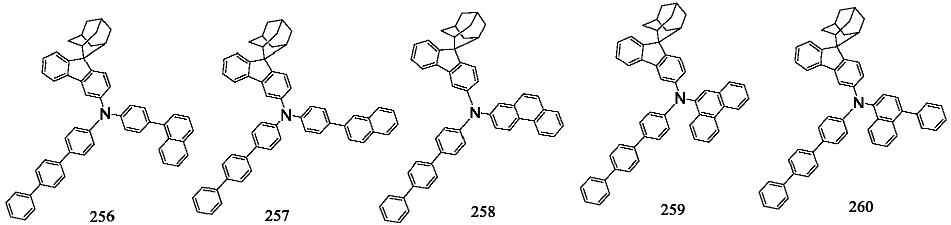

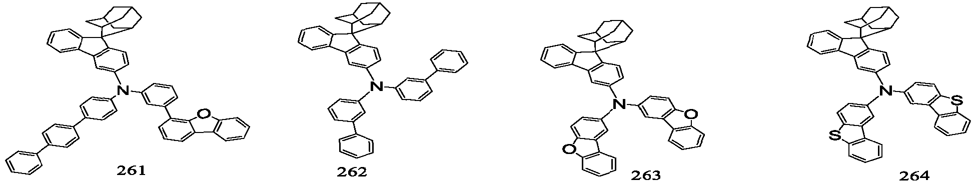

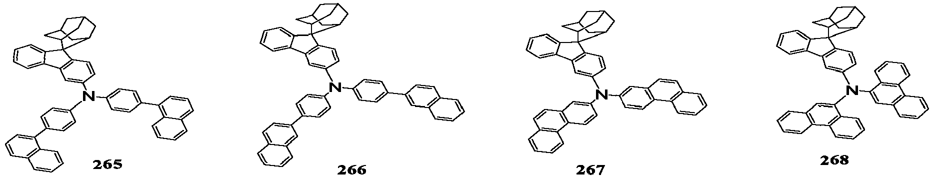

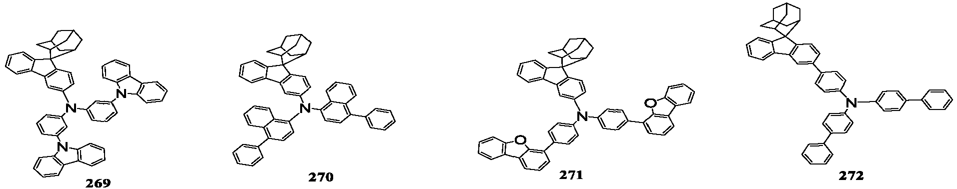

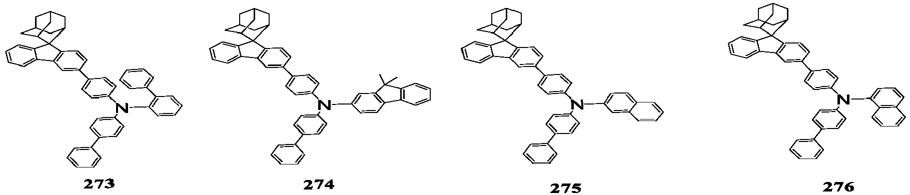

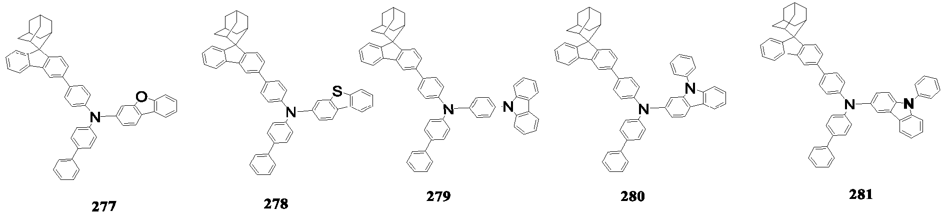

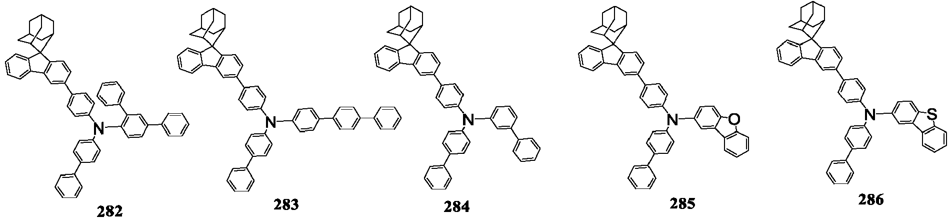

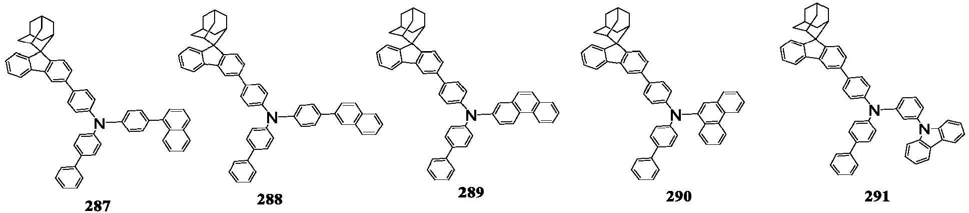

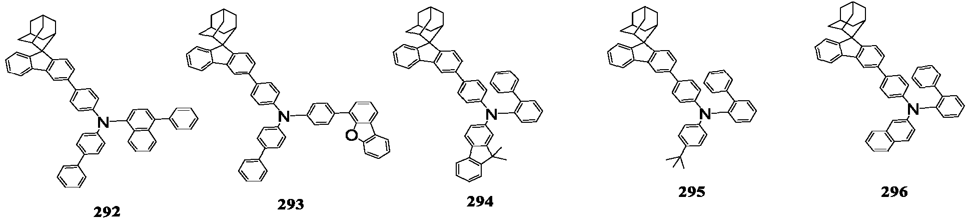

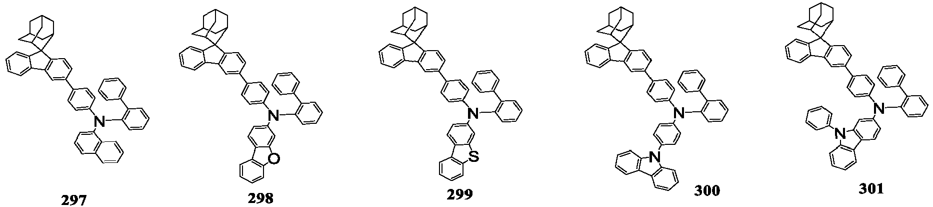

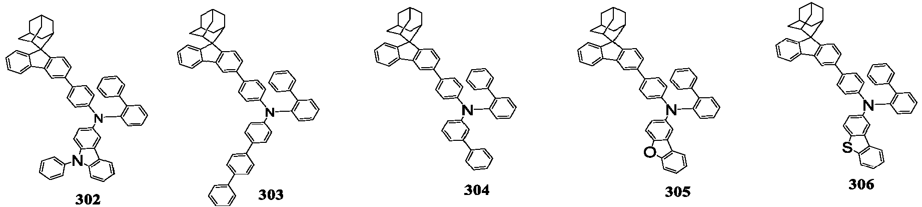

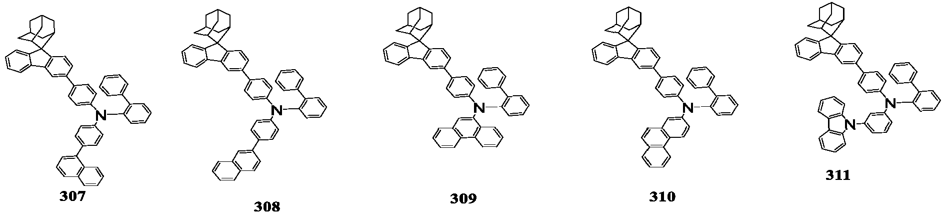

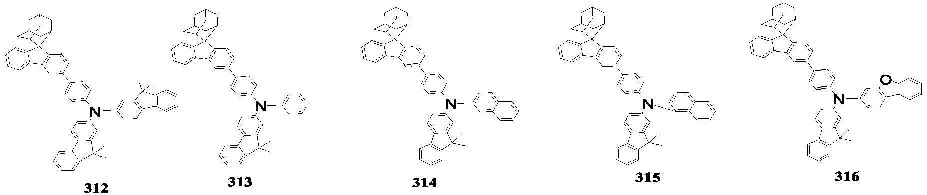

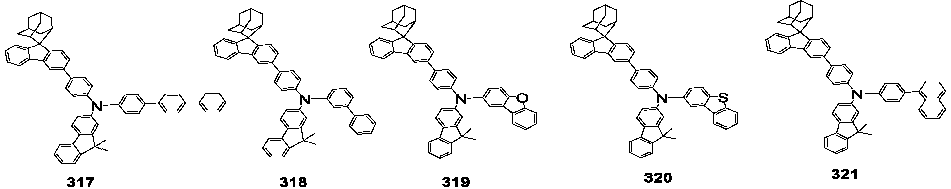

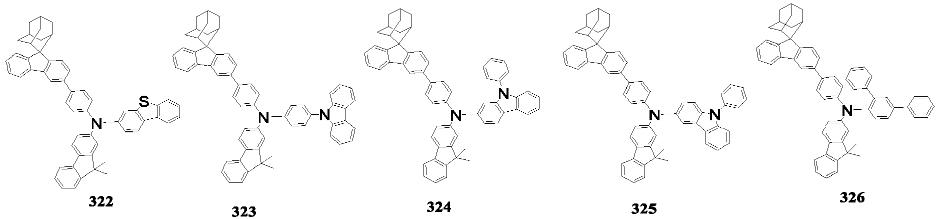

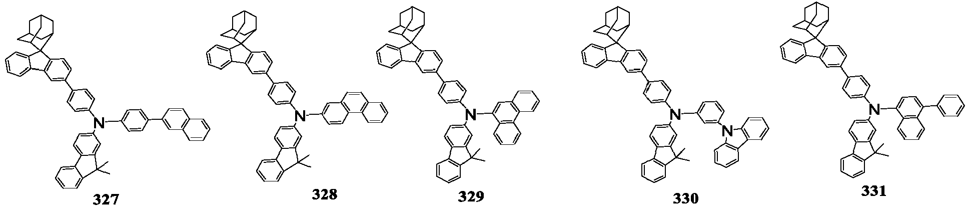

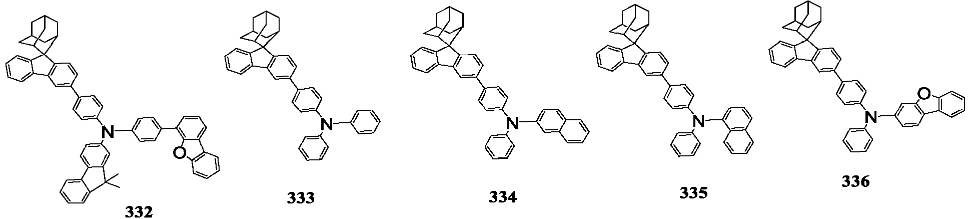

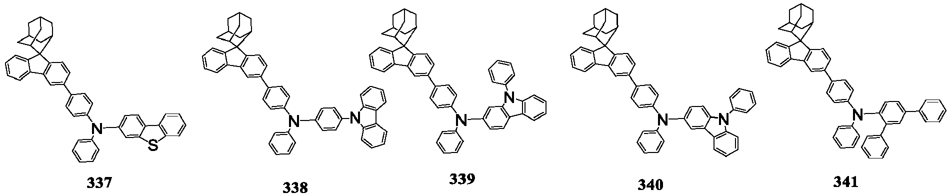

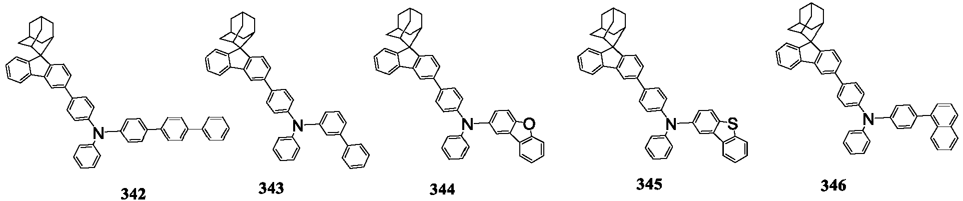

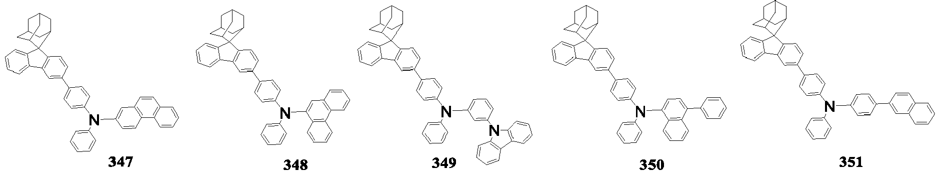

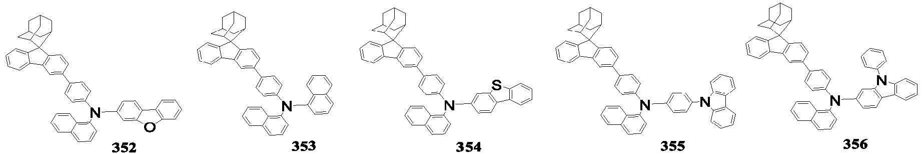

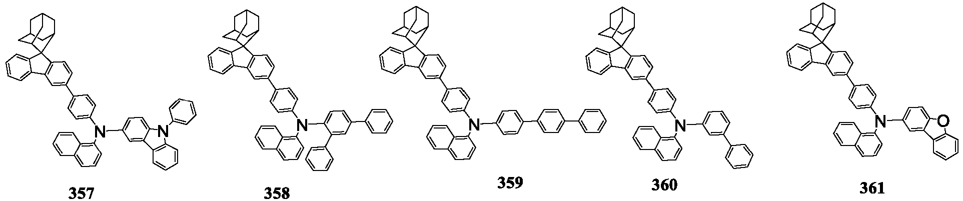

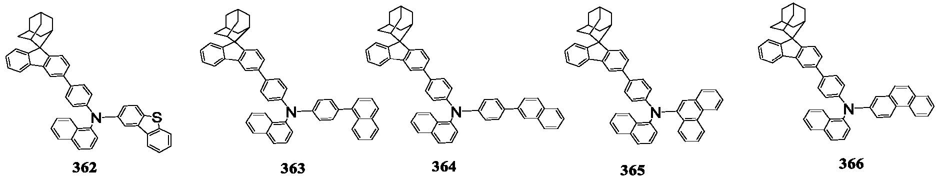

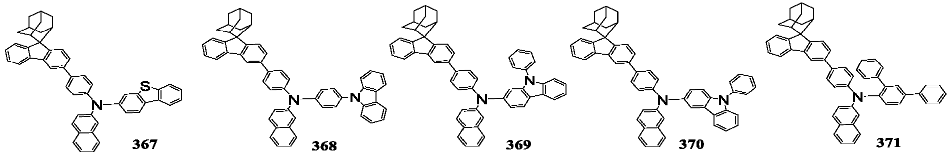

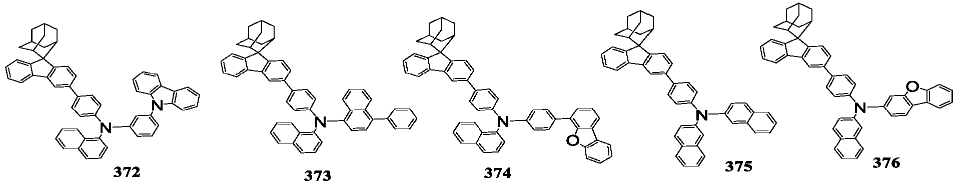

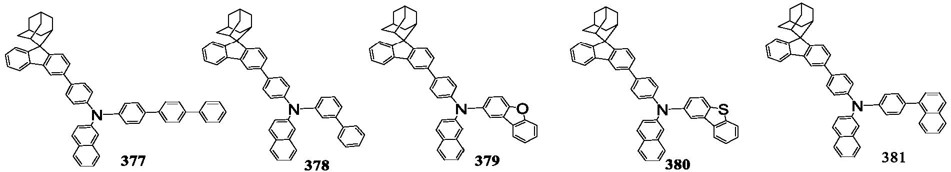

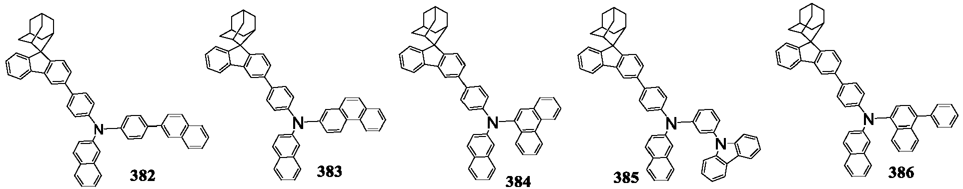

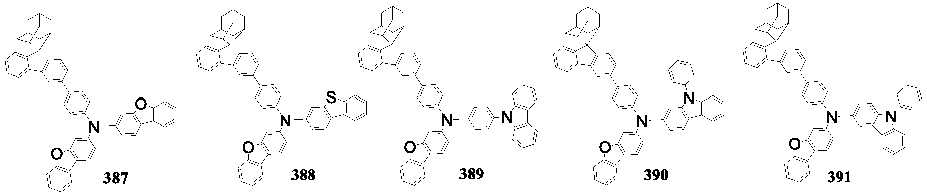

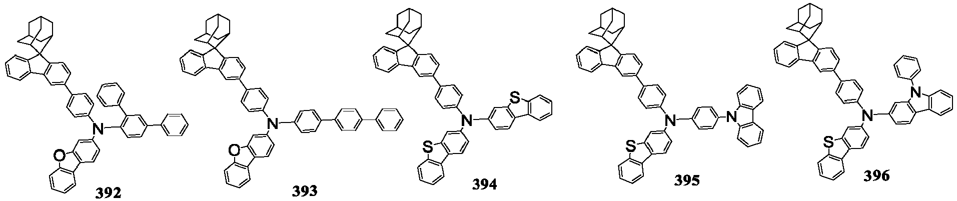

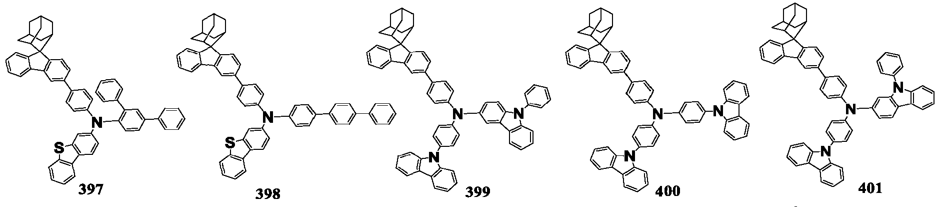

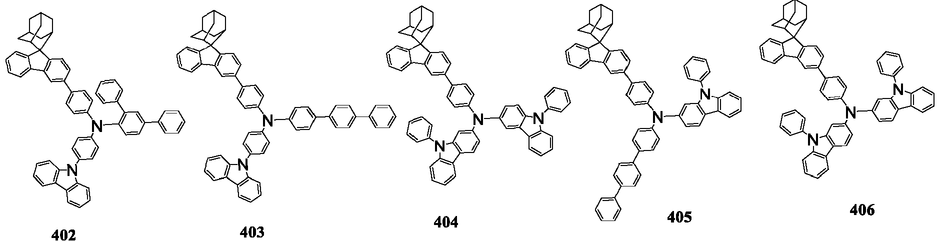

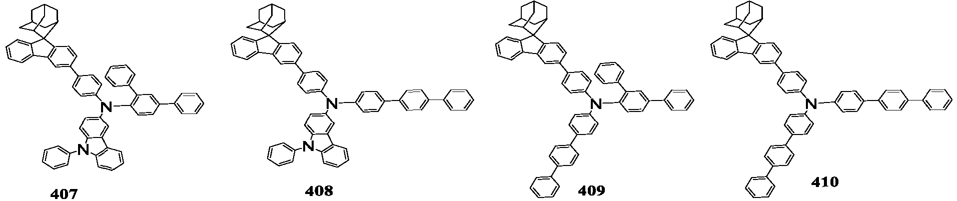

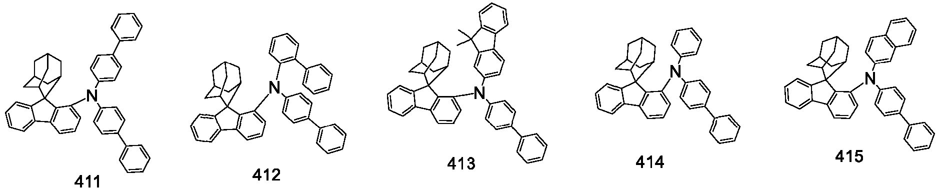

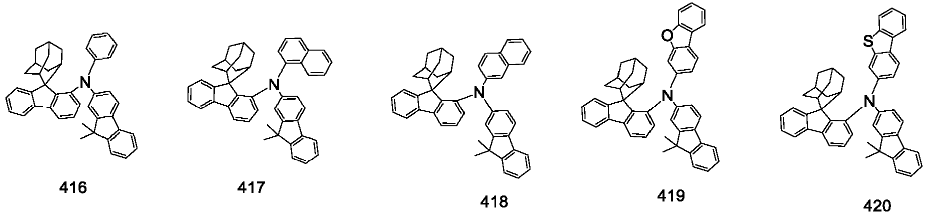

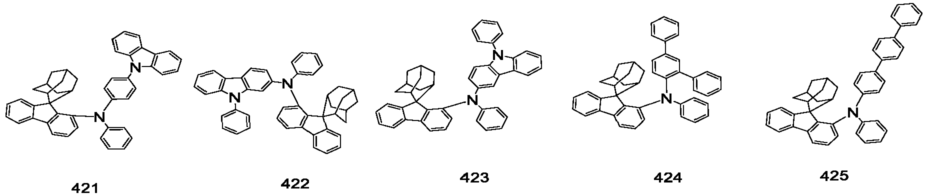

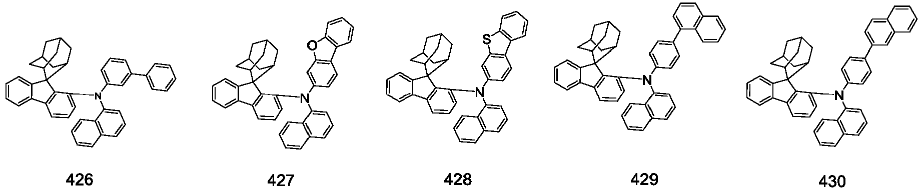

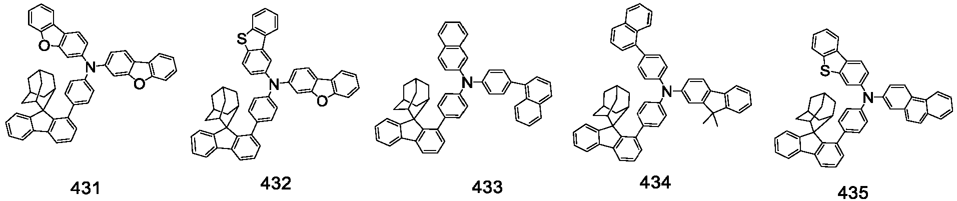

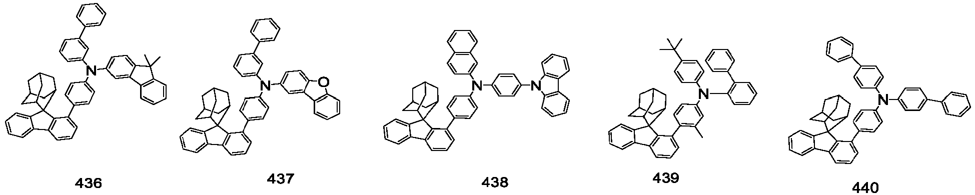

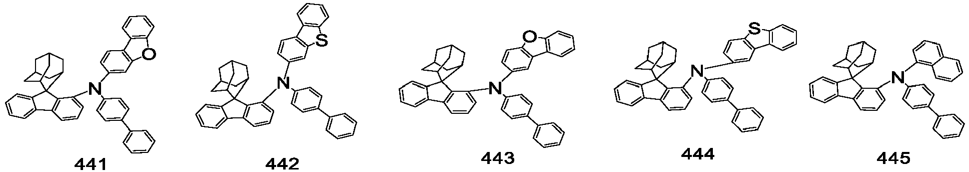

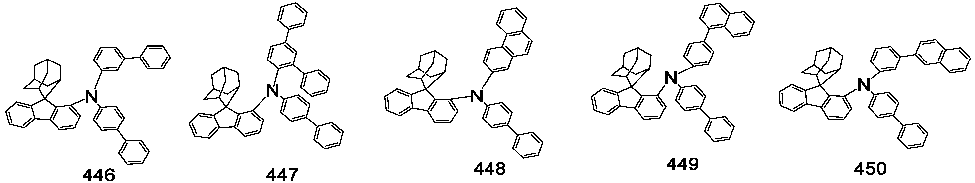

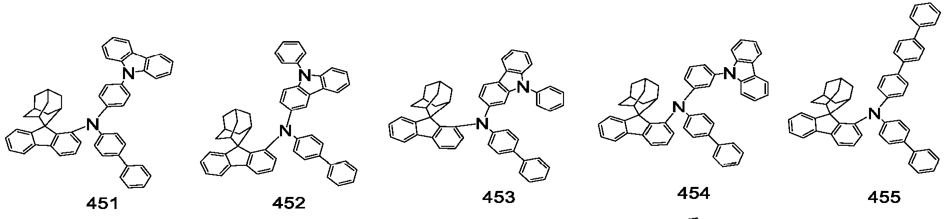

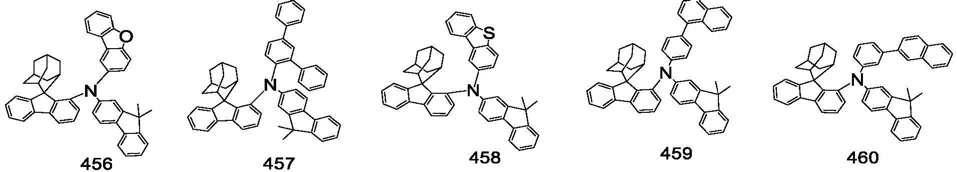

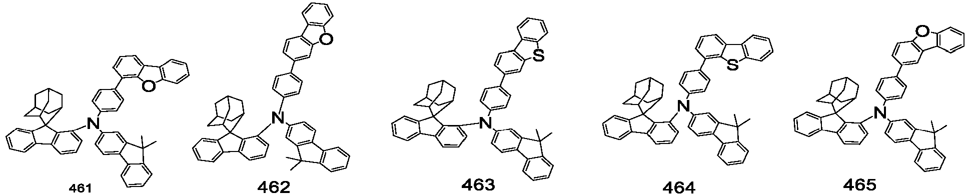

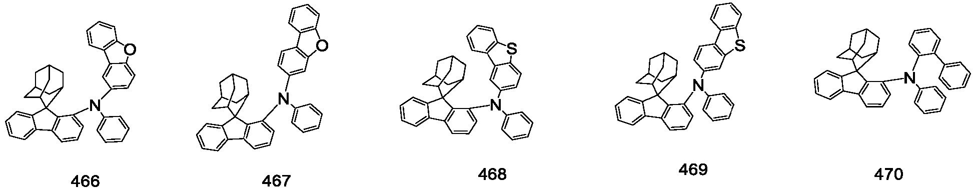

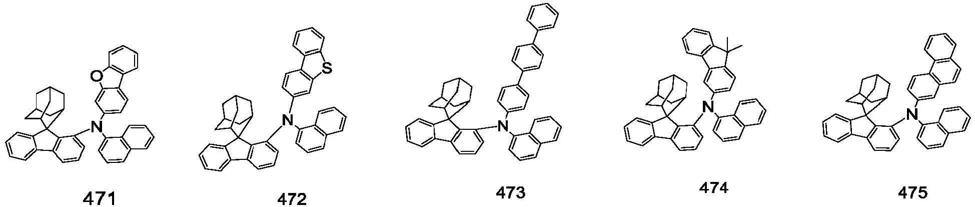

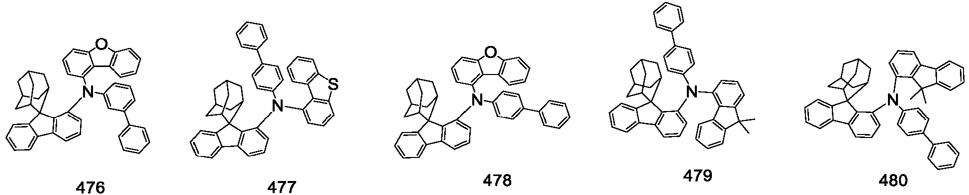

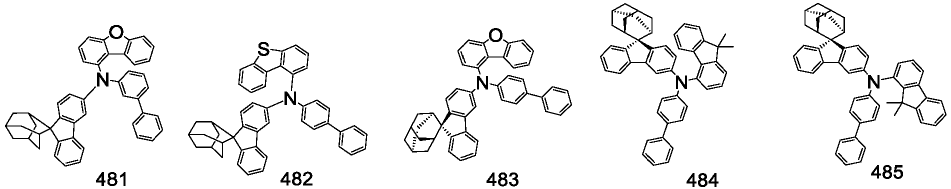

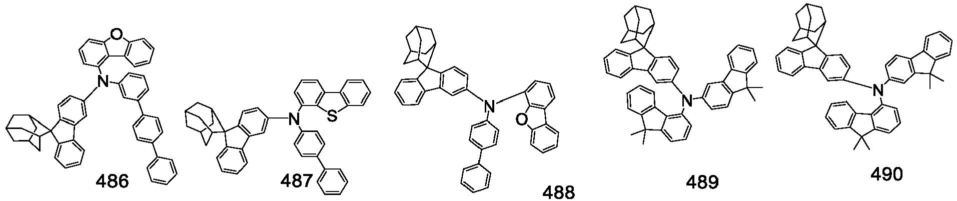

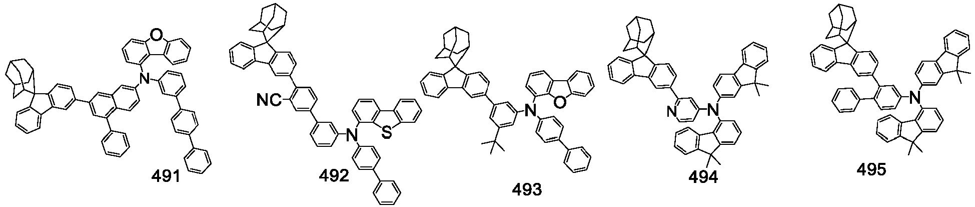

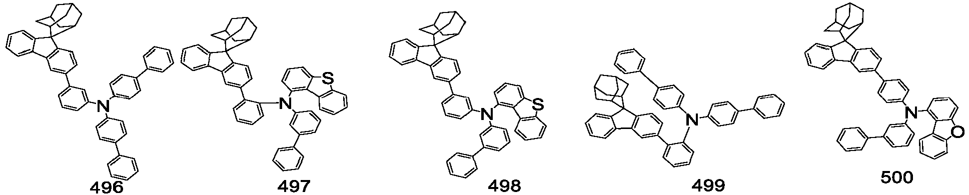

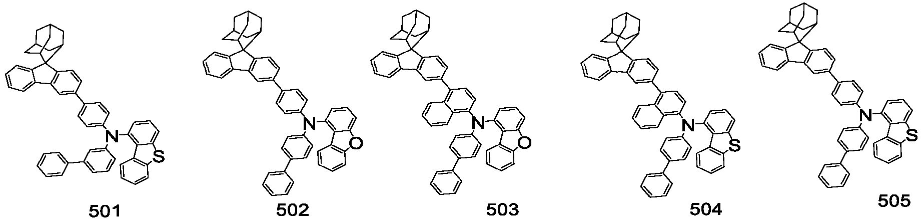

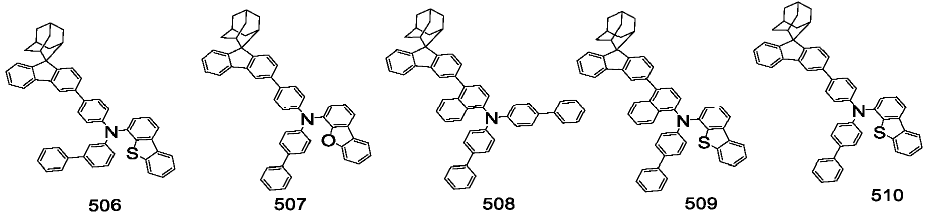

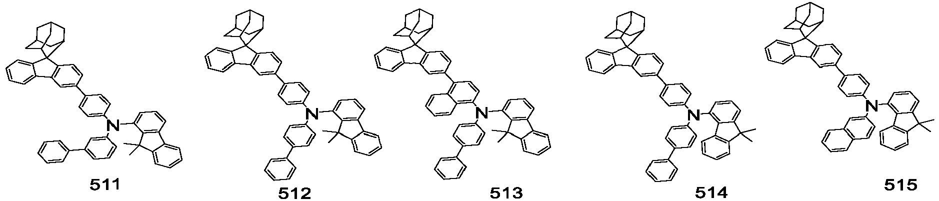

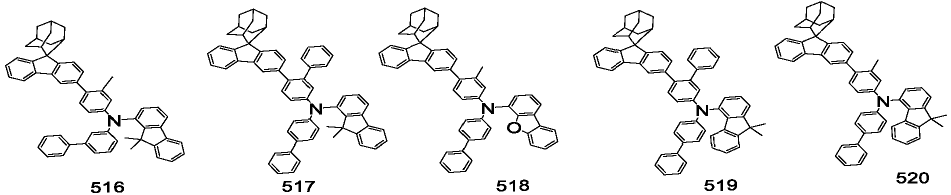

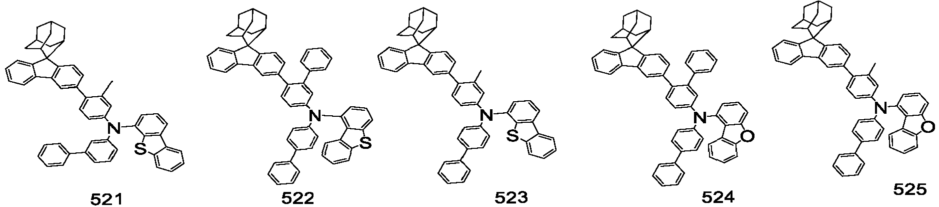

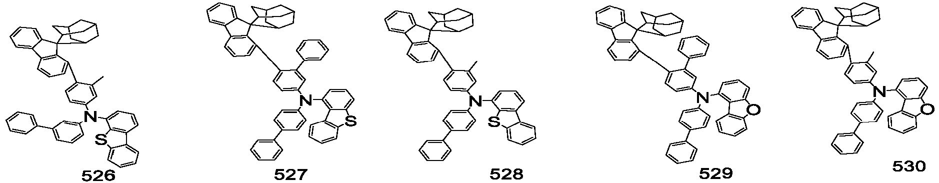

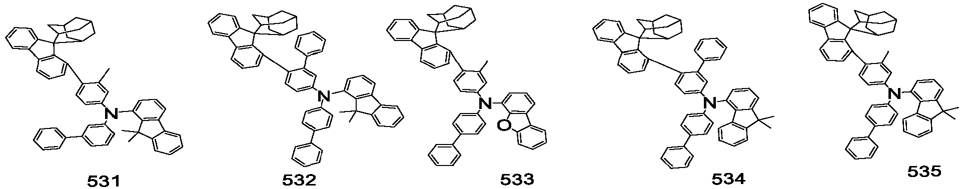

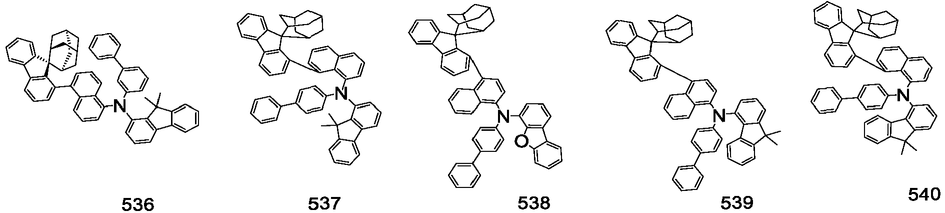

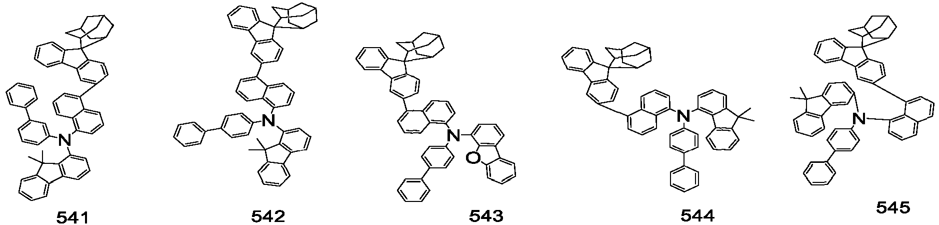

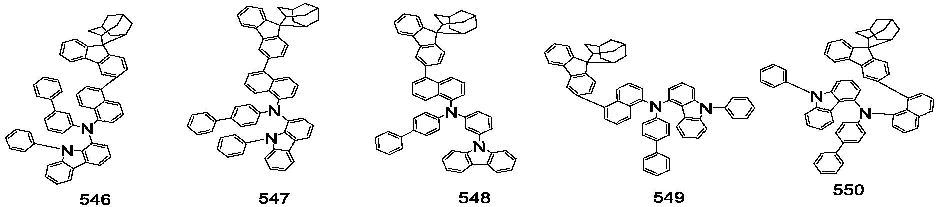

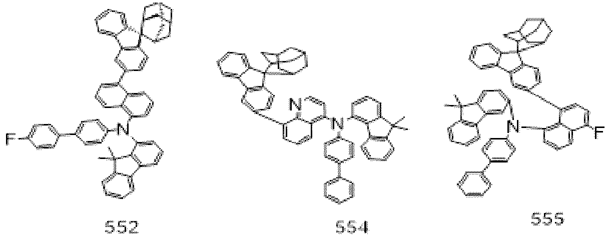

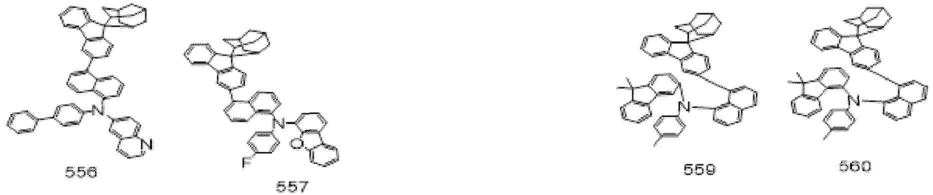

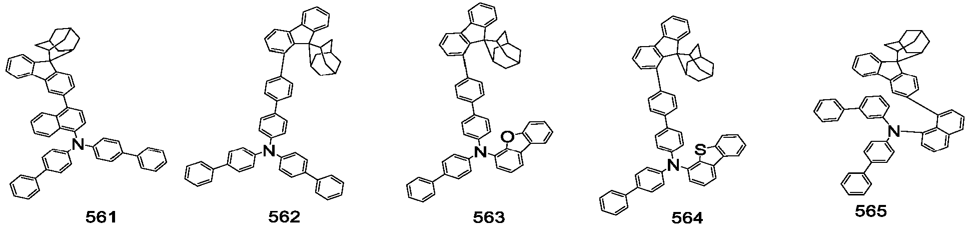

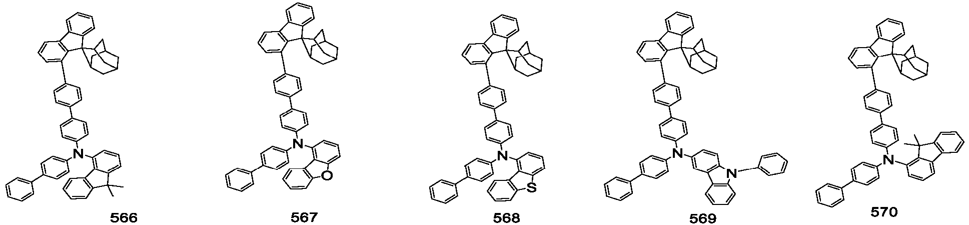

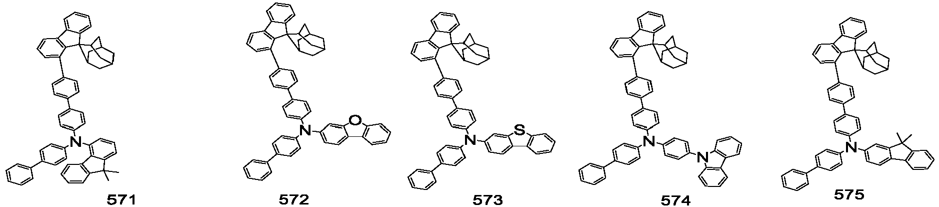

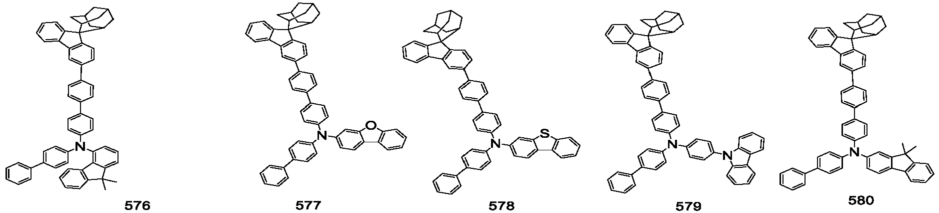

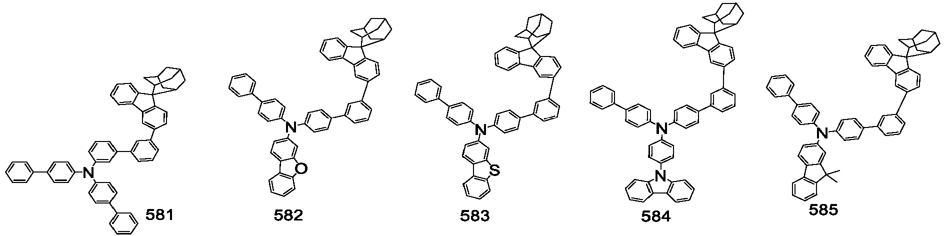

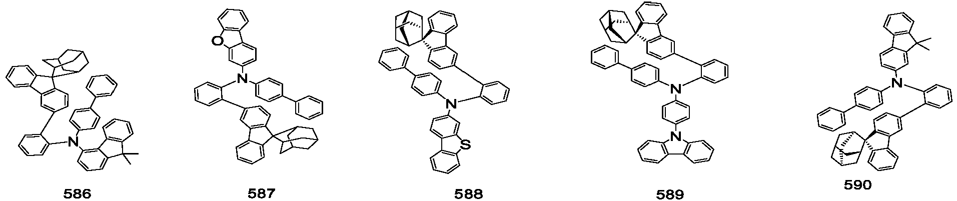

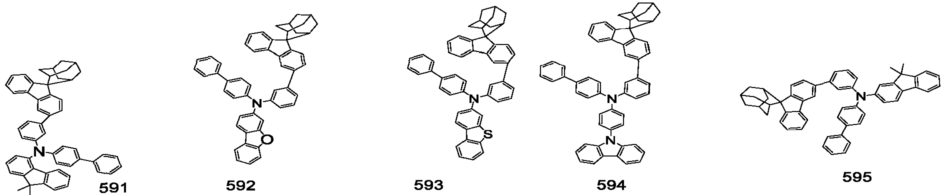

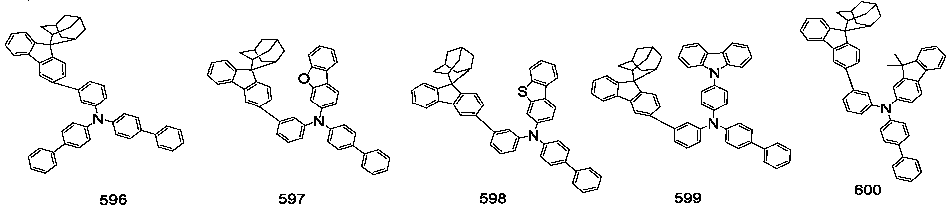

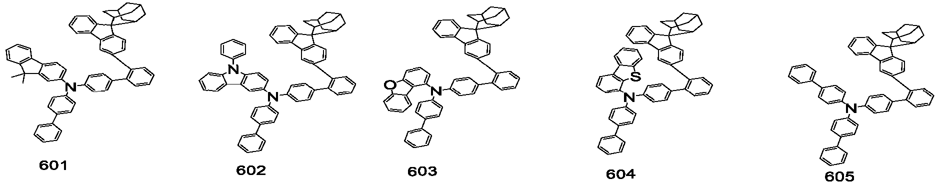

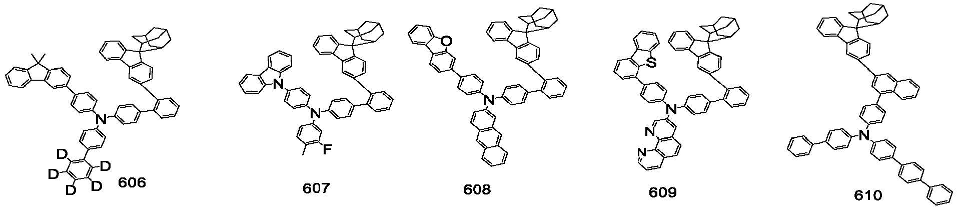

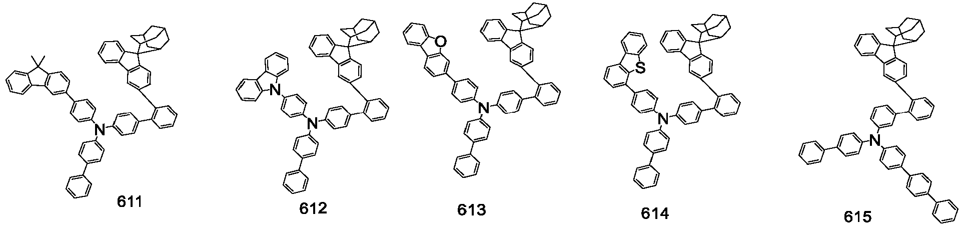

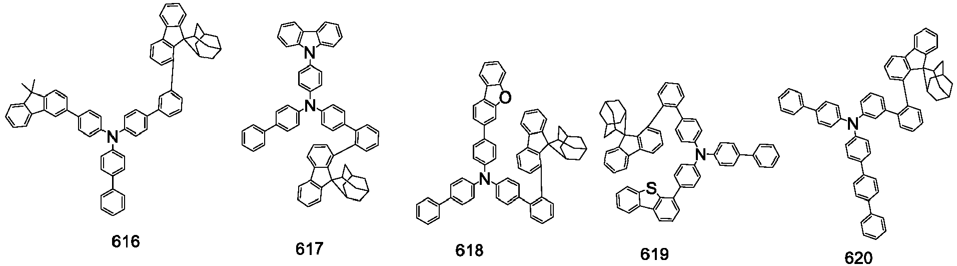

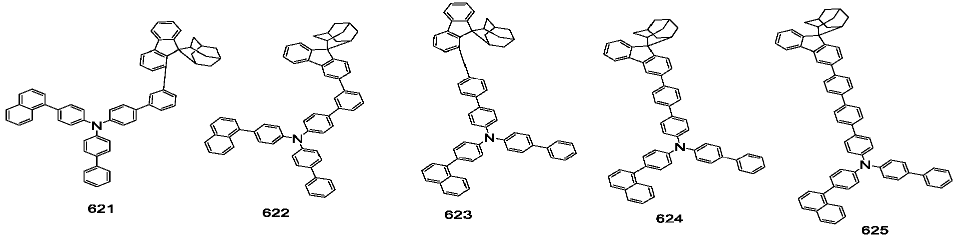

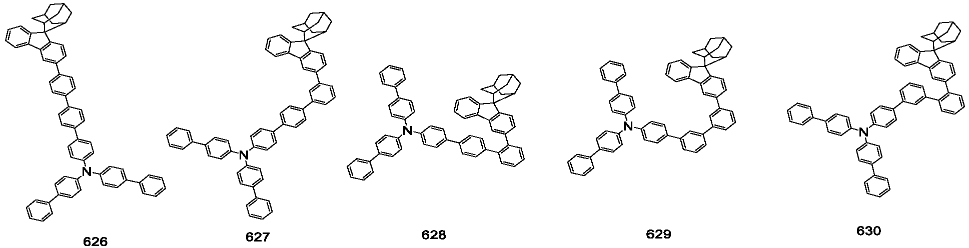

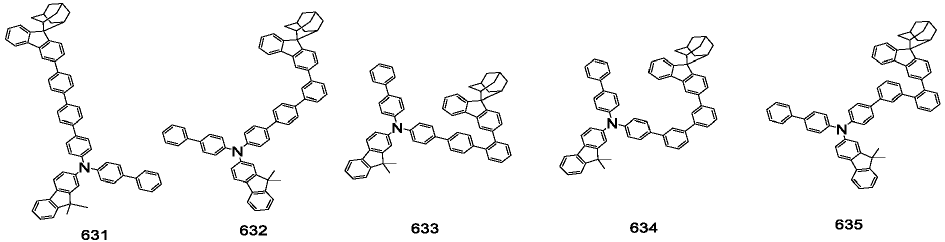

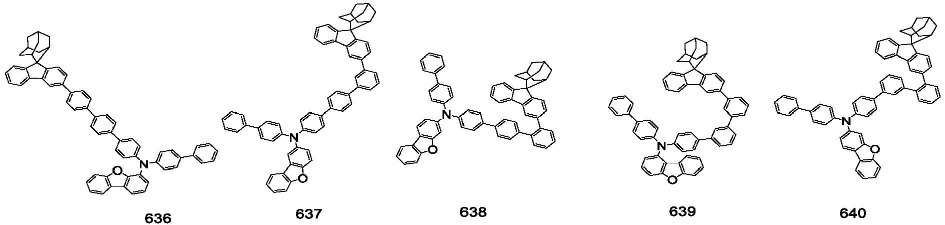

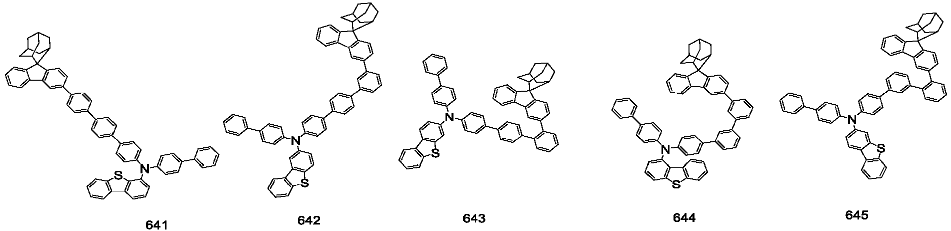

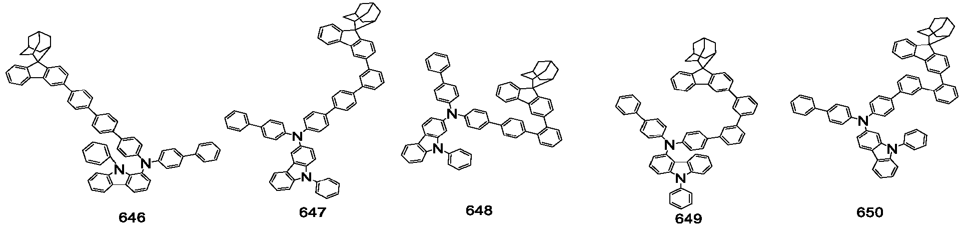

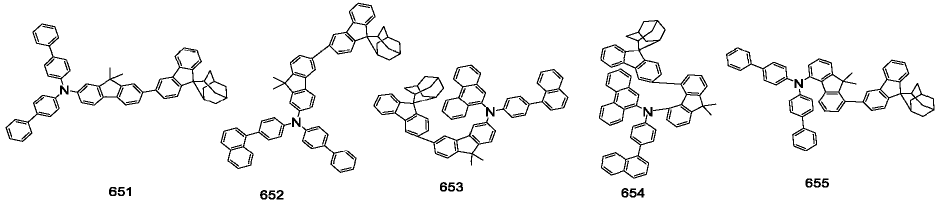

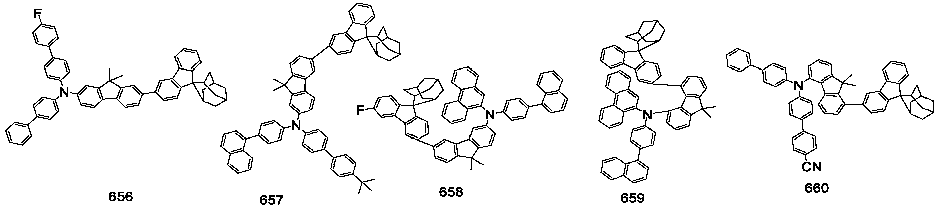

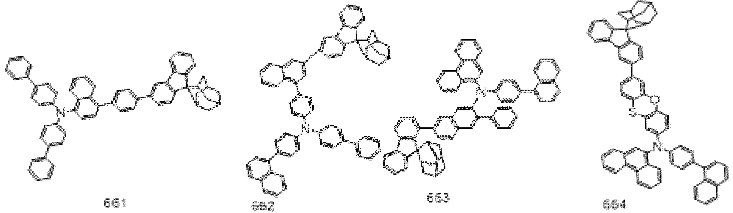

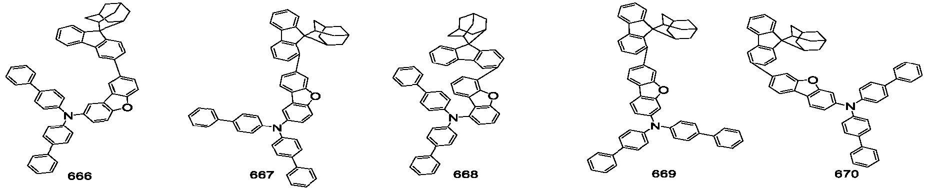

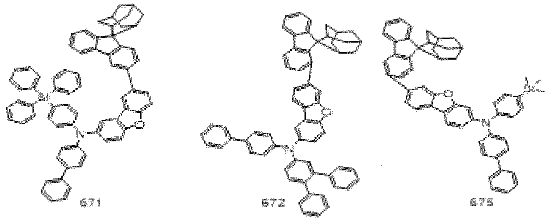

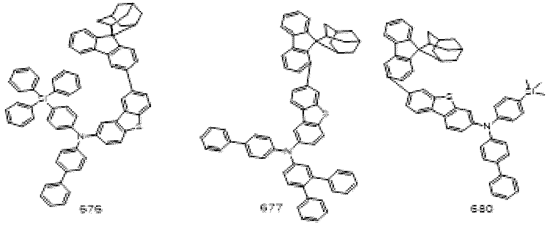

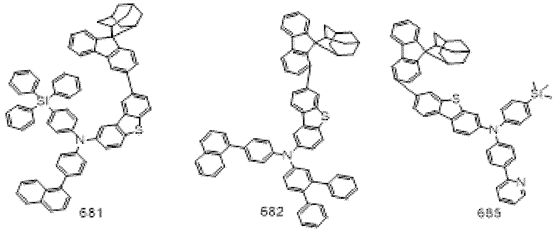

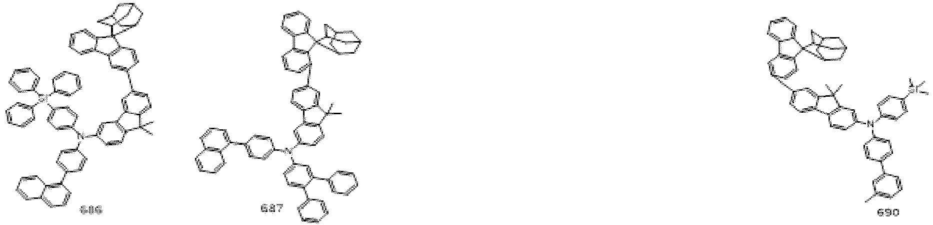

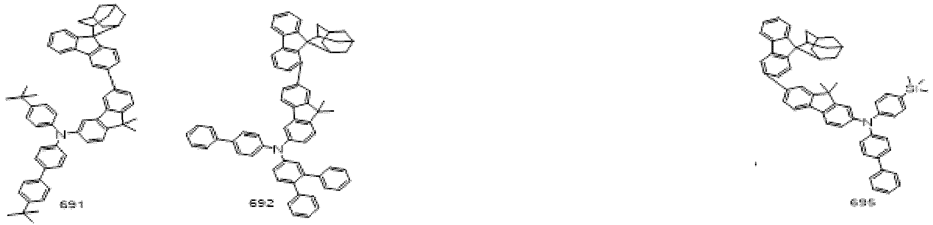

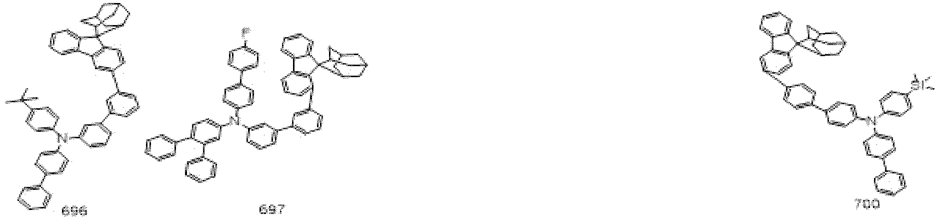

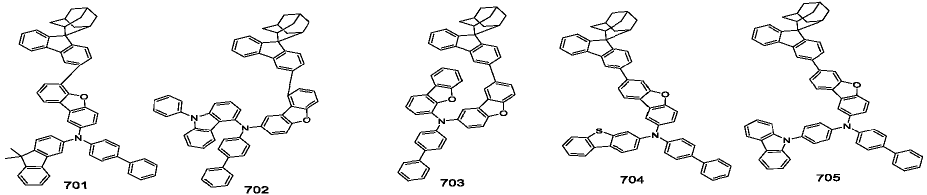

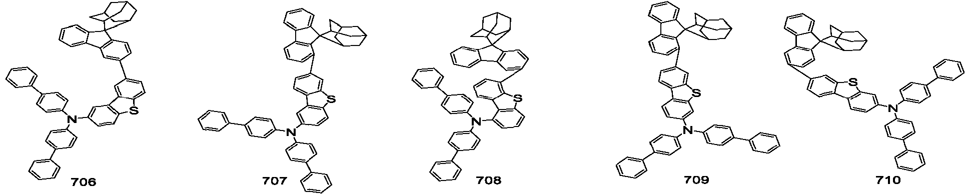

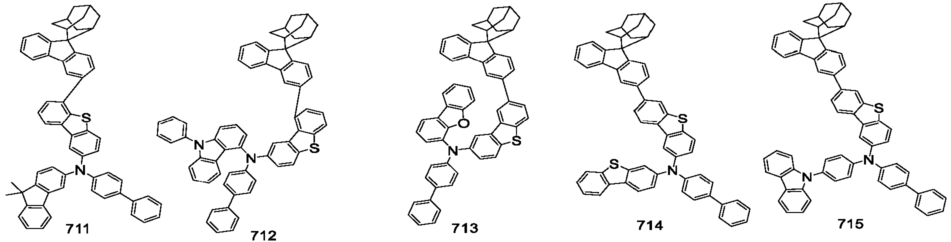

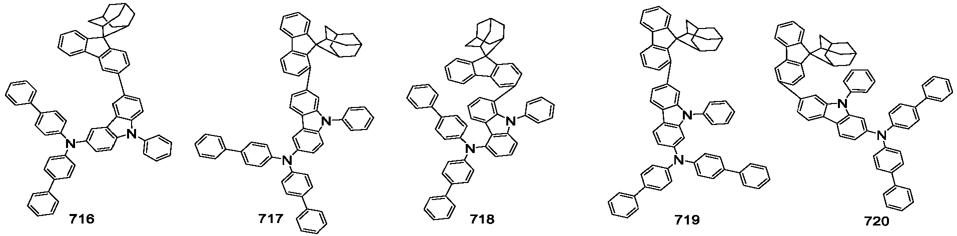

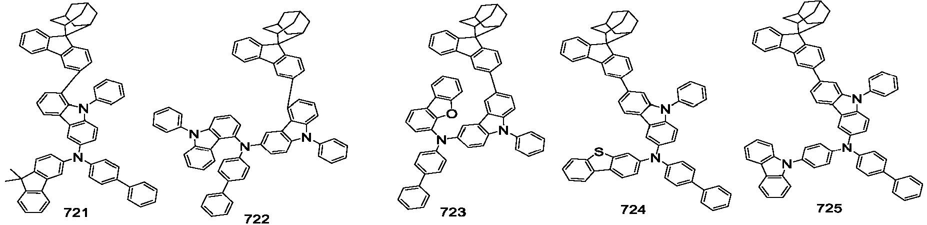

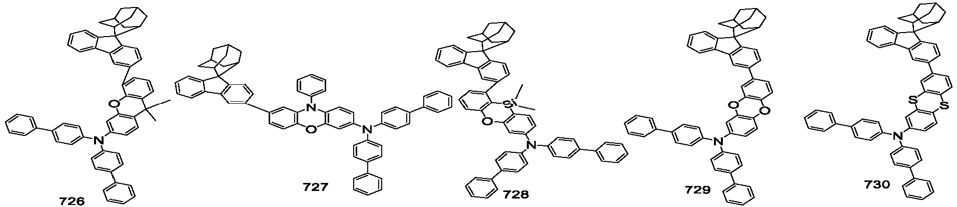

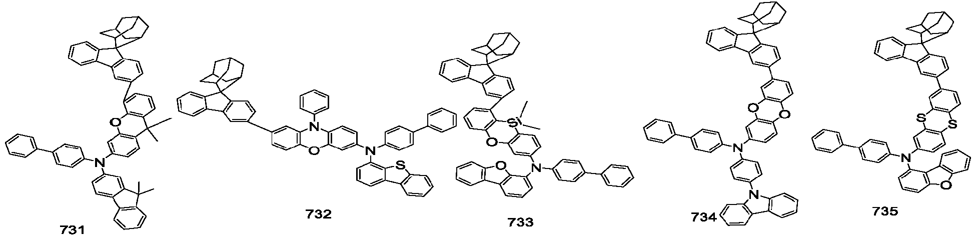

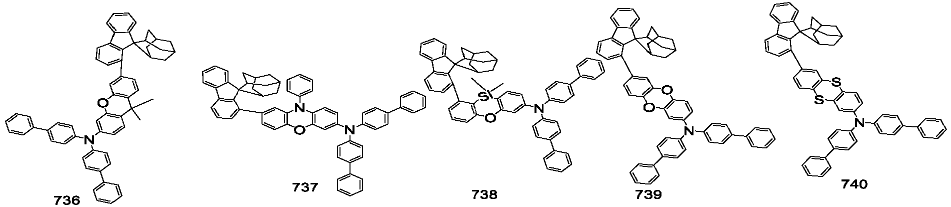

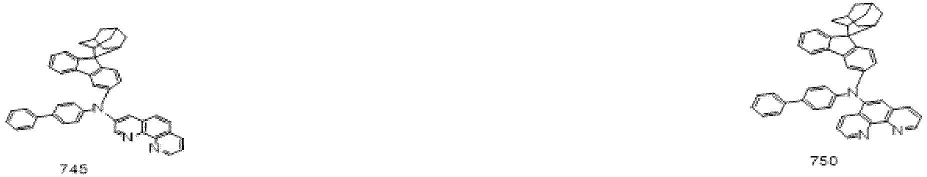

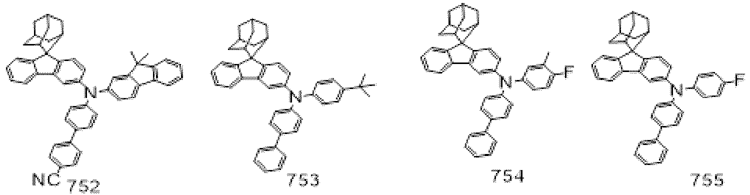

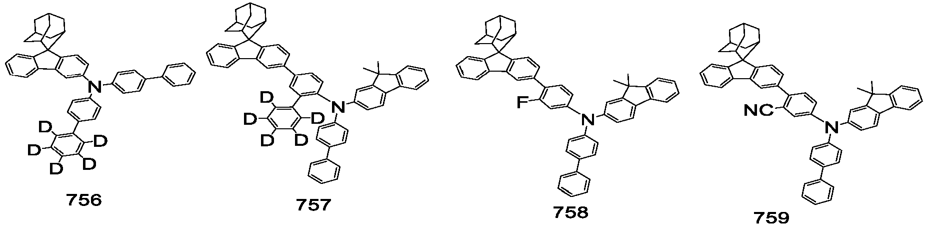

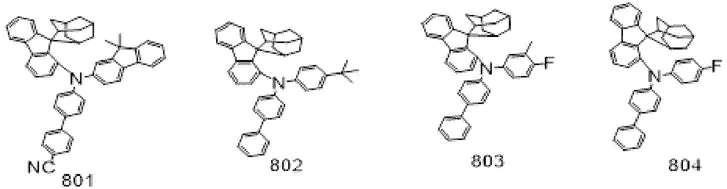

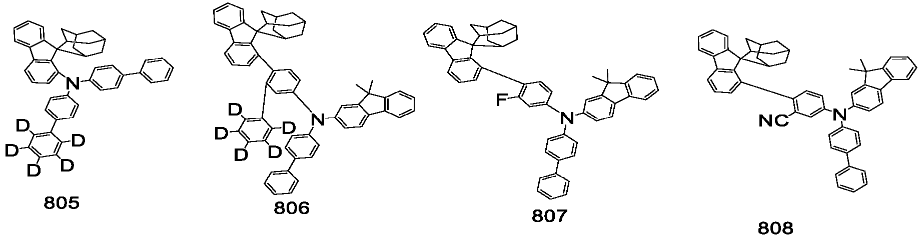

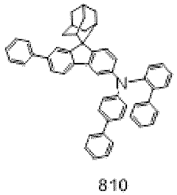

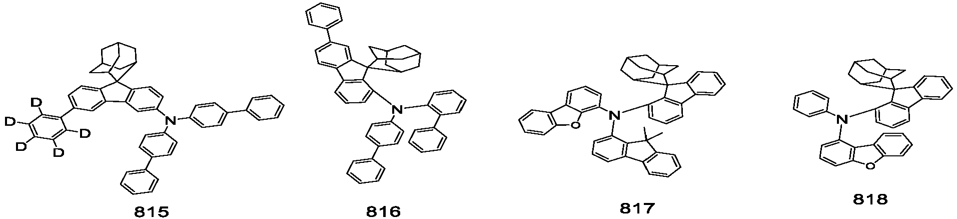

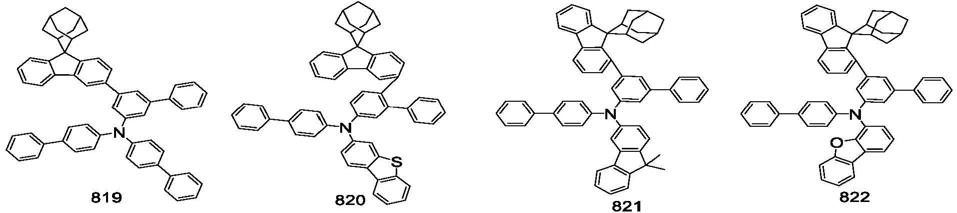

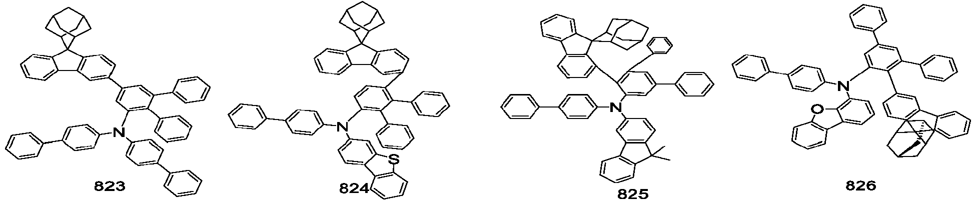

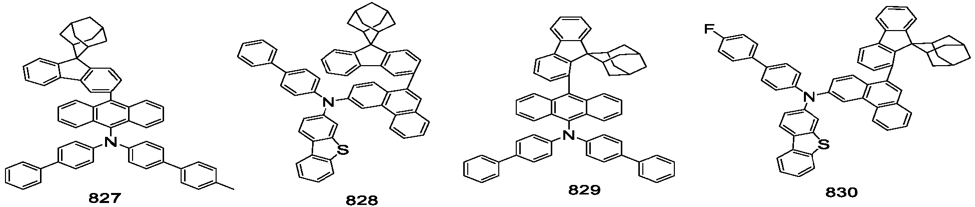

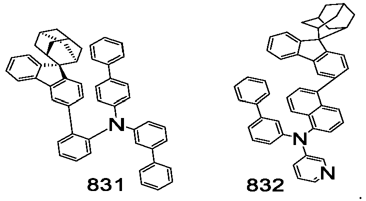

The present disclosure also provides an electronic element including an anode and a cathode disposed opposite to each other, and a functional layer disposed between the anode and the cathode; the functional layer contains the nitrogen-containing compound of the present disclosure. The electronic element may be used for implementing photoelectric conversion or electro-optic conversion.

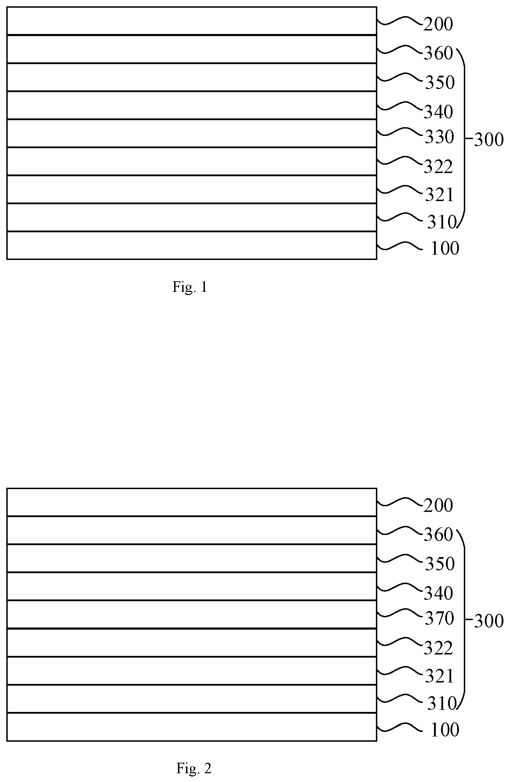

According to an embodiment, the electronic element is an organic electroluminescent device. As shown in FIG. 1, the organic electroluminescent device includes an anode 100 and a cathode 200 disposed opposite to each other, and a functional layer 300 disposed between the anode 100 and the cathode 200; and the functional layer 300 contains the nitrogen-containing compound provided by the present disclosure.

Optionally, the functional layer 300 includes an electron blocking layer 322, and the electron blocking layer 322 contains the nitrogen-containing compound provided by the present disclosure. The electron blocking layer 322 may be composed of the nitrogen-containing compound provided by the present disclosure, or may be composed of the nitrogen-containing compound provided by the present disclosure and other materials.

Optionally, the functional layer 300 includes a hole transporting layer 321 and/or a hole injecting layer 310. The hole transporting layer 321 may contain the nitrogen-containing compound provided by the present disclosure to improve the hole transmission capacity in the electronic element.

In one embodiment of the present disclosure, the organic electroluminescent device may include an anode 100, a hole transporting layer 321, an electron blocking layer 322, an organic electroluminescent layer 330 as the energy conversion layer, an electron transporting layer 350 and cathode 200 that are sequentially stacked. The nitrogen-containing compound provided by the present disclosure may be applied to the electron blocking layer 322, which can effectively improve the luminous efficiency and lifetime of the organic electroluminescent device and reduce the driving voltage of the organic electroluminescent device.

Optionally, the anode 100 includes the anode material. Preferably, it is a material having a large work function that facilitates hole injection into the functional layer. Specific examples of anode materials include: metals such as nickel, platinum, vanadium, chromium, copper, zinc and gold, or their alloys; metal oxides such as zinc oxide, indium oxide, indium tin oxide (ITO) and indium zinc oxide (IZO); combination of metals and oxides such as ZnO:Al or SnO₂: Sb; or conductive polymers such as poly(3-methylthiophene), poly[3,4-(ethylene-1,2-dioxy)thiophene] (PEDT), polypyrrole and polyaniline, but not limited thereto. It preferably includes a transparent electrode containing indium tin oxide (ITO) as the anode.

Optionally, the hole transport layer 321 may contian one or more hole transport materials. The hole transporting material may be selected from carbazole polymers, carbazole-linked triarylamine compounds, or other types of compounds, which is not specially limited in the present disclosure. For example, the hole transporting layer 321 is composed of the compound NPB.

Optionally, the organic light-emitting layer 330 may be composed of a single light-emitting material, and may also comprise a host material and a guest material. Alternatively, the organic light-emitting layer 330 is composed of a host material and a guest material. The holes injected into the organic light-emitting layer 330 and the electrons injected into the organic light-emitting layer 330 may combine in the organic light-emitting layer 330 to form excitons, and the excitons transfer energy to the host material, the host material transfers energy to the guest material, which in turn enables the guest material to emit light.

The host material of the organic light-emitting layer 330 may be a metal chelate compound, a bisstyryl derivative, an aromatic amine derivative, a dibenzofuran derivative, or other types of materials, which is not specially limited in the present disclosure. For example, the host material of the organic light-emitting layer 330 may be CBP.

The guest material of the organic light-emitting layer 330 may be a compound having a condensed aryl ring or a derivative thereof, a compound having a heteroaryl ring or a derivative thereof, an aromatic amine derivative or other materials, which is not specially limited in the present disclosure. For example, the guest material of the organic light-emitting layer 330 may be Ir(ppy)₃.

The electron transporting layer 350 may have a single layer structure or a multilayer structure, which may comprise one or more electron transporting materials, and the electron transporting materials may be selected from benzimidazole derivatives, oxadiazole derivatives, quinoxaline derivatives or other electron transporting materials, which are not specifically limited in the present disclosure. For example, the electron transporting layer 340 may be composed of ET-1 and LiQ.

Optionally, the cathode 200 includes the cathode material, which is a material having a small work function that facilitates electron injection into the functional layer. Specific examples of cathode materials include: metals such as magnesium, calcium, sodium, potassium, titanium, indium, yttrium, lithium, gadolinium, aluminum, silver, tin and lead, or their alloys; or multilayer materials such as LiF/Al, Liq/ Al, LiO₂/Al, LiF/Ca, LiF/Al, and BaF₂/Ca, but not limited thereto. It is preferable to include a metal electrode containing aluminum as the cathode.

Optionally, as shown in FIG. 1, a hole injecting layer 310 may also be provided between the anode 100 and the hole transporting layer 321 to enhance the ability to inject holes into the hole transporting layer 321. The hole injecting layer 310 may be selected from benzidine derivatives, starburst arylamine compounds, phthalocyanine derivatives, or other materials, which is not specially limited in the present disclosure. For example, the hole injecting layer 310 may be composed of m-MTDATA.

Optionally, as shown in FIG. 1, an electron injecting layer 360 may also be provided between the cathode 200 and the electron transport layer 340 to enhance the ability to inject electrons into the electron transport layer 350. The electron injecting layer 360 may include an inorganic material such as an alkali metal sulfide or an alkali metal halide, or may include a complex compound of an alkali metal and an organic compound. For example, the electron injecting layer 360 may include LiQ.

Optionally, a hole blocking layer 340 may be further provided between the organic electroluminescent layer 330 and the electron transporting layer 350.

According to another embodiment, the electronic element may be a photoelectric conversion device. As shown in FIG. 2, the photoelectric conversion device may include an anode 100 and a cathode 200 disposed opposite to each other, and a functional layer 300 disposed between the anode 100 and the cathode 200; the functional layer 300 contains the nitrogen-containing compound provided in the present disclosure.

Optionally, the functional layer 300 includes an electron blocking layer 322, and the electron blocking layer 322 contains the nitrogen-containing compound provided by the present disclosure. The electron blocking layer 322 may be composed of the nitrogen-containing compound provided by the present disclosure, or may be composed of the nitrogen-containing compound provided by the present disclosure and other materials.

Optionally, as shown in FIG. 2, the photoelectric conversion device may include an anode 100, a hole transporting layer 321, an electron blocking layer 322, a photoelectric conversion layer 370 as the energy conversion layer, an electron transporting layer 350 and a cathode 200 that are sequentially stacked. The nitrogen-containing compound provided in the present disclosure may be applied to the electron blocking layer 322 of the photoelectric conversion device, which can effectively improve the luminous efficiency and lifetime of the photoelectric conversion device and increase the open circuit voltage of the photoelectric conversion device.

Optionally, a hole injecting layer 310 may also be provided between the anode 100 and the hole transporting layer 321.

Optionally, an electron injecting layer 360 may also be provided between the cathode 200 and the electron transporting layer 350.

Optionally, a hole blocking layer 340 may also be provided between the photoelectric conversion layer 370 and the electron transport layer 350.

Optionally, the photoelectric conversion device may be a solar cell, especially an organic thin **film** solar cell. According to a specific embodiment, the solar cell includes an anode 100, a hole transport layer 321, an electron blocking layer 322, a photoelectric conversion layer 370, an electron transport layer 350 and a cathode 200 that are sequentially stacked, wherein the electron blocking layer 322 contains the nitrogen-containing compound of the present disclosure.

The present disclosure further provides an electronic device including the above-mentioned electronic element. Since the electronic device has the electronic element, it has the same beneficial effects, which will not be repeated here.

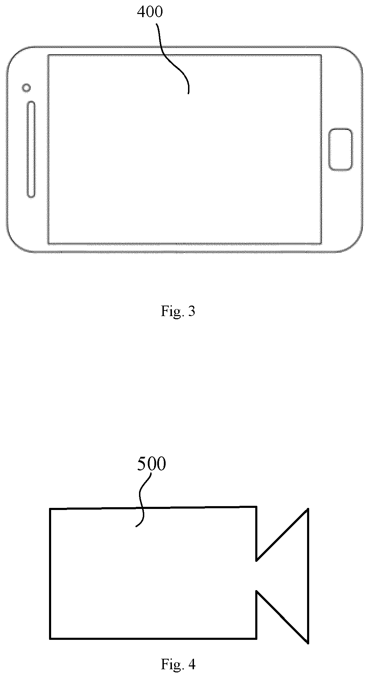

According to an embodiment, as shown in FIG. 3, the electronic device is a first electronic device 400 comprising the above-mentioned organic electroluminescent device. The first electronic device 400 may be a display device, a lighting device, an optical communication device, or other types of electronic devices. For example, the electronic device 400 may include, but is not limited to, a computer screen, a mobile phone screen, a television, an electronic paper, an emergency lighting lamp and an optical module, etc.

According to another embodiment, as shown in FIG. 4, the electronic device is a second electronic device 500 comprising the above-mentioned photoelectric conversion device. The second electronic device 500 may be a solar power generation device, a light detector, a fingerprint recognition device, an optical module, a CCD camera, or other types of electronic devices.

Hereinafter, the present disclosure will be described in further detail through examples.

### Compound Synthesis

The compounds shown in Table 1 were synthesized by the following synthetic route.

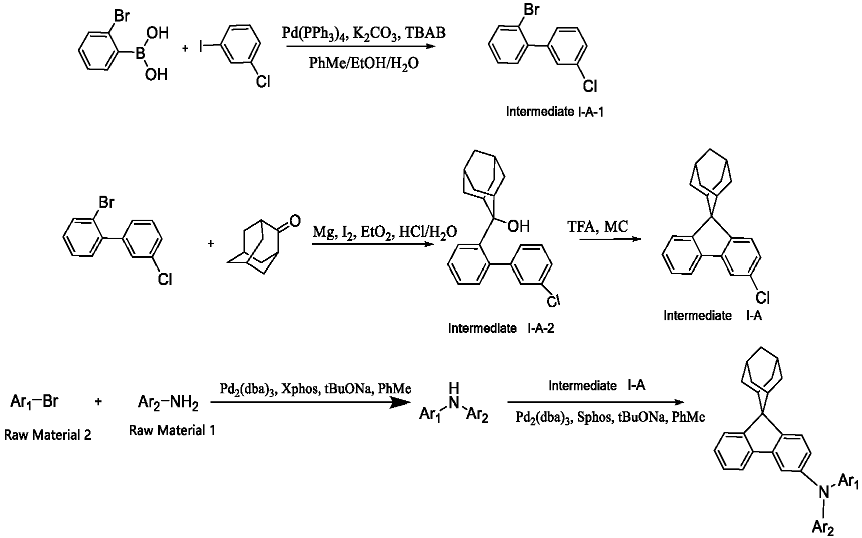

### Synthesis of Compound 1

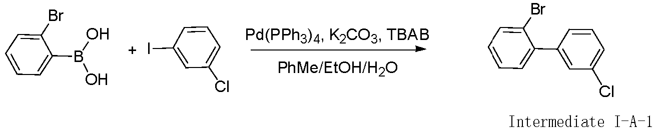

2-bromophenylboronic acid (100.0g, 500.0mmol), 1-chloro-3-iodobenzene (142.6g, 597.6mmol), tetrakis(triphenylphosphine)palladium (11.5g, 9.97mmol), potassium carbonate (102g 746mmol), tetrabutylammonium bromide (32.1g, 99.6mmol), toluene (800mL), ethanol (200mL) and deionized water (200mL) were added to the round bottom flask, heated to 78°C under nitrogen, and stirred for 2 hours, then the obtained reaction solution was cooled to room temperature, and extracted with toluene (500 mL). The organic phases were combined, dried over anhydrous magnesium sulfate, and filtered. The solvent was removed under reduced pressure. The crude product obtained was purified through silica gel column chromatography using n-heptane as the mobile phase, and then was purified by recrystallization with dichloromethane/ethanol system to obtain intermediate I-A-1 as a light yellow solid (64.0 g, yield 48%).

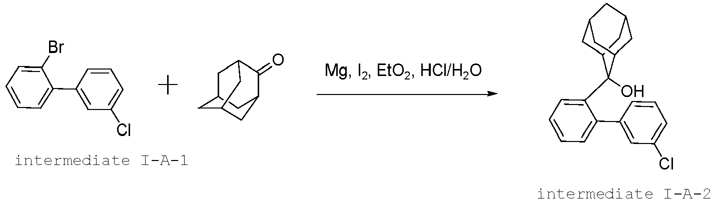

The magnesium bar (13.54 g, 564 mmol) and ether (100 mL) were placed in a dry round bottom flask under nitrogen, and iodine (100 mg) was added. Then, a solution of ether (200 mL) in which intermediate I-A-1 (64.00 g, 187.0 mmol) was dissolved was slowly dropped into the flask. After the addition was completed, the temperature was raised to 35°C and the content was stirred for 3 hours. The obtained reaction solution was lowered to 0°C, and thereto was slowly added dropwise an ether (200mL) solution of amantadone (22.45g, 149mmol) After the dropwise addition, the temperature was raised to 35°C, and stirred for 6 hours. Then, the reaction solution was cooled to room temperature, and 5wt% hydrochloric acid was added to pH< 7. After stirring for 1 hour, ether (200 mL) was added for extraction. The organic phases were combined, dried over anhydrous magnesium sulfate, filtered, and the solvent was removed under reduced pressure. The crude product obtained was purified by silica gel column chromatography using n-heptane as the mobile phase to obtain a solid intermediate I-A-2 (24g, yield 48%).

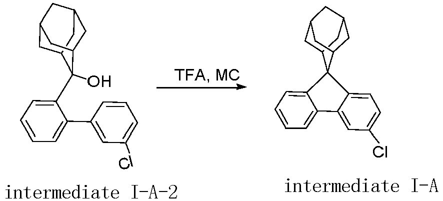

Intermediate I-A-2 (24g, 71.0mmol), trifluoroacetic acid (40.48g, 355.0mmol) and dichloromethane (200mL) were added to a round bottom flask, and stirred for 2 hours under nitrogen. Then, sodium hydroxide aqueous solution was added to pH=8. After liquid separation, the organic phase was dried over anhydrous magnesium sulfate, filtered, and the solvent was removed under reduced pressure. The crude product obtained was purified by recrystallization using dichloromethane/n-heptane (1:2) to obtain intermediate I-A as a white solid (21g, yield 92.5%). ¹HNMR(400MHz, CD₂Cl₂): 8.11(d,1H), 8.03(d,1H), 7.41-7.63(m,2H), 7.37-7.39(m,1H), 7.30-7.33(m,1H), 7.23-7.24(m,1H), 2.88-2.93(m,2H), 2.81-2.85(m,2H), 2.19(s,2H), 1.99(s,2H), 1.77-1.83(m,4H), 1.54(s,2H).

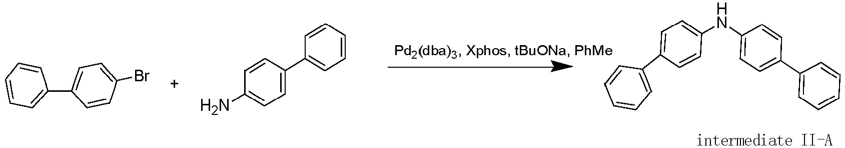

4-bromobiphenyl (4.0g, 17.16mmol), 4-aminobiphenyl (2.96g, 17.5mmol), tris(dibenzylideneacetone)dipalladium (0.16g, 0.17mmol), 2-dicyclohexylphosphino-2',4',6'-triisopropylbiphenyl (0.16g, 0.34mmol) and sodium tert-butoxide (2.47g, 25.74mmol) were added to toluene (40mL) and heated to 108°C under nitrogen protection, and stirred for 2h. Then the obtained reaction solution was cooled to room temperature, and washed with water and dried over magnesium sulfate. After filtration, the solvent was removed from the filtrate under reduced pressure. The crude product obtained was purified by recrystallization using dichloromethane/ethyl acetate system to obtain intermediate II-A as a light yellow solid (4.1g, yield 72.6%).

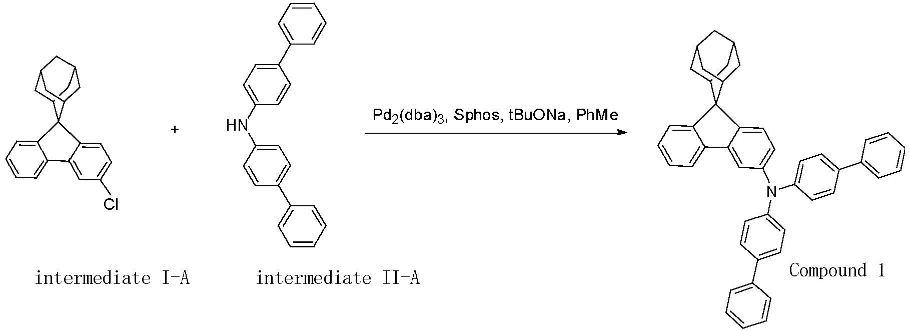

Intermediate I-A (4.1g, 12.77mmol), intermediate II-A (4.1g, 12.77mmol), tris(dibenzylideneacetone)dipalladium (0.12g, 0.13mmol), 2-dicyclohexylphosphino-2',6'-dimethoxybiphenyl (0.10g, 0.25mmol) and sodium tert-butoxide (1.84g, 19.17mmol) were added to toluene (40mL), heated to 108°C under nitrogen protection, and stirred for 1h. After cooling to room temperature, the obtained reaction solution was washed with water and then dried over magnesium sulfate. After filtration, the solvent was removed from the filtrate under reduced pressure. The crude product obtained was purified by recrystallization using toluene system to obtain Compound 1 as a white solid (4.35 g, yield 56.2%). Mass spectrum (MS): m/z=606.3 [M+H]⁺. ¹H NMR (400MHz, CD₂Cl₂): 8.11 (d, 1H), 8.02 (d, 1H), 7.64-7.60 (m, 6H), 7.55 (d, 4H), 7.43 (t, 4H), 7.33-7.24 (m, 8H), 7.06 (dd, 1H), 2.91 (m, 4H), 2.19 (m, 2H), 2.00 (s, 2H), 1.82 (d, 4H), 1.61 (s, 2H).

Referring to the synthesis method of Compound 1, and using raw material 2 to replace 4-bromobiphenyl, using raw material 1 to replace 4-aminobiphenyl, the intermediates in the fourth column of Table 1 were synthesized. The intermediates in the fourth column, which were used to replace intermediate II-A, and intermediate I-A were used to prepare other compounds in Table 1. The specific compound number, structure, raw materials, final step synthesis yield, characterization data, etc. are shown in Table 1.

**Table 1: Compound structure, preparation and characterization data**

| Compound No. | Raw material 1 | Raw material 2 | Intermediate | Compound Structure | Yield (%) | MS (m/z), [M+H]⁺ |
|---|---|---|---|---|---|---|
| 3 | 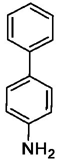 | 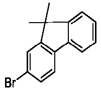 | 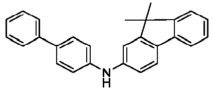 | 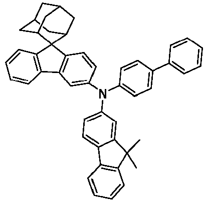 | 67 | 646.3 |
| 7 | | 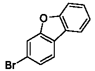 | 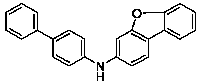 | 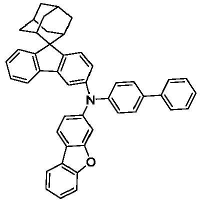 | 69 | 620.3 |
| 15 | | 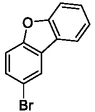 | 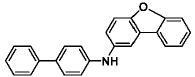 | 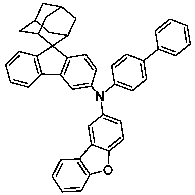 | 72 | 620.3 |
| 9 | | 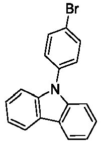 | 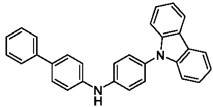 | 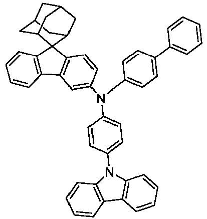 | 67 | 695.3 |
| 23 | | 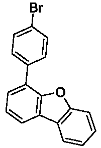 | 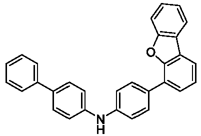 | 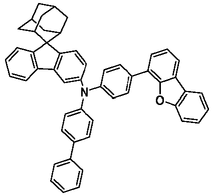 | 54 | 696.3 |
| 487 | | 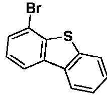 | 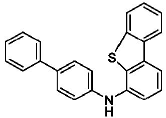 | 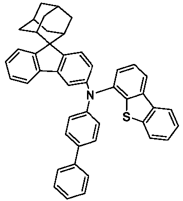 | 61 | 636.3 |
| 745 | | 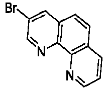 | 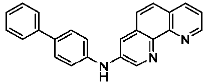 | 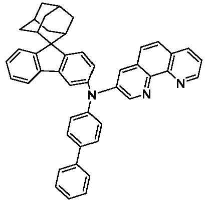 | 53 | 632.3 |
| 36 | 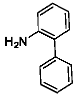 | 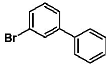 | 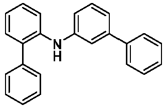 | 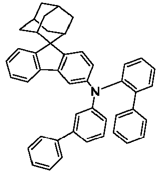 | 55 | 606.3 |
| 31 | | 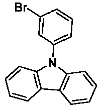 | 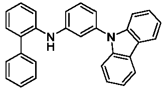 | 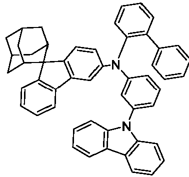 | 59 | 695.3 |
| 54 | 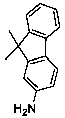 | 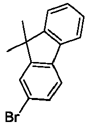 | 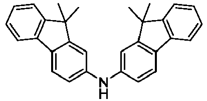 | 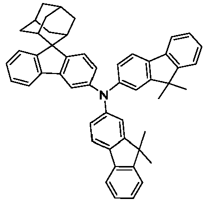 | 61 | 686.4 |
| 60 | | 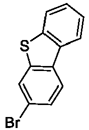 | 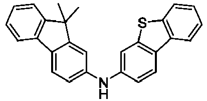 | 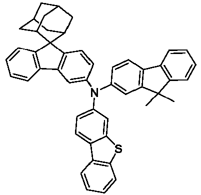 | 62 | 676.3 |
| 66 | | 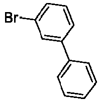 | 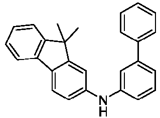 | 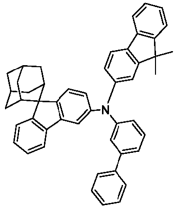 | 47 | 646.3 |
| 71 | | 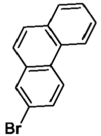 | 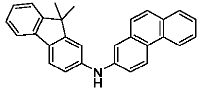 | 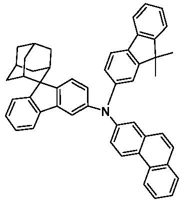 | 49 | 670.3 |
| 87 | 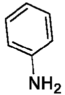 | 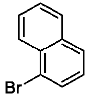 | 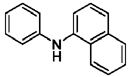 | 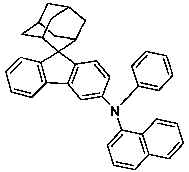 | 53 | 504.3 |
| 92 | | 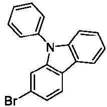 | 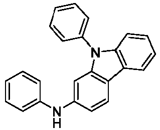 | 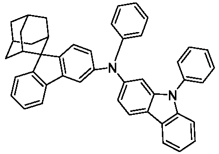 | 55 | 619.3 |
| 95 | | 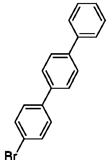 | 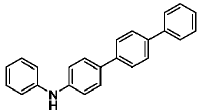 | 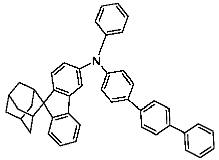 | 67 | 606.3 |
| 115 | | 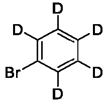 | 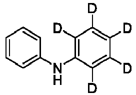 | 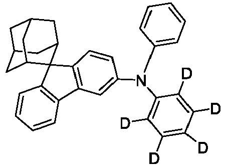 | 69 | 459.3 |
| 116 | 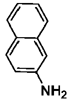 | 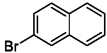 | 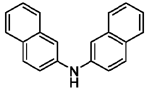 | 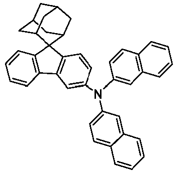 | 70 | 554.3 |
| 128 | | 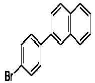 | 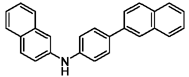 | 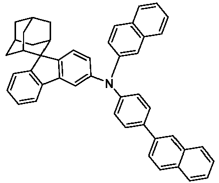 | 50 | 630.3 |
| 127 | | 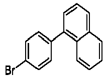 | 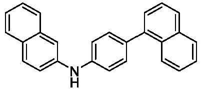 | 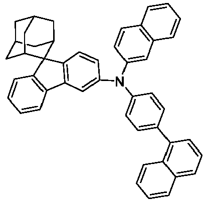 | 43 | 630.3 |
| 147 | 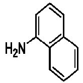 | 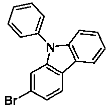 | 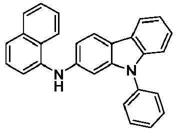 | 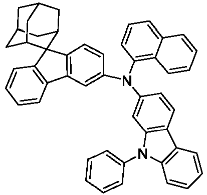 | 49 | 669.3 |
| 160 | | 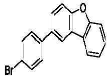 | 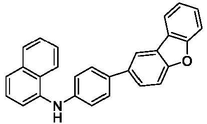 | 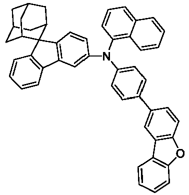 | 48 | 670.3 |
| 195 | 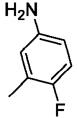 | 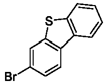 | 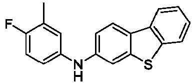 | 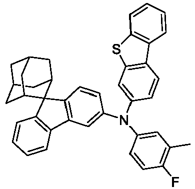 | 54 | 592.2 |
| 206 | 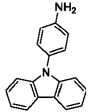 | 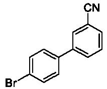 | 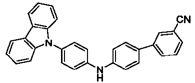 | 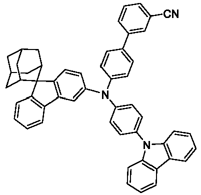 | 58 | 720.3 |

NMR data of Compound 3: ¹H NMR (CD₂Cl₂, 400MHz): 8.10 (d, 1H), 8.00 (d, 1H), 7.67-7.59 (m, 6H), 7.54 (d, 2H), 7.45-7.42 (m, 3H), 7.36-7.25 (m, 8H), 7.12 (br, 1H), 7.06 (br, 1H), 2.92 (t, 4H), 2.19 (d, 2H), 2.00 (s, 2H), 1.82 (d, 4H), 1.61 (s, 2H), 1.44 (s, 6H).

NMR data of Compound 7: ¹H NMR (CD₂Cl₂, 400MHz): 8.11 (d, 1H), 8.03 (d, 1H), 7.91 (d, 1H), 7.85 (d, 1H), 7.62-7.60 (m, 4H), 7.56 (d, 2H), 7.51 (d, 1H), 7.45-7.39 (m, 3H), 7.36-7.26 (m, 7H), 7.21 (d, 1H), 7.07 (d, 1H), 2.92 (t, 4H), 2.19 (d, 2H), 2.00 (s, 2H), 1.82 (d, 4H), 1.62 (s, 2H).

NMR data of Compound 54: ¹H NMR (400MHz, CD₂Cl₂): 8.11 (d, 1H), 7.99 (d, 1H), 7.66 (d, 2H), 7.63-7.62 (m, 3H), 7.58 (d, 1H), 7.41 (d, 2H), 7.35 (s, 2H), 7.32 (t, 2H), 7.28-7.24 (m, 4H), 7.12 (d, 2H), 7.07 (d, 1H), 2.92 (t, 4H), 2.19 (d, 2H), 2.00 (s, 2H), 1.82 (d, 4H), 1.62 (s, 2H), 1.42 (s, 12H).

### Synthesis of Compound 295

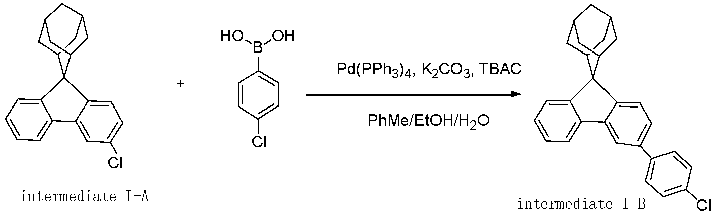

Intermediate I-A (10g, 31.17mmol), p-chlorophenylboronic acid (3.89g, 24.93mmol), tetrakis(triphenylphosphine)palladium (0.72g, 0.62mmol), potassium carbonate (6.45g 46.75mmol), Tetrabutylammonium chloride (1.73g, 6.23mmol), toluene (80mL), ethanol (20mL) and deionized water (20mL) were added to the round-bottom flask, heated to 78°C under nitrogen protection, and stirred for 6 hours. The obtained reaction solution was cooled to room temperature, and toluene (100mL) was added for extraction. The organic phases were combined, dried over anhydrous magnesium sulfate, filtered, and the solvent was removed under reduced pressure. The obtained crude product was purified by silica gel column chromatography using n-heptane as the mobile phase, and then was purified by recrystallization using chloromethane/ethyl acetate system to obtain intermediate I-B as a white solid (7.5g, yield 40.6%).

Referring to the synthesis method of intermediate I-B, the intermediates shown in the third column of Table 2 below were synthesized except that the raw materials 3 in the second column of Table 2 below were used to replace p-chlorophenylboronic acid.

**Table 2 Raw materials and intermediates**

| Intermediate No. | Raw material 3 | Intermediate Structure | Yield (%) |
|---|---|---|---|
| Intermediate I-C | 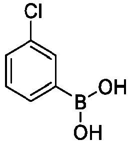 | 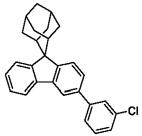 | 37 |
| Intermediate I-D | 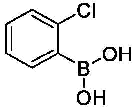 | 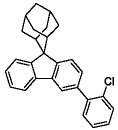 | 41 |
| Intermediate I-E | 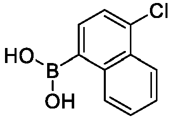 | 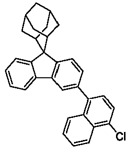 | 44 |
| Intermediate I-F | 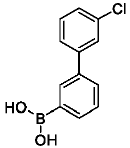 | 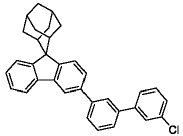 | 39 |

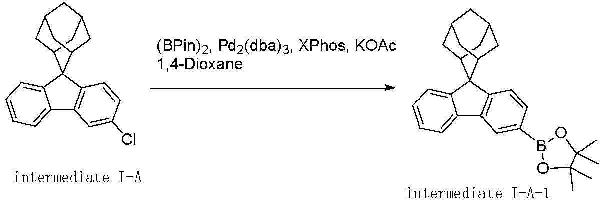

Intermediate I-A (20.4g, 63.7mmol), bis(pinacolato)diboron (19.4g, 76.5mmol), tris(dibenzylideneacetone)dipalladium (0.6g, 0.6mmol), 2-dicyclohexylphosphino-2',4',6'-triisopropylbiphenyl (0.6g; 1.3mmol), potassium acetate (12.5g; 127.4mmol) and 1,4-dioxane (150mL) were added to the flask, and stirred at 100°C with reflux for 16 hours under nitrogen. After cooling to room temperature, dichloromethane and water were added into the obtained reaction solution to separate the layers, the resulting organic phase was washed with water and dried over anhydrous magnesium sulfate, and the solvent was removed under reduced pressure to obtain the crude product. The crude product was purified through silica gel column chromatography using dichloromethane/n-heptane system to obtain intermediate I-A-1 as a white solid (13.3 g, yield 51%).

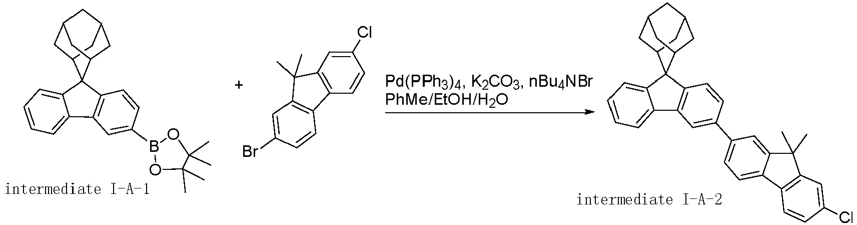

Intermediate I-A-1 (13.3g, 32.3mmol), 2-bromo-7-chloro-9,9-dimethylfluorene (7.1g, 35.5mmol), tetrakis(triphenylphosphine)palladium (0.7g, 0.6mmol), potassium carbonate (11.1g, 80.7mmol) and tetrabutylammonium bromide (2.1g, 6.5mmol) were added to the flask, and then added a mixed solvent of toluene (80mL), ethanol (20mL) and water (20mL), heated to 80°C and stirred for 24 hours maintaining the temperature under nitrogen protection. After cooling to room temperature, the stirring was stopped, the resulting reaction solution was washed with water, then the organic phase was separated from it and dried over anhydrous magnesium sulfate, and the solvent was removed under reduced pressure to obtain a crude product. The crude product was purified by silica gel column chromatography using dichloromethane/n-heptane as the mobile phase to obtain product intermediate I-A-2 as a white solid (9.0 g, yield 54.5%).

Referring to the synthesis method of intermediate I-A-2, the intermediates shown in the third column of Table 3 below were synthesized except that the raw material 41 in the second column of Table 3 below were used to replace 2-bromo-7-chloro-9,9-dimethylfluorene.

**Table 3 Raw materials and intermediates**

| Intermediate No. | Raw material 41 | Intermediate structure | Yield (%) |
|---|---|---|---|
| Intermediate I-A-3 | 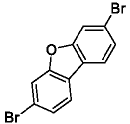 | 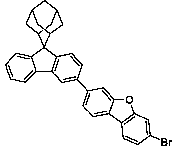 | 59 |
| Intermediate I-A-4 | 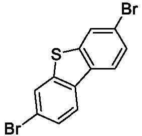 | 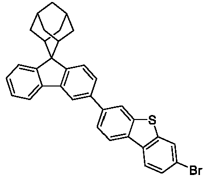 | 62 |
| Intermediate I-A-5 | 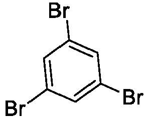 | 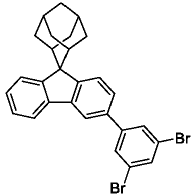 | 31 |

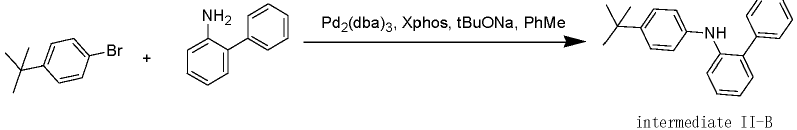

4-tert-butylbromobenzene(4.0g, 25.5mmol),2-aminobiphenyl(4.39g, 25.9mmol), tris(dibenzylideneacetone)dipalladium (0.23g, 0.25mmol), 2-dicyclohexylphosphino-2',4',6'-triisopropylbiphenyl (0.24g, 0.50mmol) and sodium tert-butoxide (3.67g, 38.22mmol) were added to toluene (40mL), heated to 108°C and stirred for 2h under nitrogen. After cooling to room temperature, the obtained reaction solution was washed with water and dried by adding magnesium sulfate. After filtration, the solvent was removed from the filtrate under reduced pressure. The crude product obtained was purified by recrystallization using dichloromethane/ethyl acetate system to obtain Intermediate II-B as a light yellow solid (3.2g, yield 56.6%).

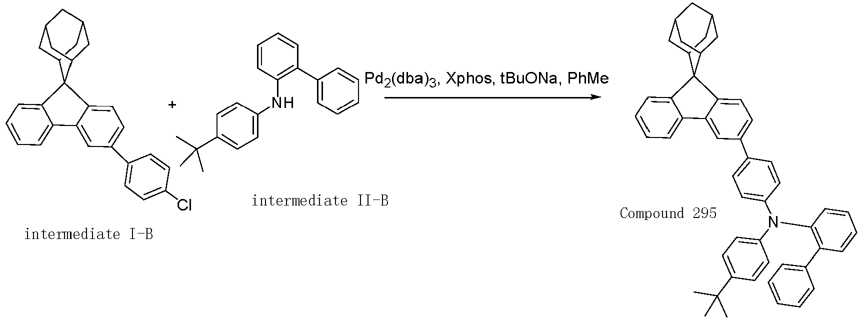

Intermediate I-B (1.50g, 3.78mmol), intermediate II-B (0.95g, 3.85mmol), tris(dibenzylideneacetone) dipalladium (0.03g, 0.04mmol), 2-dicyclohexylphosphinodicyclohexylphosphino-2',6'-dimethoxybiphenyl (0.03g, 0.07mmol) and sodium tert-butoxide (0.55g, 5.67mmol) were added to toluene (20mL), heated to 108°C and stirred for 5h under nitrogen protection. After cooling to room temperature, the obtained reaction solution was washed with water and dried by adding magnesium sulfate. After filtration, the solvent was removed from the filtrate under reduced pressure. The crude product obtained was urified by recrystallization using toluene system to obtain Compound 295 as a white solid (1.7 g, 74%). Mass spectrum: m/z=662.4 [M+H]⁺.

Refering to the synthesis method of Compound 295, and using the raw materials 4 to replace 2-aminobiphenyl, and the raw material 5 to replac 4-tert-butylbromobenzene, the intermediates in the fourth column in the following table were synthesized. Other compounds in Table 4 were prepared by replacing the intermediate II-B with the intermediates in the fourth column. The specific compound number, structure, raw materials, synthesis yield of the last step, characterization data, etc. are shown in Table 4.

**Table 4: Compound number, structure, preparation and characterization data**

| Compound No. | Raw material 4 | Raw material 5 | Intermediate | Structure | Yield (%) | MS (m/z) [M+H]⁺ |
|---|---|---|---|---|---|---|
| 304 | 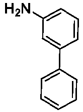 | 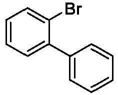 | 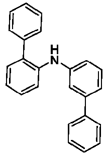 | 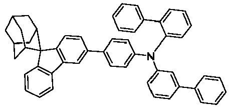 | 61 | 682.3 |
| 316 | 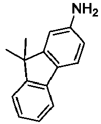 | 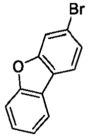 | 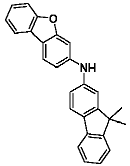 | 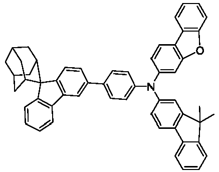 | 54 | 736.4 |
| 341 | 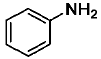 | 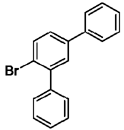 | 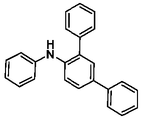 | 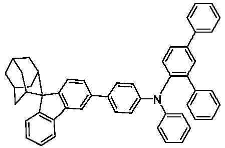 | 59 | 682.3 |
| 351 | | 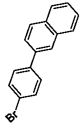 | 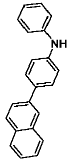 | 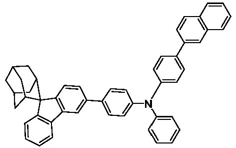 | 63 | 656.3 |
| 384 | 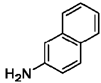 | 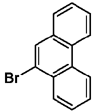 | 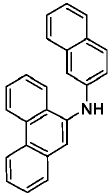 | 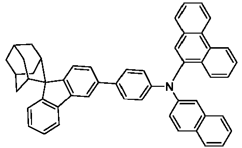 | 66 | 680.3 |
| 369 | 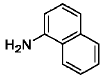 | 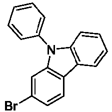 | 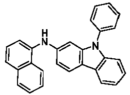 | 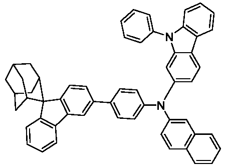 | 41 | 745.4 |

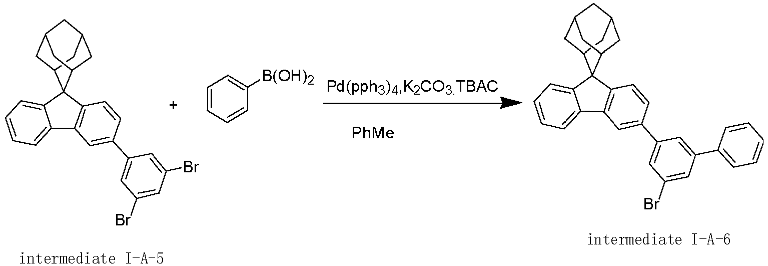

Intermediate I-A-6 was prepared according to the synthesis method of intermediate I-B, except that intermediate I-A was replaced by intermediate I-A-5, and p-chlorophenylboronic acid was replaced by phenylboronic acid to obtain intermediate I-A-6.

Referring to the synthesis method of Compound 1, the compounds shown in the forth column of Table 5 below were prepared, except that the intermediate shown in the third column of Table 5 below replaces the intermediate I-A to react with the intermediate II-A. The specific compound number, structure, raw materials, the synthetic yield of last step and characterization data are shown in Table 5.

**Table 5: Compound number, structure, preparation and characterization data**

| Compound No. | Intermediate No. | Intermediate Structure | Compound Structure | Yield(%) | MS (m/z) [M+H]⁺ |
|---|---|---|---|---|---|
| 496 | Intermediate I-C | 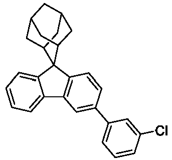 | 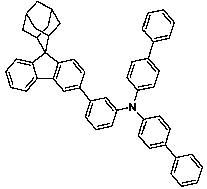 | 61 | 682.3 |
| 499 | Intermediate I-D | 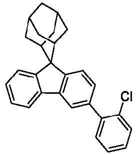 | 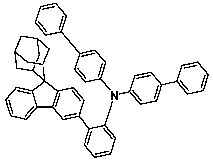 | 57 | 682.3 |
| 508 | Intermediate I-E | 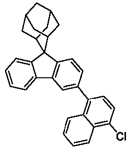 | 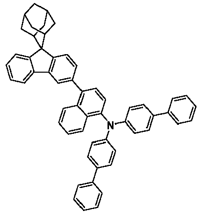 | 69 | 732.4 |
| 581 | Intermediate I-F | 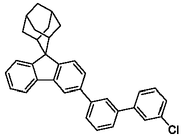 | 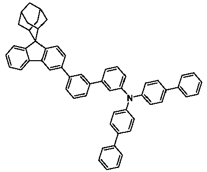 | 54 | 758.4 |
| 651 | Intermediate I-A-2 | 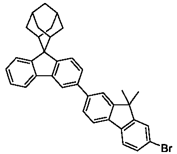 | 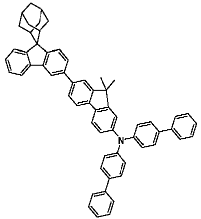 | 49 | 798.4 |
| 670 | Intermediate I-A-3 | 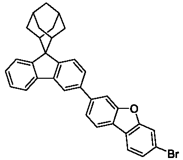 | 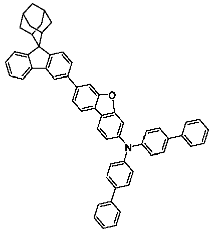 | 44 | 772.4 |
| 710 | Intermediate I-A-4 | 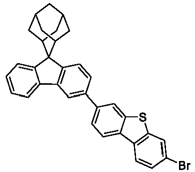 | 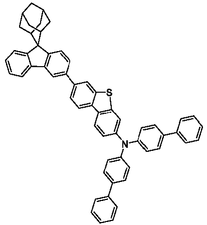 | 51 | 788.3 |
| 819 | Intermediate I-A-6 | 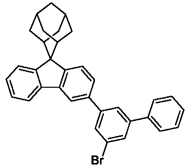 | 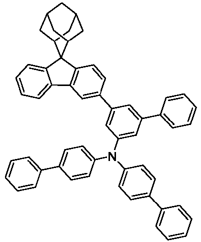 | 49 | 758.4 |

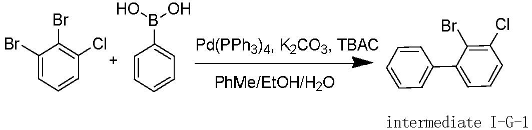

1,2-dibromo-3-chlorobenzene (80.0g, 298.7mmol), phenylboronic acid (36.5g, 298.7mmol), tetrakis(triphenylphosphine)palladium (6.9g, 6.0mmol), potassium carbonate (103.2 g, 746.7mmol), and tetrabutylammonium bromide (19.2g; 59.7mmol) were added to the flask, and then added a mixed solvent of toluene (600mL), ethanol (150mL) and water (150mL), heated to 80°C and stirred for 18 hours maintaining the temperature under nitrogen protection. After cooling to room temperature, stopped stirring, the resulting reaction liquid was washed with water, then the organic phase was separated from it and and dried over anhydrous magnesium sulfate, and the solvent was removed under reduced pressure to obtain a crude product. The crude product was prufied by silica gel column chromatography using dichloromethane/n-heptane as the mobile phase to obtain product intermediate I-G-1 as a white solid (42.0g, yield 53%).

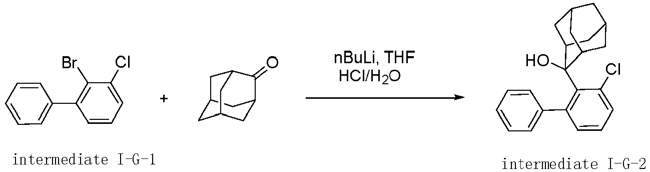

Intermediate I-G-1 (42.0g, 157.9mmol) and tetrahydrofuran (300mL) were added to the flask, cooled down -78°C under nitrogen protection, and a solution of n-butyllithium in tetrahydrofuran (2.5M) (95mL, 236.9mmol) was added dropwise under stirring. The stirring was maintained for 1 hour after the dropwise addition. Keeping at -78°C, adamantanone-(19.0g, 126.3mmol) solution in tetrahydrofuran (100mL) was added dropwise. After the addition, the temperature was maintained for 1 hour, and then warmed up to room temperature and stirred for 24 hours. A solution of hydrochloric acid (12M) (26.3mL, 315.8mmol) in water (100mL) was added to the resulting reaction solution and stirred for 1 hour. Separating the liquid, the obtained organic phase was washed to neutrality with water, and anhydrous magnesium sulfate was added for drying. The solvent was removed under reduced pressure to obtain a crude product. The crude product was purified by silica gel column chromatography using ethyl acetate/n-heptane system to obtain product intermediate I-G-2 as a white solid (25.8g, yield 48%).

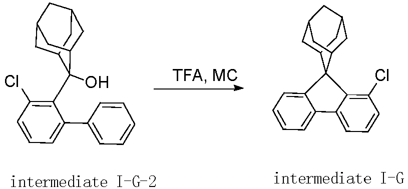

Intermediate I-G was synthesized referring to the synthesis method of intermediate I-A, except that intermediate I-G-2 was used to replace intermediate I-A-2.

Referring to the synthesis method of Compound 1, the compounds shown in the fifth column of Table 6 were prepared in which intermediate I-A was replaced by intermediate I-G, 4-aminobiphenyl was replaced by raw material 6 in the second column in the following table, 4-bromobiphenyl was replaced by raw material 7 in the third column, and intermediate II-A was replaced by the intermediates in the fourth column synthesized by raw material 6 and raw material 7. The specific compound number, structure, final step synthesis yield, characterization data, etc. are shown in Table 6.

**Table 6: Compound number, structure, preparation and characterization data**

| Compound No. | Raw material 6 | Raw material 7 | Intermediate | Compound Structure | Yield (%) | MS (m/z) [M+H]⁺ |
|---|---|---|---|---|---|---|
| 817 | 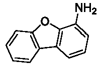 | 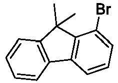 | 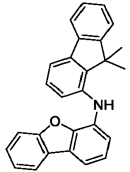 | 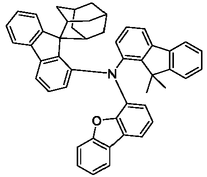 | 43 | 660.3 |
| 818 | 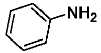 | 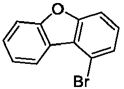 | 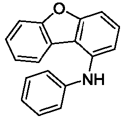 | 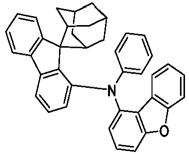 | 51 | 544.3 |

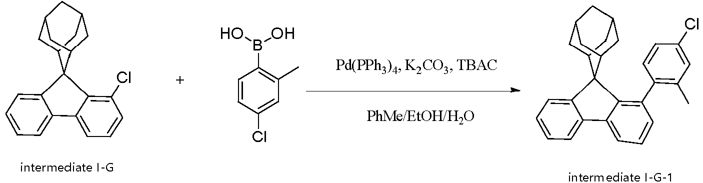

Intermediate I-G-1 was synthesized referring to the synthesis method of intermediate I-B, except that intermediate I-G was used to replace intermediate I-A.

Compound 439 was synthesized referring to the synthesis method of Compound 295, except that intermediate I-G-1 was used to replace intermediate I-B. The specific compound number, structure, final step synthesis yield, characterization data, etc. are shown in Table 7.

**Table 7 Compound number, structure, preparation and characterization data**

| Compound No. | Compound Structure | yield (%) | Mass (m/z) [M+H]⁺ |
|---|---|---|---|
| 439 | 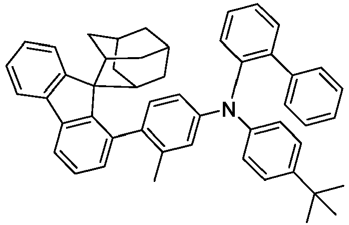 | 37 | 676.4 |

### Preparation and evaluation of organic electroluminescent devices

### Example 1

The green organic electroluminescent device was manufactured by the following method.

The ITO substrate (made by Corning) with an ITO thickness of 1500Å was cut into a size of 40mm (length) × 40mm (width) × 0.7mm (thickness), then making it into an experimental substrate with cathode, anode and insulating layer patterns by the photolithography process. The experimental substrate was treated with ultraviolet ozone and O₂:N₂ plasma to increase the work function of the anode (experimental substrate) and remove scum.

m-MTDATA was vacuum-evaporated on the experimental substrate (anode) to form a hole injecting layer (HIL) with a thickness of 100 Å, and NPB was vacuum-evaporated on the hole injecti layer to form a first hole transporting layer with a thickness of 1000Å.

Compound 1 was evaporated on the first hole transporting layer to form a second hole transporting layer with a thickness of 350Å.

CBP as the host material was doped with Ir(ppy)₃ at a film thickness ratio of 100:5 simultaneously to form a light-emitting layer (EML) with a thickness of 380Å.

ET-1 and LiQ were mixed in a weight ratio of 1:1 and evaporated to form an electron transport ing layer (ETL) with a thickness of 300Å. LiQ was vapor-deposited on the electron transporting layer to form an electron injecting layer (EIL) with a thickness of 10Å. Magnesium (Mg) and silver (Ag) were then mixed and vacuum-evaporated at a vapor deposition rate of 1:9 on the electron injecting layer to form a cathode with a thickness of 120Å.

In addition, CP-1 was vapor-deposited on the cathode with a thickness of 650 Å, thereby completing the manufacture of the organic light-emitting device.

The chemical structures of some materials used during the manufacture of the above electroluminescent device are shown as follows:

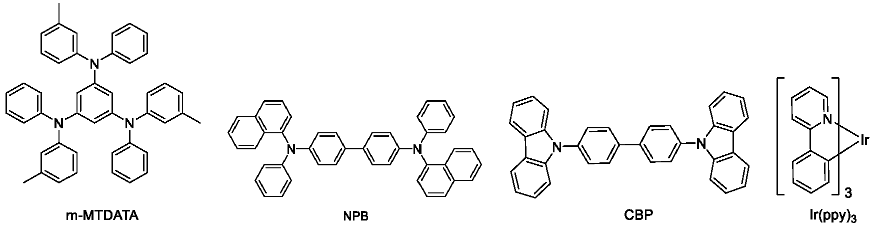

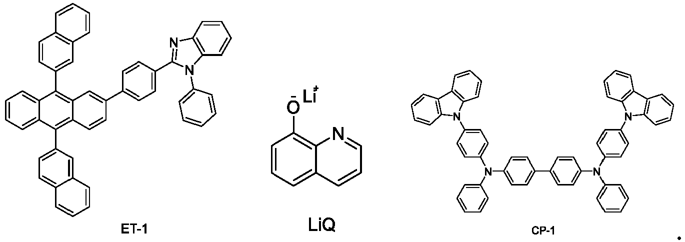

### Example 2 to Example 43

The devices of Examples 2 to 43 were manufactured by referring to the method of Example 1, except that the compounds synthesized shown in Table 8 below were used to replace Compound 1, respectively, to form the second hole transporting layer with a thickness of 350Å, thereby completing the manufacture of corresponding green organic electroluminescent devices.

### Comparative Example 1 to Comparative Example 7

The devices of Comparative Example 1 to Comparative Example 7 were manufactured by referring to the method of Example 1, except that Compound A, Compound B, Compound C, Compound D, Compound E, Compound F and Compound G were used to replace Compound 1, respectively, thereby completing the manufacture of corresponding green organic electroluminescent devices.

The chemical structures of Compound A, Compound B, Compound C, Compound D, Compound E, Compound F and Compound G are shown as follows:

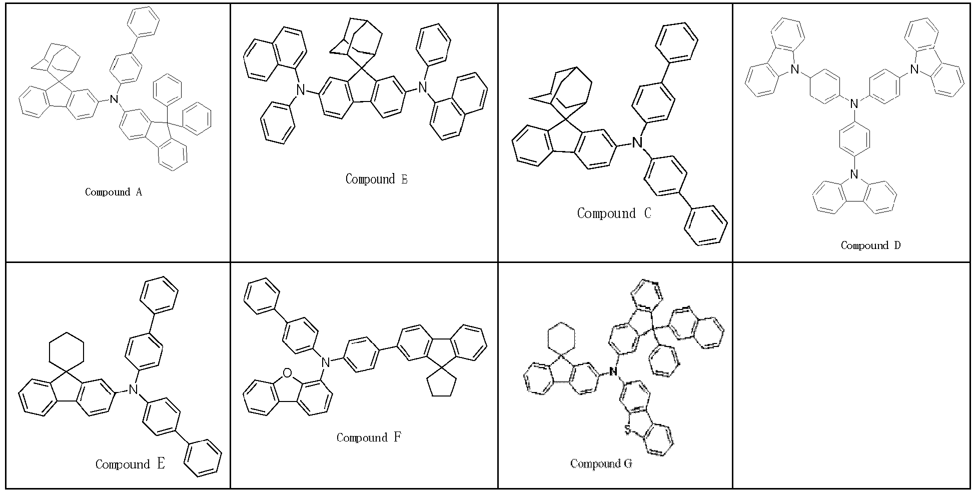

The green organic electroluminescent devices manufactured in Examples 1 to 43 and Comparative Examples 1 to 7 were tested for performance. Specifically, the IVL performance of the devices was tested under the condition of 10mA/cm², and the T95 lifetime of the devices was tested under the initial brightness of 17,000 nit. The test results are shown in Table 8.

**Table 8 Performance test results of green organic electroluminescent devices**

| Examples | Second hole transporting layer material | Driving voltage (V) | Current efficiency (Cd/A) | Color coordinateCI Ex ,CIEy | External quantum efficiency EQE(%) | T95 lifetime (h) |
|---|---|---|---|---|---|---|
| Example 1 | Compound 1 | 4.11 | 86.92 | 0.266,0.700 | 32.7 | 160 |
| Example 2 | Compound 3 | 4.14 | 86.84 | 0.266,0.701 | 32.5 | 158 |
| Example 3 | Compound 7 | 4.10 | 87.01 | 0.265,0.700 | 33.1 | 161 |
| Example 4 | Compound 9 | 4.13 | 86.96 | 0.262,0.702 | 32.9 | 155 |
| Example 5 | Compound 15 | 4.12 | 86.92 | 0.262,0.702 | 32.9 | 160 |
| Example 6 | Compound 23 | 4.14 | 86.78 | 0.261,0.704 | 32.5 | 158 |
| Example 7 | Compound 36 | 4.09 | 87.01 | 0.266,0.700 | 33.1 | 161 |
| Example 8 | Compound 31 | 4.07 | 86.93 | 0.266,0.700 | 32.5 | 162 |
| Example 9 | Compound 54 | 4.09 | 86.80 | 0.266,0.701 | 32.5 | 158 |
| Example 10 | Compound 60 | 4.08 | 87.01 | 0.265,0.700 | 33.1 | 162 |
| Example 11 | Compound 66 | 4.12 | 86.95 | 0.262,0.702 | 32.8 | 156 |
| Example 12 | Compound 71 | 4.15 | 86.92 | 0.262,0.702 | 32.9 | 160 |
| Example 13 | Compound 87 | 4.14 | 86.78 | 0.261,0.704 | 32.5 | 158 |
| Example 14 | Compound 92 | 4.18 | 87.04 | 0.266,0.700 | 32.9 | 163 |
| Example 15 | Compound 95 | 4.07 | 86.92 | 0.266,0.700 | 32.7 | 160 |
| Example 16 | Compound 115 | 4.09 | 86.84 | 0.266,0.701 | 32.5 | 158 |
| Example 17 | Compound 116 | 4.12 | 87.02 | 0.265,0.700 | 33.0 | 160 |
| Example 18 | Compound 128 | 4.14 | 86.94 | 0.262,0.702 | 32.9 | 155 |
| Example 19 | Compound 127 | 4.09 | 86.92 | 0.262,0.702 | 32.9 | 160 |
| Example 20 | Compound 147 | 4.11 | 86.79 | 0.261,0.704 | 32.4 | 158 |
| Example 21 | Compound 162 | 4.15 | 87.01 | 0.266,0.700 | 33.1 | 161 |
| Example 22 | Compound 195 | 4.12 | 86.78 | 0.261,0.704 | 32.5 | 158 |
| Example 23 | Compound 206 | 4.11 | 87.00 | 0.266,0.700 | 33.3 | 165 |
| Example 24 | Compound 295 | 4.08 | 86.89 | 0.262,0.702 | 32.9 | 155 |
| Example 25 | Compound 304 | 4.14 | 86.92 | 0.263,0.702 | 33.0 | 160 |
| Example 26 | Compound 316 | 4.17 | 86.80 | 0.261,0.704 | 32.4 | 158 |
| Example 27 | Compound 341 | 4.15 | 87.12 | 0.266,0.700 | 33.1 | 161 |
| Example 28 | Compound 351 | 4.18 | 87.12 | 0.266,0.700 | 33.1 | 161 |
| Example 29 | Compound 384 | 4.12 | 86.83 | 0.262,0.703 | 32.8 | 155 |
| Example 30 | Compound 369 | 4.10 | 86.90 | 0.262,0.703 | 32.9 | 160 |
| Example 31 | Compound 439 | 4.15 | 84.90 | 0.263,0.703 | 32.8 | 155 |
| Example 32 | Compound 487 | 4.15 | 84.73 | 0.262,0.703 | 31.8 | 161 |
| Example 33 | Compound 496 | 4.06 | 83.17 | 0.262,0.700 | 32.9 | 160 |
| Example 34 | Compound 499 | 4.09 | 85.44 | 0.262,0.704 | 30.2 | 159 |
| Example 35 | Compound 508 | 4.18 | 86.32 | 0.266,0.701 | 31.5 | 160 |
| Example 36 | Compound 581 | 4.11 | 83.69 | 0.263,0.703 | 32.8 | 155 |
| Example 37 | Compound 651 | 4.17 | 82.7 | 0.262,0.702 | 31.9 | 159 |
| Example 38 | Compound 670 | 4.10 | 83.62 | 0.262,0.702 | 30.2 | 157 |
| Example 39 | Compound 710 | 4.09 | 85.17 | 0.263,0.703 | 32.5 | 155 |
| Example 40 | Compound 745 | 4.17 | 82.69 | 0.263,0.703 | 31.5 | 154 |
| Example 41 | Compound 817 | 4.11 | 85.74 | 0.262,0.702 | 30.9 | 160 |
| Example 42 | Compound 818 | 4.07 | 87.19 | 0.261,0.704 | 32.2 | 159 |
| Example 43 | Compound 819 | 4.14 | 86.81 | 0.261,0.704 | 32.2 | 159 |
| Comparative Example 1 | Compound A | 4.67 | 86.89 | 0.262,0.702 | 25.8 | 149 |
| Comparative Example 2 | Compound B | 4.73 | 85.04 | 0.262,0.702 | 24.2 | 145 |
| Comparative Example 3 | Compound C | 4.62 | 85.54 | 0.263,0.703 | 24.4 | 155 |
| Comparative Example 4 | Compound D | 4.66 | 82.69 | 0.263,0.703 | 24.5 | 154 |
| Comparative Example 5 | Compound E | 4.65 | 85.04 | 0.262,0.702 | 24.2 | 140 |
| Comparative Example 6 | Compound F | 4.68 | 85.64 | 0.263,0.703 | 24.4 | 143 |
| Comparative Example 7 | Compound G | 4.72 | 84.54 | 0.263,0.703 | 24.4 | 150 |

According to the above table, the luminous efficiency (Cd/A) and lifetime of the organic electroluminescent devices manufactured in Examples 1 to 43 are comparable to those of Comparative Examples 1 to 7 in the case of little difference in the color coordinates, while the overall device voltages of Comparative Examples 1 to 7 are relatively high. As shown in the table above, the driving voltages of Examples 1 to 43 are reduced by at least 0.45V compared to those of Comparative Examples 1 to 7. Therefore, an organic electroluminescent device with a lower operating voltage can be produced by using the nitrogen-containing compound of the present disclosure in the second hole transporting layer.

The nitrogen-containing compound of the present disclosure introduces an adamantane structure at the fluorene to enhance the electron density of the fluorene ring and the conjugate system of the entire compound through the hyperconjugation effect, which can enhance the hole conductivity and electron tolerance of the nitrogen-containing compound. In addition, the introduction of adamantyl can also increase the molecular weight of the nitrogen-containing compound and reduce the molecular symmetry, can increase the glass transition temperature and evaporation temperature of the compound of the present disclosure, control the crystallinity of the nitrogen-containing compound, makes the nitrogen-containing compound has higher physical and thermal stability when being mass-produced, which is convenient for mass production stability of organic electroluminescent devices and photoelectric conversion devices. Compared with Comparative Examples 1, 2 and 3, connecting amine to fluorene at positions 1 and 3 can increase the steric hindrance of the arylamine structure to a certain extent, and increase the twist angles of the fluorene plane and the arylamine plane (especially the triarylamine plane), which reduces the conjugation degree of the nitrogen-containing compound. When the nitrogen-containing compound is used as the second hole transporting layer (also known as the electron blocking layer), it may better match the HOMO energy level of the adjacent layer, thereby reducing the operating voltage of the organic electroluminescent device.

The organic electroluminescent devices manufactured in Example 1 were divided into two groups, and one group of devices was directly subjected to performance tests without heat treatment, and the test results are shown in Table 9. The other group was subjected to heat-treated (placed at 110°C for 1 hour) before performance tests. The test results for performance are shown in Table 10. Referring to the aforementioned method, the performance parameters without heat treatment and performance parameters after heat treatment were also obtained for the organic electroluminescent devices manufactured in Example 14, Example 21, Example 32, Example 35, Example 38, Example 41, and Comparative Examples 1 to 7.

**Table 9 Performance parameters of organic electroluminescent devices without heat treatment**

| Example | Compound | Operating voltage (V) | Current efficiency (Cd/A) | External quantum efficiency EQE (%) | Color coordinate CIEx,CIEy | T95 lifetime (hr) |
|---|---|---|---|---|---|---|
| Example 1 | Compound 1 | 4.11 | 86.92 | 32.7 | 0.266,0.700 | 160 |
| Example 14 | Compound 92 | 4.18 | 87.04 | 32.9 | 0.266,0.700 | 163 |
| Example 21 | Compound 162 | 4.17 | 87.01 | 33.1 | 0.266.0.700 | 161 |
| Example 31 | Compound 439 | 4.15 | 84.90 | 32.8 | 0.263,0.703 | 155 |
| Example 35 | Compound 508 | 4.18 | 86.32 | 31.5 | 0.266,0.701 | 160 |
| Example 38 | Compound 670 | 4.10 | 83.62 | 30.2 | 0.262,0.702 | 157 |
| Example 41 | Compound 817 | 4.11 | 85.74 | 30.9 | 0.262,0.702 | 160 |
| Comparative Example 1 | Compound A | 4.67 | 86.89 | 25.8 | 0.262,0.702 | 149 |
| Comparative Example 2 | Compound B | 4.73 | 85.04 | 24.2 | 0.262,0.702 | 145 |
| Comparative Example 3 | Compound C | 4.62 | 85.54 | 24.4 | 0.263,0.703 | 155 |
| Comparative Example 4 | Compound D | 4.66 | 82.69 | 24.5 | 0.263,0.703 | 154 |
| Comparative Example 5 | Compound E | 4.65 | 85.04 | 24.2 | 0.262,0.702 | 140 |
| Comparative Example 6 | Compound F | 4.68 | 85.64 | 24.4 | 0.263,0.703 | 143 |
| Comparative Example 7 | Compound G | 4.72 | 84.54 | 24.4 | 0.263,0.703 | 150 |

**Table 10 Performance parameters of organic electroluminescent devices after heat treatment**

| Example | Compound | Operating voltage (V) | Current efficiency (Cd/A) | External quantum efficiency EQE(%) | Color coordinate CIEx,CIEy | T95 lifetime (h) |
|---|---|---|---|---|---|---|
| Example 1 | Compound 1 | 4.05 | 83.25 | 32.7 | 0.263,0.703 | 160 |
| Example 14 | Compound 92 | 4.07 | 86.84 | 32.9 | 0.266,0.700 | 154 |
| Example 21 | Compound 162 | 4.13 | 86.72 | 33.1 | 0.266.0.700 | 167 |
| Example 31 | Compound 439 | 4.08 | 87.01 | 31.5 | 0.263,0.703 | 164 |
| Example 35 | Compound 508 | 4.10 | 86.32 | 31.5 | 0.266,0.701 | 160 |
| Example 38 | Compound 670 | 4.06 | 84.62 | 30.2 | 0.262,0.702 | 157 |
| Example 41 | Compound 817 | 4.03 | 85.94 | 30.9 | 0.262,0.702 | 160 |
| Comparative Example 1 | Compound A | 4.66 | 66.89 | 16.7 | 0.262,0.702 | 134 |
| Comparative Example 2 | Compound B | 4.75 | 65.04 | 16.0 | 0.262,0.702 | 130 |
| Comparative Example 3 | Compound C | 4.65 | 80.54 | 20.4 | 0.263,0.703 | 145 |
| Comparative Example 4 | Compound D | 4.68 | 62.69 | 16.1 | 0.263,0.703 | 134 |
| Comparative Example 5 | Compound E | 4.67 | 65.04 | 16.8 | 0.262,0.702 | 127 |
| Comparative Example 6 | Compound F | 4.68 | 62.64 | 16.0 | 0.263,0.703 | 123 |
| Comparative Example 7 | Compound G | 4.73 | 64.54 | 15.6 | 0.263,0.703 | 125 |

From the results in Table 9 and Table 10, it can be seen that for the organic electroluminescent devices of Comparative Examples 1, Comparative Examples 2, and Comparative Examples 4 to 7, compared with those without heat treatment, the luminous efficiency and external quantum efficiency are reduced by more than 23% after heat treatment, and the lifetimes are reduced by more than 10% after heat treatment. Compared with that without heat treatment, the efficiency and lifetime of the organic electroluminescent device of Comparative Example 3 after heat treatment are reduced by 5.8%, and the lifetime is reduced by 6.5%. However, for the organic electroluminescent devices of Example 1, Example 14, Example 21, Example 35, Example 38 and Example 41 after heat treatment, they maintained the comparable efficiency and lifetime as those without heat treatment.

From the device results in Table 8 to Table 10, it can be seen that the voltages of the devices using the compounds of the present disclosure are reduced, comparing those of the devices using the the compounds of Comparative Example 1 and Comparative Example 3. The reason may be that the compounds of the present disclosure inwhich the aromatic amine is connected at the 1st or 3rd positions of adamantane fluorene have a deeper HOMO than the compounds inwhich the aromatic amine is connected at the 2nd position of adamantane fluorene, so as to make the injection of holes into the light-emitting layer smoother. Compared with the compounds of Comparative Example 5, Comparative Example 6 and Comparative Example 7, the application of the compounds of the present disclosure can reduce the voltage and improve the thermal stability for the device. The reason may be that the cycloalkyl group formed on the 9,9-dimethylfluorene of the present disclosure is the rigid and large-volume adamantyl group, which has a stronger ability to reduce the molecular stacking, compared with the single ring structure. The material can achieve a more stable film state.

It can be seen that, according to Table 8 to Table 10 for the results of the organic electroluminescent device of the examples, when the arylamine compound with adamantane-fluorene as the core is used as the second hole transporting layer material of the green light device, it can produce organic electroluminescent devices with excellent characteristics in terms of driving voltage, luminous efficiency, external quantum efficiency and thermal stability, etc. For exmple, it can produce a organic electroluminescent device with high-efficiency, high heat-resistance and long lifetime.

## Claims

1. A nitrogen-containing compound having a structure shown in Chemical Formula 1: 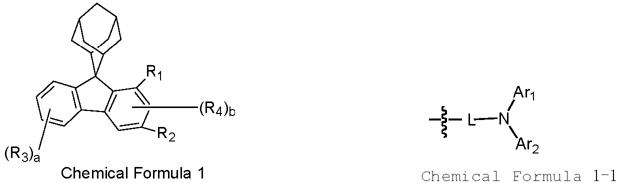
wherein 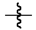 represents a chemical bond;
R₁ and R₂ are each independently selected from hydrogen or a group represented by Chemical Formula 1-1, and one and only one of R₁ and R₂ has the group represented by Chemical Formula 1-1; when R₁ or R₂ is selected from hydrogen, said R₁ and R₂ may be replaced by R₄;
R₃, R₄ are each independently selected from the group consisting of deuterium and phenyl;
a is selected from 0, 1, 2, 3, or 4; when a is greater than or equal to 2, any two R₃ are the same or different;
b is selected from 0, 1, 2, or 3, when b is greater than or equal to 2, any two R₄ are the same or different;
L is selected from the group consisting of single bond and the following groups: 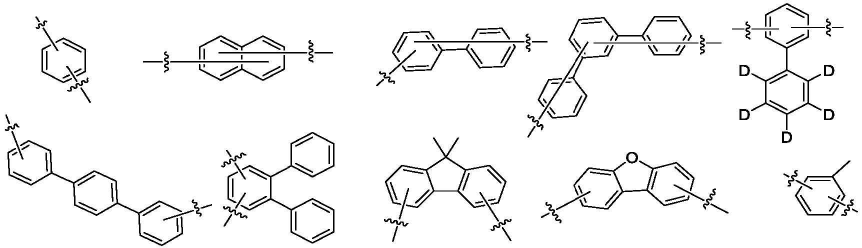 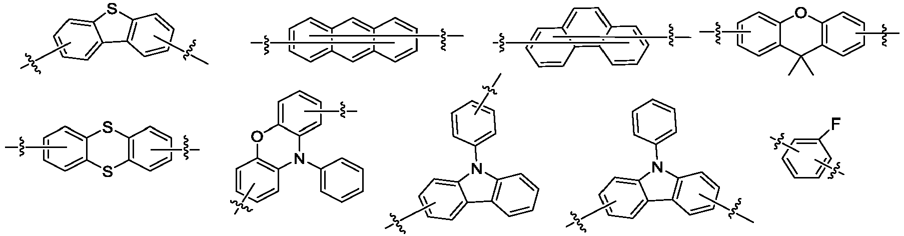 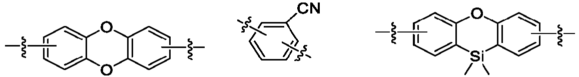 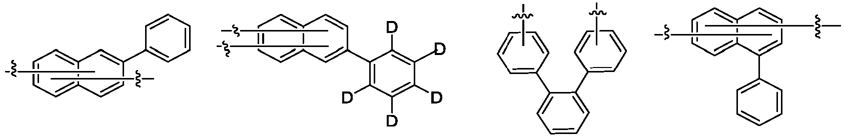 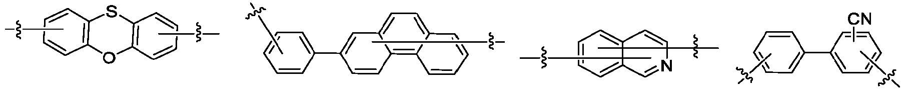 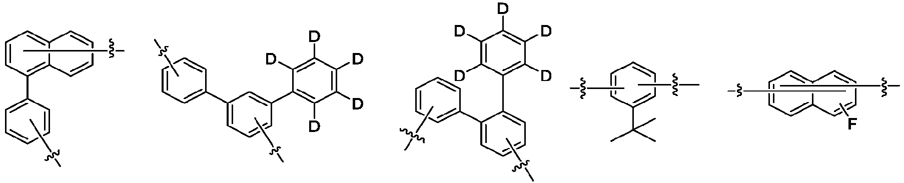 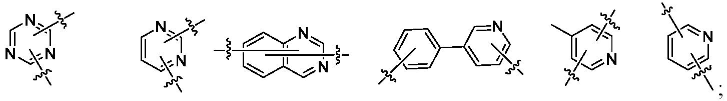 and
Ar₁ and Ar₂ are each independently selected from the group consisting of the following groups:

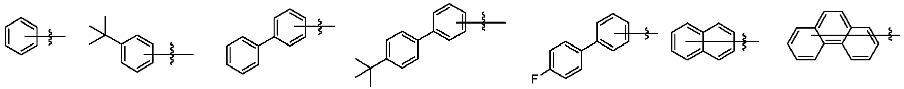

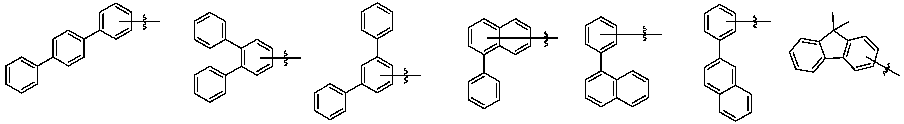

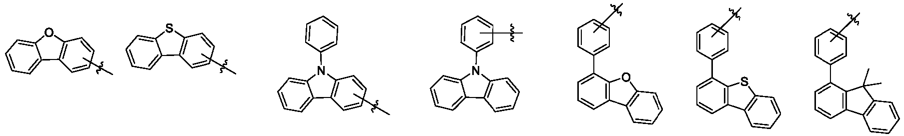

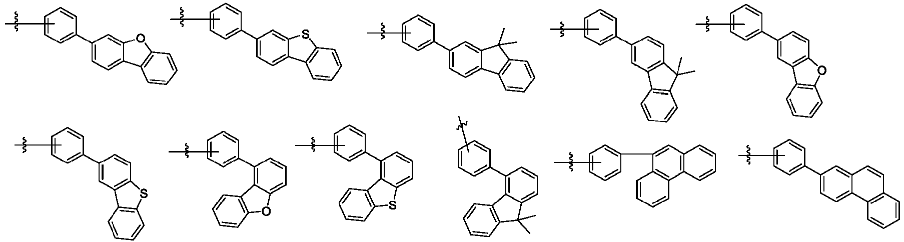

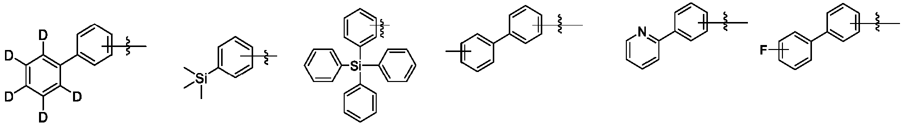

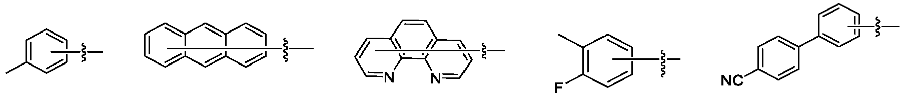

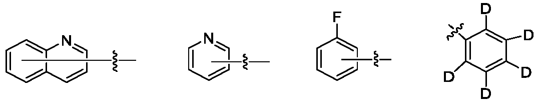

2. The nitrogen-containing compound of claim 1, wherein the nitrogen-containing compound is selected from the group consisting of the following compounds:

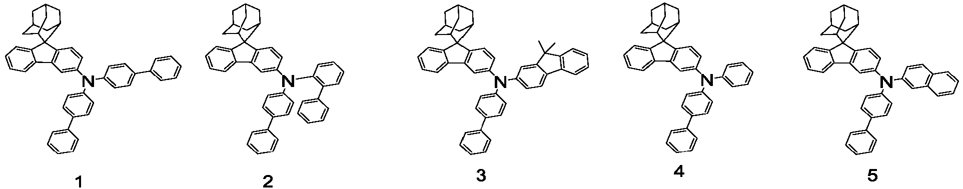

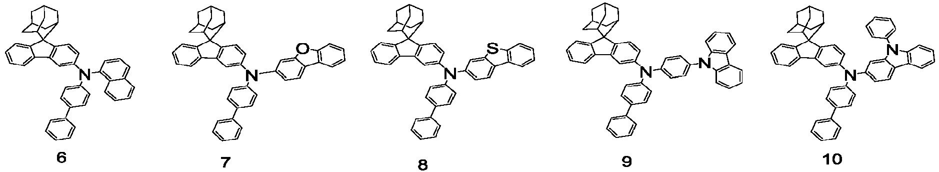

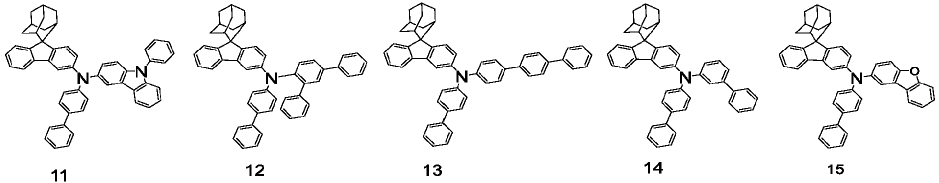

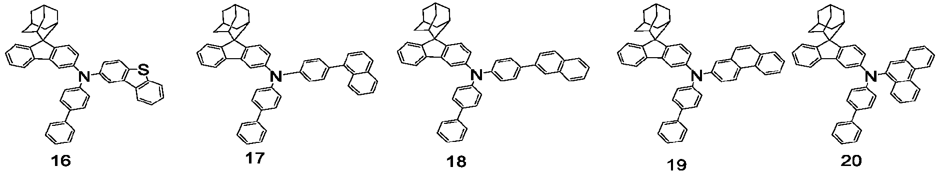

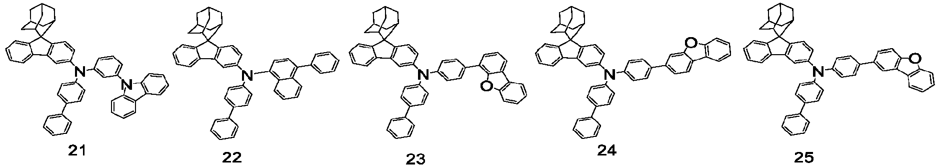

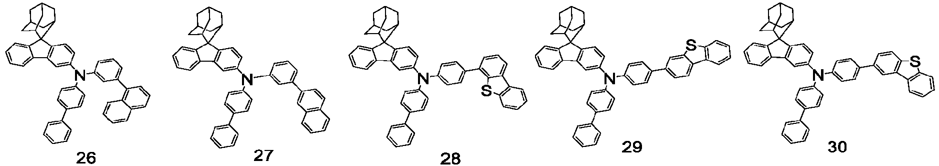

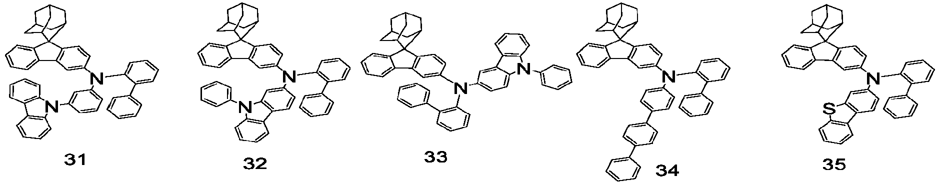

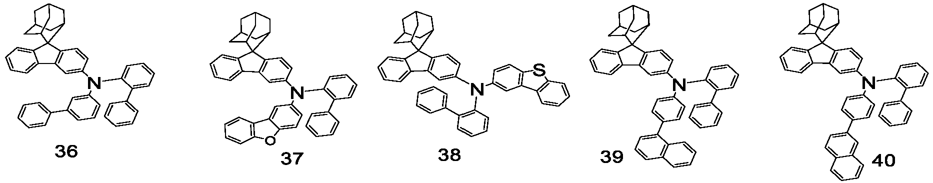

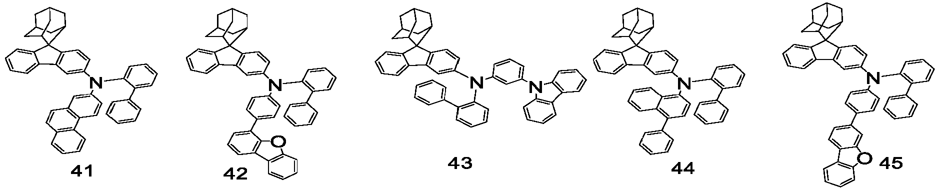

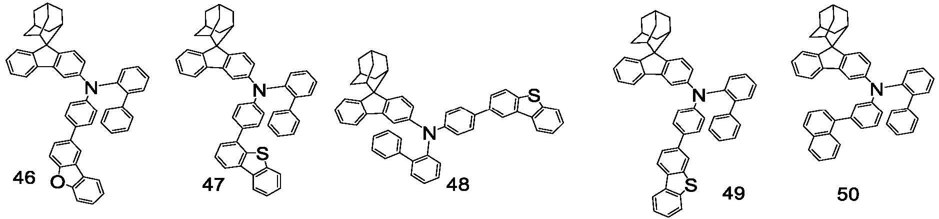

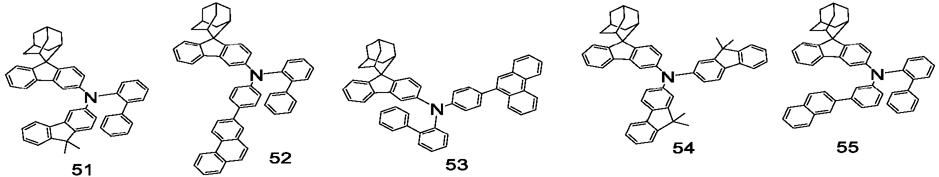

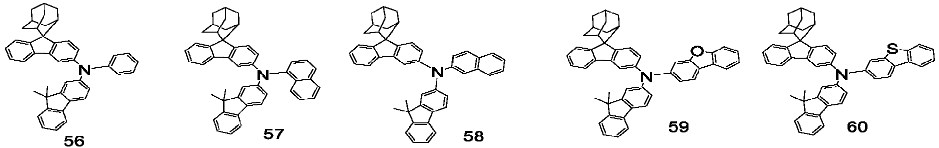

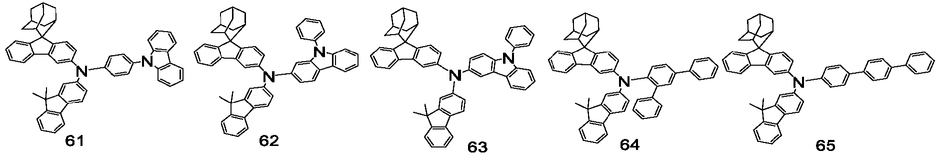

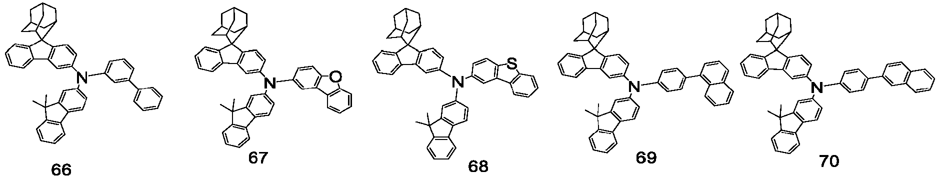

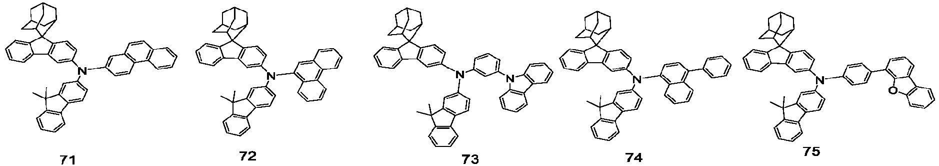

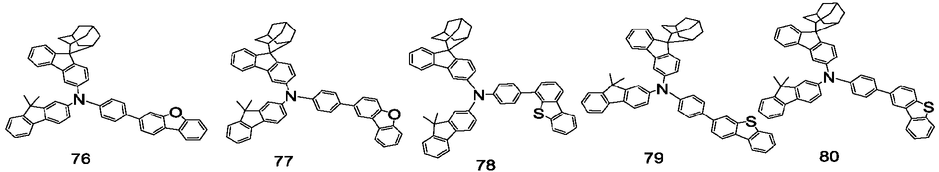

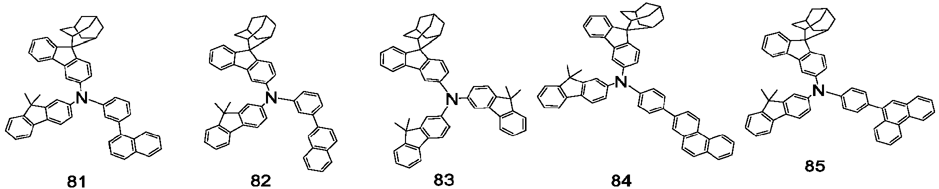

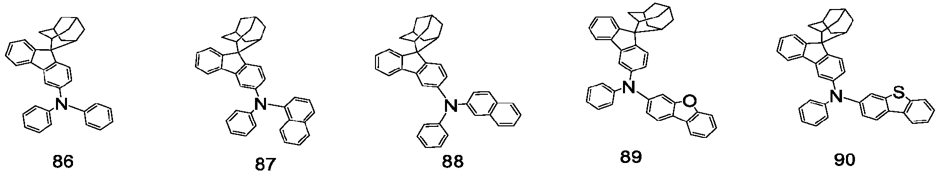

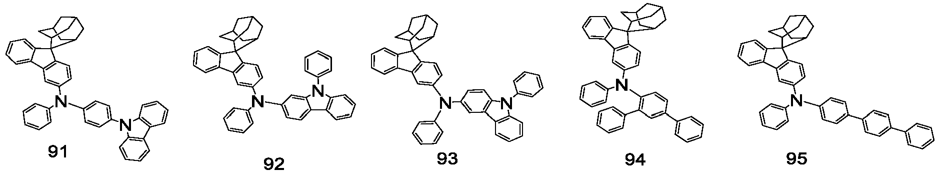

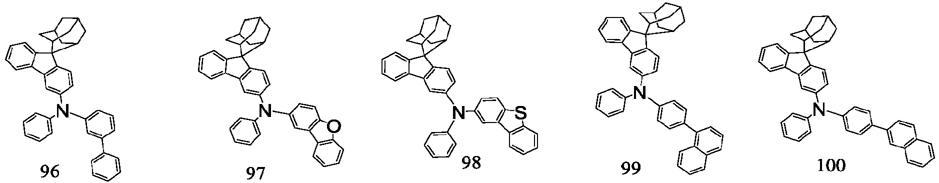

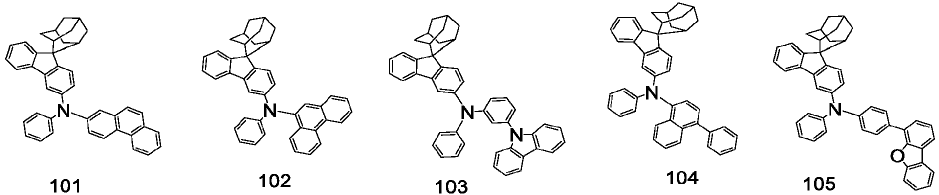

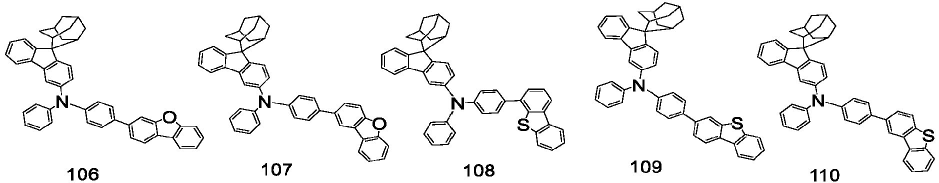

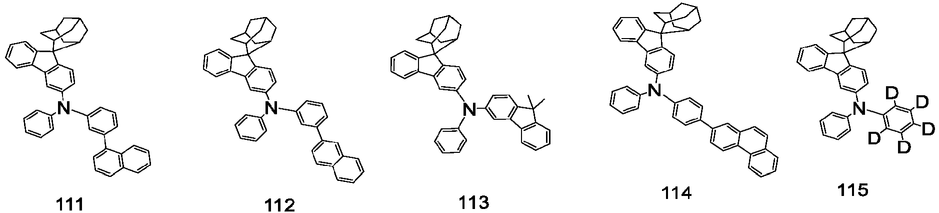

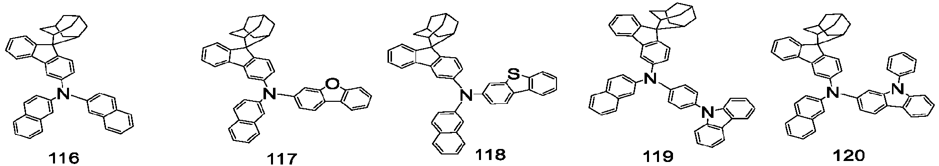

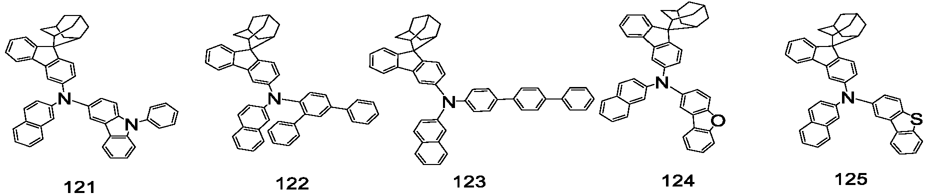

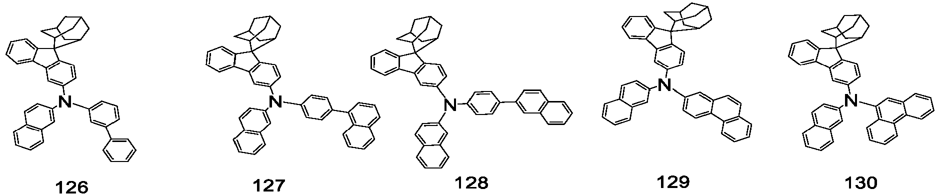

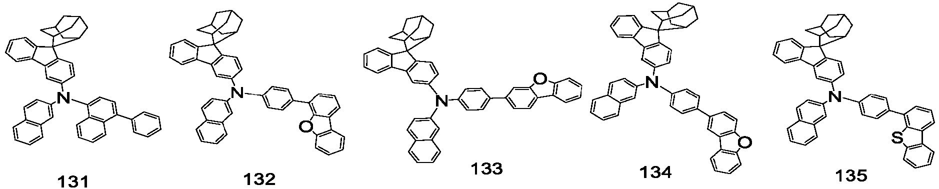

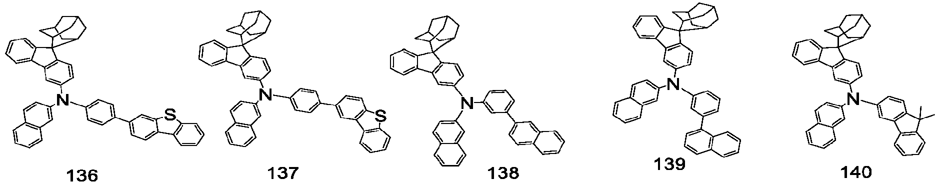

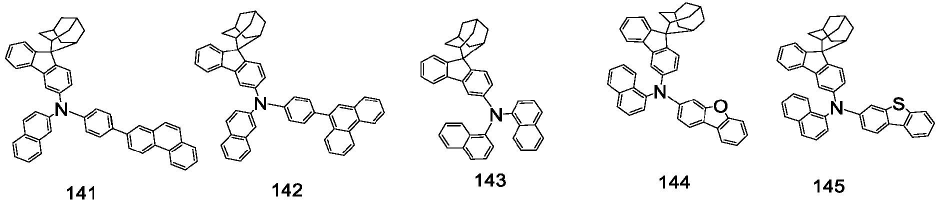

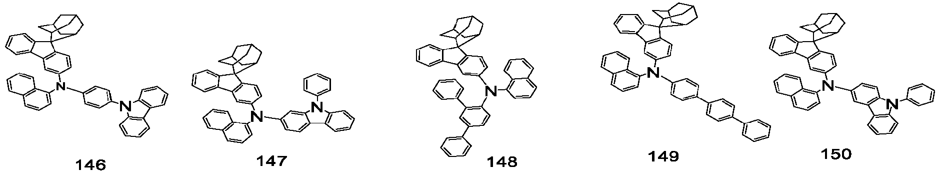

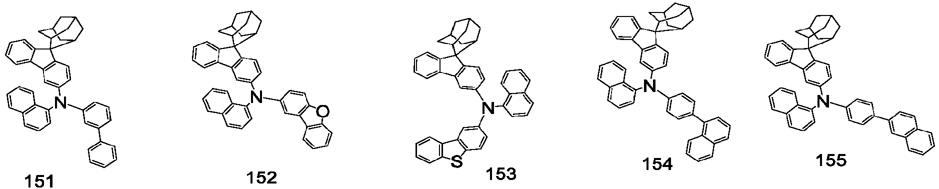

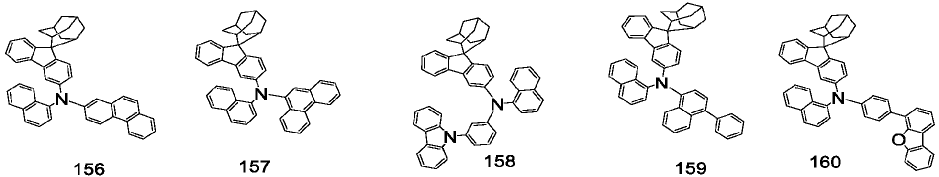

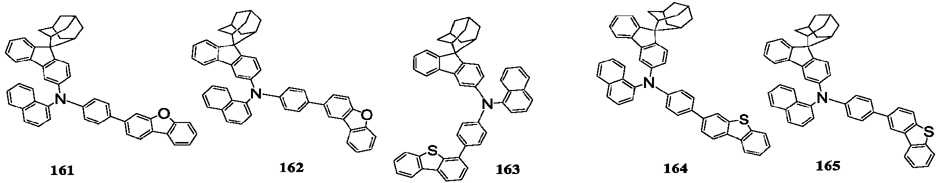

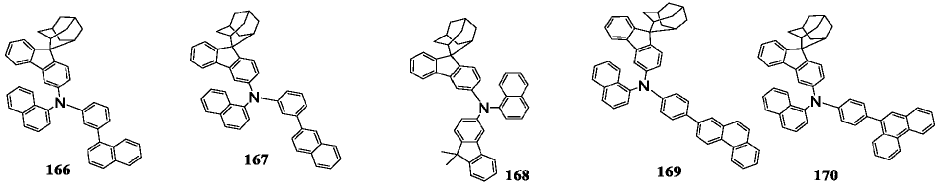

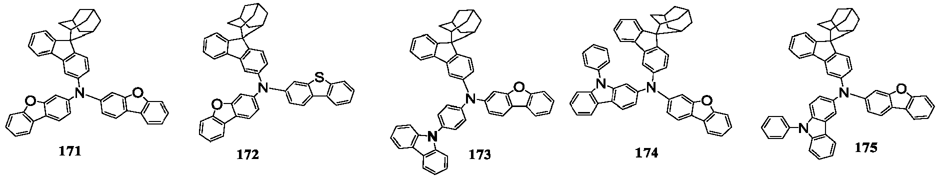

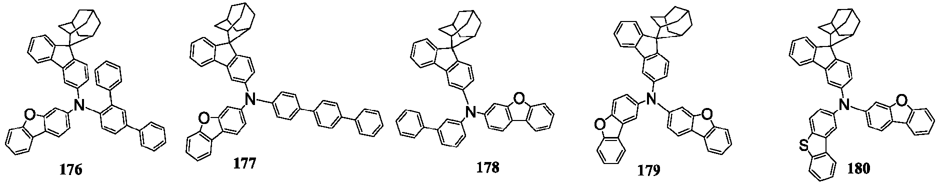

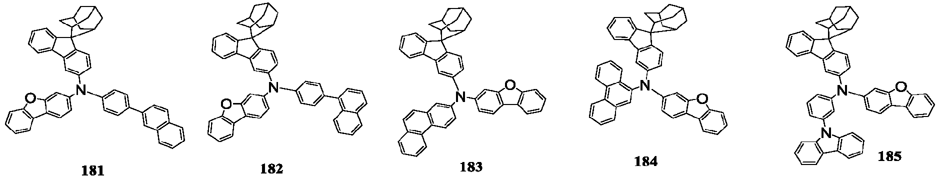

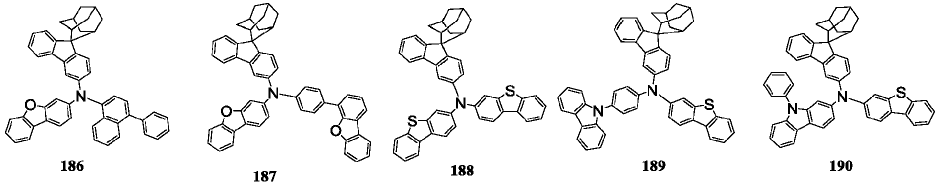

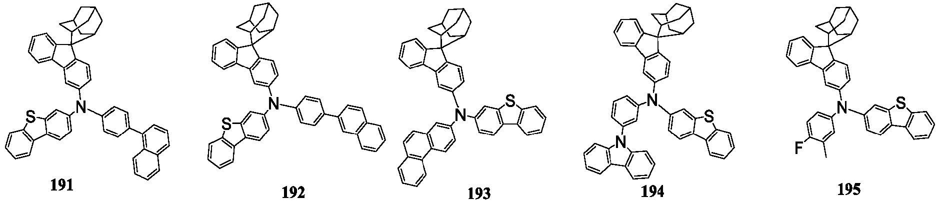

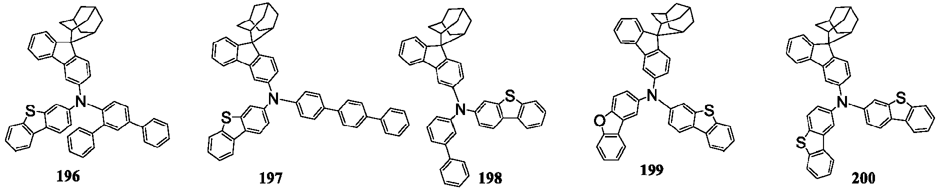

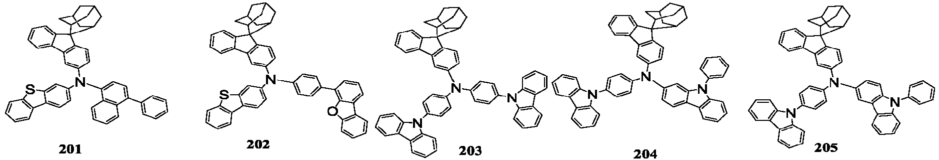

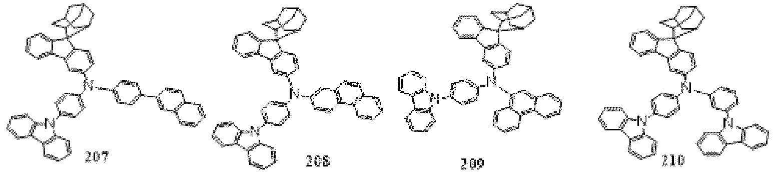

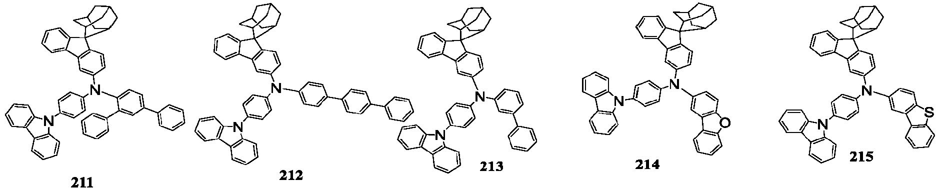

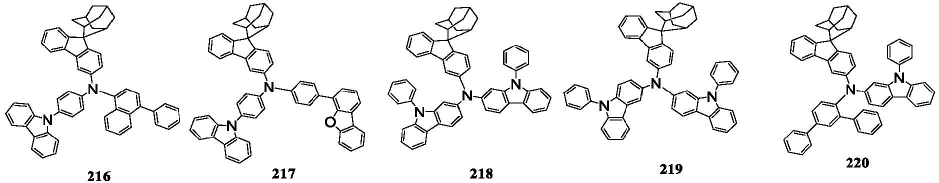

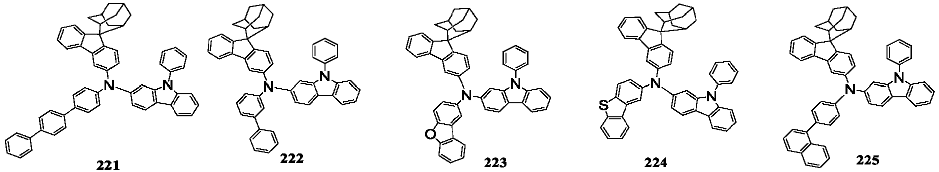

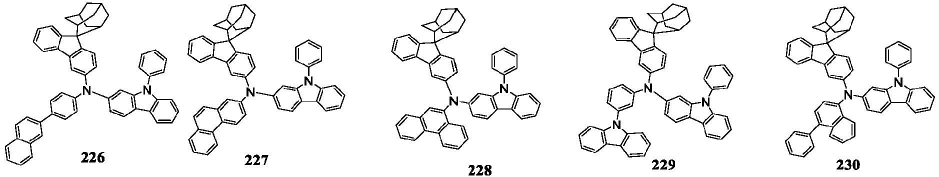

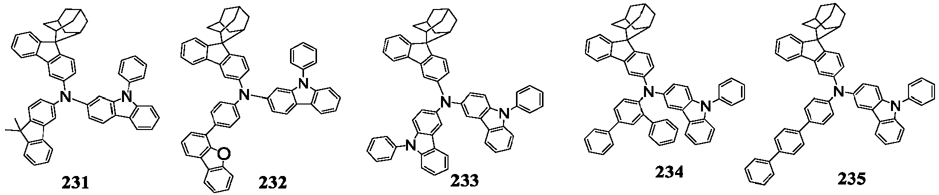

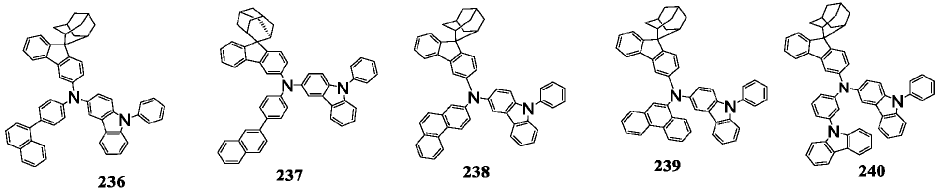

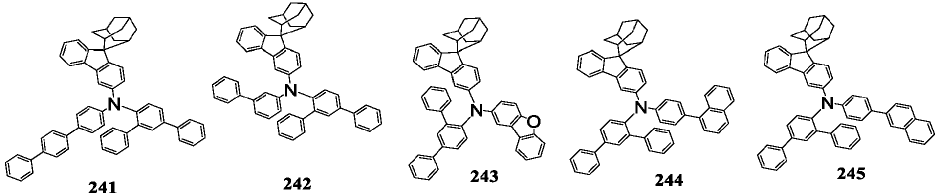

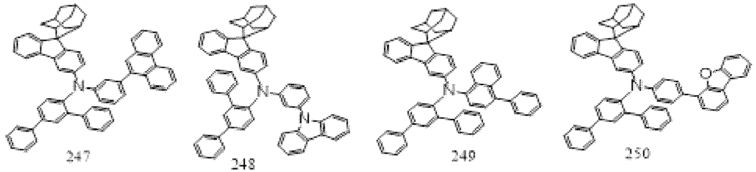

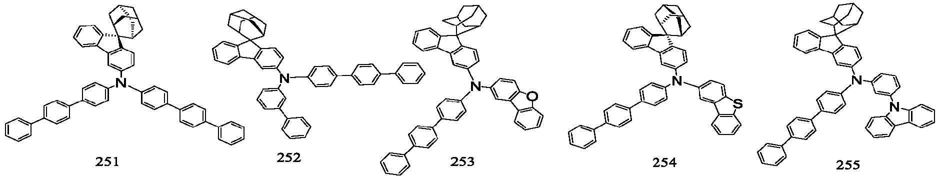

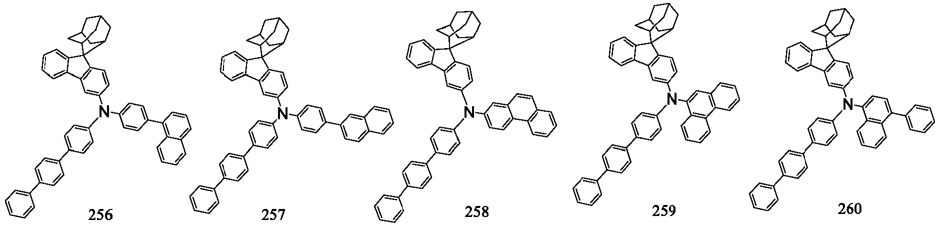

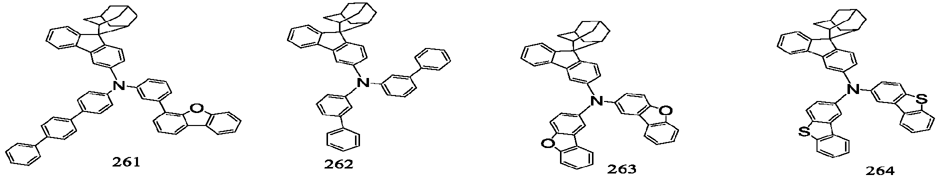

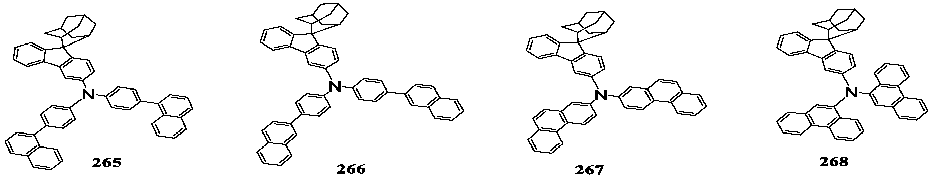

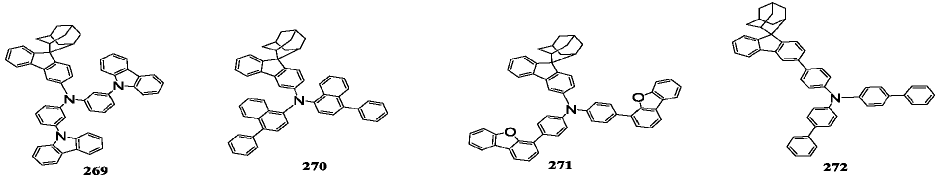

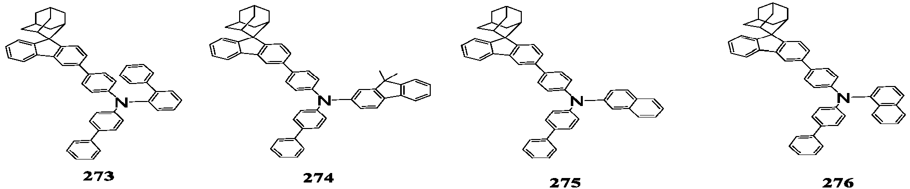

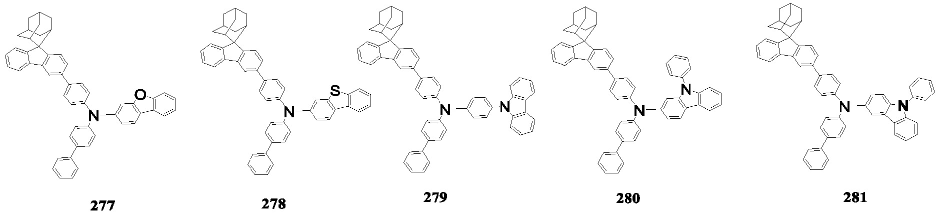

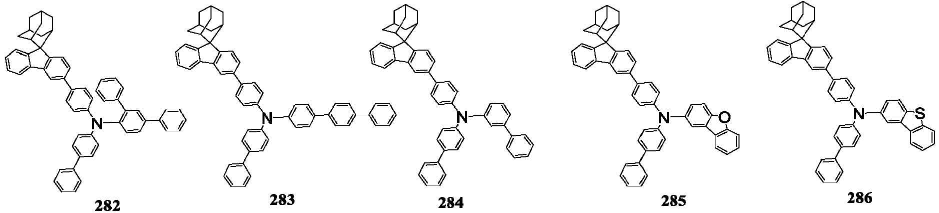

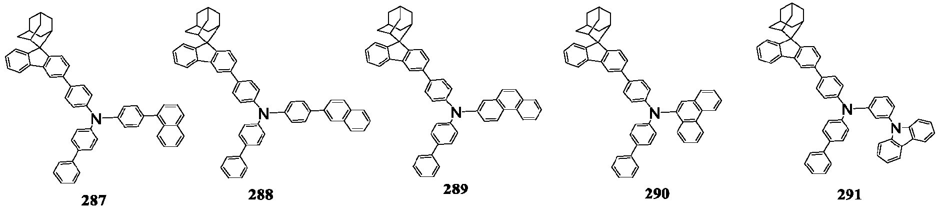

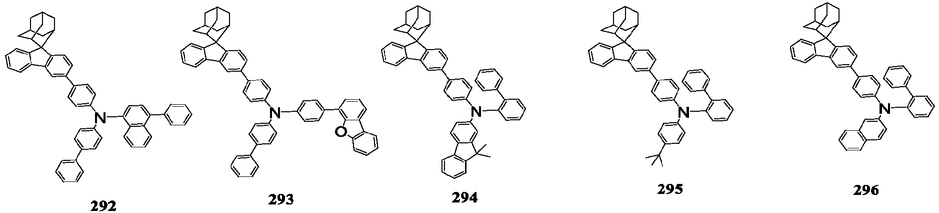

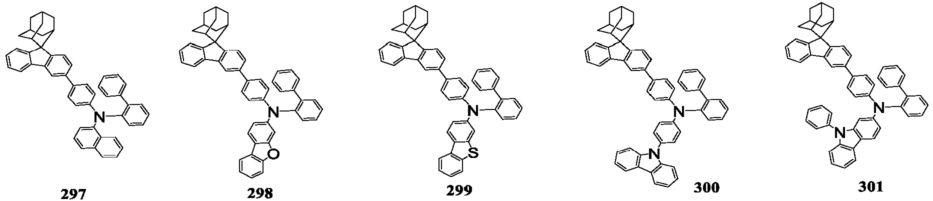

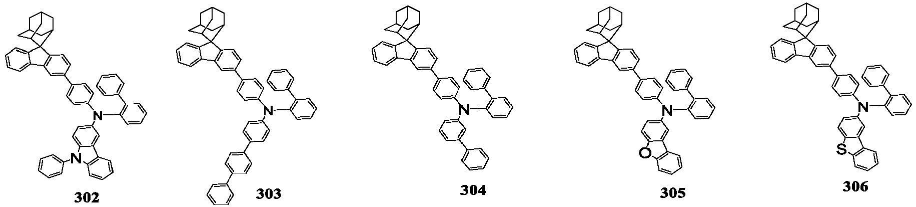

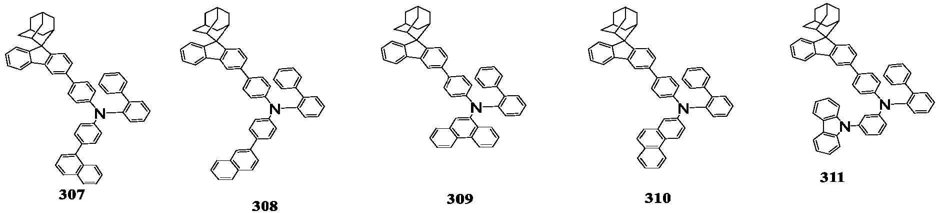

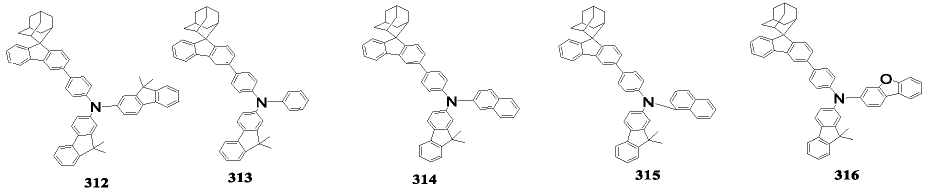

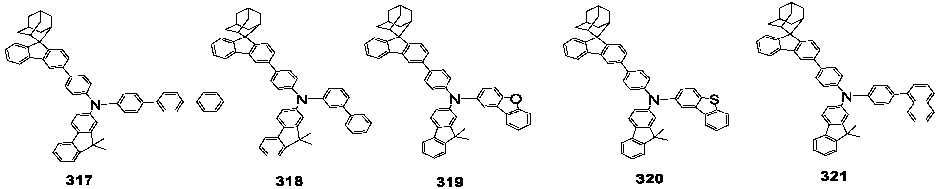

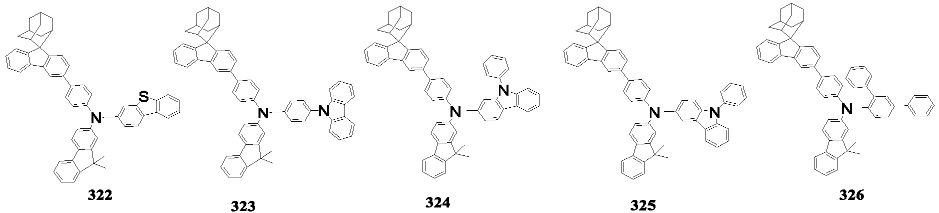

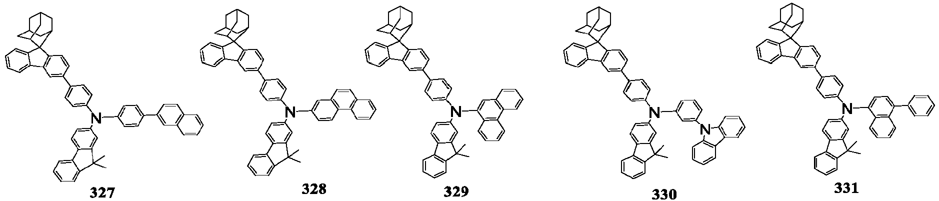

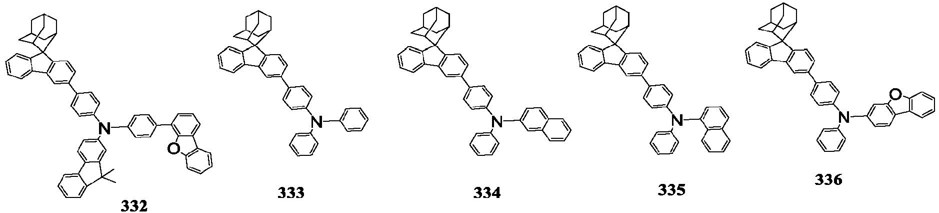

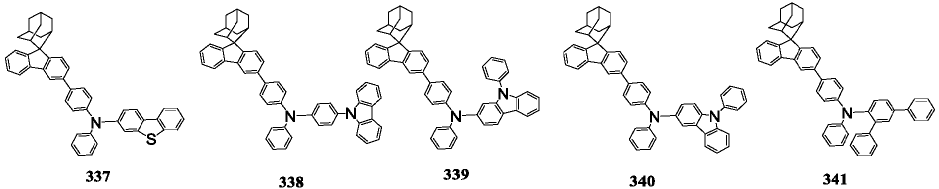

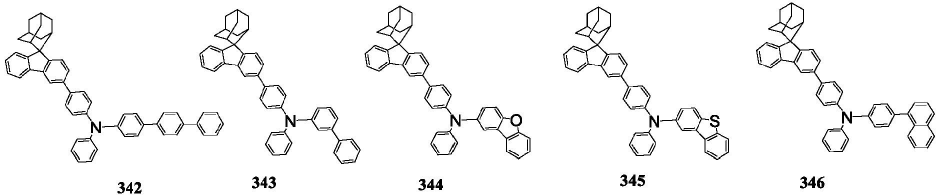

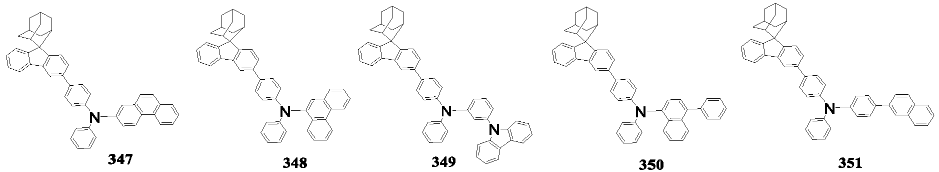

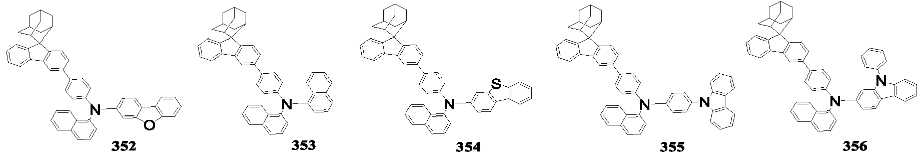

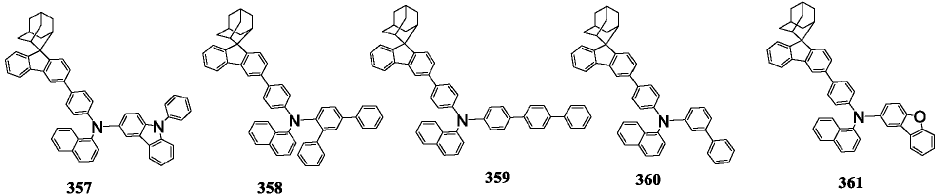

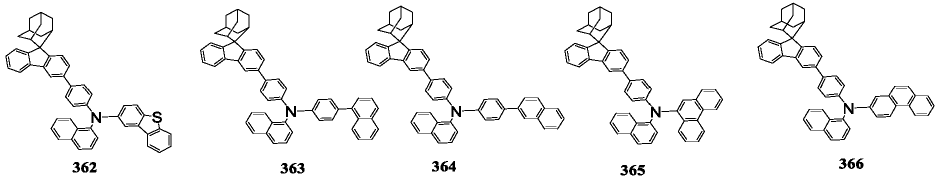

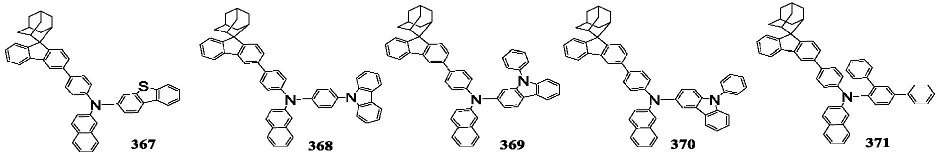

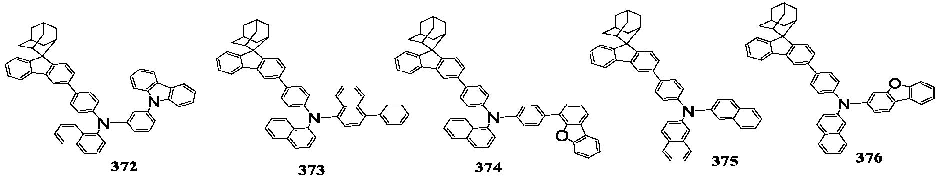

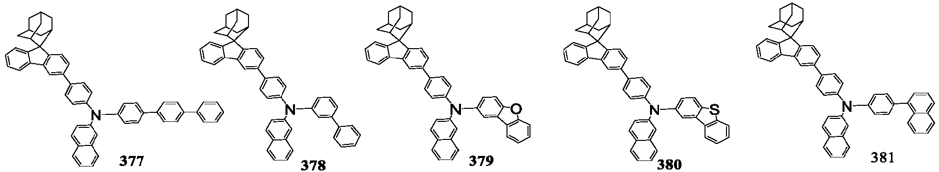

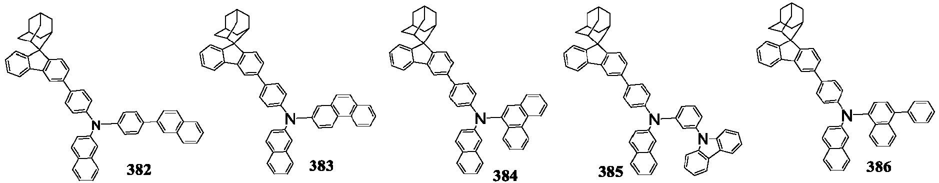

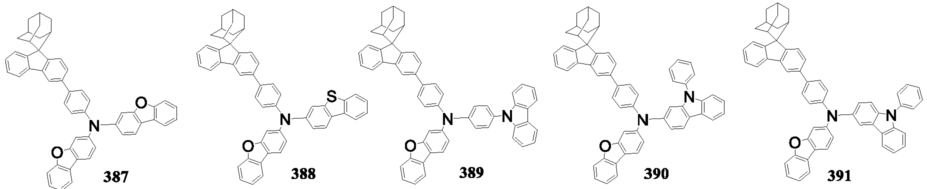

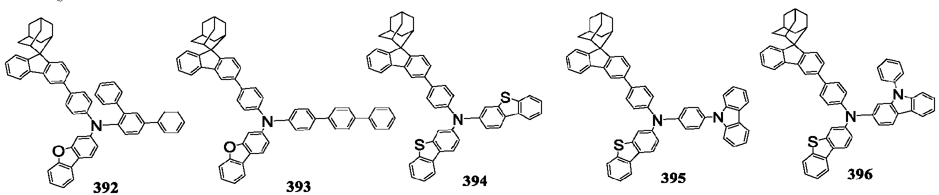

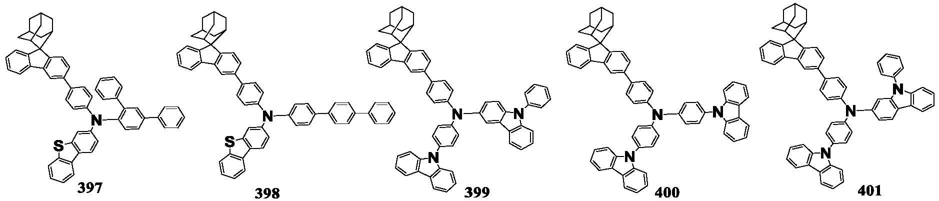

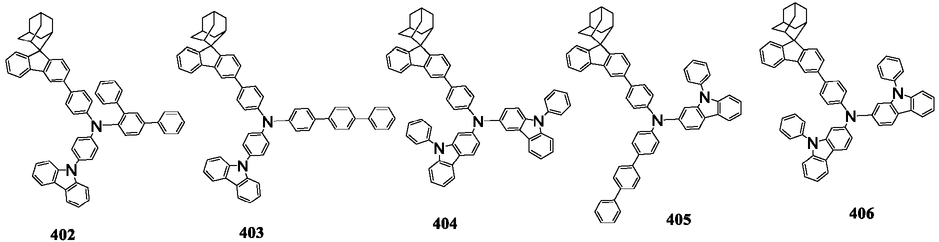

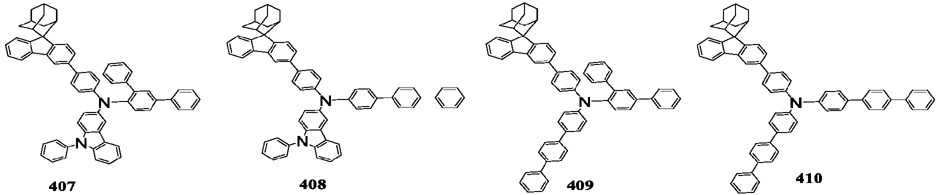

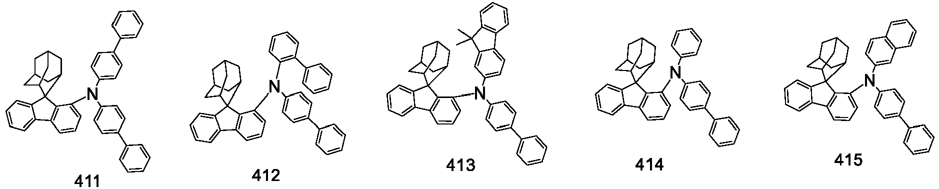

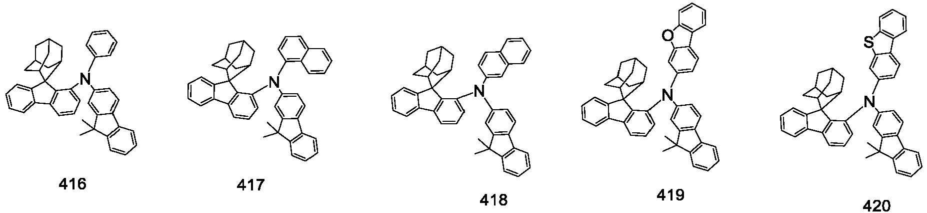

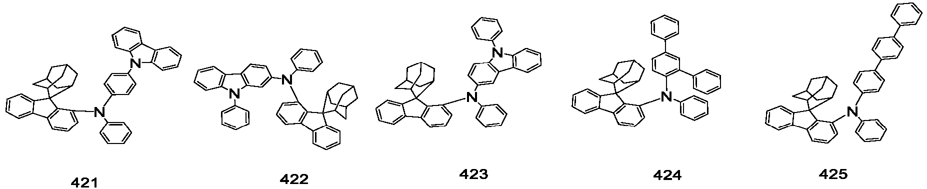

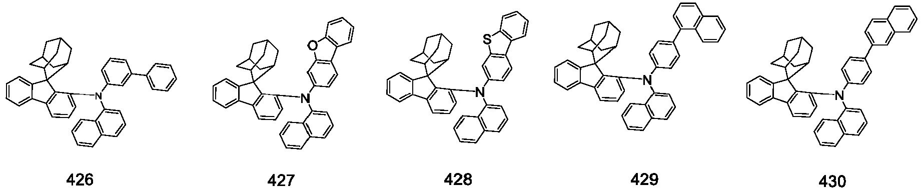

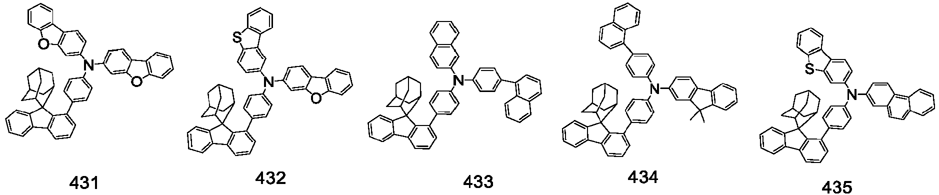

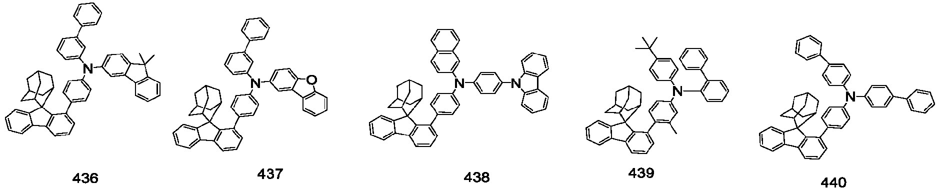

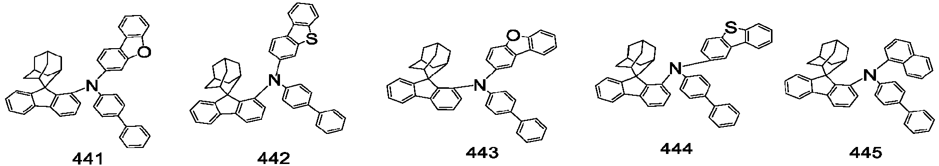

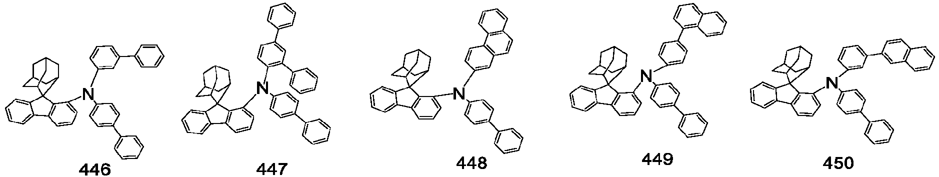

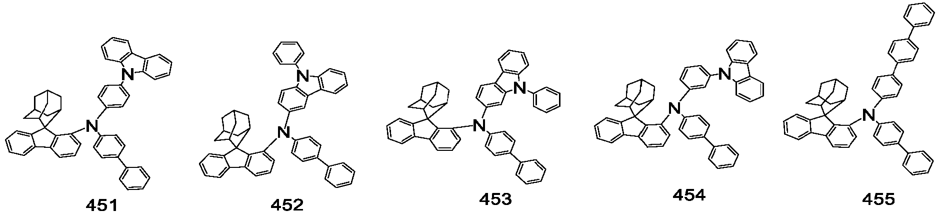

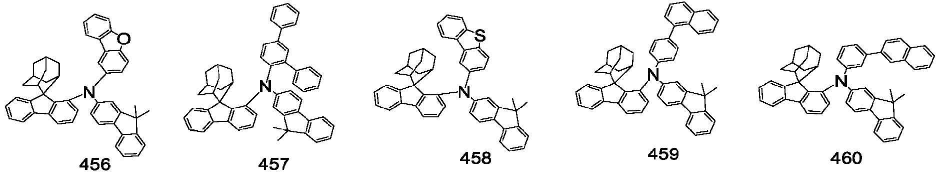

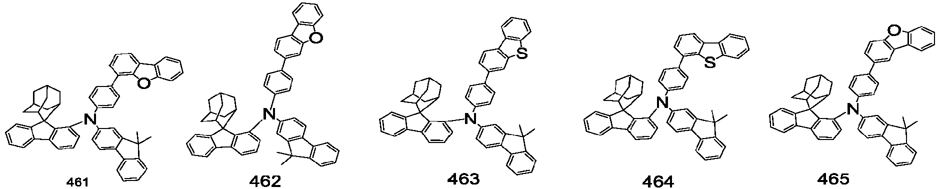

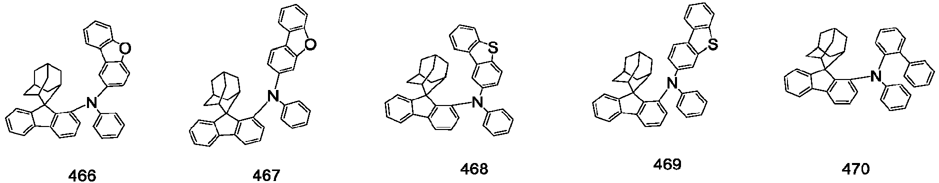

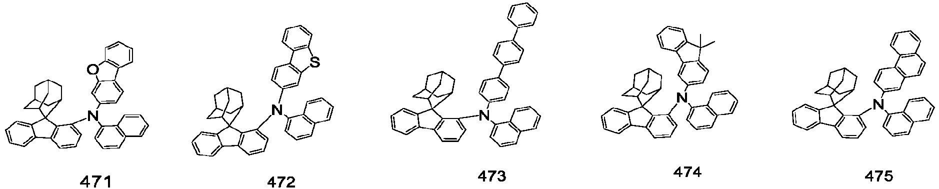

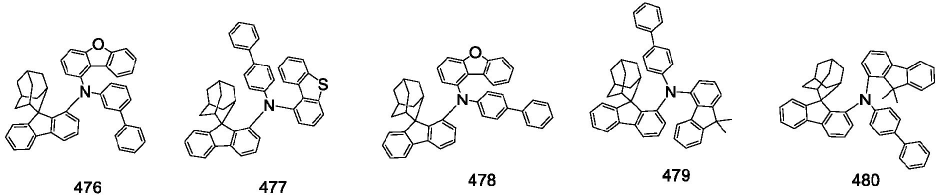

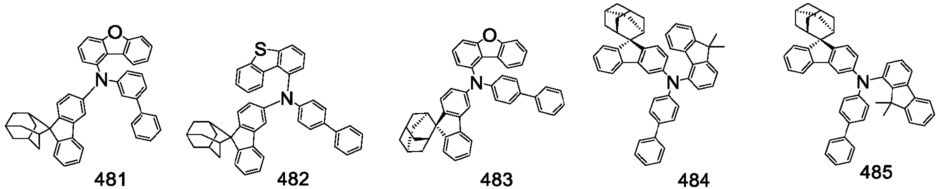

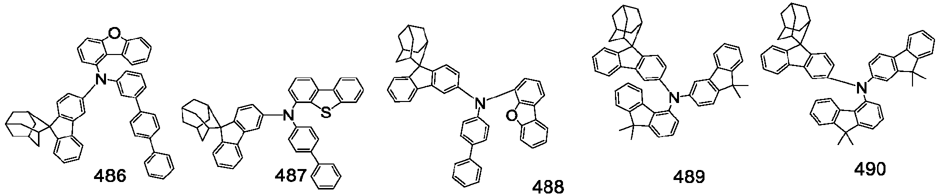

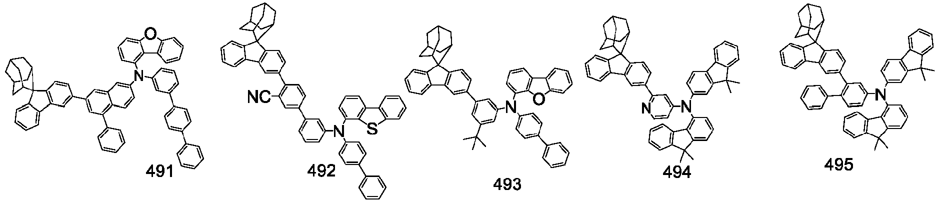

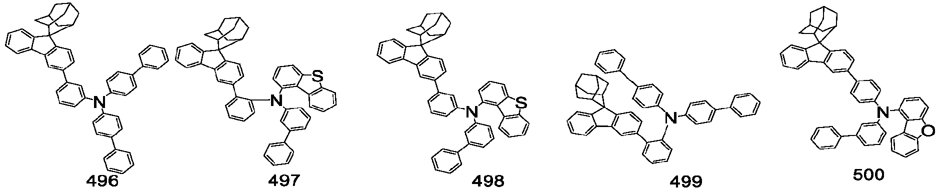

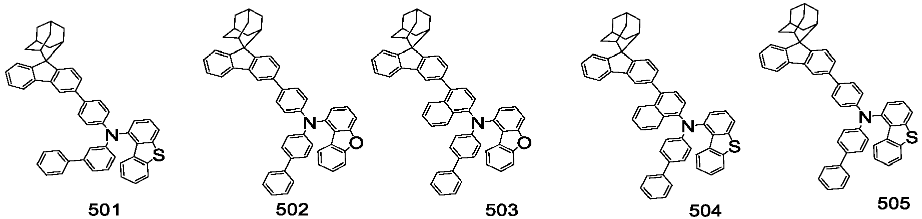

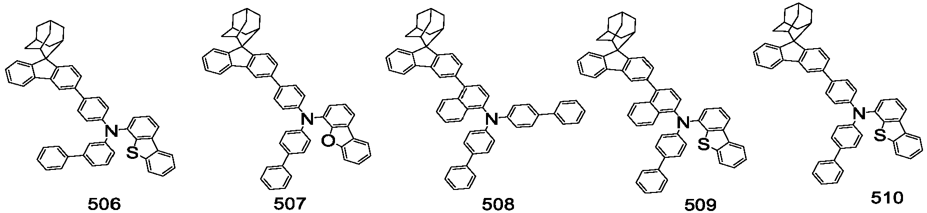

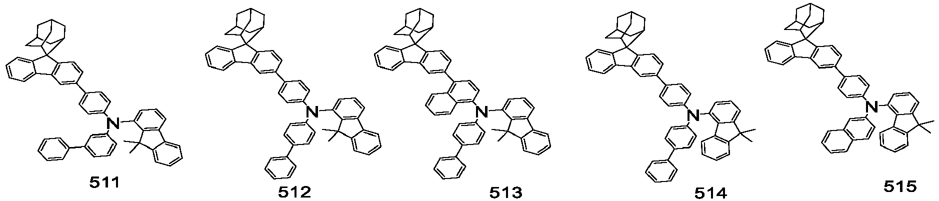

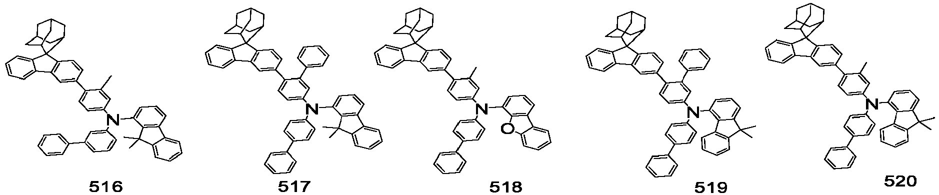

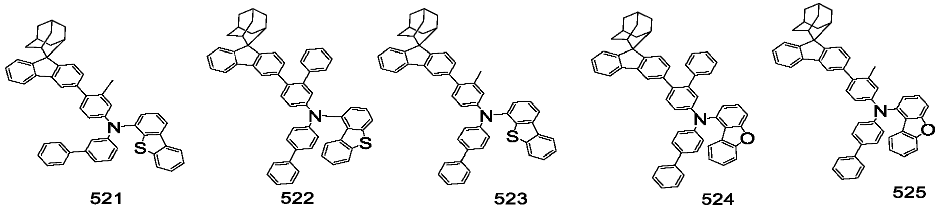

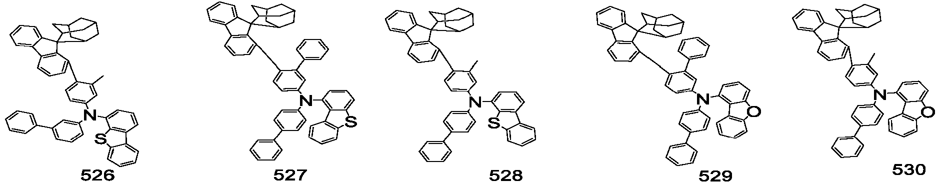

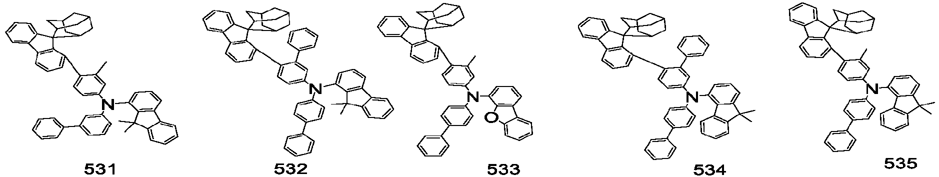

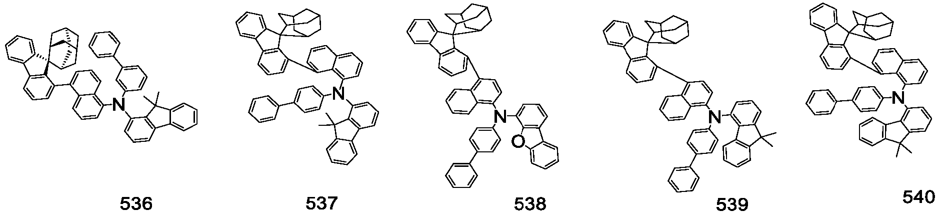

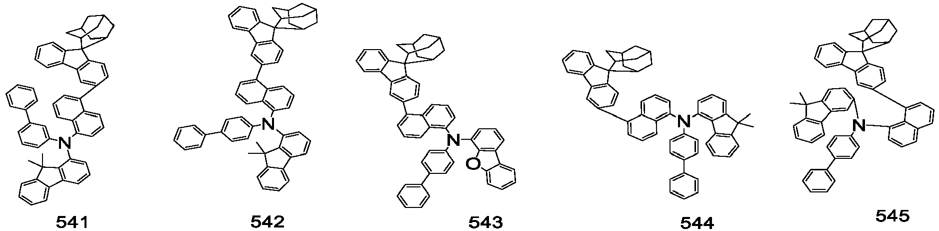

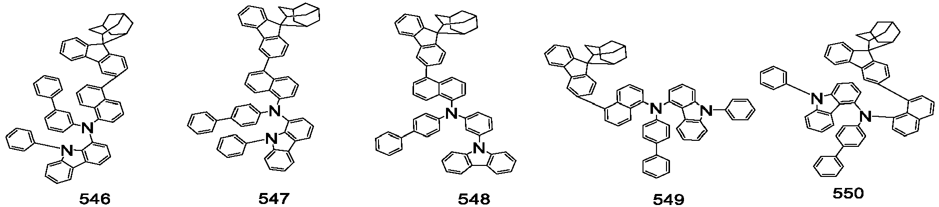

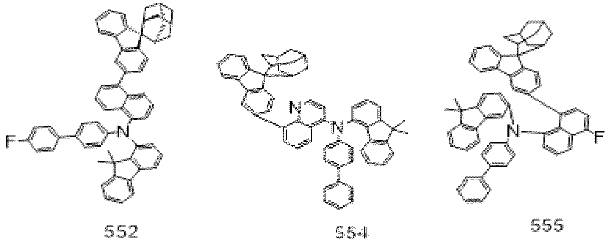

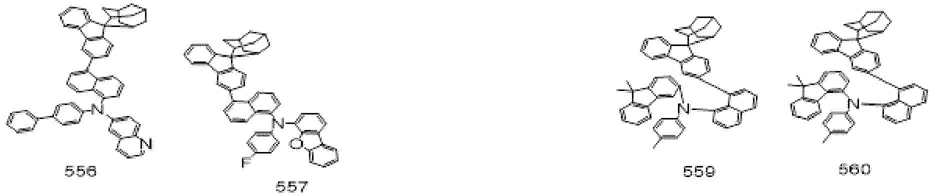

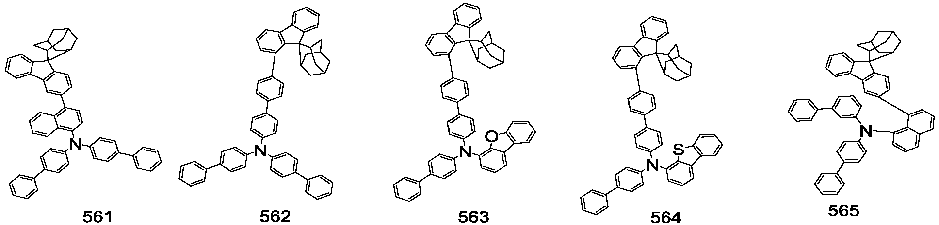

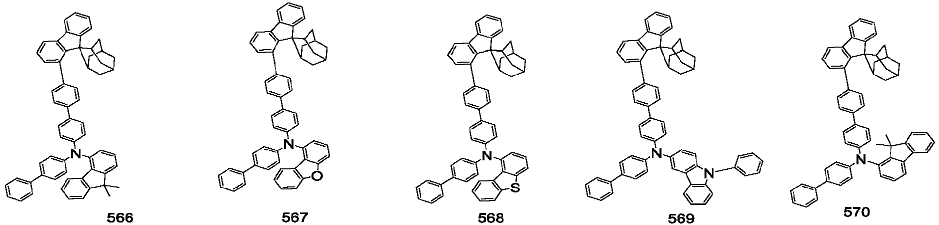

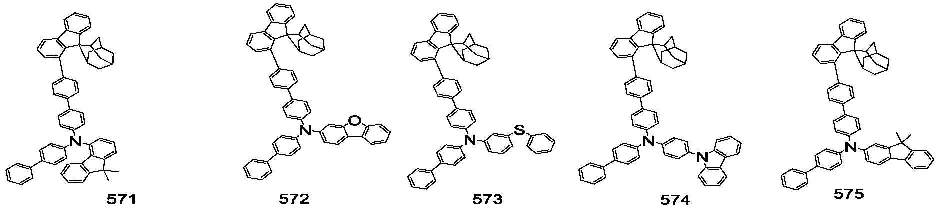

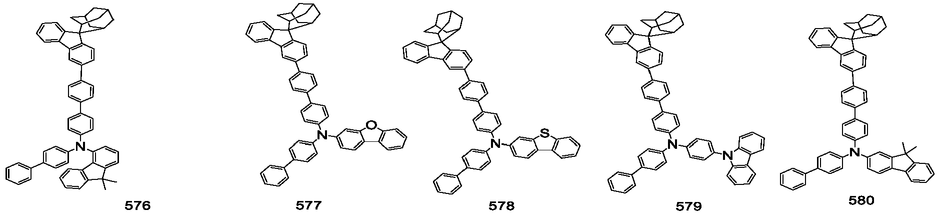

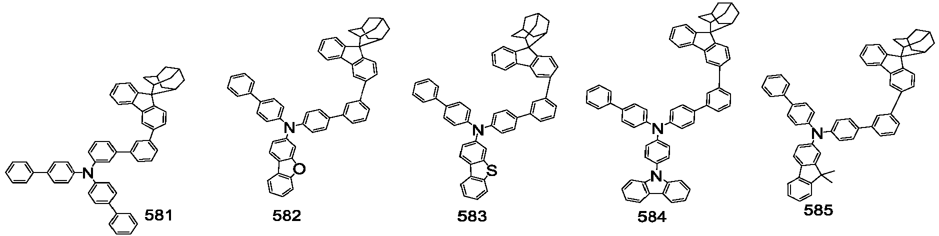

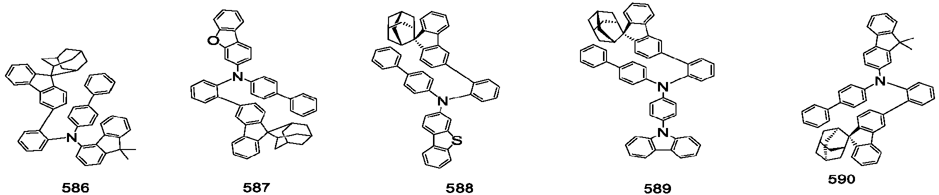

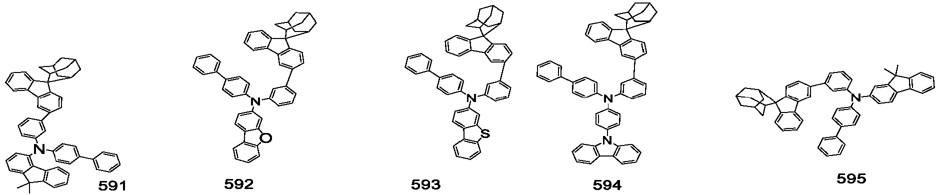

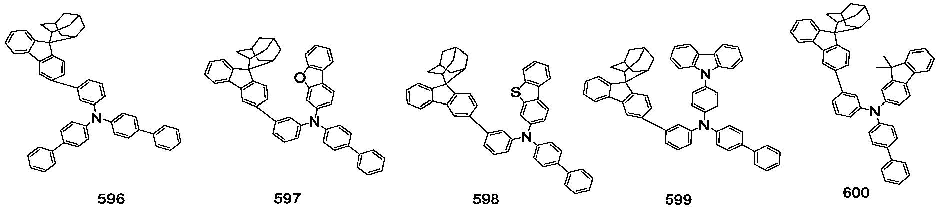

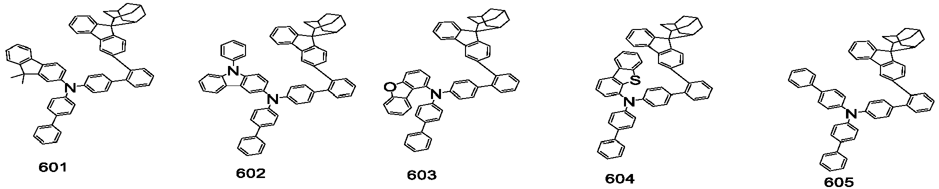

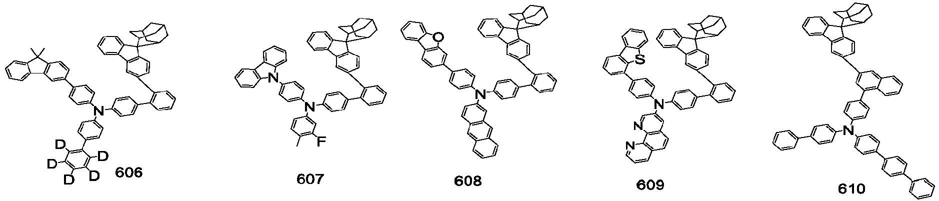

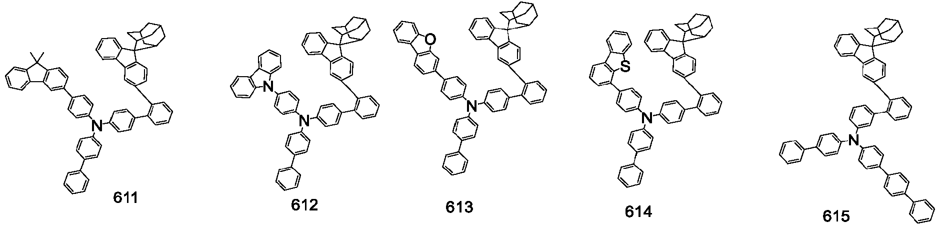

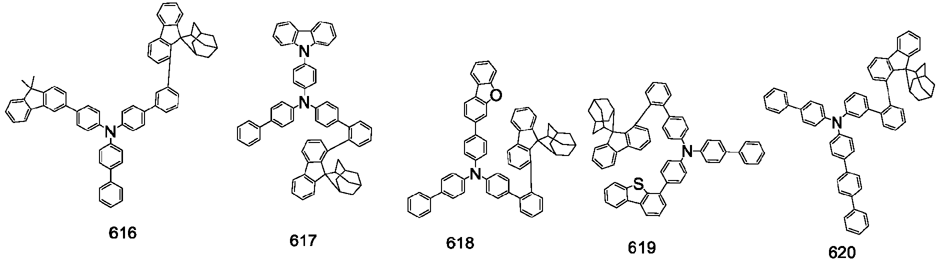

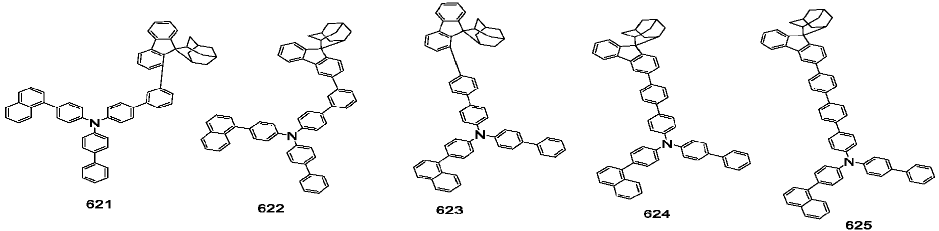

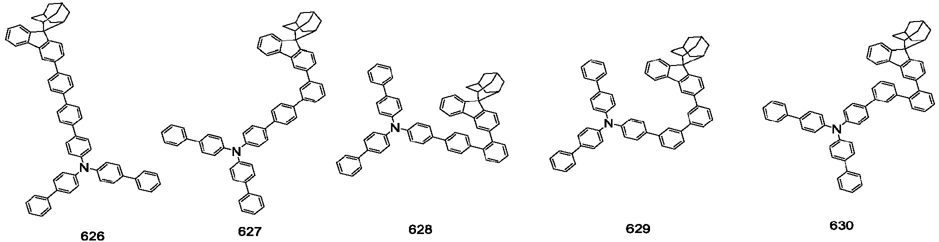

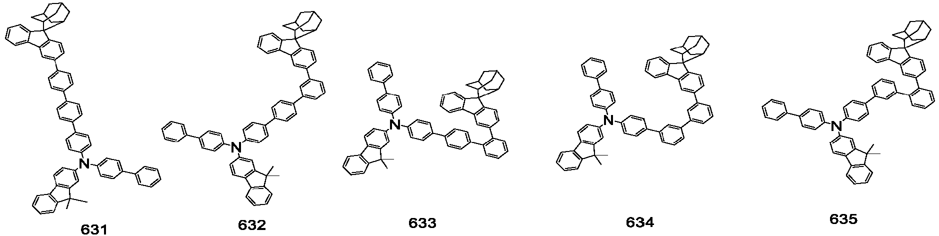

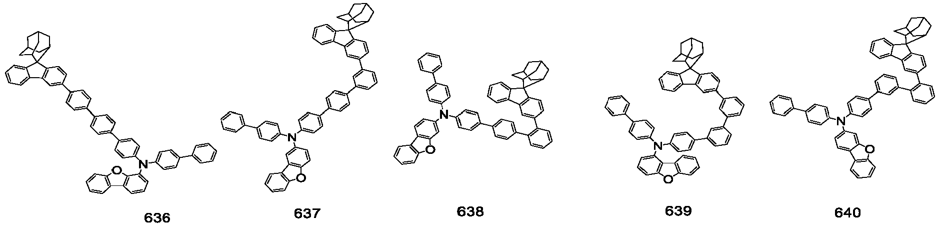

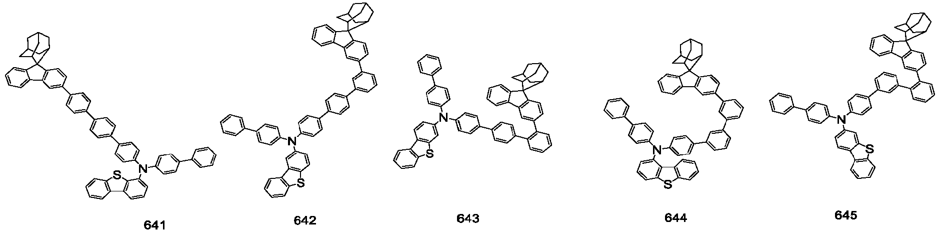

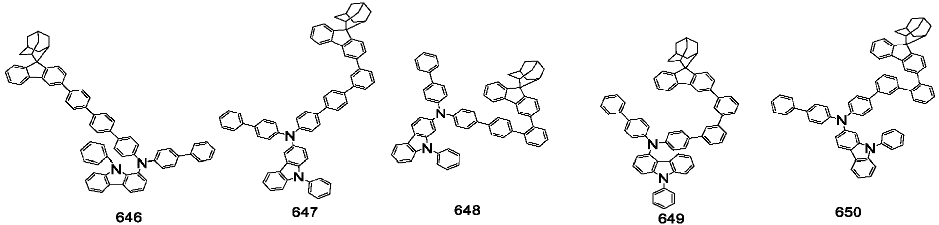

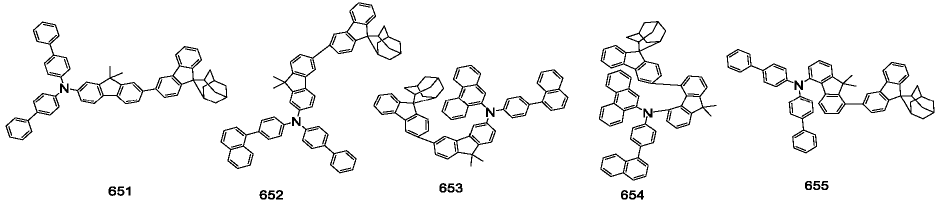

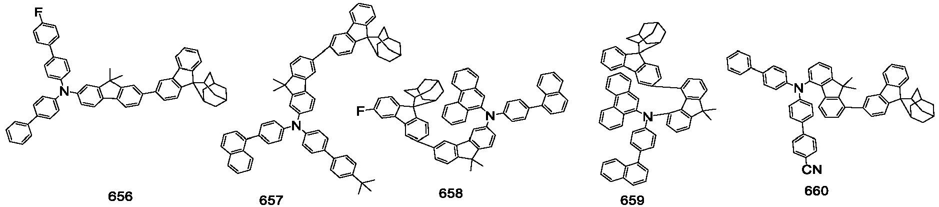

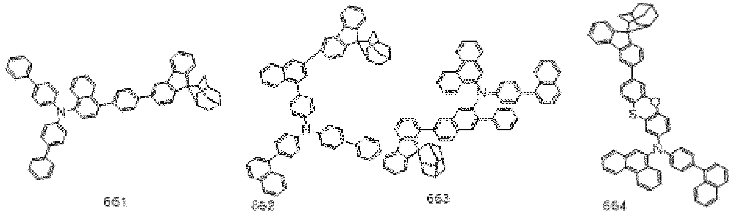

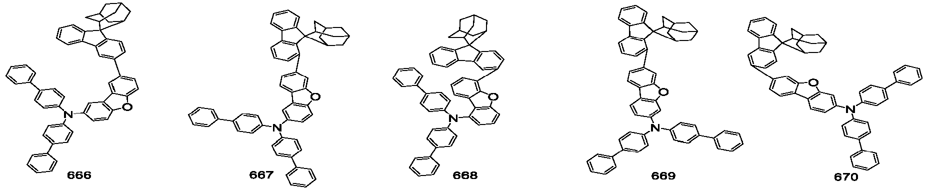

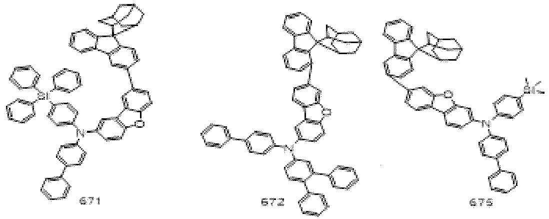

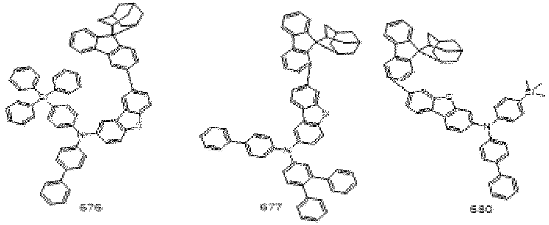

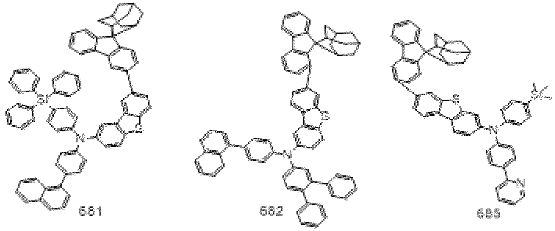

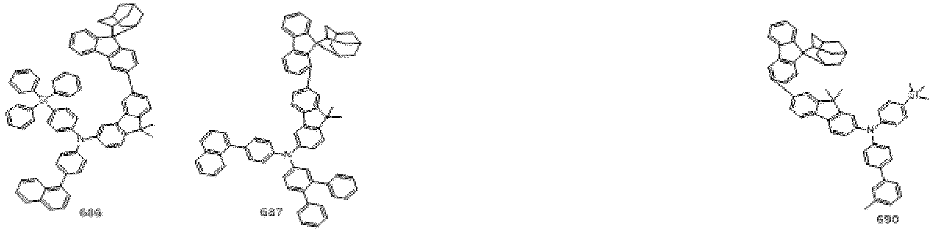

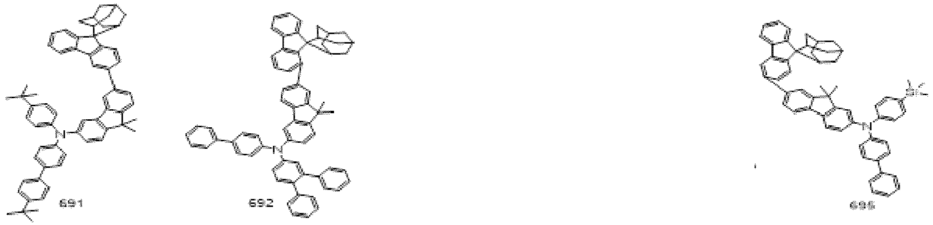

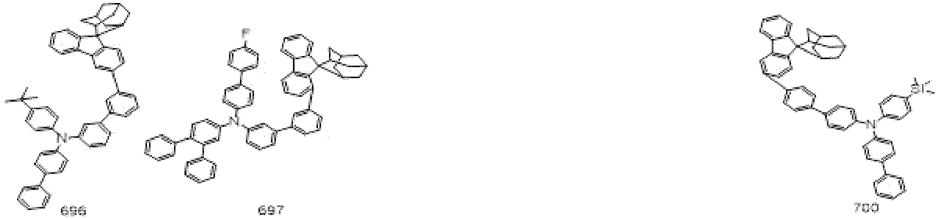

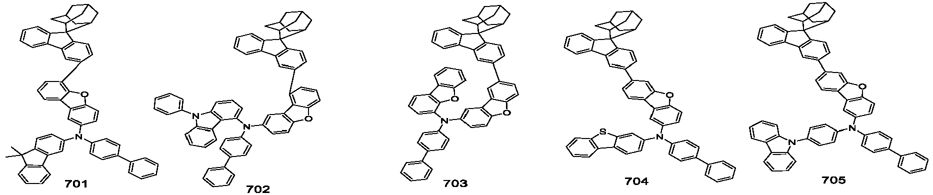

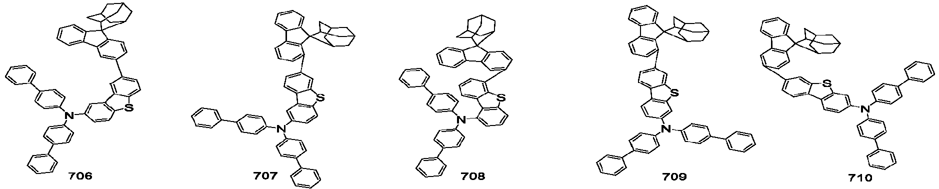

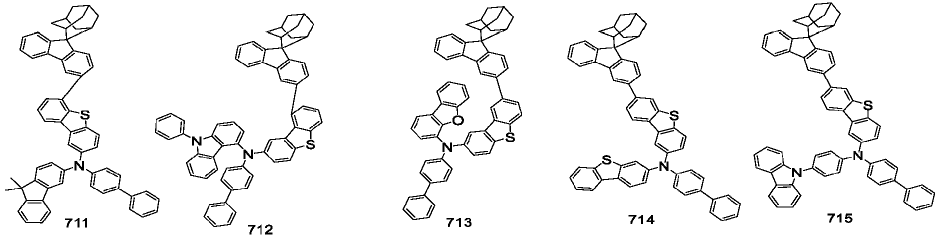

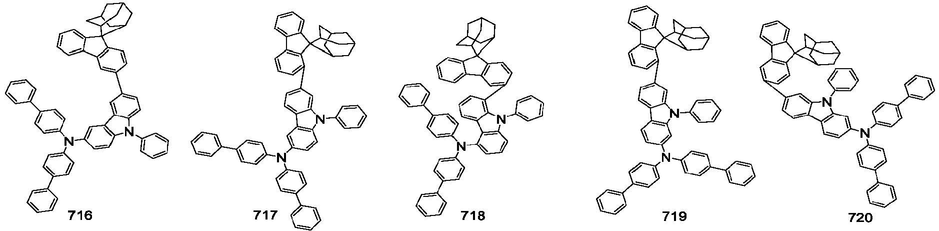

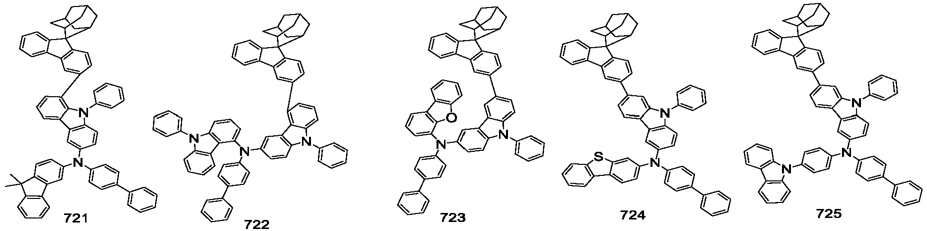

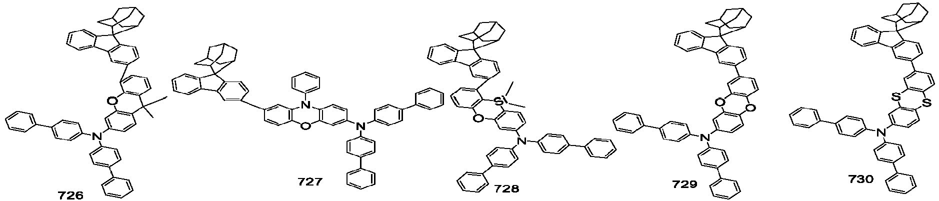

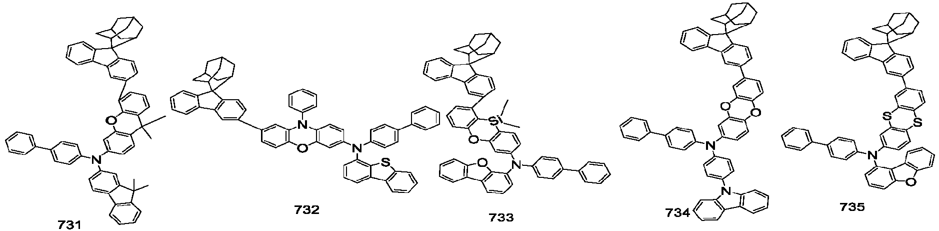

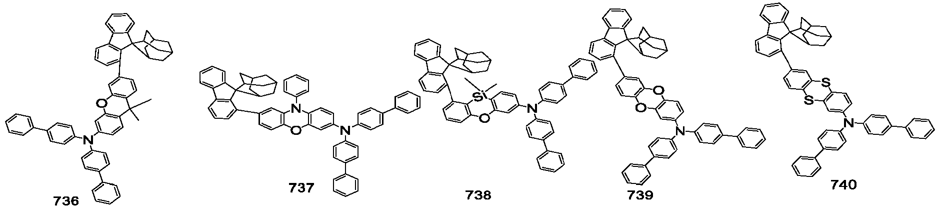

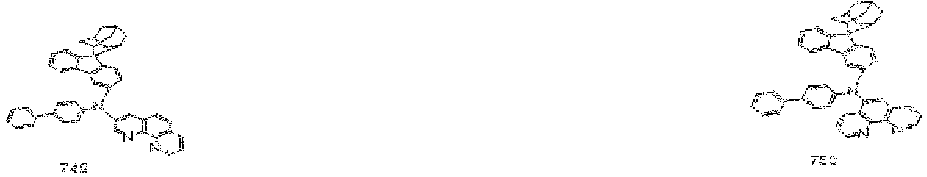

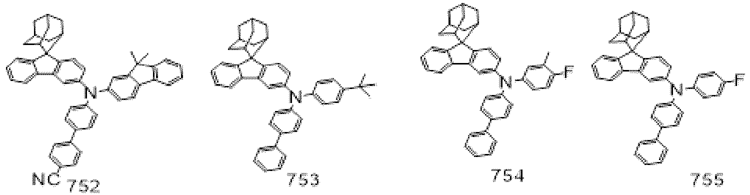

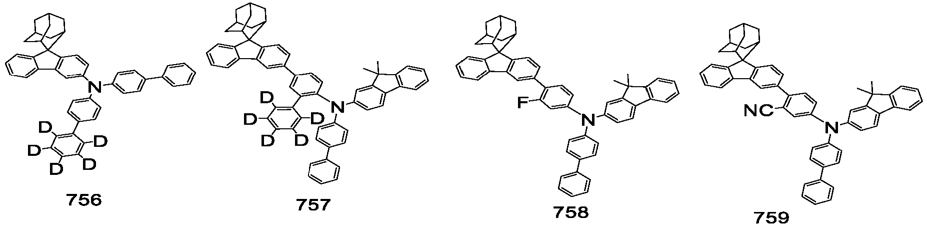

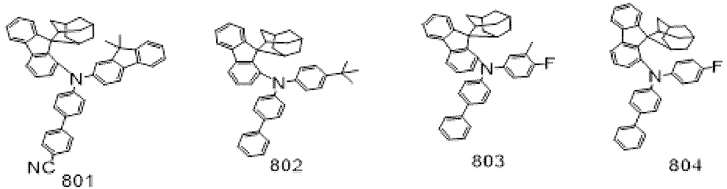

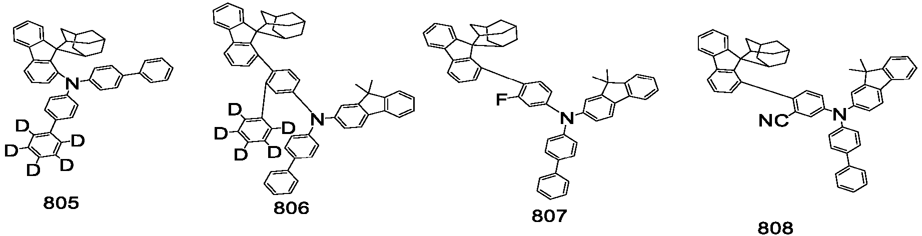

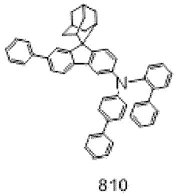

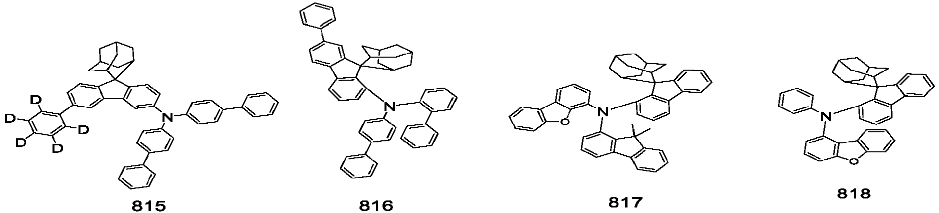

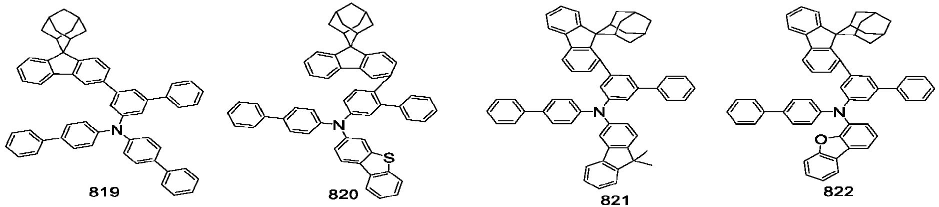

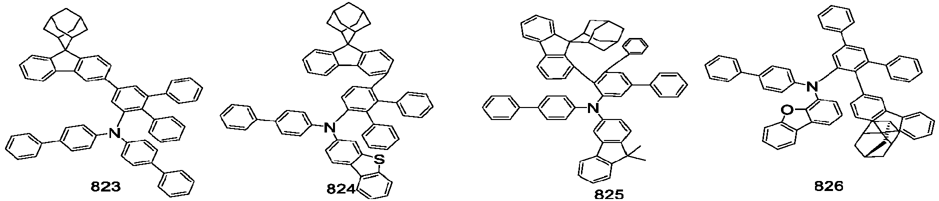

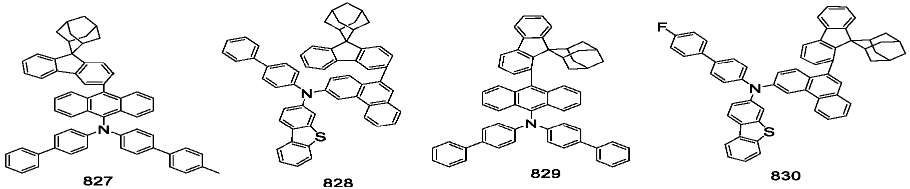

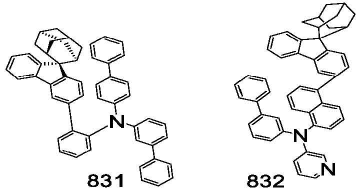

3. An electronic element including an anode and a cathode disposed opposite to each other, and a functional layer disposed between the anode and the cathode; wherein the functional layer contains the nitrogen-containing compound any one of claims 1 or 2.

4. The electronic element of claim 3, wherein the functional layer includes an electron blocking layer, and the electron blocking layer contains the nitrogen-containing compound.

5. The electronic element of claim 3, wherein the electronic element is an organic electroluminescence device or a photoelectric conversion device; preferably, the organic electroluminescent device is a green light device

6. An electronic device including the electronic element of any of claims 3 to 5.

## Patentansprüche

1. Stickstoffhaltige Verbindung mit einer in der chemischen Formel 1 dargestellten Struktur: 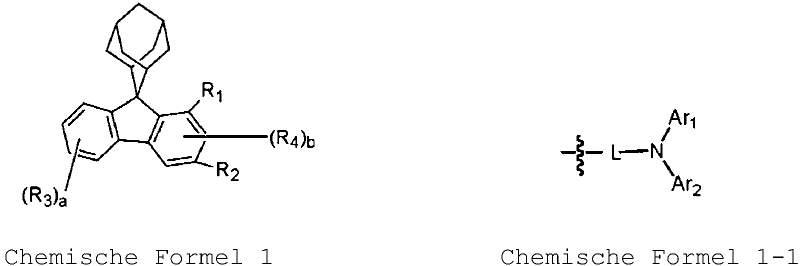
wobei 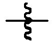 eine chemische Bindung darstellt;
R₁ und R₂ jeweils unabhängig voneinander aus Wasserstoff oder einer durch die chemische Formel 1-1 dargestellten Gruppe ausgewählt sind, und eines und nur eines von R₁ und R₂ die durch die chemische Formel 1-1 dargestellte Gruppe aufweist; wenn R₁ oder R₂ aus Wasserstoff ausgewählt ist, können das genannte R₁ und R₂ durch R₄ ersetzt werden;
R₃ und R₄ jeweils unabhängig voneinander aus der aus Deuterium und Phenyl bestehenden Gruppe ausgewählt sind;
a aus 0, 1, 2, 3, oder 4 ausgewählt ist; wenn a größer als oder gleich 2 ist, sind jeweils zwei beliebige R₃ gleich oder verschieden;
b aus 0, 1, 2 oder 3 ausgewählt ist, wenn b größer als oder gleich 2 ist, sind jeweils zwei beliebige R₄ gleich oder verschieden;
L aus der aus einer Einfachbindung und den folgenden Gruppen bestehenden Gruppe ausgewählt ist: 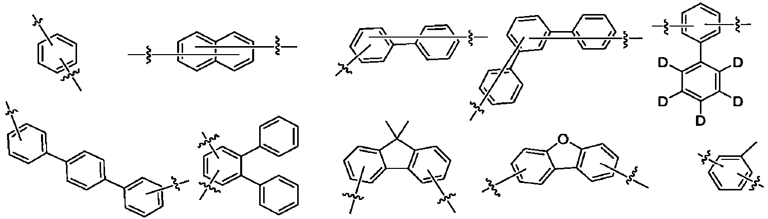 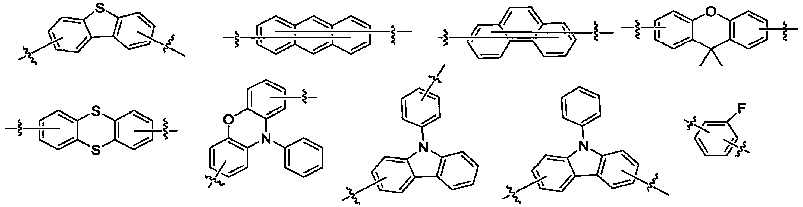 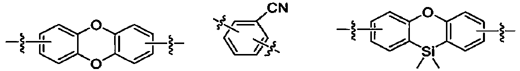 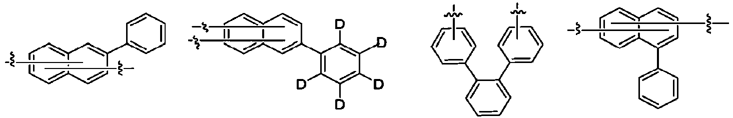 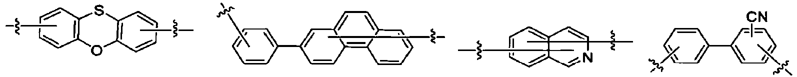 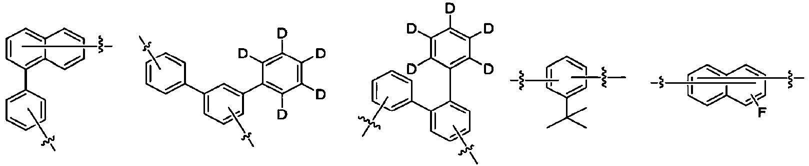 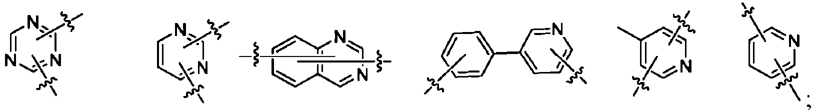 und
Ar₁ und Ar₂ jeweils unabhängig voneinander aus der aus den folgenden Gruppen bestehenden Gruppe ausgewählt sind:

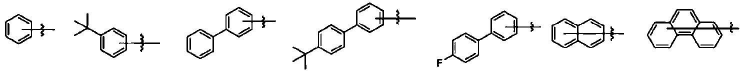

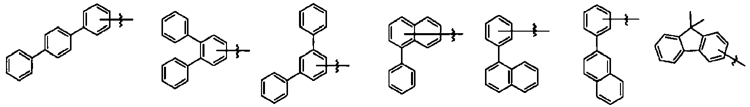

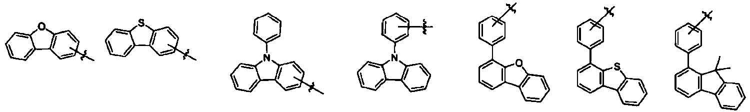

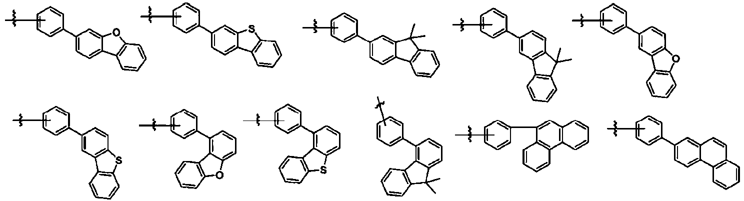

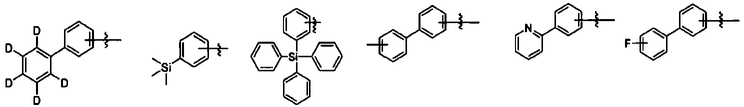

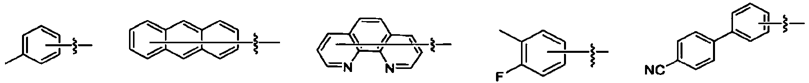

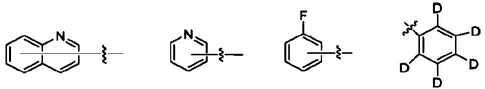

2. Stickstoffhaltige Verbindung nach Anspruch 1, wobei die stickstoffhaltige Verbindung aus der aus den folgenden Verbindungen bestehenden Gruppe ausgewählt ist:

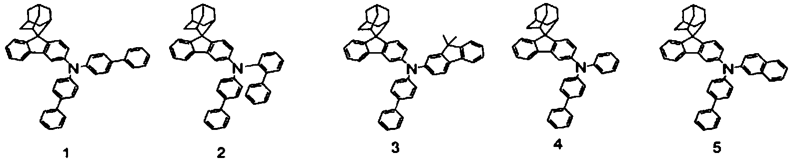

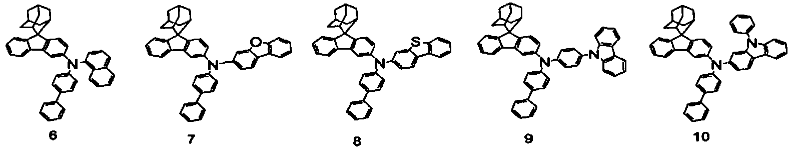

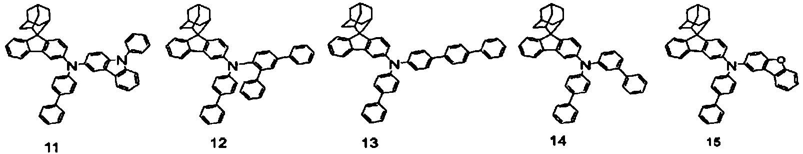

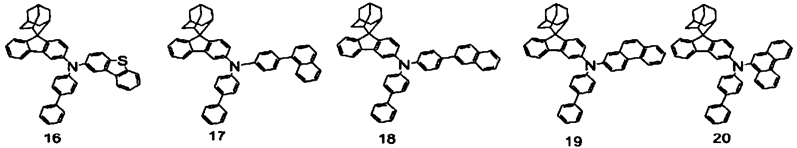

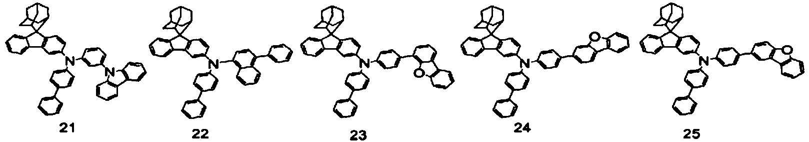

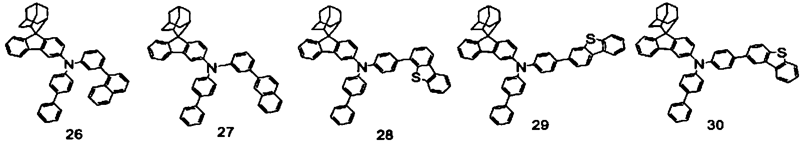

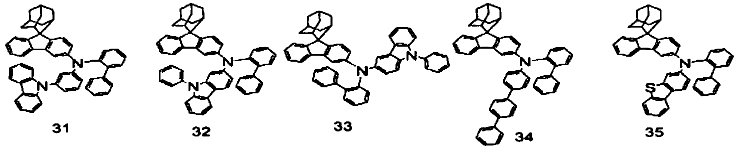

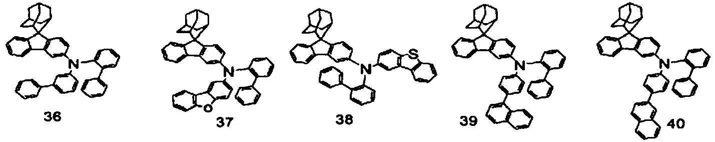

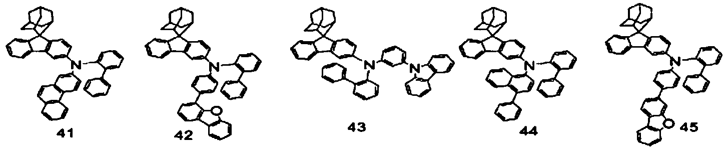

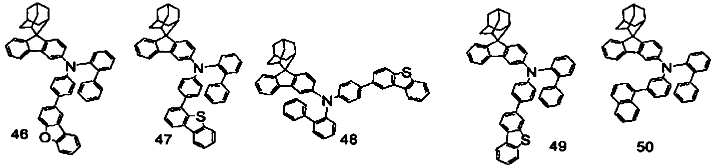

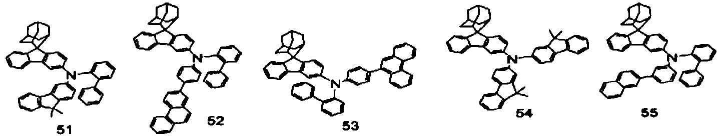

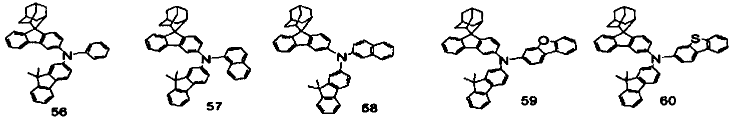

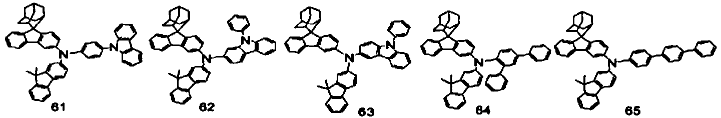

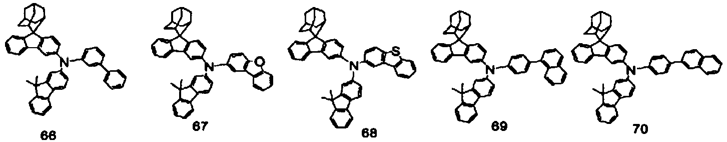

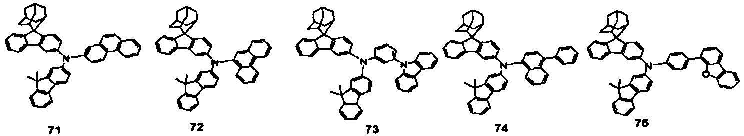

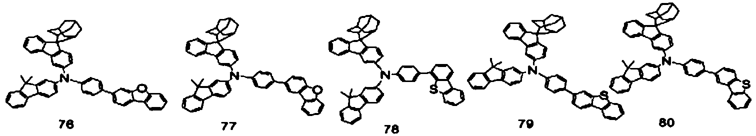

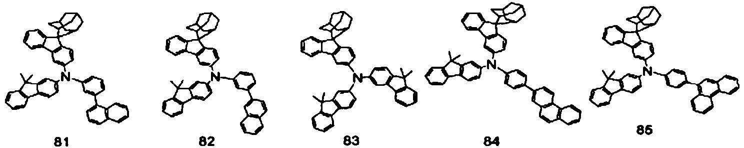

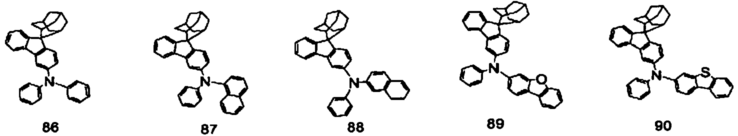

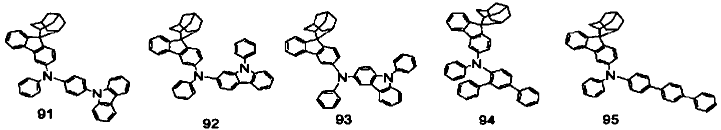

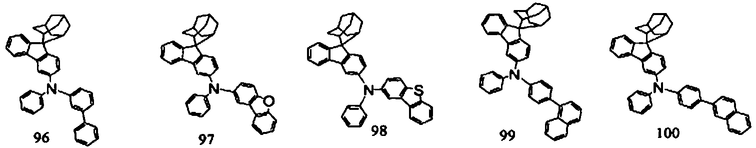

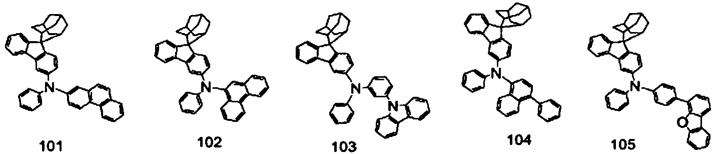

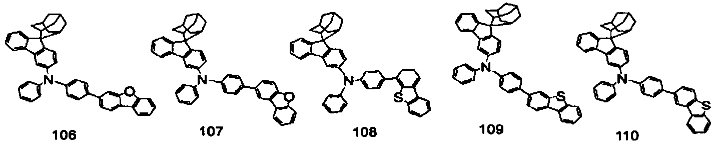

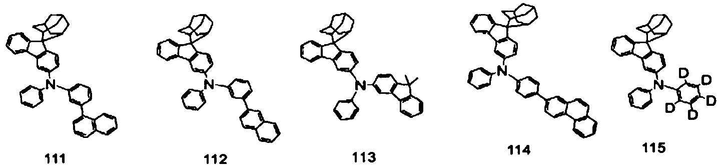

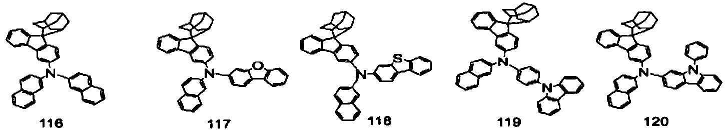

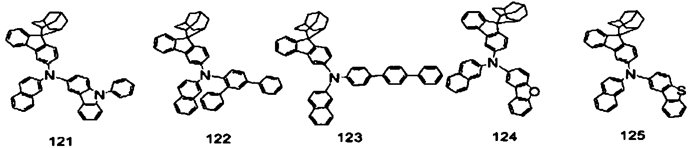

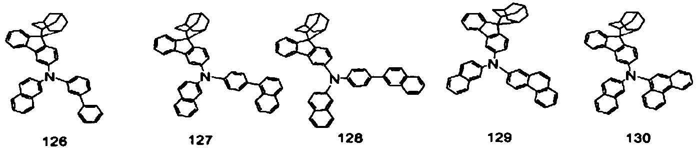

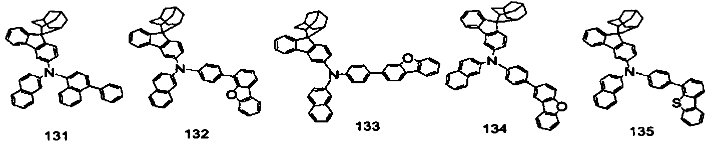

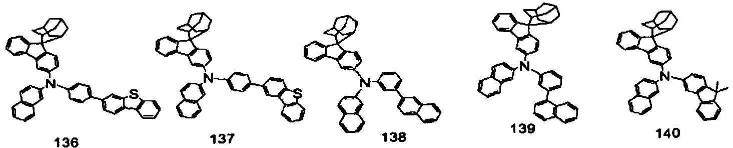

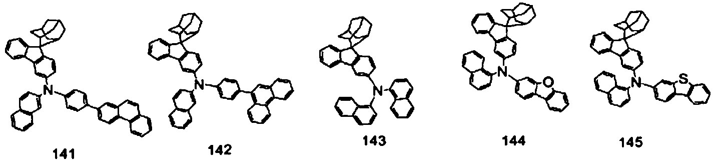

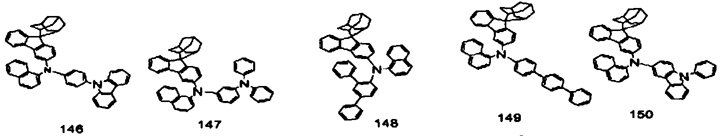

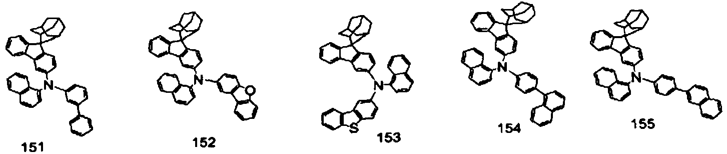

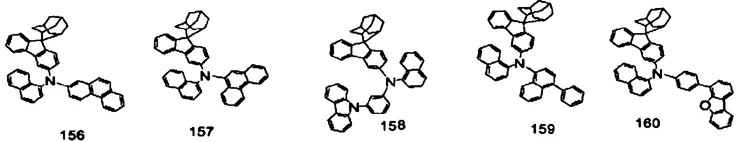

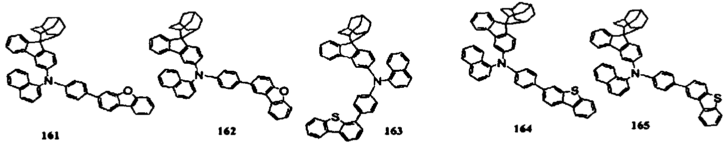

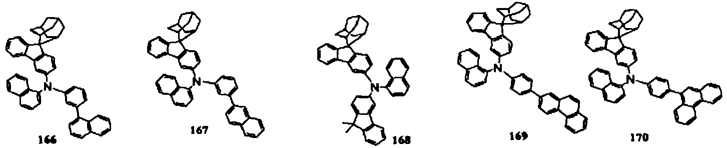

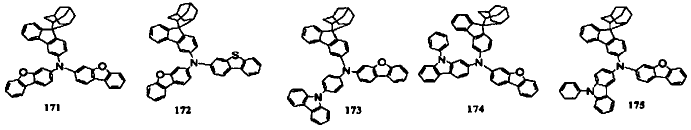

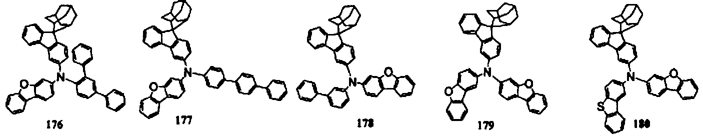

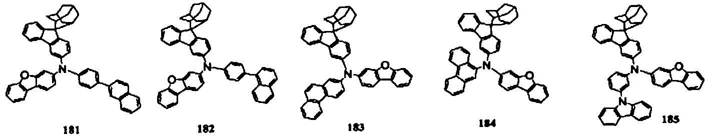

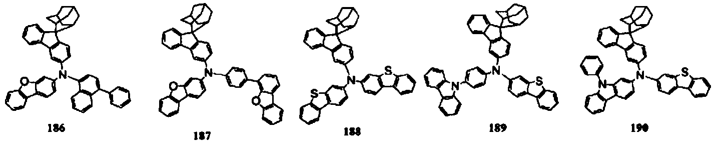

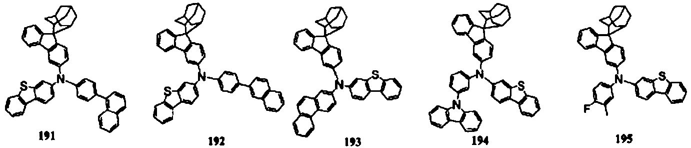

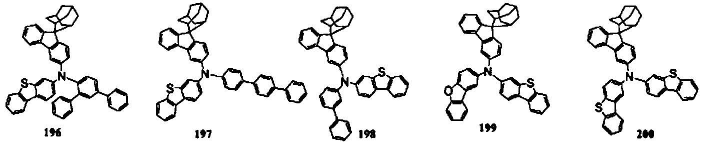

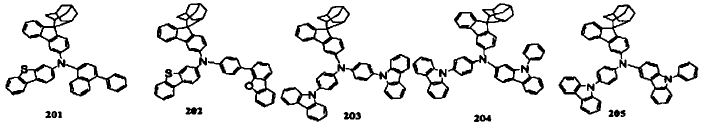

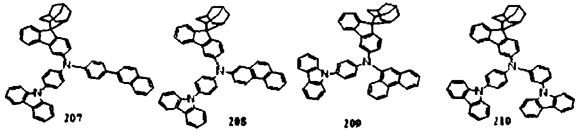

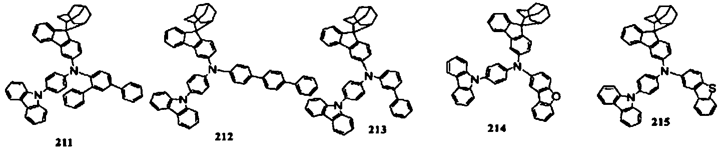

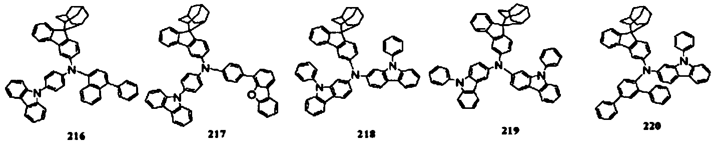

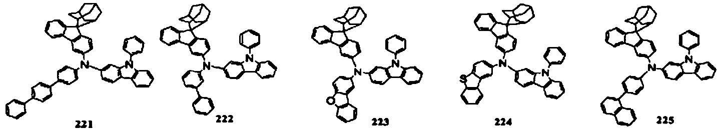

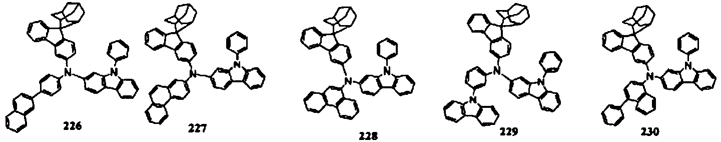

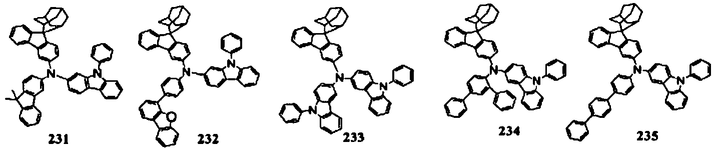

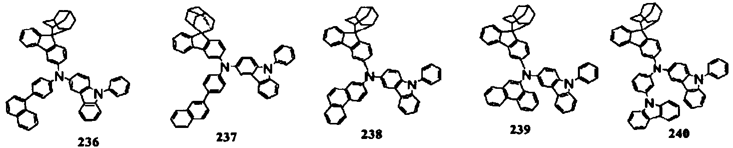

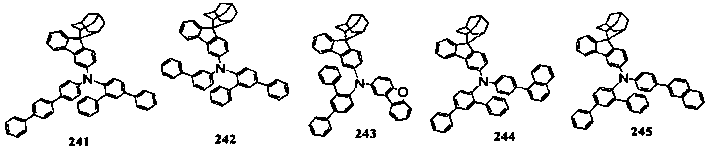

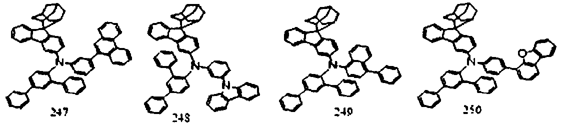

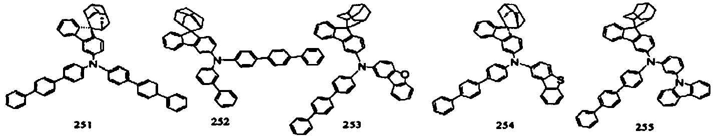

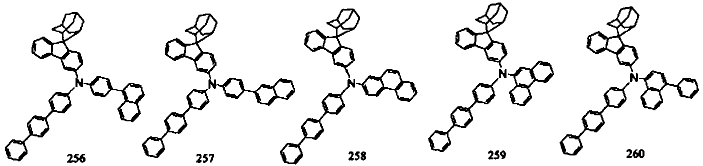

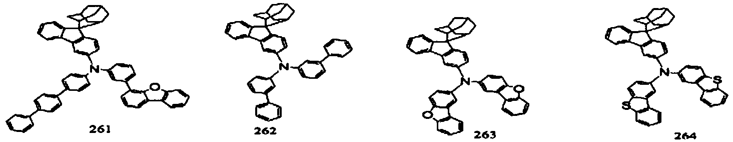

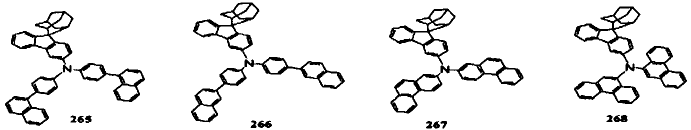

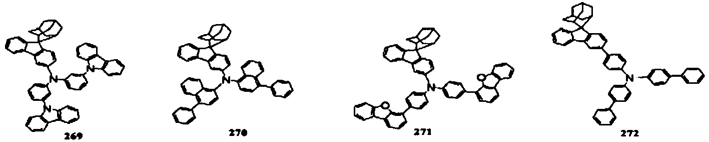

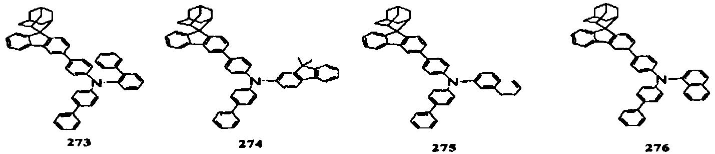

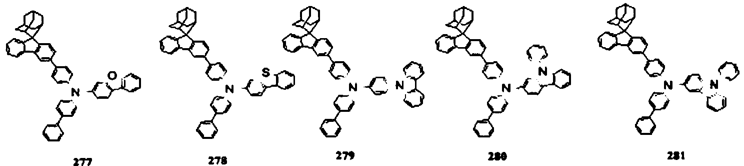

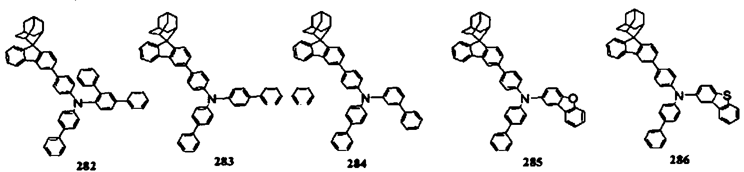

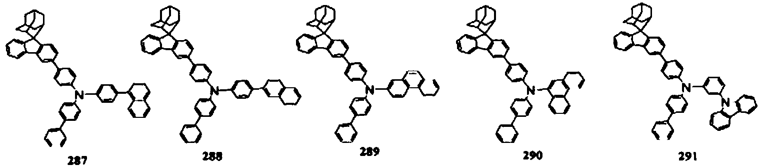

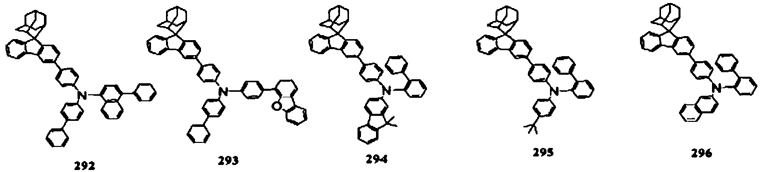

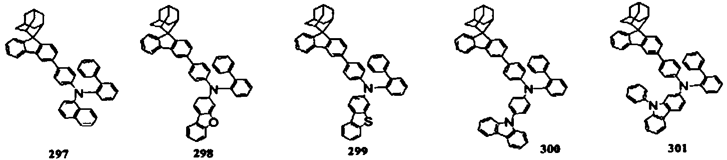

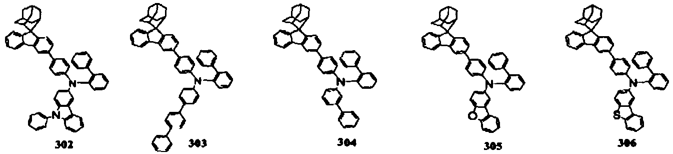

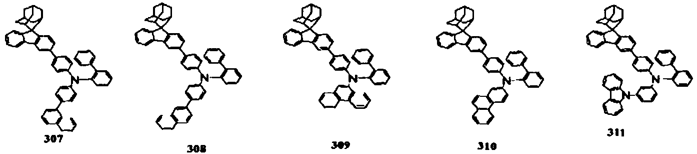

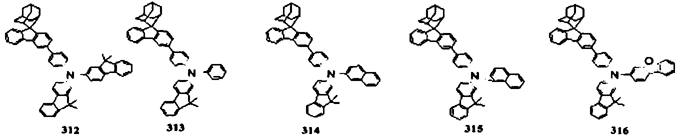

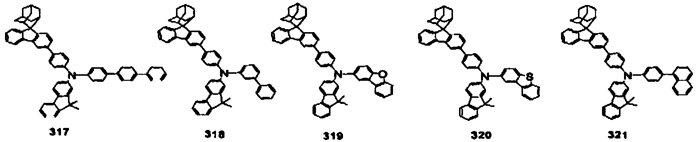

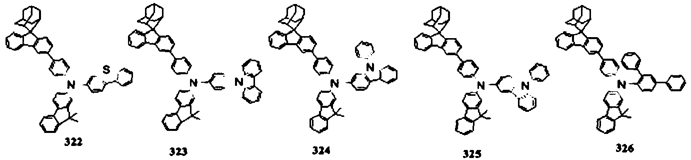

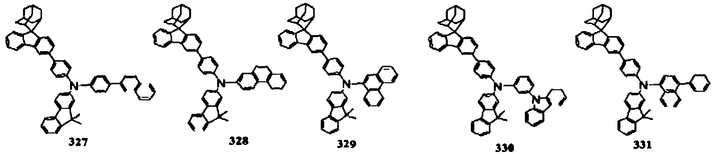

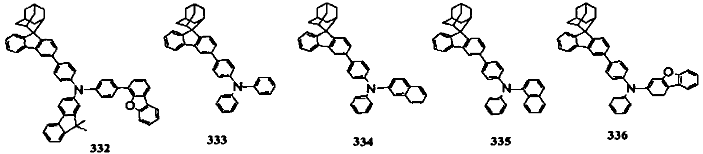

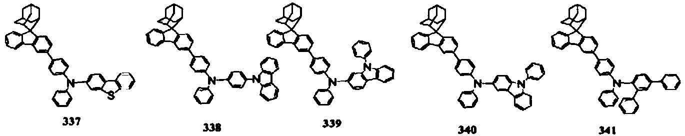

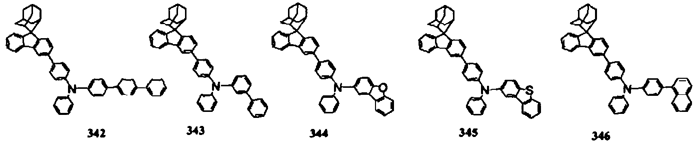

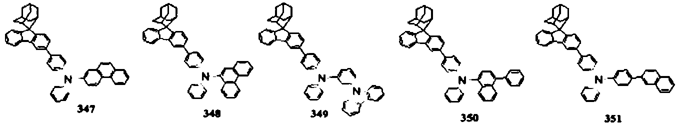

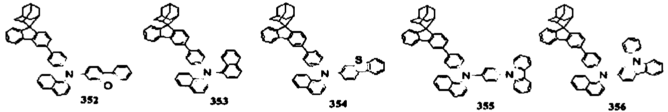

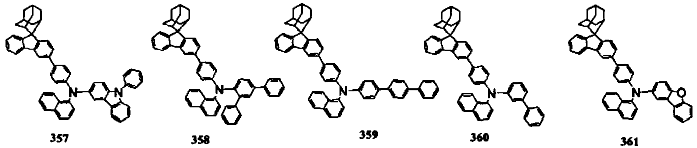

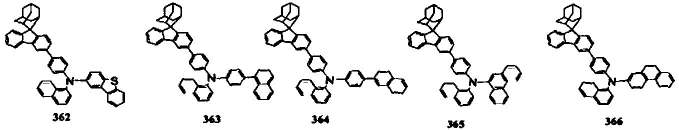

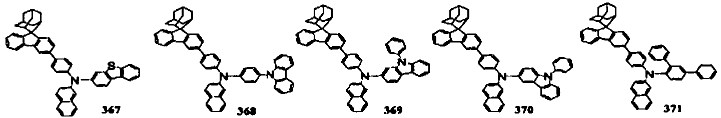

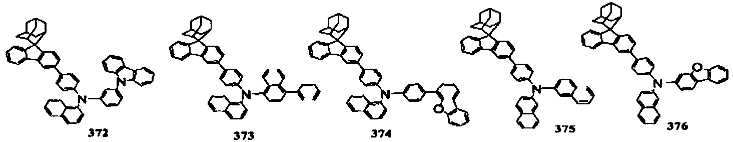

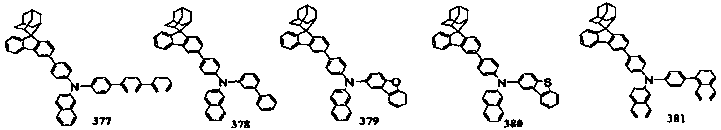

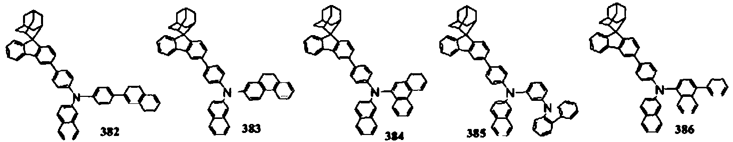

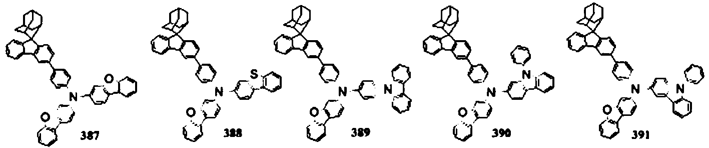

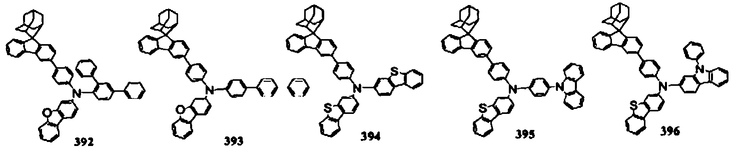

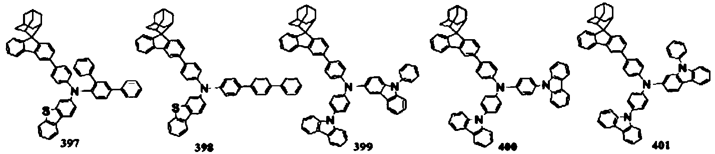

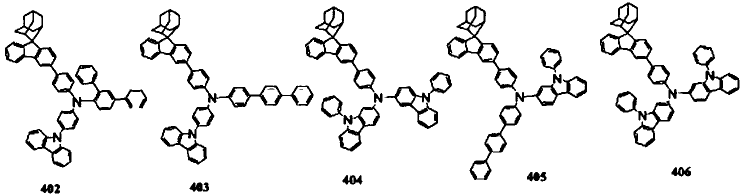

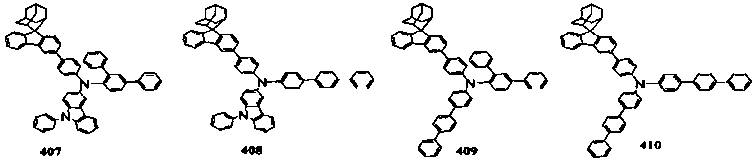

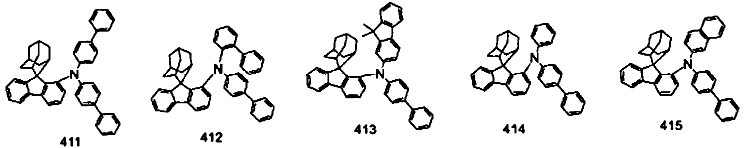

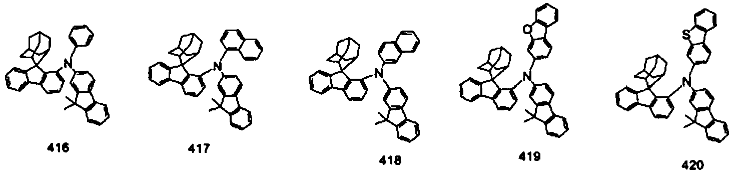

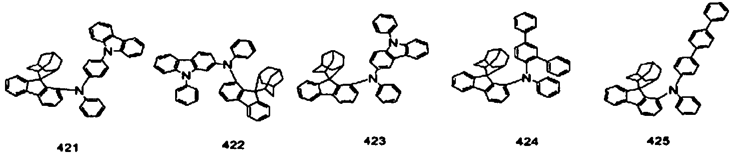

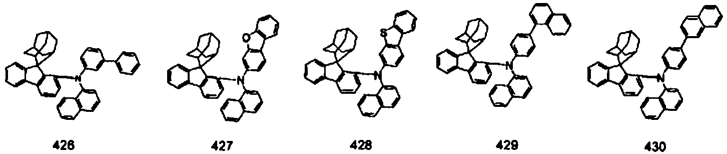

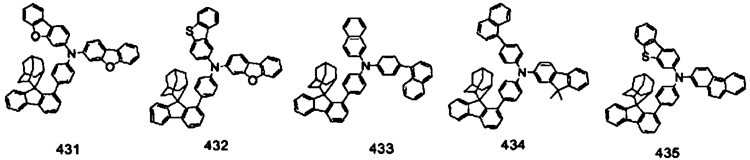

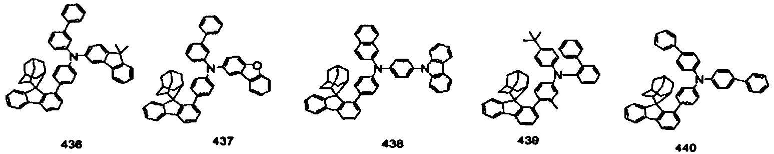

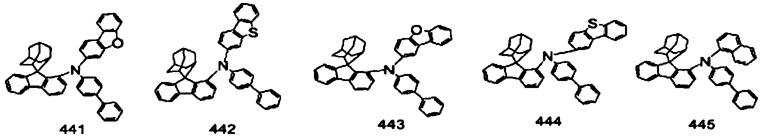

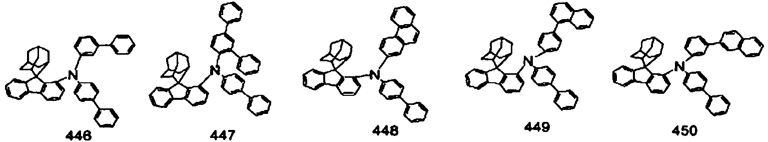

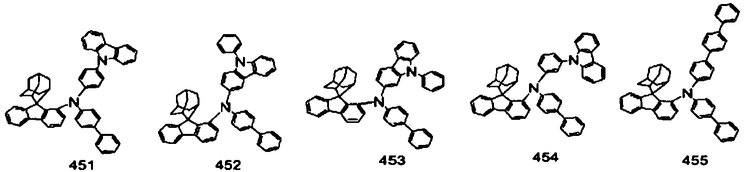

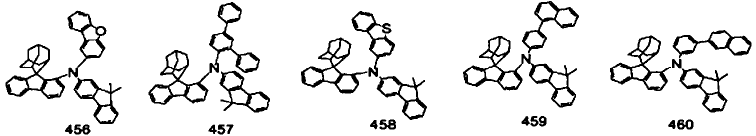

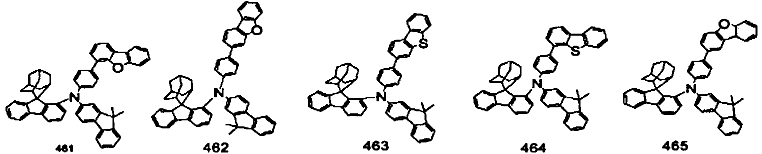

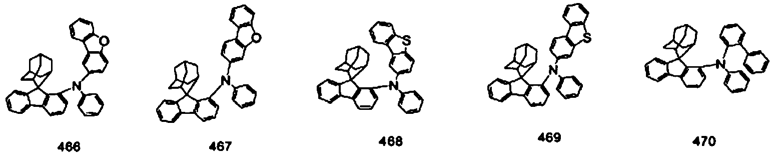

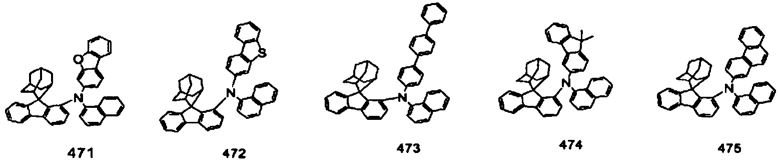

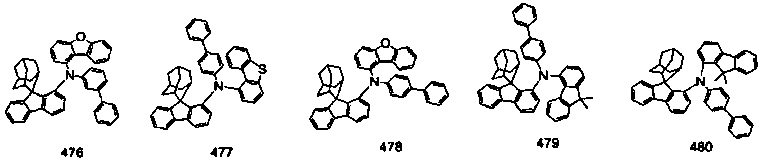

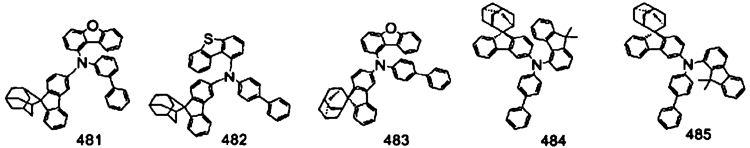

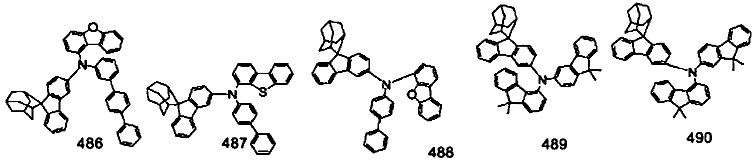

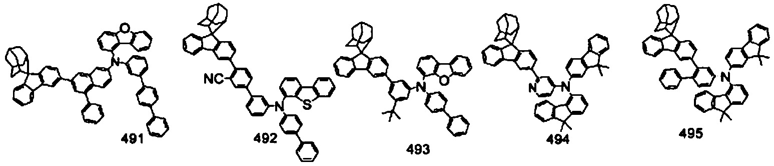

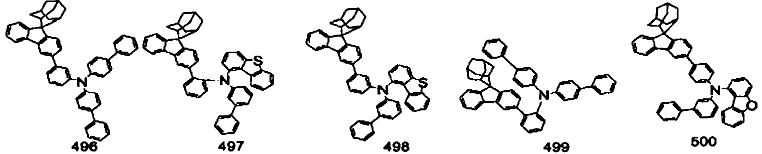

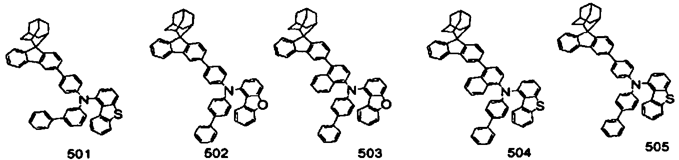

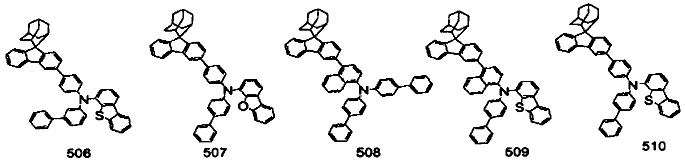

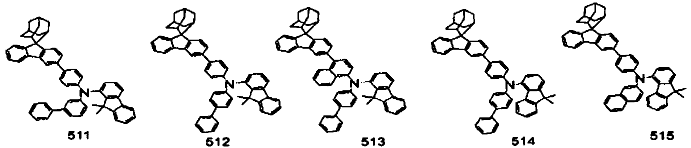

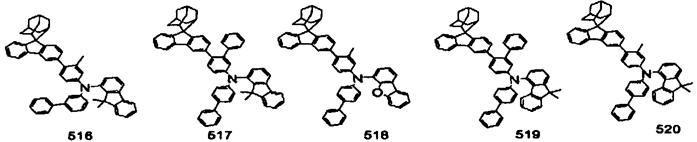

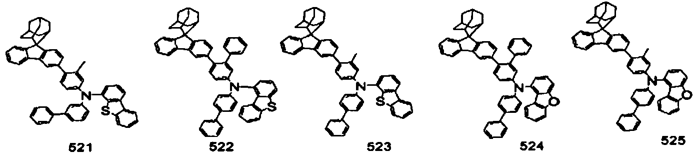

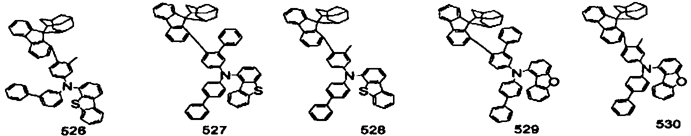

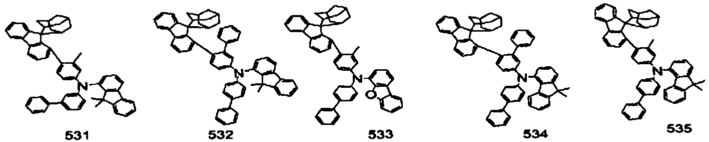

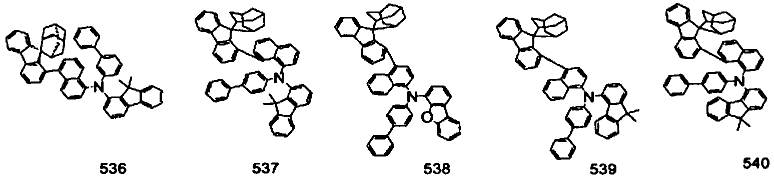

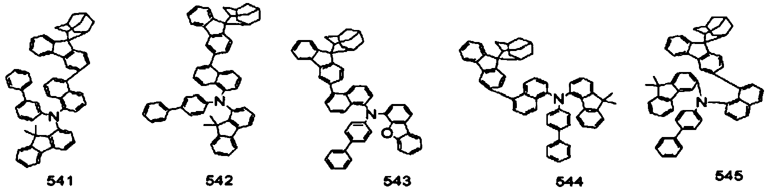

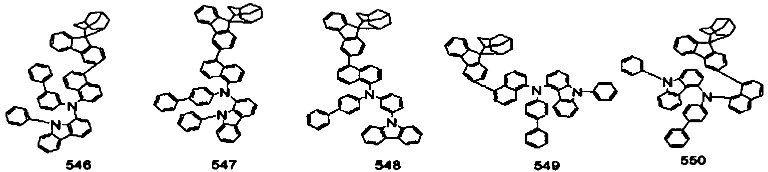

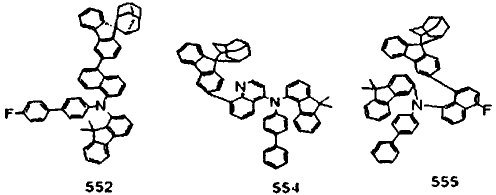

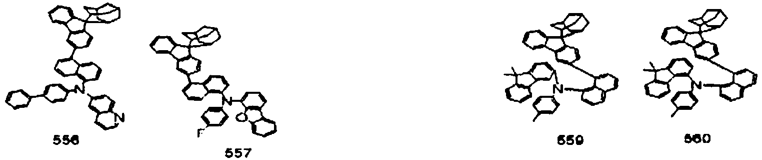

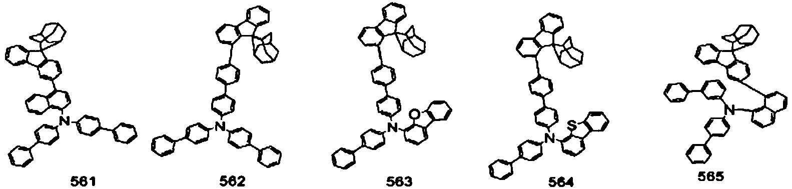

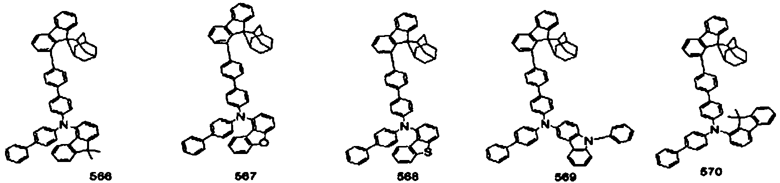

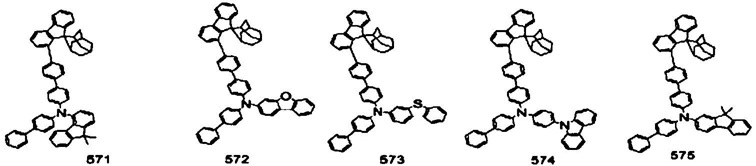

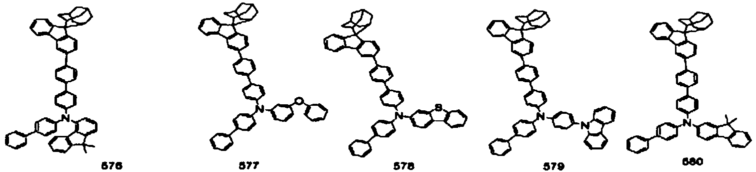

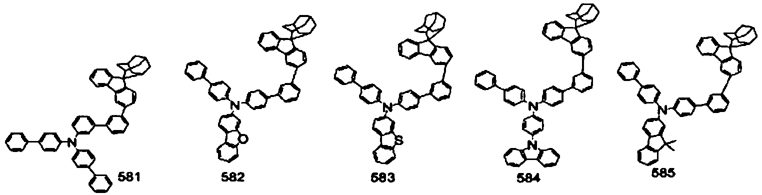

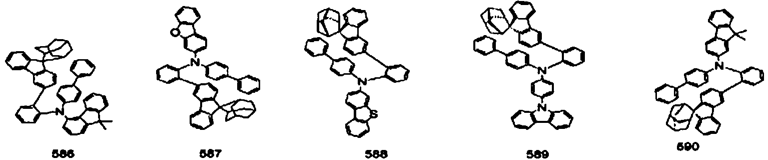

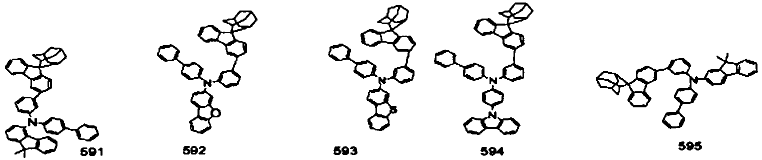

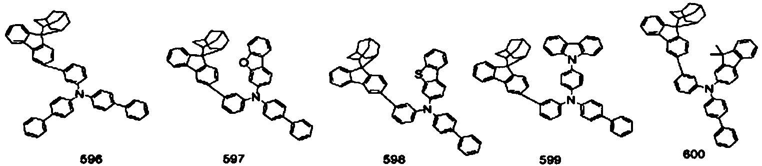

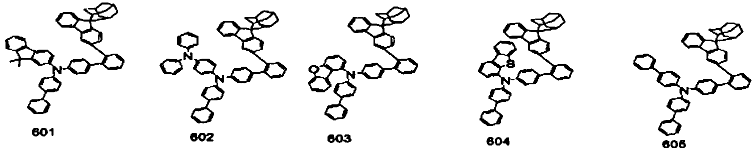

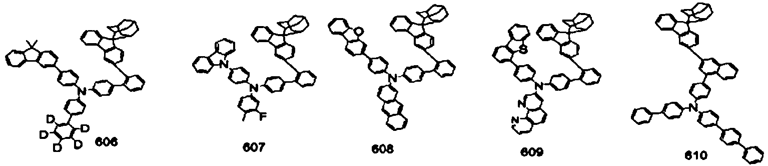

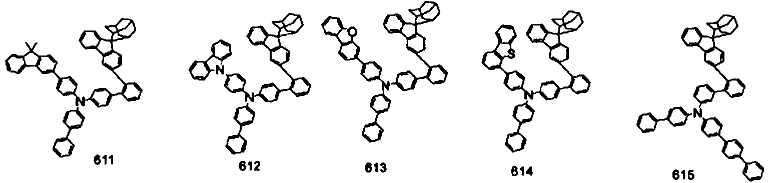

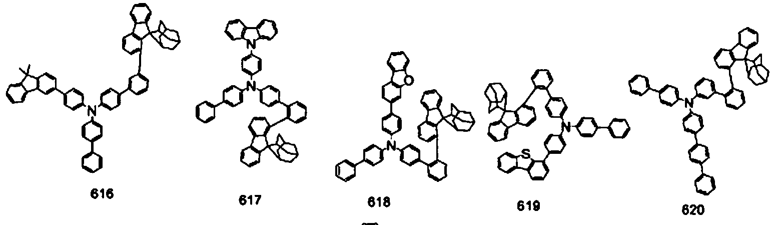

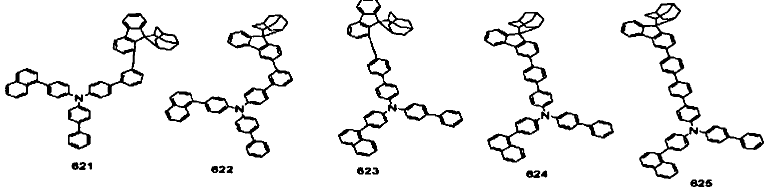

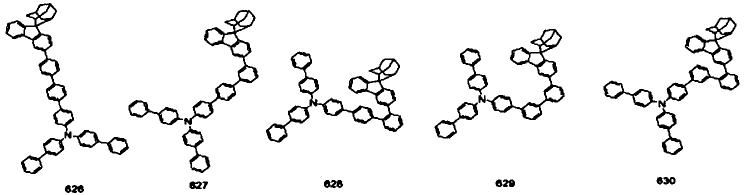

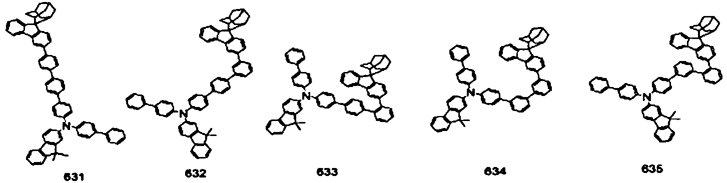

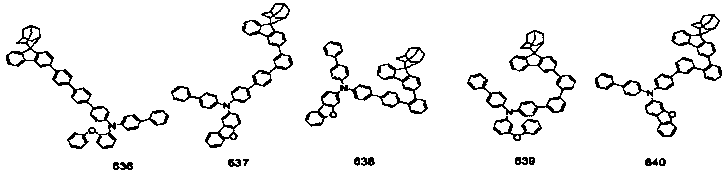

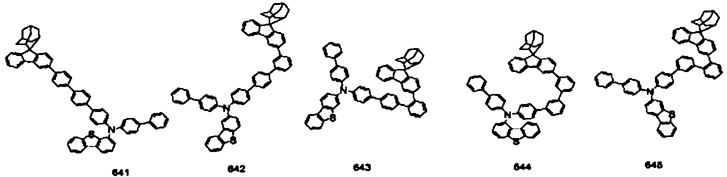

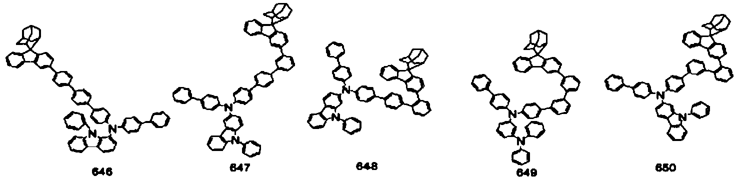

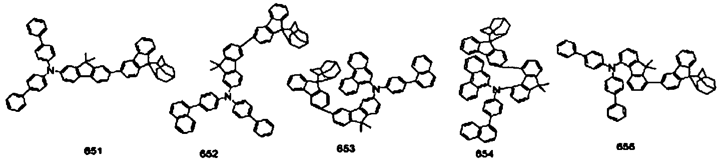

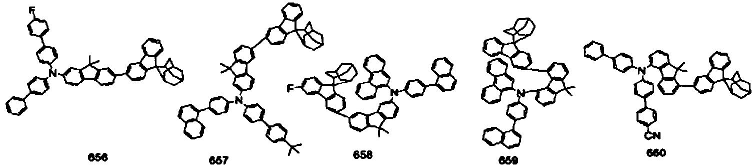

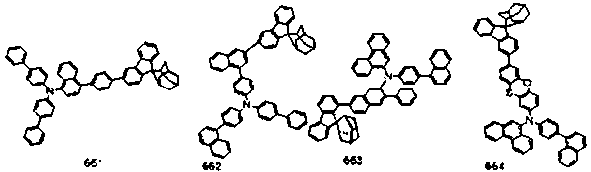

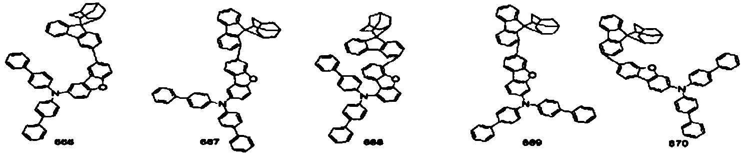

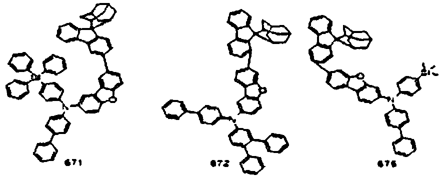

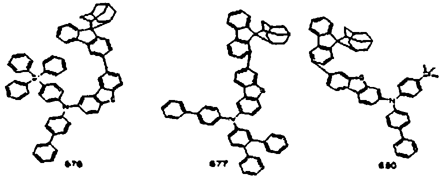

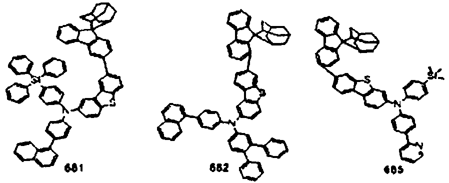

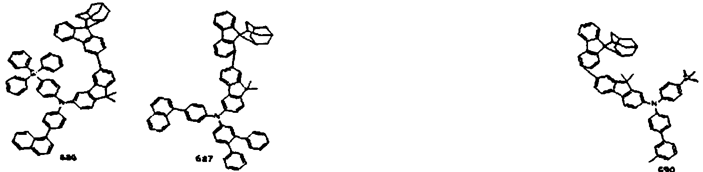

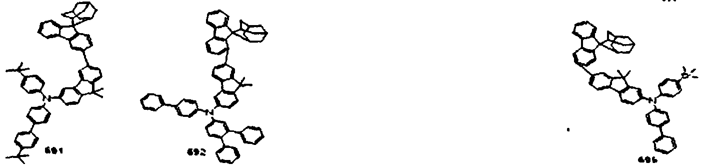

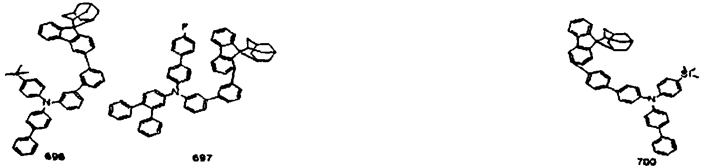

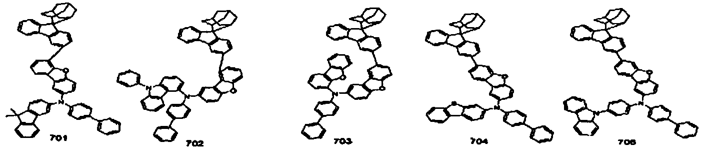

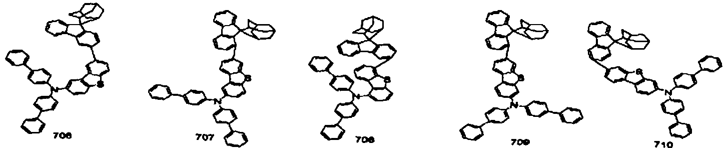

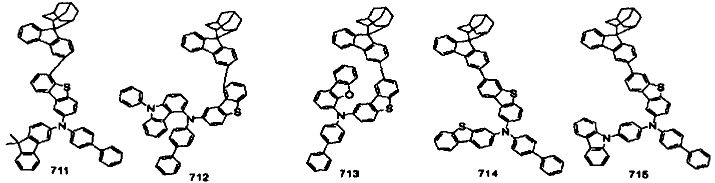

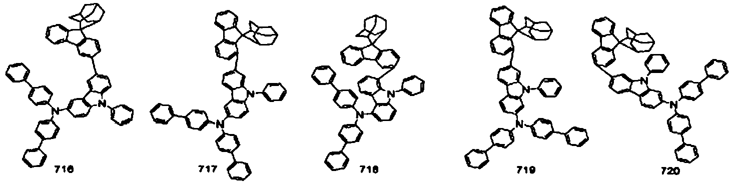

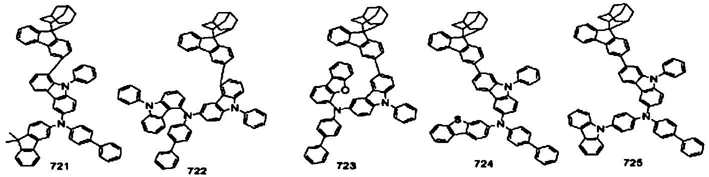

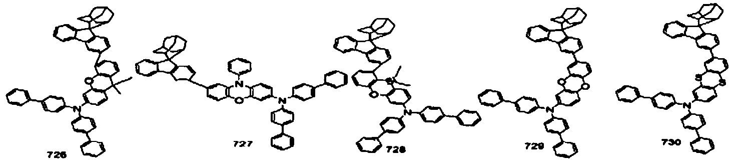

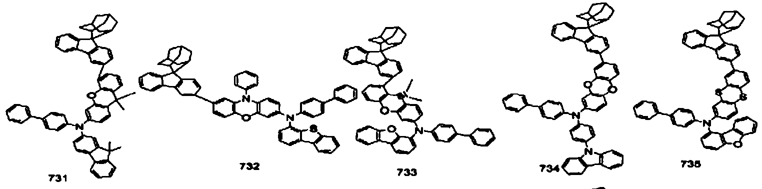

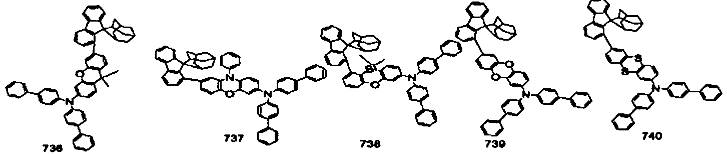

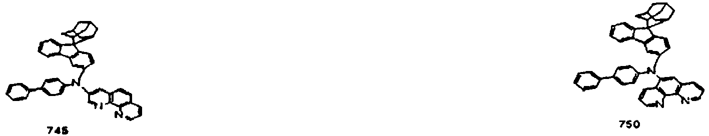

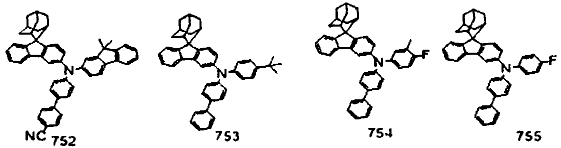

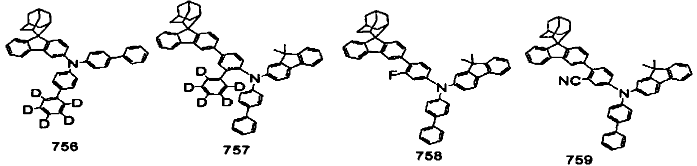

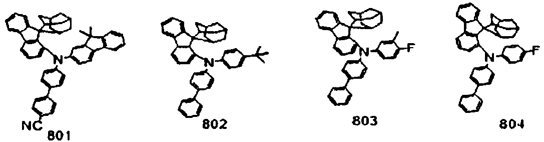

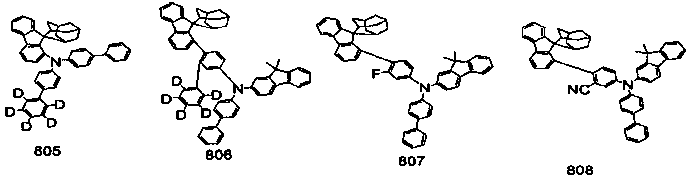

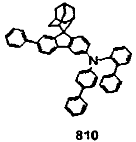

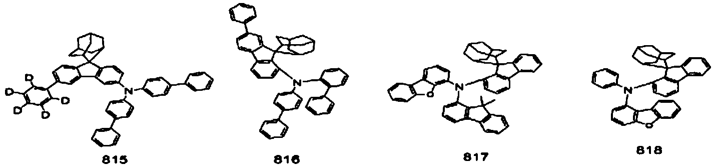

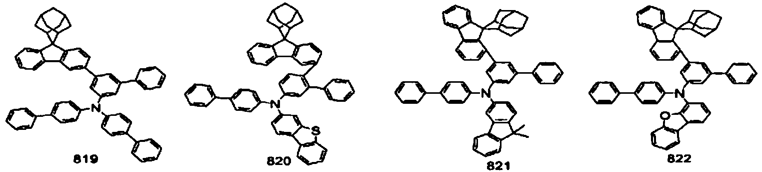

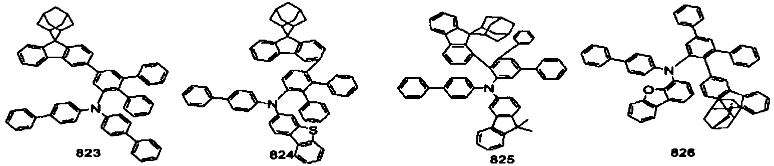

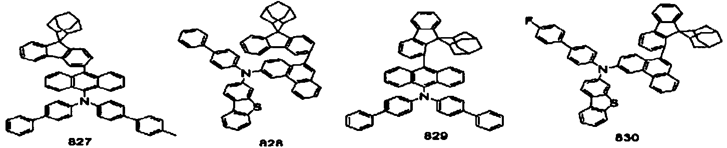

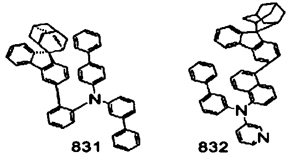

3. Elektronisches Element, umfassend eine Anode und eine Kathode, die einander gegenüberliegend angeordnet sind, sowie eine zwischen der Anode und der Kathode angeordnete Funktionsschicht; wobei die Funktionsschicht die stickstoffhaltige Verbindung nach einem der Ansprüche 1 oder 2 enthält.

4. Elektronische Element nach Anspruch 3, wobei die Funktionsschicht eine Elektronenblockierschicht umfasst und die Elektronenblockierschicht die stickstoffhaltige Verbindung enthält.

5. Elektronische Element nach Anspruch 3, wobei das elektronische Element eine organische Elektrolumineszenzvorrichtung oder eine photoelektrische Umwandlungsvorrichtung ist; vorzugsweise ist die organische Elektrolumineszenzvorrichtung eine Grünlichtvorrichtung

6. Elektronische Vorrichtung, umfassend das elektronische Element nach einem der Ansprüche 3 bis 5.

## Revendications

1. Composé contenant de l'azote présentant une structure représentée par la formule chimique 1: 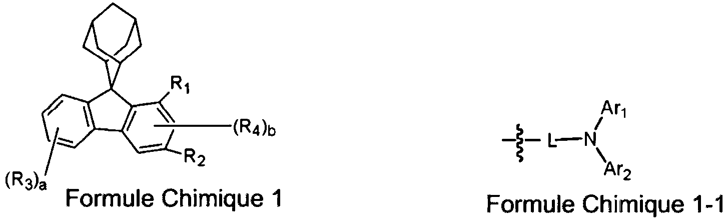
dans laquelle 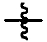 représente une liaison chimique;
R₁ et R₂ sont chacun indépendamment sélectionnés parmi l'hydrogène ou un groupe représenté par la formule chimique 1-1, et un seul et unique parmi R₁ et R₂ comporte le groupe représenté par la formule chimique 1-1; lorsque R₁ ou R₂ est sélectionné parmi l'hydrogène, lesdits R₁ et R₂ peuvent être remplacés par R₄;
R₃, R₄ sont chacun indépendamment sélectionnés dans le groupe constitué du deutérium et du phényle;
a est sélectionné parmi 0, 1, 2, 3, ou 4; lorsque a est supérieur ou égal à 2, deux quelconques R₃ sont identiques ou différents;
b est sélectionné parmi 0, 1, 2, ou 3; lorsque b est supérieur ou égal à 2, deux quelconques R₄ sont identiques ou différents;
L est sélectionné dans le groupe constitué d'une liaison simple et des groupes suivants: 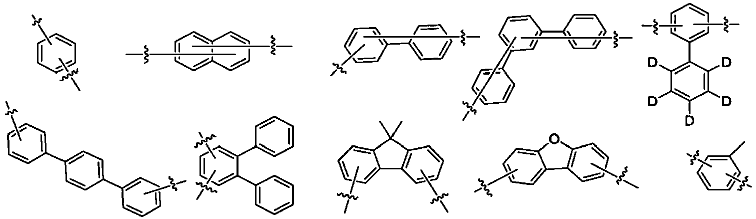 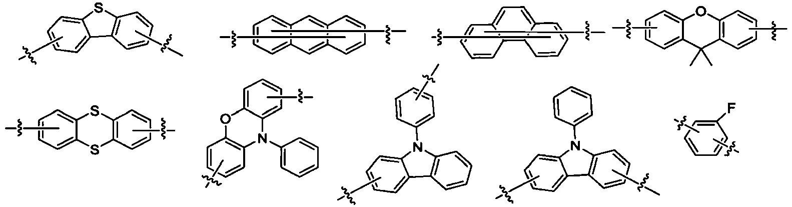 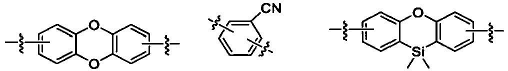 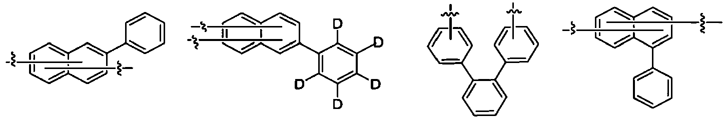 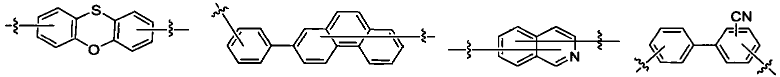 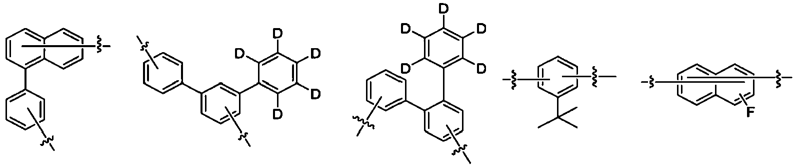 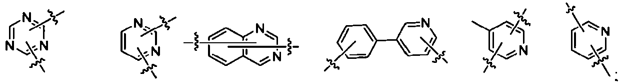 et
Ar₁ et Ar₂ sont chacun indépendamment sélectionnés dans le groupe constitué des groupes suivants:

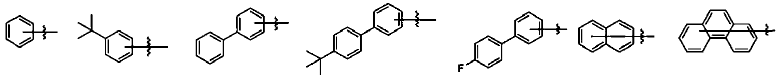

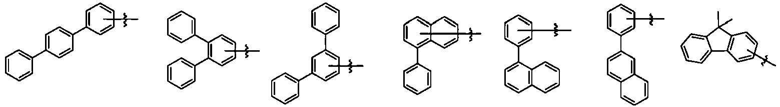

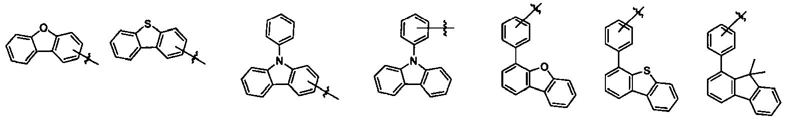

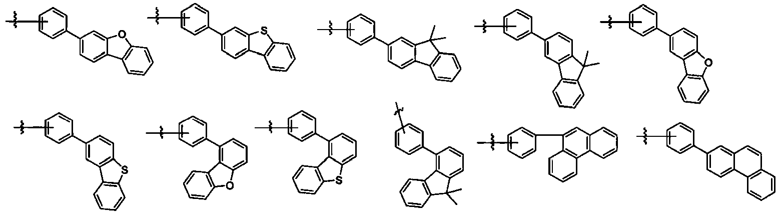

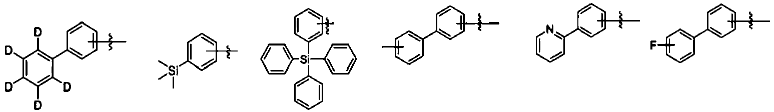

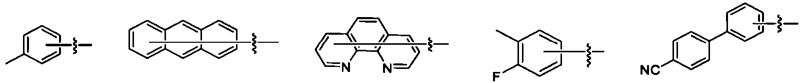

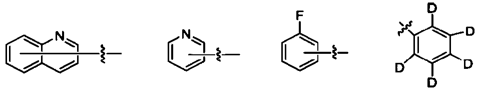

2. Composé contenant de l'azote selon la revendication 1, dans lequel le composé contenant de l'azote est sélectionné dans le groupe constitué des composés suivants:

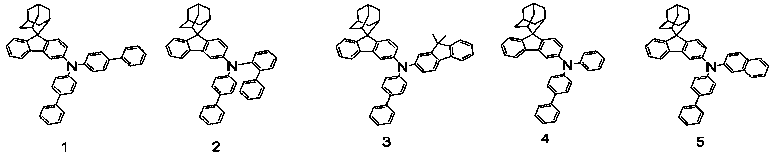

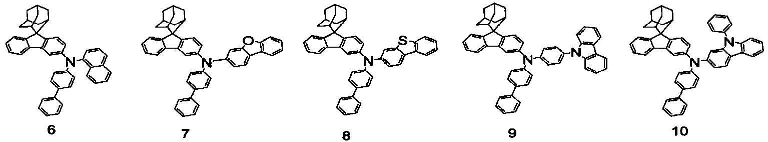

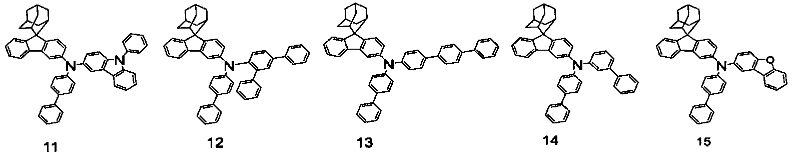

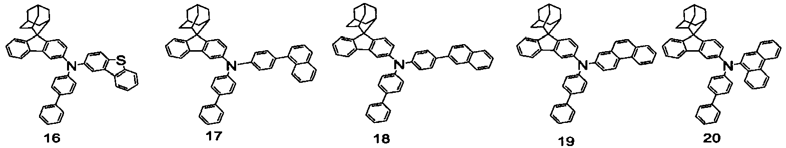

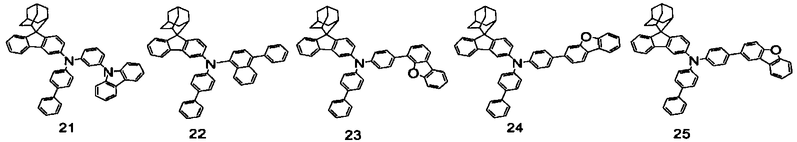

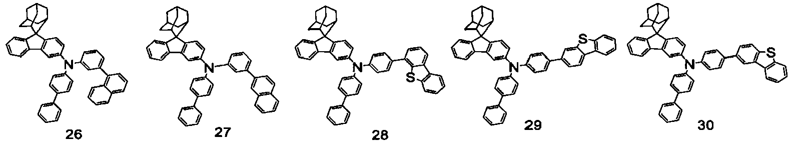

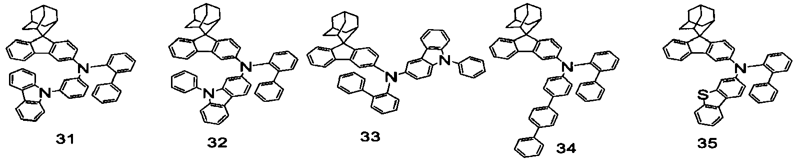

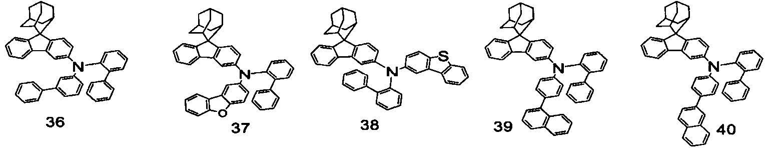

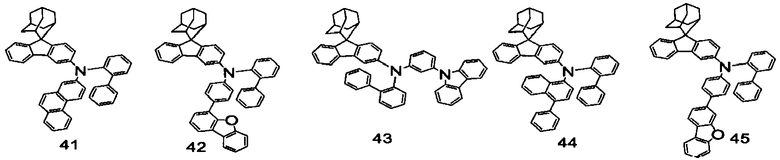

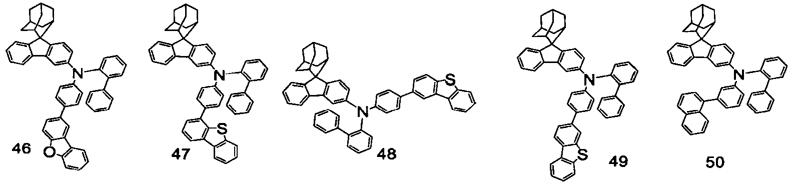

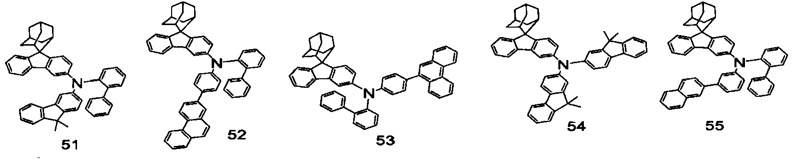

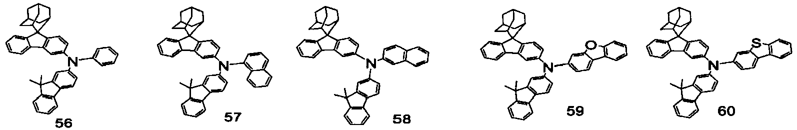

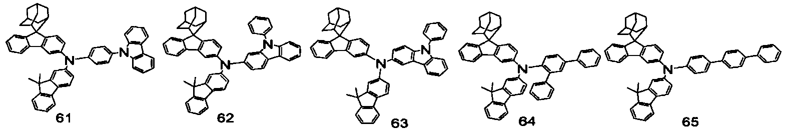

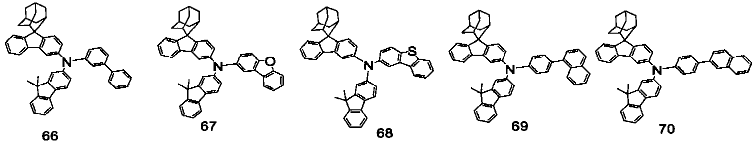

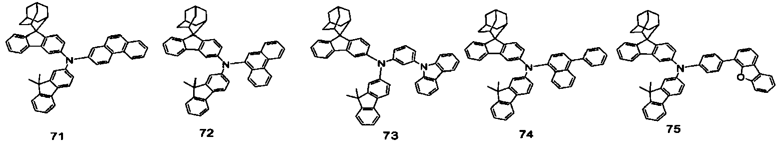

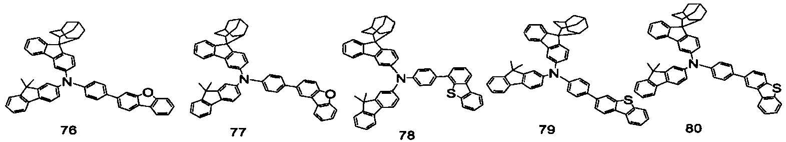

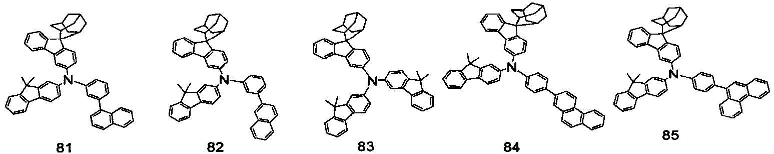

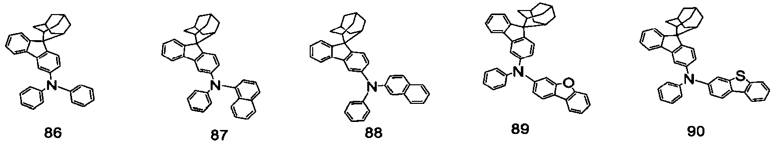

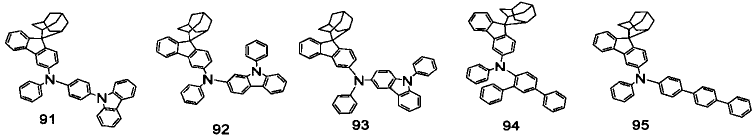

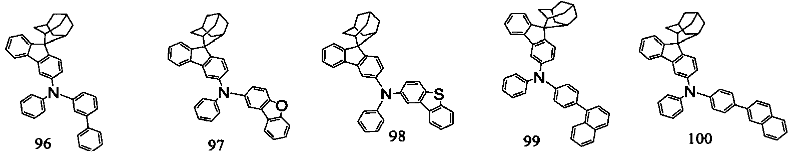

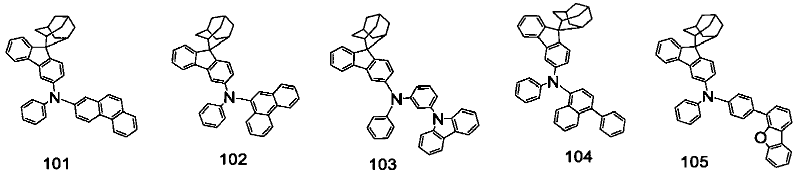

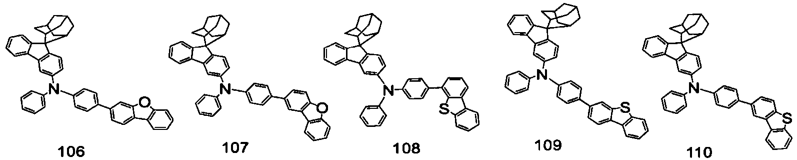

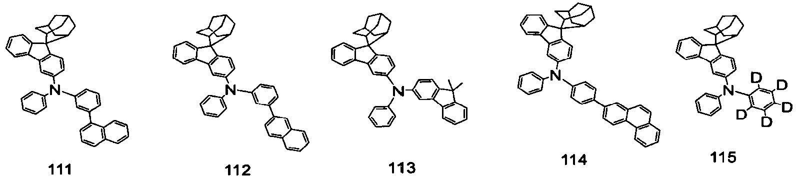

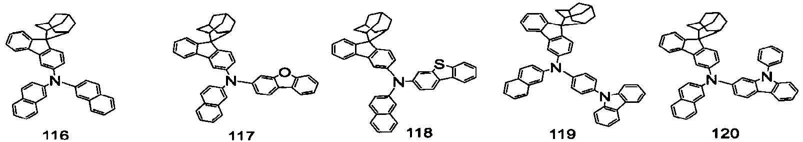

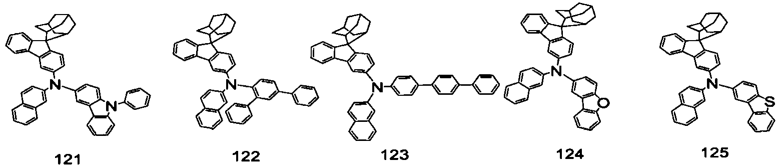

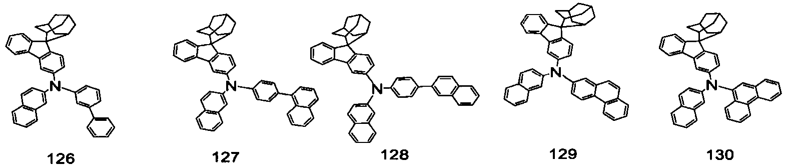

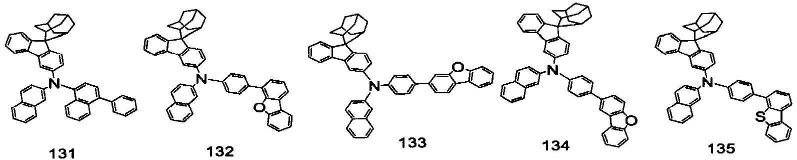

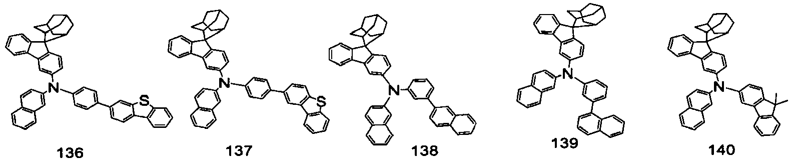

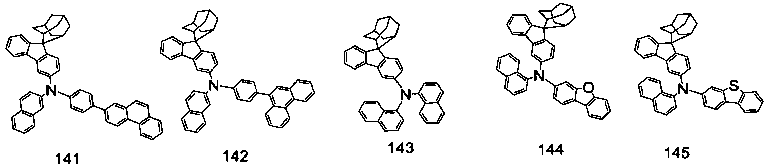

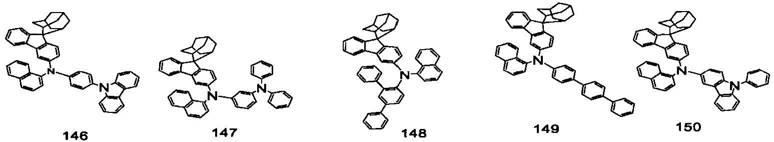

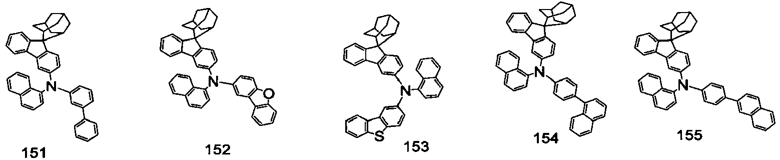

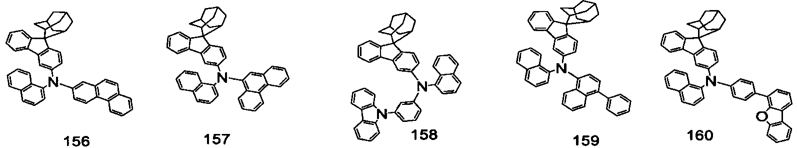

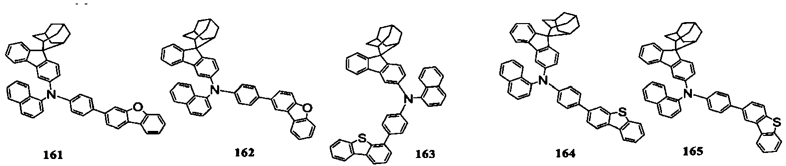

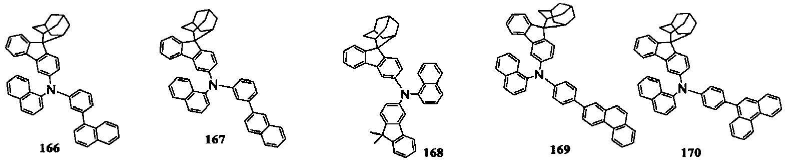

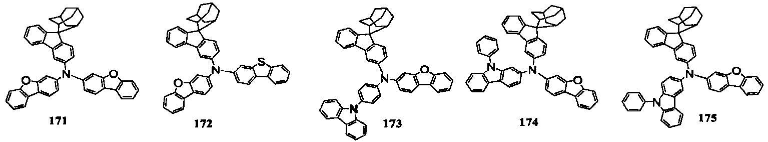

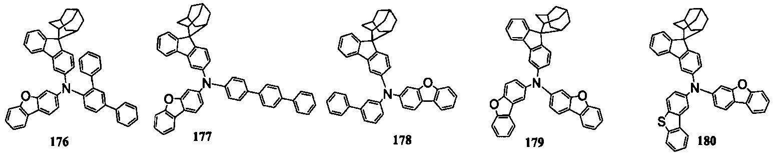

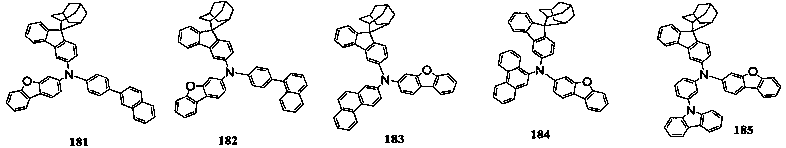

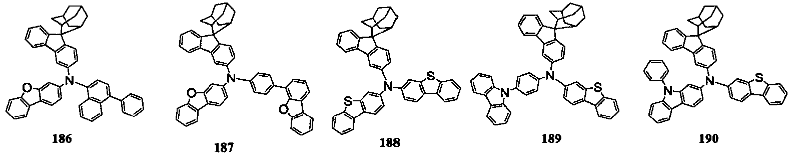

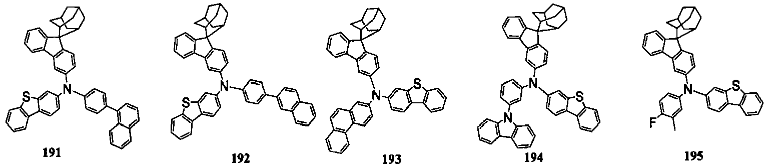

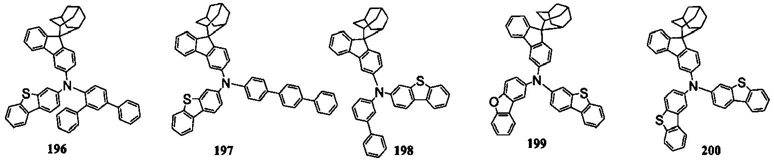

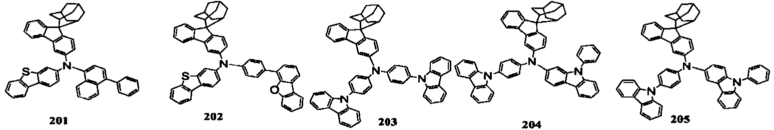

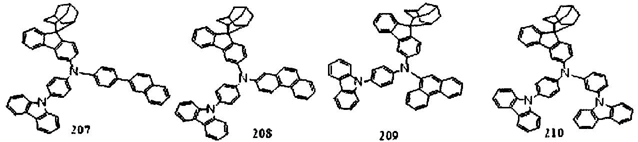

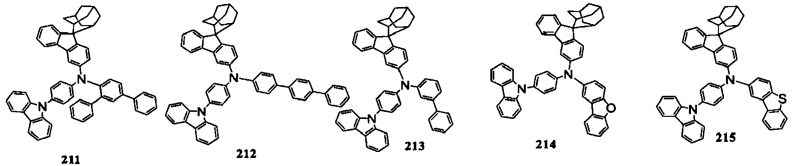

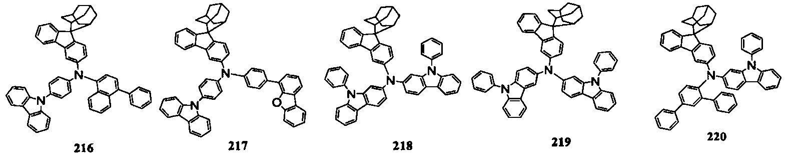

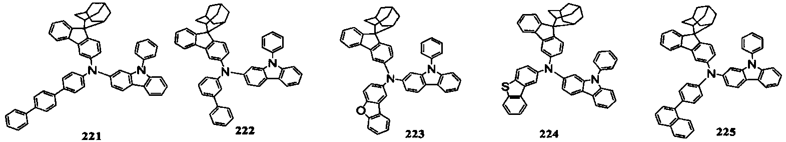

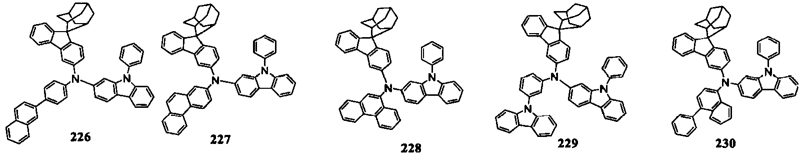

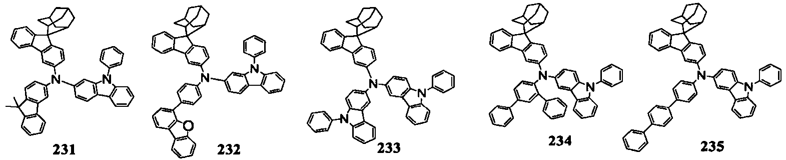

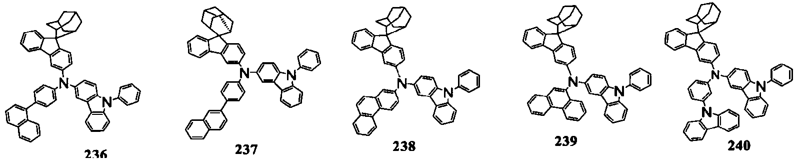

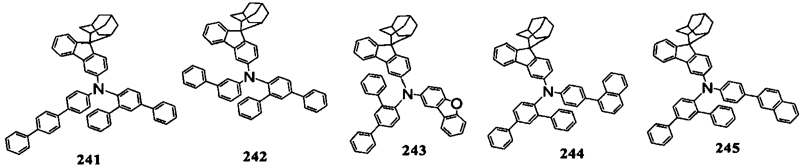

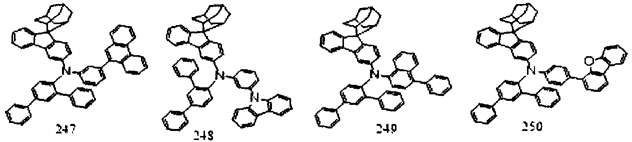

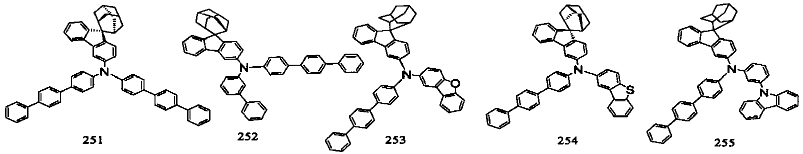

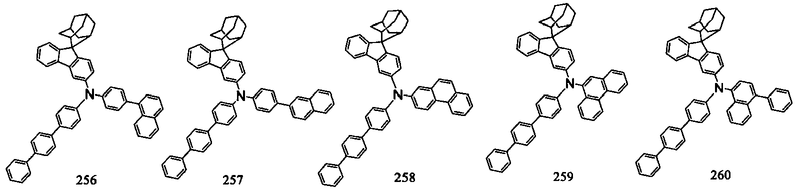

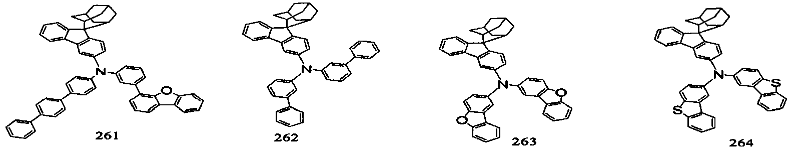

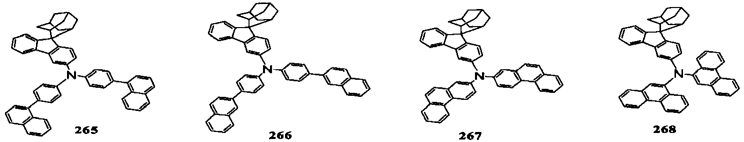

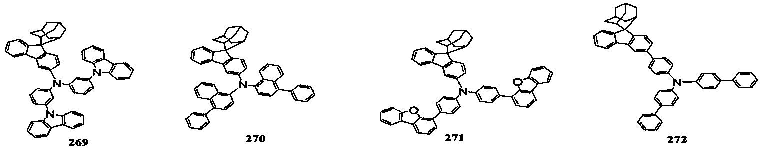

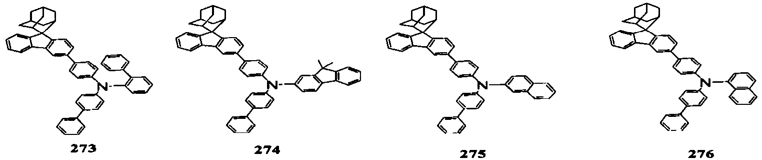

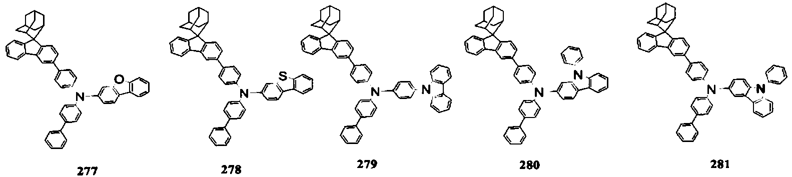

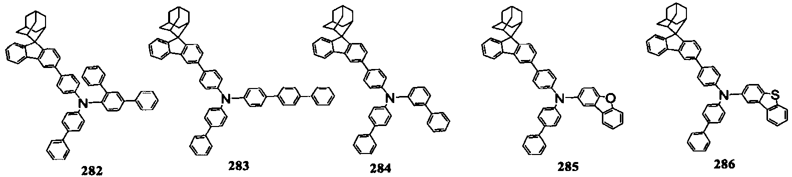

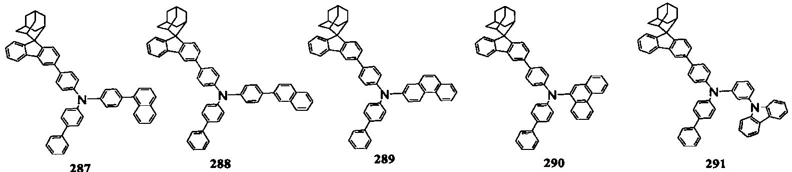

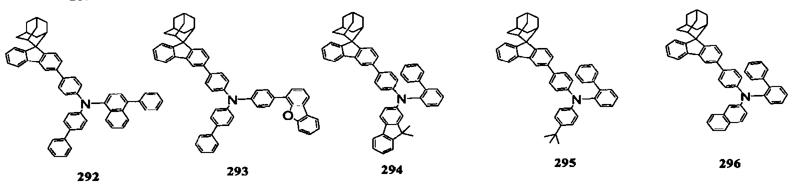

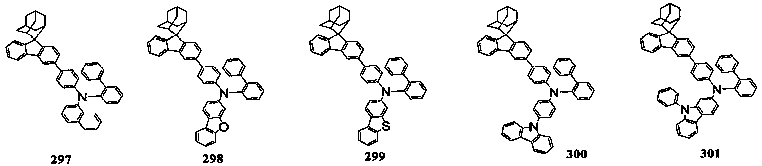

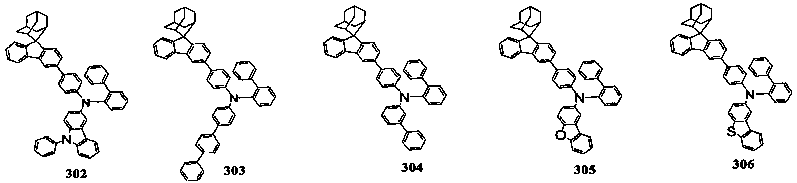

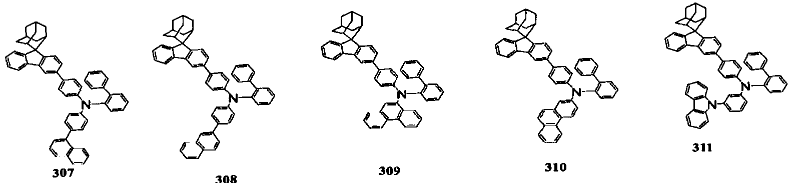

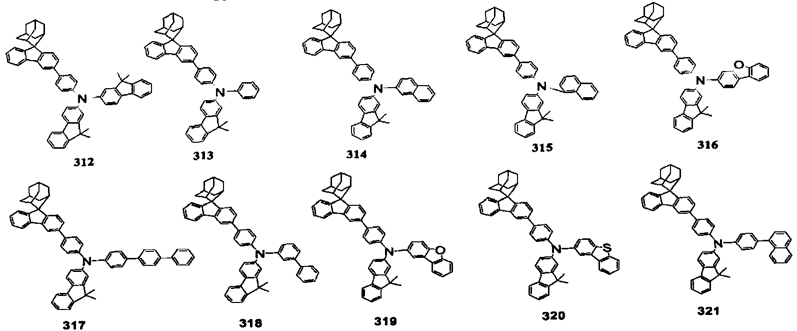

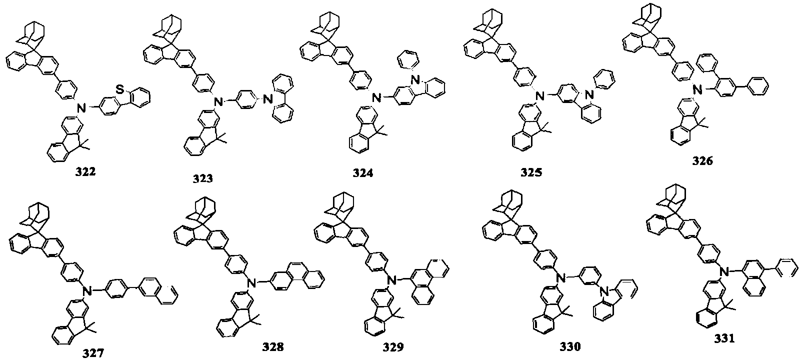

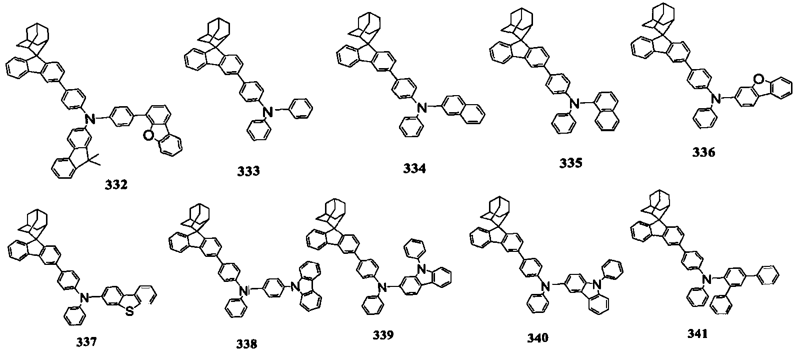

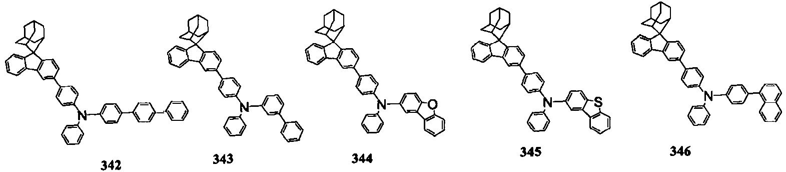

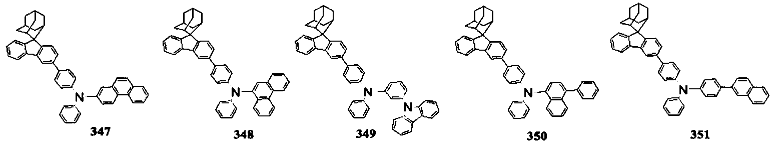

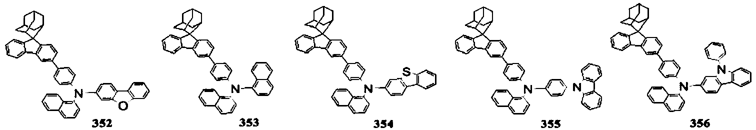

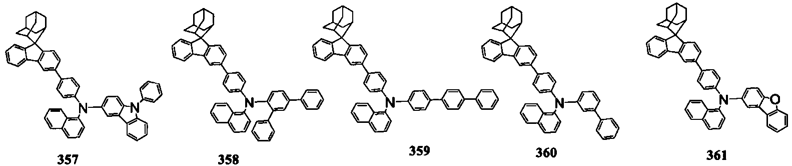

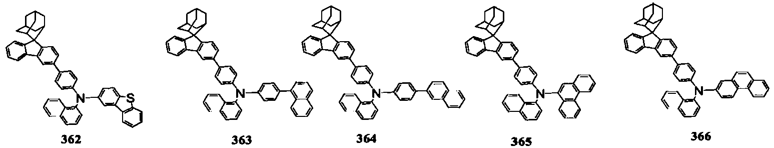

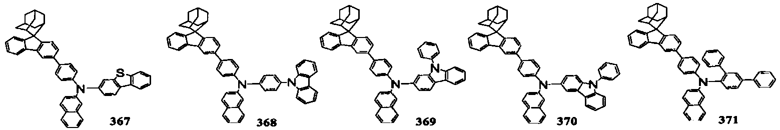

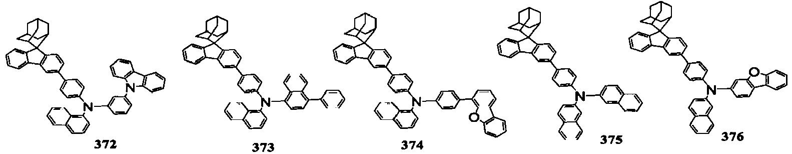

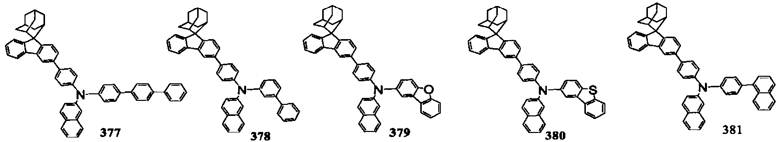

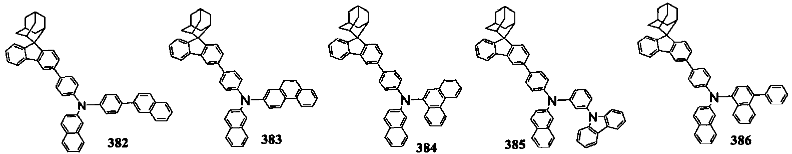

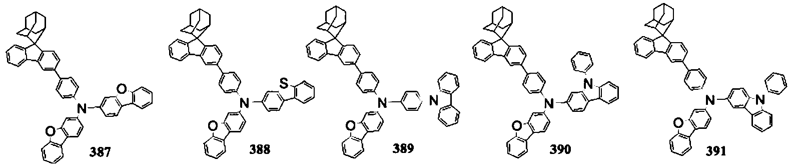

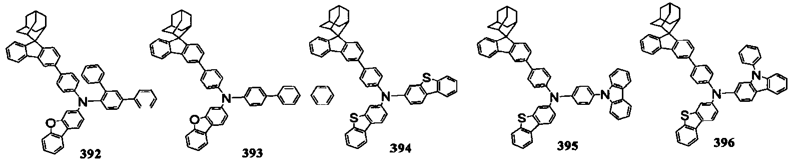

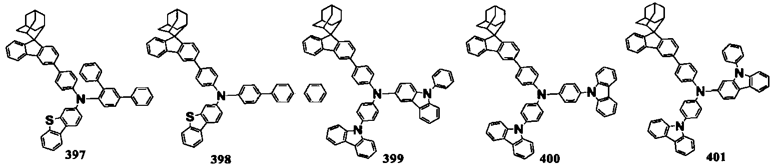

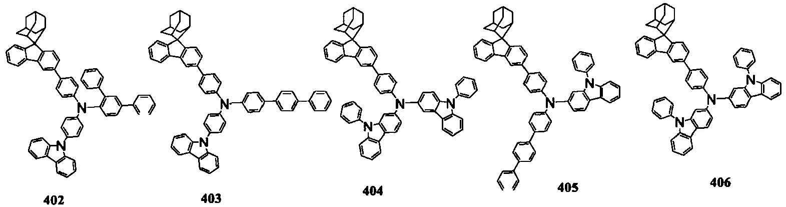

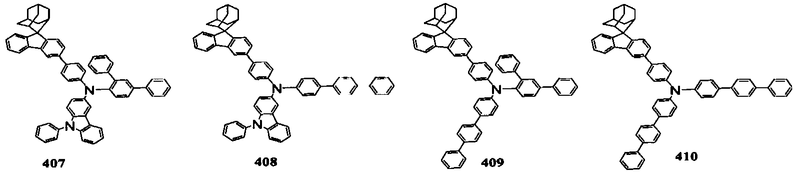

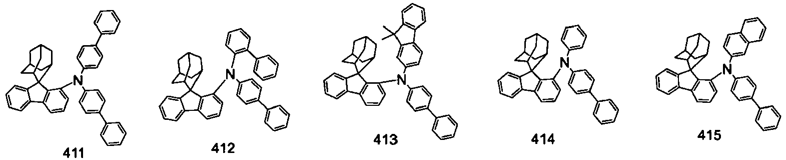

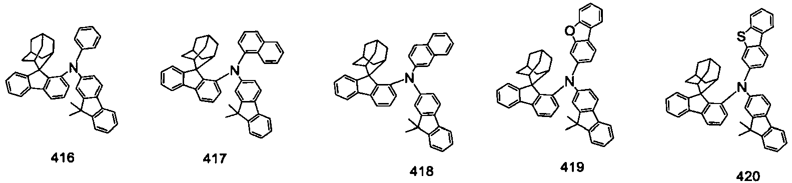

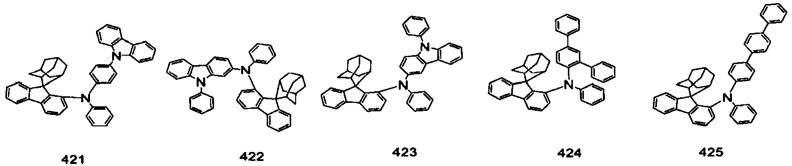

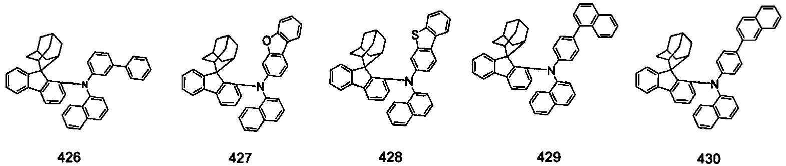

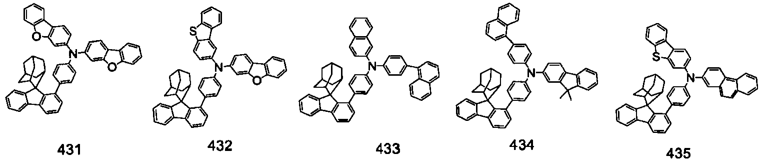

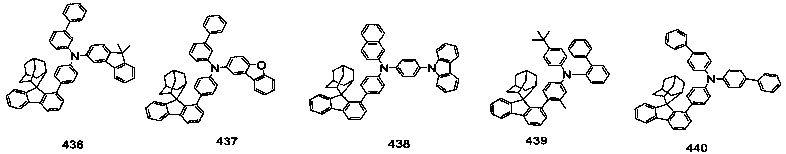

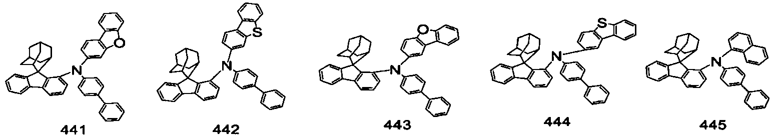

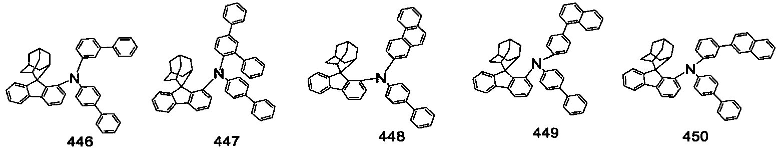

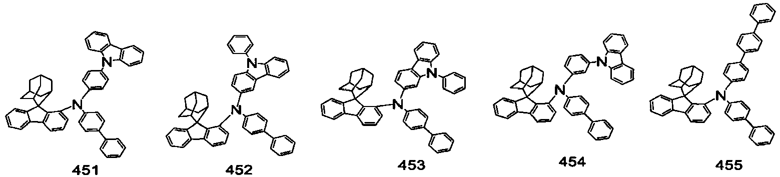

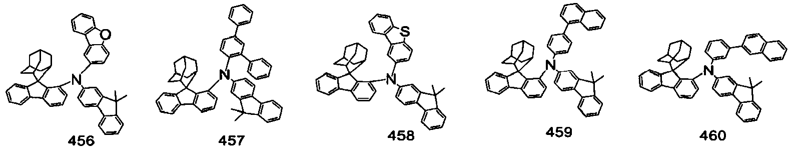

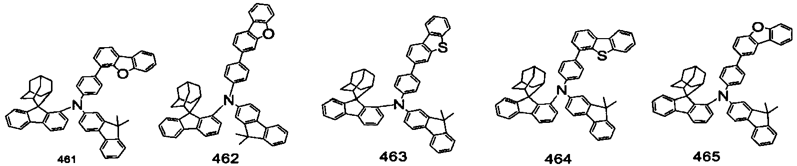

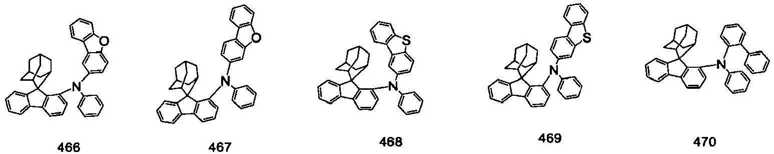

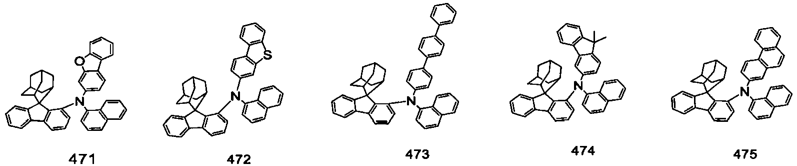

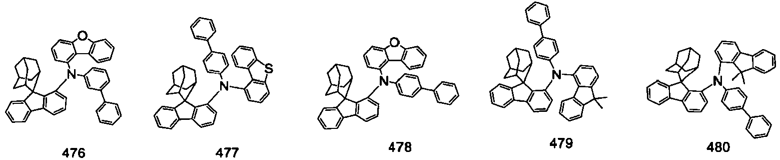

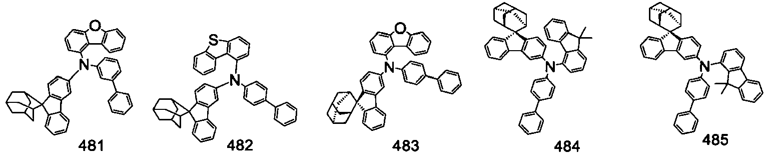

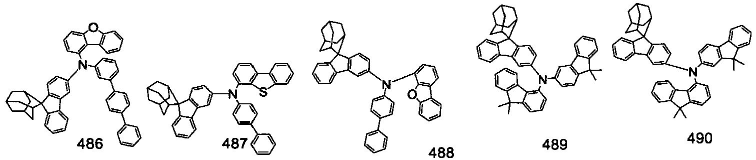

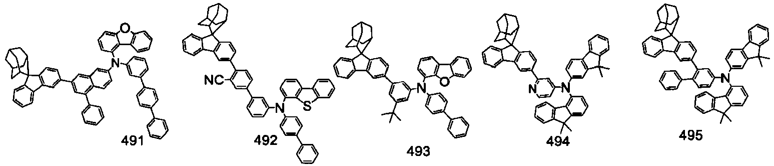

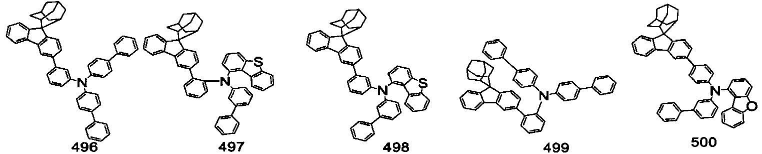

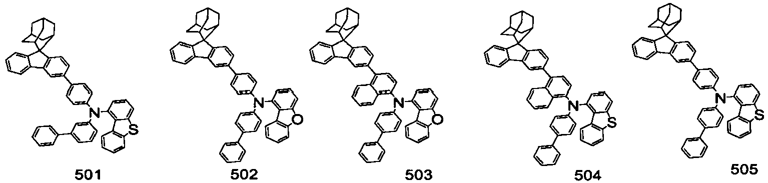

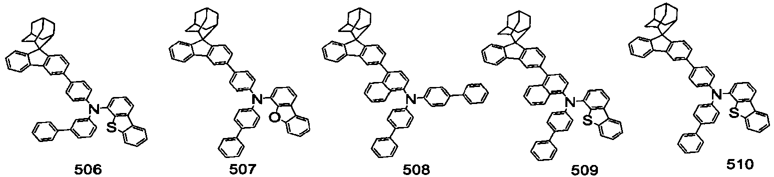

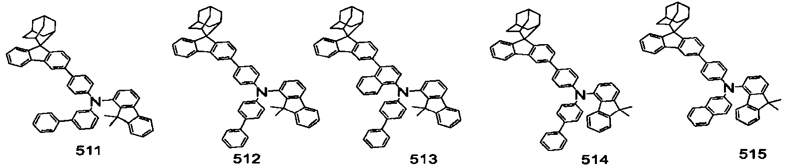

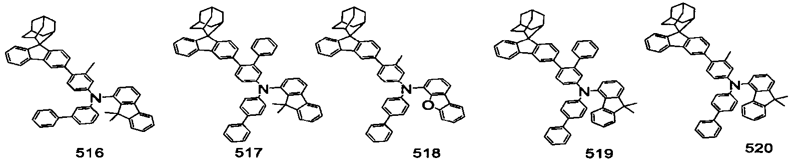

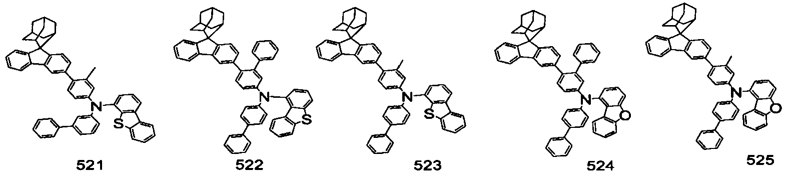

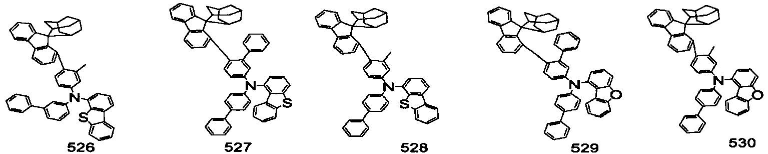

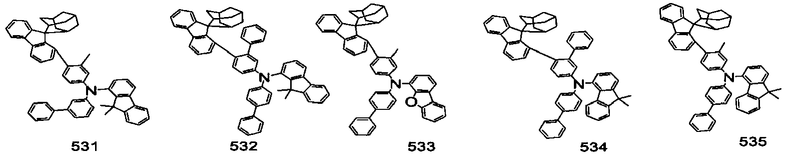

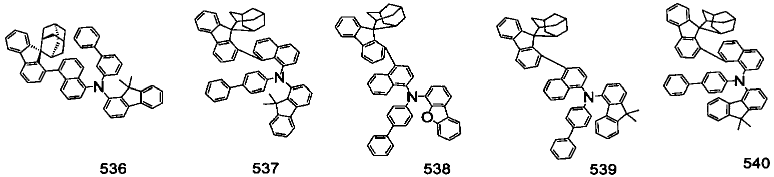

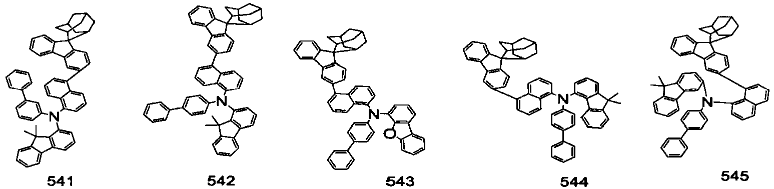

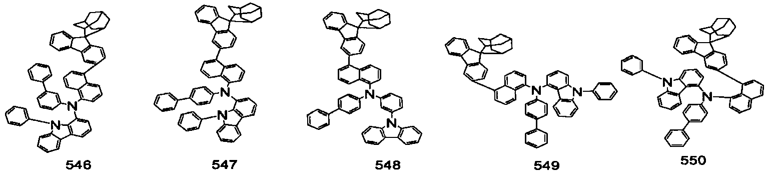

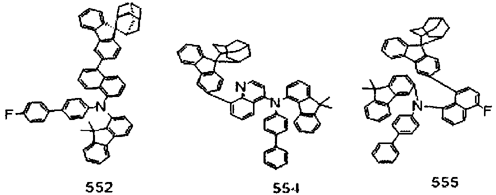

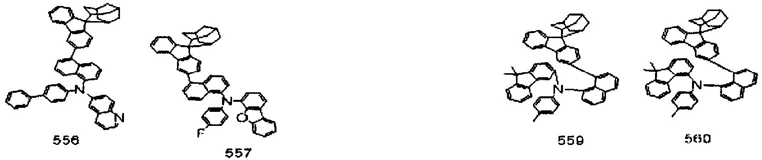

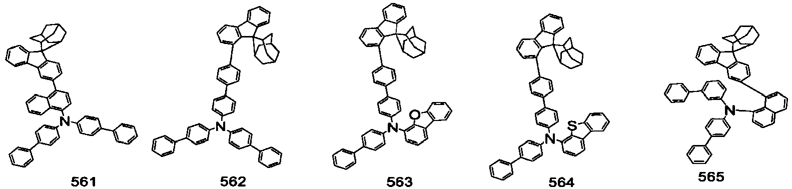

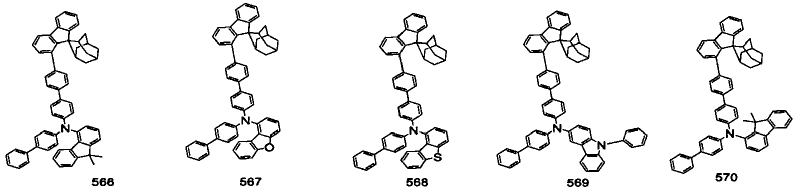

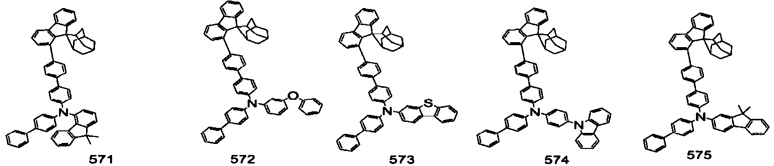

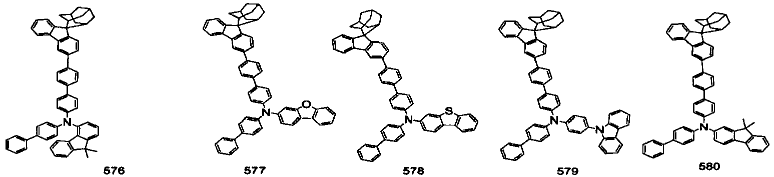

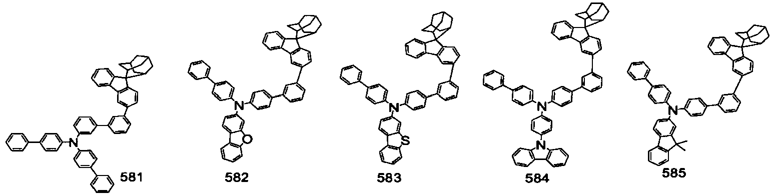

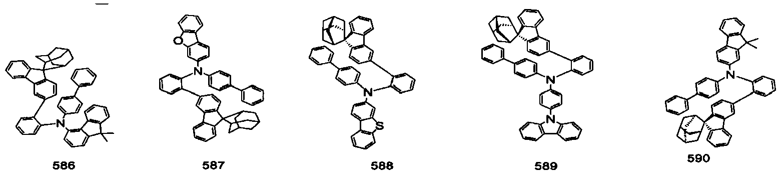

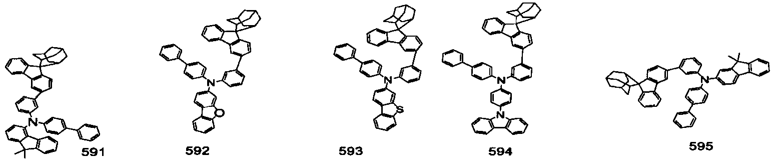

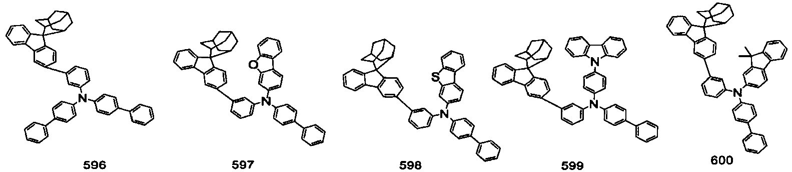

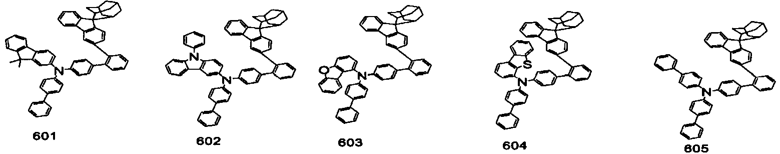

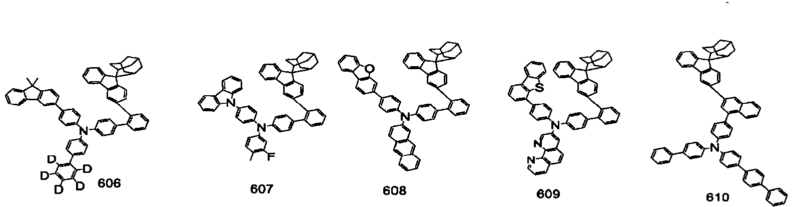

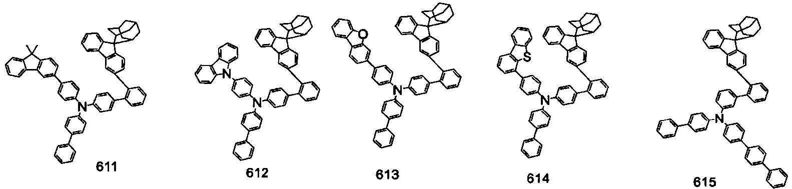

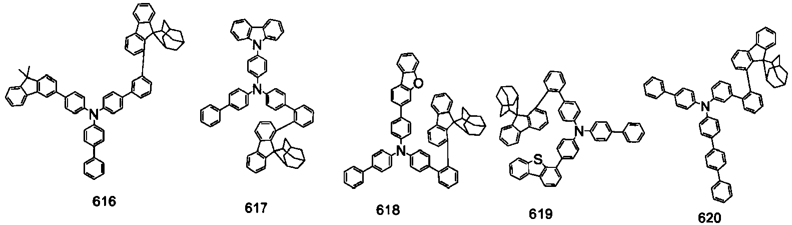

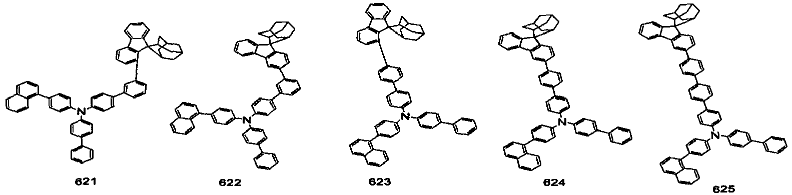

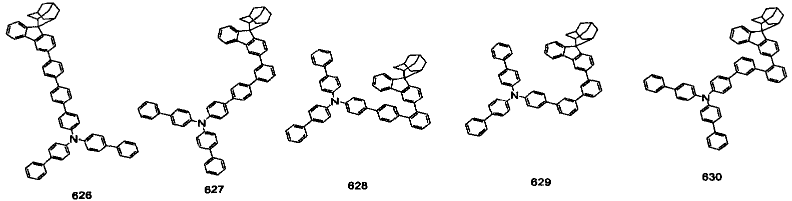

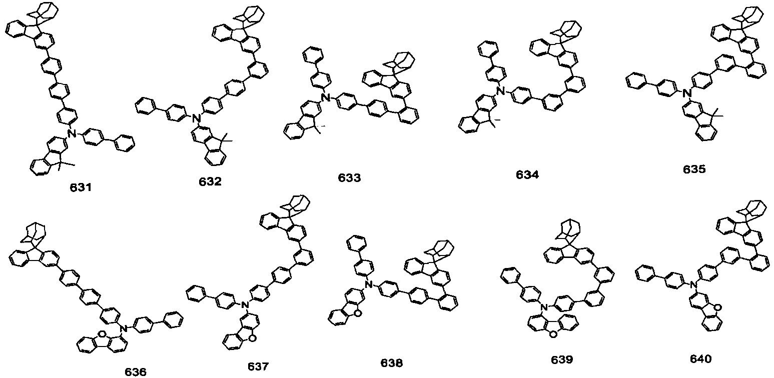

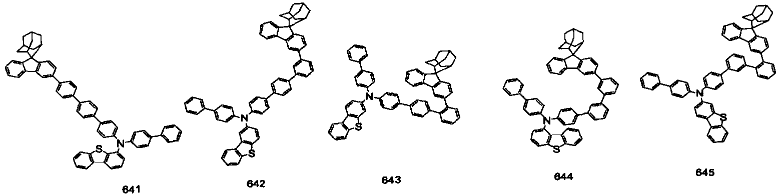

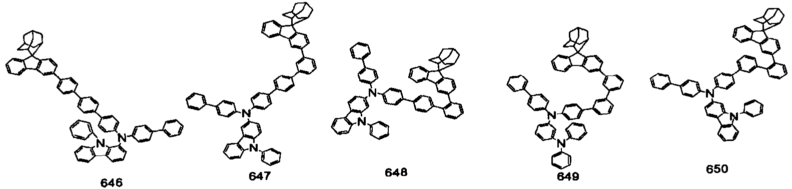

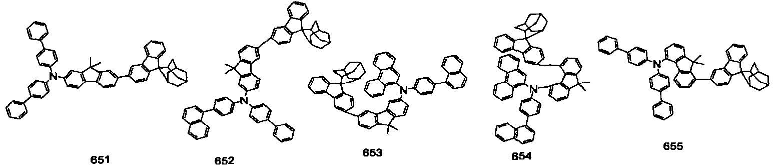

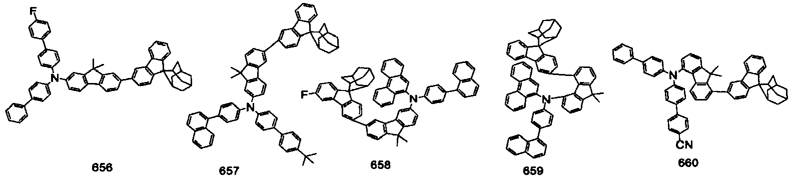

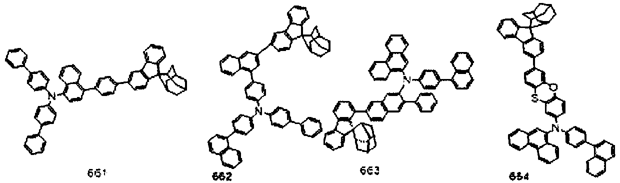

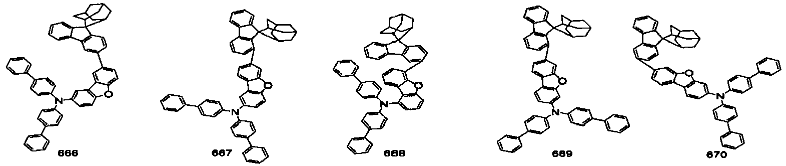

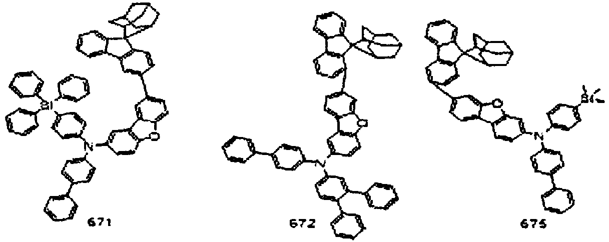

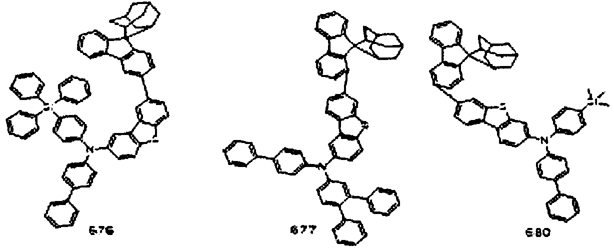

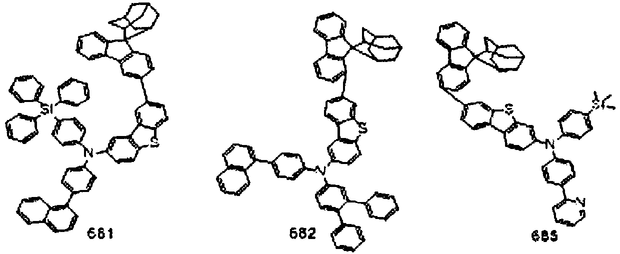

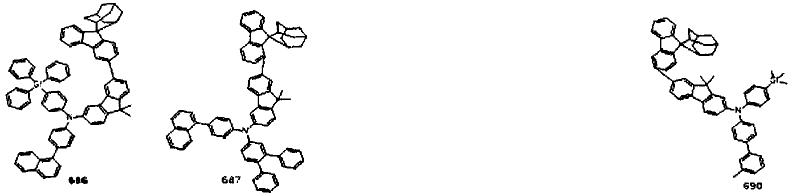

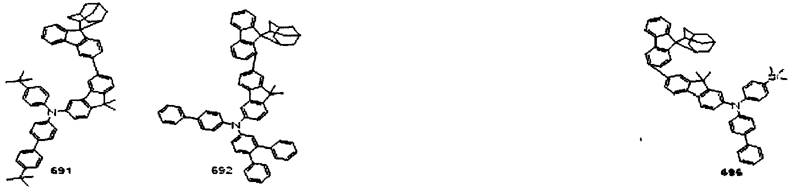

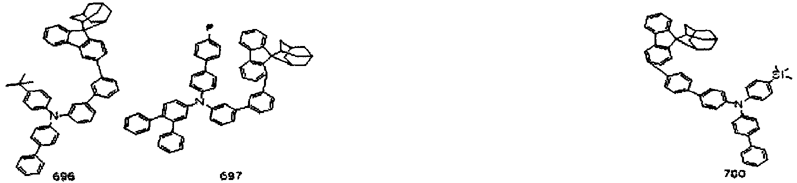

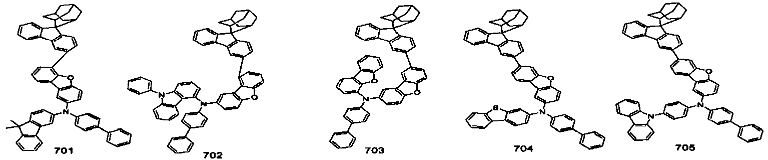

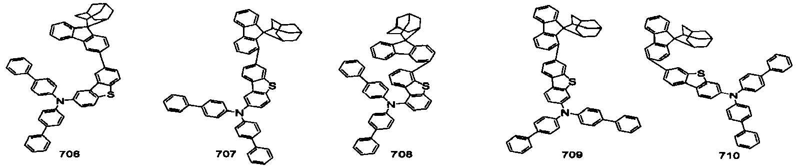

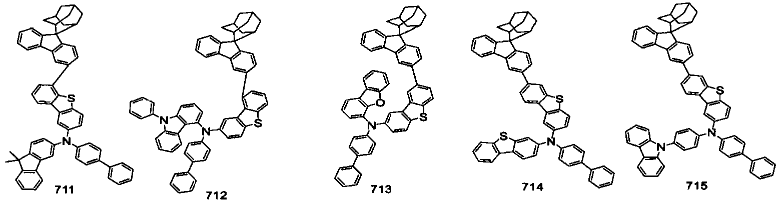

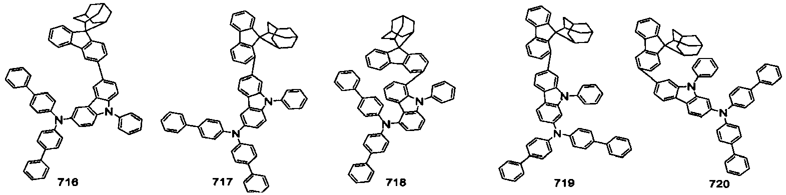

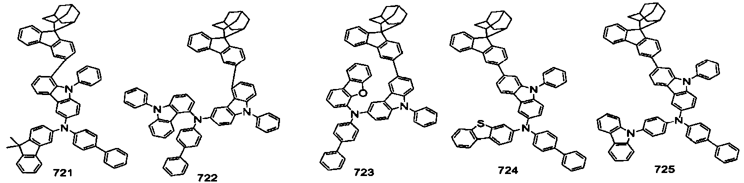

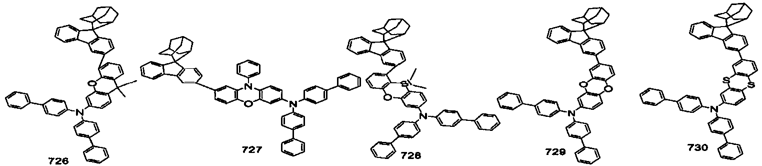

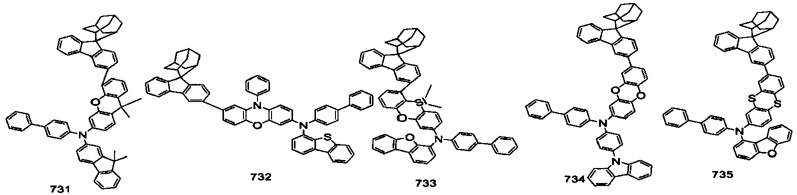

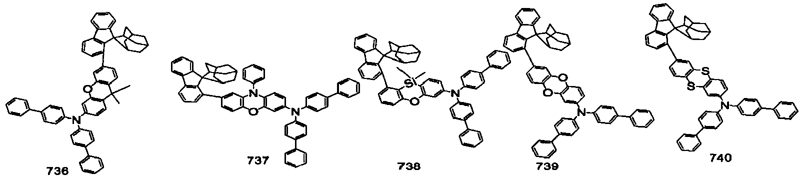

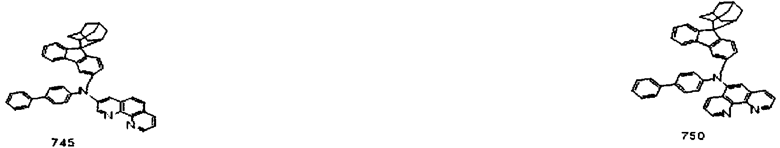

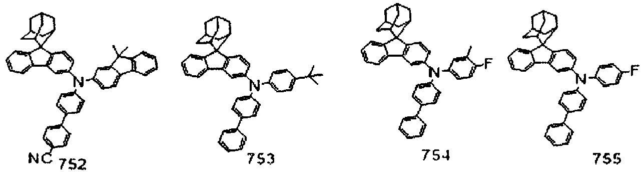

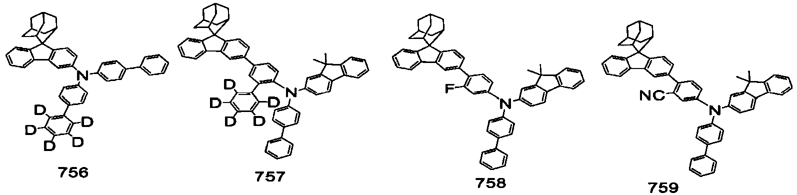

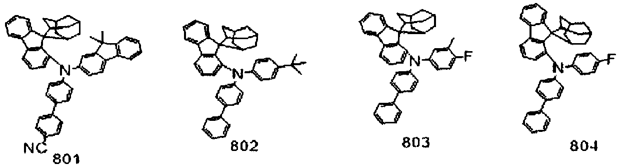

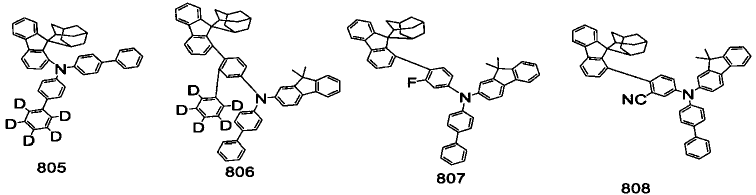

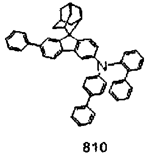

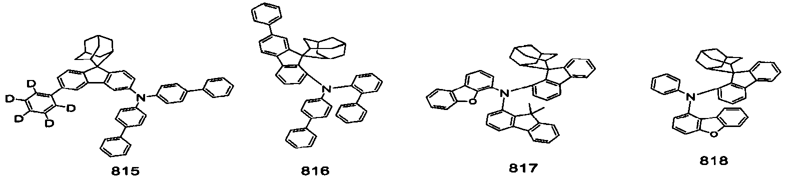

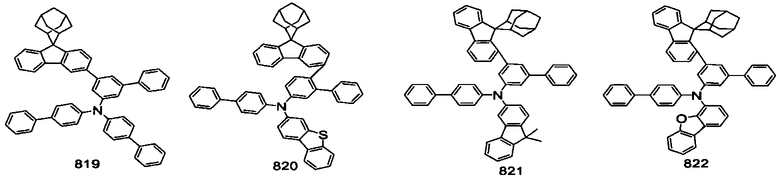

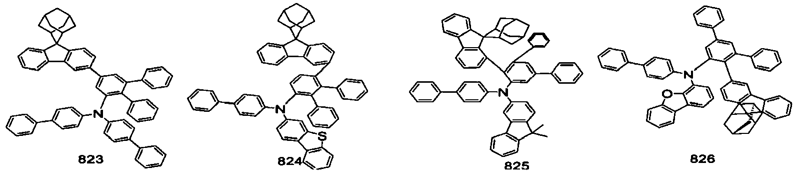

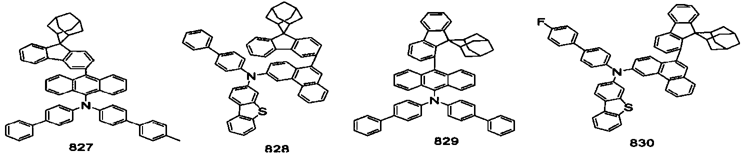

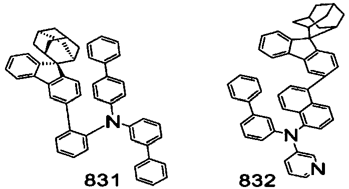

3. Élément électronique comprenant une anode et une cathode disposées de manière opposée l'une par rapport à l'autre, et une couche fonctionnelle disposée entre l'anode et la cathode; dans lequel la couche fonctionnelle contient le composé contenant de l'azote selon l'une quelconque des revendications 1 ou 2.

4. Élément électronique selon la revendication 3, dans lequel la couche fonctionnelle comprend une couche de blocage des électrons, et dans lequel la couche de blocage des électrons contient le composé contenant de l'azote.

5. Élément électronique selon la revendication 3, dans lequel l'élément électronique est un dispositif d'électroluminescence organique ou un dispositif de conversion photoélectrique;
de préférence, le dispositif d'électroluminescence organique est un dispositif émettant une lumière verte

6. Dispositif électronique comprenant l'élément électronique selon l'une quelconque des revendications 3 à 5.
